(19) Europäisches Patentamt
European Patent Office
Office européen des brevets

(11) Veröffentlichungsnummer: **0 184 550 B1**

# (12) EUROPÄISCHE PATENTSCHRIFT

(45) Veröffentlichungstag der Patentschrift: **18.03.92**

(21) Anmeldenummer: **85810523.2**

(22) Anmeldetag: **08.11.85**

Die Akte enthält technische Angaben, die nach dem Eingang der Anmeldung eingereicht wurden und die nicht in dieser Patentschrift enthalten sind.

(51) Int. Cl.⁵: **C07D 233/64**, C07D 403/12, C07D 401/12, C07D 405/12, C07D 215/48, C07D 209/42, C07D 209/52, C07F 9/6506, C07F 7/18, A61K 31/415, A61K 31/40

## (54) 5-Amino-4-hydroxyvalerylamid-Derivate.

(30) Priorität: **13.11.84 CH 5426/84**
**17.07.85 CH 3094/85**

(43) Veröffentlichungstag der Anmeldung:
**11.06.86 Patentblatt 86/24**

(45) Bekanntmachung des Hinweises auf die Patenterteilung:
**18.03.92 Patentblatt 92/12**

(84) Benannte Vertragsstaaten:
**AT BE CH DE FR GB IT LI LU NL SE**

(56) Entgegenhaltungen:
**EP-A- 0 111 266**
**EP-A- 0 118 223**
**EP-A- 0 127 235**
**EP-A- 0 128 762**
**EP-A- 0 143 746**
**EP-A- 0 173 481**
**EP-B- 0 045 665**
**EP-B- 0 077 029**
**US-A- 4 470 971**

(73) Patentinhaber: **CIBA-GEIGY AG**
**Klybeckstrasse 141**
**CH-4002 Basel(CH)**

(72) Erfinder: **Bühlmayer, Peter, Dr.**
**Im Baumgarten 3**
**CH-4414 Arlesheim(CH)**
Erfinder: **Rasetti, Vittorio, Dr.**
**Passwangstrasse 6**
**CH-4059 Basel(CH)**
Erfinder: **Fuhrer, Walter, Dr.**
**Im Budler 3**
**CH-4419 Lupsingen(CH)**
Erfinder: **Stanton, James Lawrence, Dr.**
**Hammerstrasse 150**
**CH-4057 Basel(CH)**
Erfinder: **Göschke, Richard, Dr.**
**Felixhäglistrasse 21**
**CH-4103 Bottmingen(CH)**

EP 0 184 550 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

CHEMICAL ABSTRACTS, Band 96, Nr. 23, 7. Juni 1982, Columbus, Ohio, US; PERRIN, CHARLES L.; ARRHENIUS, GLORIA MEICHIA L.: "A critical test of the theory of stereoelectronic control" Seite 592, Spalte 1, Zusammenfassung-Nr. 198 797p

CHEMICAL ABSTRACTS, Band 89, Nr. 19, 6. November 1978, Columbus, Ohio, US; HJEDS, HANS; HONORE, TAGE: "Structural analogs of gamma-aminobutyric acic (GABA). Syntheses of a series of aminoalkanehydroxamic acid hydrochlorides" Seite 610, Spalte 1, Zusammenfassung-Nr. 163 932g

CHEMICAL ABSTRACTS, Band 95, Nr. 3, 20. Juli 1981, Columbus, Ohio, US; SAURIOL-LORD, FRANCOISE; GRINDLEY, T. BRUCE: "Saymmetric induction in additions to epocides. Addition of alpha-anions of N,N-disubstituted carbocamides" Seite 613, Spalte 1, Zusammenfassung-Nr. 24 154r

CHEMICAL ABSTRACTS, Band 87, Nr. 17, 24. Oktober 1977, Columbus, Ohio, US; HULLOT, PIERRE; CUVIGNY, THERESE; LARCHEVEOUE, MARC; NORMANT, HENRI: "Hyperbasic media: preparation of alpha-carbanions N,N-disubstituted carboxamides. Synthesis of beta- and gamma-hydroxyamides and gamma-butyrolactones" Seite 662, Spalte 2, Zusammenfassung-Nr. 134 387m

## Beschreibung

Die Erfindung betrifft Verbindungen der Formel

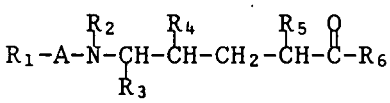

(I),

worin $R_1$ $C_1$-$C_7$-Alkanoyl, Phenoxy-$C_1$-$C_7$-alkanoyl, Naphthoxy-$C_1$-$C_7$-alkanoyl, $\alpha$-Naphthoxy-carboxy-$C_1$-$C_7$-alkanoyl, $\alpha$-Naphthoxy-$C_1$-$C_7$-alkoxycarbonyl-$C_1$-$C_7$-alkanoyl, $\alpha$-Naphthoxy-benzyloxycarbonyl-$C_1$-$C_7$-alkanoyl, $\alpha$-Naphthoxy-carbamoyl-$C_1$-$C_7$-alkanoyl, $\alpha$-Naphthoxy-cyano-$C_1$-$C_7$-alkanoyl, $\alpha$-Naphthoxy-di-$C_1$-$C_7$-alkylamino-$C_1$-$C_7$-alkanoyl, $\alpha$-Naphthoxy-oxo-$C_1$-$C_7$-alkanoyl, Phenyl-, $\alpha$-Naphthyl- oder $\beta$-Naphthyl-$C_1$-$C_7$-alkanoyl, worin $C_1$-$C_7$-Alkanoyl unsubstituiert oder durch Hydroxy, $C_1$-$C_7$-Alkoxy, $C_1$-$C_7$-Alkanoyloxy, Carboxy, $C_1$-$C_7$-Alkoxy-carbonyl, Carbamoyl, $C_1$-$C_7$-Alkylcarbamoyl, Di-$C_1$-$C_7$-alkylcarbamoyl, Cyano, Phosphono, Di-$C_1$-$C_7$-alkoxy-phosphoryl, Benzofuranyl und/oder Oxo substituiert sein kann und gegebenenfalls verzweigt ist, über eine Carbonylgruppe -CO- gebundenes 2-Phenyläthyl, 3-Phenyl-2-propyl, 4-Phenyl-3-butyl, 2-$\alpha$-Naphthyläthyl, 3-$\alpha$-Naphthyl-2-propyl oder 4-$\alpha$-Naphthyl-3-butyl, das jeweils in 1-Stellung durch Acyloxy in Form von Aethylaminocarbonyloxy, 2-Benzyloxycarbonylamino-2-methyl-propionyloxy, 2-Amino-2-methylpropionyloxy oder Acetoacetoxy, durch verestertes Carboxy in Form von Benzyloxycarbonyl, durch substituiertes Carbamoyl in Form von Carboxymethylcarbamoyl, tert-Butoxycarbonylmethylcarbamoyl, 2-Dimethylaminoäthylcarbamoyl, 3-Hydroxy-2-propylcarbamoyl, 2,2-Dimethoxyäthylcarbamoyl oder 5-Amino-5-carboxypentylcarbamoyl oder durch verestertes Phosphono in Form von Hydroxymethoxyphosphoryl substituiert ist, 3-Phenyl-2-äthoxy-hydroxyphosphorylpropionyl, 3-Phenyl- oder 3-$\alpha$-Naphthyl-2-dimethylaminomethylpropionyl, 4-Phenyl- oder 4-$\alpha$-Naphthyl-3-benzyloxycarbonyl-butyryl, 2-Benzyl- oder 2-$\alpha$-Naphthylmethyl-4,4-dimethyl-3-oxo-pentanoyl, 2-Benzyl- oder 2-$\alpha$-Naphthylmethyl-5-dimethylamino-pentanoyl, 2-Benzyl-oder 2-$\alpha$-Naphthylmethyl-5-dimethylamino-4-oxo-pentanoyl, 2-Benzyl- oder 2-$\alpha$-Naphthylmethyl-5,5-dimethyl-4-oxo-hexanoyl, $\alpha$,p-Diamino-phenylacetyl, $\alpha$,p-Dibenzyloxycarbonylamino-phenylacetyl, $\alpha$-Pivaloylamino-p-benzyloxycarbonylamino-phenylacetyl, 2-(o,o-Dichloroanilino)-phenylacetyl, 2-(o,o-Dichloro-N-benzylanilino)-phenylacetyl, $\alpha$- oder $\beta$-Naphthylcarbonyl, 1,8-Naphthalindicarbonyl, Pyrrolylcarbonyl, Cyclohepta[b]pyrrolyl-5-carbonyl, Indolylcarbonyl, 1-Benzyl-indolyl-3-carbonyl, 4,5,6,7-Tetrahydroindolyl-2-carbonyl, Chinolylcarbonyl oder Oxamoyl,
A den bivalenten Rest der Aminosäuren Leucin, Norleucin, Phenylalanin, N-Methyl-phenylalanin, $\beta$-Phenylserin, Cyclohexylalanin, Glutamin, Histidin oder N-Methyl-histidin, $R_2$ Wasserstoff, $R_3$ Isobutyl oder Cyclohexylmethyl, $R_4$ Hydroxy, $R_5$ Isopropyl, Cyclohexylmethyl, $\alpha$-Decahydronaphthyl oder Dimethylamino, und
$R_6$ $C_1$-$C_7$-Alkylamino, Di-$C_1$-$C_7$-alkylamino, Hydroxy-$C_1$-$C_7$-alkylamino, 2-Phenoxyäthylamino, 2-(3-Carbamoyl-4-hydroxyphenoxy)-äthylamino, Carboxy-alkylamino oder Amino-carboxy-alkylamino, worin der Carboxyrest nicht in 1-Stellung des Alkylrests, welcher bis zu 10 C-Atome enthält, steht, $C_1$-$C_7$-Alkoxycarbonyl-alkylamino oder ($C_1$-$C_7$-Alkanoylamino-, $C_1$-$C_7$-Alkoxycarbonylamino- oder Benzyloxycarbonylamino-)-$C_1$-$C_7$-alkoxycarbonyl-alkylamino, worin der Carbonylrest nicht in 1-Stellung des Alkylrests, welcher bis zu 10 C-Atome enthält, steht, 4-Tris-(hydroxymethyl)-methylcarbamoyl-n-butylamino, Amino-$C_1$-$C_7$-alkylamino, Di-$C_1$-$C_7$-alkylamino-$C_1$-$C_7$-alkylamino, $C_1$-$C_7$-Alkoxycarbonylamino-$C_1$-$C_7$-alkylamino, Morpholino-$C_1$-$C_7$-alkylamino, Cycloalkyl-$C_1$-$C_7$-alkylamino mit 4-10 C-Atomen, Benzylamino, Pyridyl-$C_1$-$C_7$-alkylamino, Imidazolyl-$C_1$-$C_7$-alkylamino oder 2-(2-[4-Imidazolyl]-äthylamino)-äthylamino darstellt, sowie pharmazeutisch annehmbare Salze von diesen Verbindungen mit salzbildenden Gruppen, mit Ausnahme der Verbindung der Formel I, worin $R_1$ $\alpha$-Naphthoxyacetyl, A den bivalenten Rest der Aminosäure Histidin, $R_2$ Wasserstoff, $R_3$ Isobutyl, $R_4$ Hydroxy, $R_5$ Isopropyl und $R_6$ Cyclohexylmethylamino bedeuten, Verfahren zu ihrer Herstellung, pharmazeutische Präparate mit diesen Verbindungen und die Verwendung dieser Verbindungen als Arzneimittel oder zur Herstellung von pharmazeutischen Präparaten, sowie Zwischenprodukte zur Herstellung von Verbindungen der Formel I.

Die Erfindung betrifft auch Verbindungen der Formel I, worin $R_1$ Phenoxy-$C_1$-$C_7$-alkanoyl, Naphthoxy-$C_1$-$C_7$-alkanoyl, Phenyl-$C_1$-$C_7$-alkanoyl, worin $C_1$-$C_7$-Alkanoyl unsubstituiert oder substituiert durch $C_1$-$C_7$-Alkanoyloxy, Carboxy, $C_1$-$C_7$-Alkoxycarbonyl, Carbamoyl, $C_1$-$C_7$-Alkylcarbamoyl, Cyano, Di-$C_1$-$C_7$-alkoxyphosphoryl oder $C_1$-$C_7$-Alkyl und Oxo oder Benzofuranyl und Oxo substituiert sein kann und gegebenenfalls verzweigt ist, Naphthyl-$C_1$-$C_7$-alkanoyl, worin $C_1$-$C_7$-Alkanoyl unsubstituiert oder durch $C_1$-$C_7$-Alkanoyloxy, Carboxy, $C_1$-$C_7$-Alkoxycarbonyl, Carbamoyl, Cyano oder $C_1$-$C_7$-Alkyl und Oxo substituiert sein kann und gegebenenfalls verzweigt ist, 2-tert-Butylcarbamoyl-3-$\alpha$-naphthyl-propionyl, 2-Carboxymethylcarbamoyl-3-$\alpha$-naphthyl-propionyl, Indolyl-2-carbonyl oder Cyclohepta[b]pyrrolyl-5-carbonyl, A den bivalenten Rest der

Aminosäure L-Histidin, $R_2$ Wasserstoff, $R_3$ Isobutyl oder Cyclohexylmethyl, $R_4$ Hydroxy, $R_5$ Isopropyl oder Cyclohexylmethyl und $R_6$ $C_1$-$C_7$-Alkylamino oder Amino-carboxy-alkylamino, worin die Substituenten nicht in 1-Stellung des Alkylrests stehen, welcher bis zu 7 C-Atome enthält, bedeuten und die die Reste $R_3$ und $R_4$ tragenden C-Atome die S-Konfiguration aufweisen, ferner pharmazeutisch annehmbare Salze von diesen Verbindungen, Verfahren zu ihrer Herstellung, pharmazeutische Präparate mit diesen Verbindungen und die Verwendung dieser Verbindungen als Arzneimittel oder zur Herstellung von pharmazeutischen Präparaten, sowie Zwischenprodukte zur Herstellung von Verbindungen der Formel I,
und die Verbindungen der Formel I mit den Bezeichnungen

N-(N-Benzyl-indolyl-2-carbonyl)-His-Leu$\stackrel{C}{-}$Val-methylamid,
N-(3(R,S)-Benzyloxycarbonyl-4-α-naphthylbutyryl)-His-Leu$\stackrel{C}{-}$Val-methylamid,

N-Benzyloxycarbonyl-(p-benzyloxycarbonylamino-Phe)-His-Leu$\stackrel{C}{-}$Val-n-butylamid,
N-Pivaloyl-(p-benzyloxycarbonylamino-Phe)-His-Leu$\stackrel{C}{-}$Val-n-butylamid,
N-(Indolyl-2-carbonyl)-His-Leu$\stackrel{C}{-}$Val-4-[tris-(tert-butyldimethyl-silyloxymethyl)-methylcarbamoyl]-butylamid,
N-(2-Aethylaminocarbonyloxy-4-phenyl-butyryl)-His-Cha$\stackrel{C}{-}$Val-n-butyl-amid

und deren pharmazeutisch annehmbare Salze, Verfahren zu ihrer Herstellung, pharmazeutische Präparate mit diesen Verbindungen und die Verwendung dieser Verbindungen als Arzneimittel oder zur Herstellung von pharmazeutischen Präparaten, sowie Zwischenprodukte zur Herstellung von Verbindungen der Formel I.

Die Europäische Patente EP-B-0 045 665 und EP-A-0 118 223 beschreiben als Reninhemmer wirksame Peptidanaloga, die anstelle der durch Renin spaltbaren Peptidbindung im natürlichen Reninsubstrat bestimmte nicht mehr proteolytisch spaltbare Peptidisostere enthalten.

EP-A-0 143 746 als älteres Recht offenbart in den Ansprüchen 31 und 32 Verbindungen, die der Formel VIII des hier vorgelegten Textes entsprechen, und Verfahren zu deren Herstellung, wobei jedoch die Bedeutungen von $R_3$ unterschiedlich sind. Die auf Seite 51 des älteren Rechtes an zweiter Stelle gezeigte Formel für Zwischenproduckte unterscheidet sich von der Formel I für die hier vorliegenden Verbindungen da bei letzerer $R_1$ nicht H bedeutet.

EP-A-0 173 481 als älteres Recht offenbart die Verbindung der Formel I, worin $R_1$ $\alpha$-Naphtoxyacetyl, A den bivalenten Rest der Aminosäure Histidin, $R_2$ Wasserstoff, $R_3$ Isobutyl, $R_4$ Hydroxy, $R_5$ Isopropyl und $R_6$ Cyclohexylmethylamino bedeutet, als "NOA-His-LVA-CHA" (Tabelle A, Seite 114, Verbindung Nr. 132). Diese Verbindung ist durch einen Disclaimer aus der hier vorliegenden Anmeldung ausgeschlossen.

In der Beschreibung der vorliegenden Erfindung bedeutet der bei der Definition von Gruppen oder Resten, z.B. Niederalkyl, Niederalkoxy, Niederalkanoyl etc., verwendete Ausdruck "Nieder", dass die so definierten Gruppen oder Reste, falls nicht ausdrücklich anders definiert, bis einschliesslich 7 und bevorzugt bis einschliesslich 4 C-Atome enthalten.

Die durch $R_3$, $R_4$ und $R_5$ substituierten C-Atome können die R-, S- oder R,S-Konfiguration haben. Bevorzugt sind Verbindungen der Formel I, worin die durch $R_3$ und $R_4$ substituierten C-Atome die S-Konfiguration aufweisen.

Die in der Beschreibung der vorliegenden Erfindung verwendeten allgemeinen Ausdrücke und Bezeichnungen haben vorzugsweise die folgenden Bedeutungen:
Niederalkyl hat vorzugsweise 1-7 C-Atome und ist z.B. Methyl, Aethyl, n-Propyl, Isopropyl, n-Butyl oder tert-Butyl.

A ist ein bivalenter Rest einer der genannten $\alpha$-Aminosäuren mit der L-Konfiguration.

Salze sind in erster Linie die pharmazeutisch verwendbaren, nichttoxischen Salze von Verbindungen der Formel I.

Solche Salze werden beispielsweise von Verbindungen der Formel I mit einer sauren Gruppe, z.B. einer

Carboxygruppe, gebildet und sind in erster Linie geeignete Alkalimetall-, z.B. Natrium- oder Kalium-, oder Erdalkalimetallsalze, z.B. Magnesium- oder Calciumsalze, ferner Zinksalze oder Ammoniumsalze, auch solche Salze, welche mit organischen Aminen, wie gegebenenfalls durch Hydroxy substituierten Mono-, Di- oder Trialkylaminen gebildet werden, z.B. Diäthylamin, Di-(2-hydroxyäthyl)-amin, Triäthylamin, N,N-Dimethyl-N-(2-hydroxyäthyl)-amin, Tri-(2-hydroxy-äthyl)-amin oder N-Methyl-D-glucamin. Die Verbindungen der Formel I mit einer basischen Gruppe, z.B. einer Aminogruppe, können Säureadditionssalze bilden, z.B. mit anorganischen Säuren, z.B. Salzsäure, Schwefelsäure oder Phosphorsäure, oder mit organischen Carbon-, Sulfon- oder Sulfosäuren, z.B. Essigsäure, Propionsäure, Glykolsäure, Bernsteinsäure, Maleinsäure, Hydroxymaleinsäure, Methylmaleinsäure, Fumarsäure, Aepfelsäure, Weinsäure, Zitronensäure, Benzoesäure, Zimtsäure, Mandelsäure, Salicylsäure, 4-Aminosalicylsäure, 2-Phenoxybenzoesäure, 2-Acetoxybenzoesäure, Embonsäure, Nicotinsäure oder Isonicotinsäure, ferner Aminosäuren, wie z.B. den weiter vorn genannten α-Aminosäuren, sowie Methansulfonsäure, Aethansulfonsäure, 2-Hydroxyäthansulfonsäure, Aethan-1,2-disulfonsäure, Benzolsulfonsäure, 4-Methylbenzolsulfonsäure oder Naphthalin-2-sulfonsäure, oder mit anderen sauren organischen Verbindungen, wie Ascorbinsäure. Verbindungen der Formel I mit sauren und basischen Gruppen können auch innere Salze bilden.

Zur Isolierung oder Reinigung können auch pharmazeutisch ungeeignete Salze Verwendung finden.

Die Verbindungen der vorliegenden Erfindung weisen Enzym-hemmende Wirkungen auf; insbesondere hemmen sie die Wirkung des natürlichen Enzyms Renin. Letzteres gelangt aus den Nieren in das Blut und bewirkt dort die Spaltung von Angiotensinogen unter Bildung des Dekapeptids Angiotensin I, das dann in der Lunge, den Nieren und anderen Organen zum Octapeptid Angiotensin II gespalten wird. Letzteres erhöht den Blutdruck sowohl direkt durch arterielle Konstriktion, als auch indirekt durch die Freisetzung des Natriumionen zurückhaltenden Hormons Aldosteron aus den Nebennieren, womit ein Anstieg des extrazellulären Flüssigkeitsvolumens verbunden ist. Dieser Anstieg ist auf die Wirkung von Angiotensin II selber oder des daraus als Spaltprodukt gebildeten Heptapeptids Angiotensin III zurückzuführen. Hemmer der enzymatischen Aktivität von Renin bewirken eine Verringerung der Bildung von Angiotensin I. Als Folge davon entsteht eine geringere Menge Angiotensin II. Die verminderte Konzentration dieses aktiven Peptidhormons ist die unmittelbare Ursache für die blutdrucksenkende Wirkung von Renin-Hemmern.

Die Wirkung von Renin-Hemmern wird unter anderem experimentell mittels in vitro-Tests nachgewiesen, wobei die Verminderung der Bildung von Angiotensin I in verschiedenen Systemen (Humanplasma, gereinigtes humanes Renin zusammen mit synthetischem oder natürlichem Reninsubstrat) gemessen wird. Unter anderem wird der folgende in vitro Test verwendet: Ein Extrakt von menschlichem Renin aus der Niere (0,5 mGU [Milli-Goldblatt-Einheiten]/ml) wird eine Stunde lang bei 37°C und pH 7,2 in 1-molarer wässriger 2-N-(Tris-hydroxymethylmethyl)-amino-äthansulfonsäure-Pufferlösung mit 23 µg/ml synthetischem Renin-Substrat, dem Tetradecapeptid H-Asp-Arg-Val-Tyr-Ile-His-Pro-Phe-His-Leu-Leu-Val-Tyr-Ser-OH, inkubiert. Die Menge des gebildeten Angiotensins I wird in einem Radioimmunoassay ermittelt. Die erfindungsgemässen Hemmstoffe werden dem Inkubationsgemisch jeweils in verschiedenen Konzentrationen zugefügt. Als $IC_{50}$ wird diejenige Konzentration des jeweiligen Hemmstoffes bezeichnet, die die Bildung von Angiotensin I um 50 % reduziert. Die Verbindungen der vorliegenden Erfindung zeigen in den in vitro-Systemen Hemmwirkungen bei minimalen Konzentrationen von etwa $10^{-6}$ bis etwa $10^{-9}$ Mol/l.

An salzverarmten Tieren bewirken Renin-Hemmer einen Blutdruckabfall. Das menschliche Renin unterscheidet sich von Renin anderer Spezies. Zur Prüfung von Hemmern des humanen Renins werden Primaten (Marmosets, Callithrix jacchus) verwendet, weil humanes Renin und Primaten-Renin im enzymatisch aktiven Bereich weitgehend homolog sind. Unter anderem wird der folgende in vivo Test benutzt: Die Testverbindungen werden an normotensiven Marmosets beider Geschlechter mit einem Körpergewicht von etwa 300 g, die bei Bewusstsein sind, geprüft. Blutdruck und Herzfrequenz werden mit einem Katheter in der Oberschenkelarterie gemessen. Die endogene Freisetzung von Renin wird durch die intravenöse Injektion von Furosemid (5 mg/kg) angeregt. 30 Minuten nach der Injektion von Furosemid werden die Testsubstanzen entweder über einen Katheter in der lateralen Schwanzvene durch einmalige Injektion oder durch kontinuierliche Infusion verabreicht und ihre Wirkung auf Blutdruck und Herzfrequenz ausgewertet. Die Verbindungen der vorliegenden Erfindung sind in dem beschriebenen in vivo Test bei Dosen von etwa 0,1 bis etwa 1,0 mg/kg i.v. wirksam.

Die Verbindungen der vorliegenden Erfindung können als Antihypertensiva, ferner zur Behandlung von Herzinsuffizienz verwendet werden.

Die Erfindung betrifft in erster Linie Verbindungen der Formel I, worin $R_1$ Niederalkanoyl mit 1-7 C-Atomen, z.B. Formyl, Acetyl, Propionyl oder Pivaloyl, Phenoxyniederalkanoyl, z.B. Phenoxyacetyl, Naphthoxyniederalkanoyl, z.B. α- oder β-Naphthoxyacetyl, α-Naphthoxy-carboxy-niederalkanoyl, z.B. 2-α-Naphthoxy-4-carboxy-butyryl, α-Naphthoxy-niederalkoxycarbonyl-niederalkanoyl, z.B. α-Naphthoxy-äthoxycarbonyl-acetyl, 2-α-Naphthoxy-3-äthoxycarbonyl-propionyl oder 2-α-Naphthoxy-4-tert-

butoxycarbonyl-butyryl, α-Naphthoxy-benzyloxycarbonyl-niederalkanoyl, z.B. 2-α-Naphthoxy-3-benzyloxycarbonyl-propionyl, α-Naphthoxy-carbamoyl-niederalkanoyl, z.B. 2-α-Naphthoxy-4-carbamoyl-butyryl, α-Naphthoxy-cyano-niederalkanoyl, z.B. α-Naphthoxy-cyano-acetyl oder 2-α-Naphthoxy-4-cyano-butyryl, α-Naphthoxy-diniederalkylamino-niederalkanoyl, z.B. 2-α-Naphthoxy-5-dimethylamino-pentanoyl, α-Naphthoxy-oxo-niederalkanoyl, z.B. 2-α-Naphthoxy-4-oxo-pentanoyl, Phenyl-, α-Naphthyl-oder β-Naphthyl-niederalkanoyl,worin Niederalkanoyl unsubstituiert oder substituiert z.B. durch Hydroxy, Niederalkoxy, Niederalkanoyloxy, Carboxy, Niederalkoxycarbonyl, Carbamoyl, Niederalkylcarbamoyl, Diniederalkylcarbamoyl, Cyano, Phosphono, Diniederalkoxyphosphoryl, Benzofuranyl und/oder Oxo sein kann und gegebenenfalls verzweigt ist, über eine Carbonylgruppe -CO- gebundenes 2-Phenyläthyl, 3-Phenyl-2-propyl, 4-Phenyl-3-butyl, 2-α-Naphthyläthyl, 3-α-Naphthyl-2-propyl oder 4-α-Naphthyl-3-butyl, das jeweils in 1-Stellung durch Acyloxy in Form von Aethylaminocarbonyloxy, 2-Benzyloxycarbonylamino-2-methyl-propionyloxy, 2-Amino-2-methyl-propionyloxy oder Acetoacetoxy, durch verestertes Carboxy in Form von Benzyloxycarbonyl, durch substituiertes Carbamoyl in Form von Carboxymethylcarbamoyl, tert-Butoxycarbonylmethylcarbamoyl, 2-Dimethylaminoäthylcarbamoyl, 3-Hydroxy-2-propylcarbamoyl, 2,2-Dimethoxyäthylcarbamoyl oder 5-Amino-5-carboxypentylcarbamoyl oder durch verestertes Phosphono in Form von Hydroxymethoxyphosphoryl substituiert ist, z.B. Phenylacetyl, α-Naphthylacetyl, β-Naphthylacetyl, 3-Phenylpropionyl, 3-α- oder β-Naphthylpropionyl, 3-Phenyl- oder 3-α-Naphthyl-2-hydroxy-propionyl, 3-Phenyl- oder 3-α-Naphthyl-2-niederalkoxy-propionyl, wie 3-Phenyl- oder 3-α-Naphthyl-2-neopentyloxy-propionyl, 3-Phenyl-oder 3-α-Naphthyl-2-niederalkanoyloxy-propionyl, wie 3-Phenyl-2-pivaloyloxy- oder 2-acetoxy-propionyl, 3-α-Naphthyl-2-pivaloyloxy- oder 2-acetoxy-propionyl, 3-α-Naphthyl-2-acetoacetoxy-propionyl,3-α-Naphthyl-2-äthylaminocarbonyloxy-propionyl oder 3-α-Naphthyl-2-(2-amino- oder 2-benzyloxycarbonylamino-2-methyl-propionyloxy)-propionyl, 3-Phenyl- oder 3-α-Naphthyl-2-carboxymethyl-propionyl, 3-Phenyl- oder 3-α-Naphthyl-2-niederalkoxycarbonyl-propionyl, wie 3-α-Naphthyl-2-äthoxycarbonyl-propionyl, 3-Phenyl- oder 3-α-Naphthyl-2-benzyloxycarbonylmethyl-propionyl, 3-Phenyl- oder 3-α-Naphthyl-2-carbamoyl-propionyl, 3-Phenyl- oder 3-α-Naphthyl-2-tert-butylcarbamoyl-propionyl, 3-Phenyl- oder 3-α-Naphthyl-2-(2-dimethylaminoäthyl)-carbamoyl-propionyl, 3-α-Naphthyl-2-(carboxy- oder tert-butoxycarbonyl)-methylcarbamoyl-propionyl, 3-Phenyl- oder 3-α-Naphthyl-2-(3-hydroxy-2-propyl)-carbamoyl-propionyl, 3-Phenyl- oder 3-α-Naphthyl-2-(2,2-dimethoxyäthyl)-carbamoyl-propionyl, 3-Phenyl- oder 3-α-Naphthyl-2-(5-Amino-5-carboxypentyl)-carbamoyl-propionyl, 3-Phenyl- oder 3-α-Naphthyl-2-cyano-propionyl, 3-Phenyl- oder 3-α-Naphthyl-2-cyanomethyl-propionyl, 3-Phenyl-2-phosphono-oder phosphonomethyl-propionyl, 3-Phenyl-2-dimethoxyphosphoryl- oder dimethoxyphosphorylmethyl-propionyl, 3-Phenyl-2-diäthoxyphosphoryl- oder diäthoxyphosphorylmethyl-propionyl, 3-Phenyl-2-äthoxy- oder methoxy-hydroxyphosphoryl-propionyl, 3-Phenyl- oder 3-α-Naphthyl-2-acetonyl-propionyl, 3-Phenyl- oder 3-α-Naphthyl-2-dimethylaminomethyl-propionyl, 2-Benzyl- oder 2-α-Naphthylmethyl-4-cyano-butyryl, 4-Phenyl- oder 4-α-Naphthyl-3-carboxy-butyryl, 4-Phenyl- oder 4-α-Naphthyl-3-benzyloxycarbonyl-butyryl, 2-Benzyl-4-(2-benzofuranyl)-4-oxo-butyryl, 2-Benzyl- oder 2-α-Naphthylmethyl-4-oxo-pentanoyl, 2-Benzyl- oder 2-α-Naphthylmethyl-4,4-dimethyl-3-oxo-pentanoyl, 2-Benzyl- oder 2-α-Naphthylmethyl-5-dimethylaminopentanoyl, 2-Benzyl- oder 2-α-Naphthylmethyl-5-dimethylamino-4-oxo-pentanoyl, 2-Benzyl- oder 2-α-Naphthylmethyl-5,5-dimethyl-4-oxo-hexanoyl, α,p-Diamino-phenylacetyl, α,p-Dibenzyloxycarbonylamino-phenylacetyl, α-Pivaloylamino-p-benzyloxycarbonylamino-phenylacetyl, ferner 2-(o,o-Dichloroanilino)-phenylacetyl, 2-(o,o-Dichloro-N-benzylanilino)-phenylacetyl, α- oder β-Naphthylcarbonyl, 1,8-Naphthalindicarbonyl, Pyrrolylcarbonyl, z.B. 2- oder 3-Pyrrolylcarbonyl, Cyclohepta[b]pyrrolyl-5-carbonyl, Indolylcarbonyl, z.B. 2-, 3- oder 5-Indolylcarbonyl, 1-Benzylindolyl-3-carbonyl, 4,5,6,7-Tetrahydroindolyl-2-carbonyl, Chinolylcarbonyl, z.B. 2-, 3-oder 4-Chinolylcarbonyl, oder Oxamoyl,

A den bivalenten Rest der Aminosäuren Leucin, Norleucin, Phenylalanin, N-Methyl-phenylalanin, β-Phenylserin, Cyclohexylalanin, Glutamin, Histidin oder N-Methyl-histidin, $R_2$ Wasserstoff, $R_3$ Isobutyl oder Cyclohexylmethyl, $R_4$ Hydroxy, $R_5$ Isopropyl, Cyclohexylmethyl, α-Decahydronaphthyl oder Dimethylamino und $R_6$ Niederalkylamino mit 1-7 C-Atomen, z.B. Methyl-, Aethyl-, n-Propyl-, Isopropyl-, n-Butyl-, Isobutyl-, n-Pentyl oder Isopentylamino, Diniederalkylamino, z.B. Dimethylamino, Hydroxyniederalkylamino, z.B. 2-Hydroxyäthylamino, 1-Hydroxybut-2-ylamino oder 5-Hydroxypentylamino, 2-Phenoxyäthylamino, 2-(3-Carbamoyl-4-hydroxyphenoxy)-äthylamino, Carboxyalkylamino oder Amino-carboxyalkylamino, worin der Carboxyrest nicht in 1-Stellung des Alkylrests, welcher bis zu 10 C-Atome enthält, steht, z.B. 4-Carboxy-n-butylamino, 5-Amino-5-carboxy-n-pentylamino, 7-Carboxy-n-heptylamino oder 8-Carboxy-n-octylamino, Niederalkoxycarbonylalkylamino oder (Niederalkanoylamino-, Niederalkoxycarbonylamino- oder Benzyloxycarbonylamino)-niederalkoxycarbonyl-alkylamino, worin der Carbonylrest nicht in 1-Stellung des Alkylrests, welcher bis zu 10 C-Atome enthält, steht, z.B. 4-tert-Butoxycarbonyl-n-butylamino, 5-tert-Butoxycarbonylamino-5-methoxycarbonyl-n-pentylamino oder 7-tert-Butoxycarbonyl-n-heptylamino, 4-Tris-(hydroxymethyl)-methylcarbamoyl-n-butylamino, Aminoniederalkylamino, z.B. 2-Aminoäthylamino, Diniederalkylaminoniederalkylamino, z.B. 2-Dimethylaminoäthylamino oder 3-Dimethylaminopropylamino, Niederalkoxycarbonylaminoniederalkylamino, z.B. 2-tert-Butoxycarbonylamino-äthylamino, Morpholinoniederalkylamino, z.B. 2-Morpholino-äthylamino, Cycloalkylniederalkylamino mit 4-10 C-Atomen, z.B. Cyclopropylmethylamino oder Cyclohexylmethylamino, Benzylamino, Pyridylniederalkylamino, z.B. 2-Pyridylmethylamino, Imidazolylniederalkylamino, z.B. 2-(4-Imidazolyl)-äthylamino, oder 2-(2-[4-Imidazolyl]-äthylamino)-äthylamino, darstellt, sowie pharmazeutisch annehmbare Salze von diesen Verbindungen mit salzbildenden Gruppen mit Ausnahme der Verbindung der Formel I, worin $R_1$ $\alpha$-Naphthoxyacetyl, A den bivalenten Rest der Aminosäure Histidin, $R_2$ Wasserstoff, $R_3$ Isobutyl, $R_4$ Hydroxy, $R_5$ Isopropyl und $R_6$ Cyclohexylmethylamino bedeuten.

Die Erfindung betrifft in erster Linie auch Verbindungen der Formel I, worin $R_1$ Phenoxyniederalkanoyl, z.B. Phenoxyacetyl, Naphthoxyniederalkanoyl, z.B. $\alpha$- oder $\beta$-Naphthoxyacetyl, Phenylniederalkanoyl, worin Niederalkanoyl unsubstituiert oder substituiert durch Niederalkanoyloxy, Carboxy, Niederalkoxycarbonyl, Carbamoyl, Niederalkylcarbamoyl, Cyano, Diniederalkoxyphosphoryl, oder Niederalkyl und Oxo oder Benzofuranyl und Oxo sein kann und gegebenenfalls verzweigt ist, z.B. 3-Phenylpropionyl, 2-Acetoxy-3-phenyl-propionyl, 2-Pivaloyloxy-3-phenyl-propionyl, 2-Aethoxy- oder -Methoxycarbonyl-3-phenyl-propionyl, 2-tert-Butylcarbamoyl-3-phenyl-propionyl, 2-Benzyl-3-cyano-propionyl, 2-Dimethoxyphoshoryl-3-phenyl-propionyl, 2-Benzyl-5,5-dimethyl-4-oxo-hexanoyl, 2-Benzyl-4,4-dimethyl-3-oxo-pentanoyl oder 4-(2-Benzofuranyl)-2-benzyl-4-oxo-butyryl, Naphthylniederalkanoyl, worin Niederalkanoyl unsubstituiert oder substituiert durch Niederalkanoyloxy, Carboxy, Niederalkoxycarbonyl, Carbamoyl, Cyano oder Niederalkyl und Oxo sein kann und gegebenenfalls verzweigt ist, z.B. 3-$\alpha$- oder $\beta$-Naphthyl-propionyl, 2-Acetoxy-3-$\alpha$-naphthyl-propionyl, 2-Pivaloyloxy-3-$\alpha$-naphthyl-propionyl, 2-Aethoxy-oder Methoxycarbonyl-3-$\alpha$-naphthyl-propionyl, 2-Carbamoyl-3-$\alpha$-naphthyl-propionyl, 3-Cyano-2-$\alpha$-naphthylmethyl-propionyl, 5,5-Dimethyl-2-$\alpha$-naphthylmethyl-4-oxo-hexanoyl oder 4,4-Dimethyl-2-$\alpha$-naphthylmethyl-3-oxo-pentanoyl, ferner 2-tert-Butylcarbamoyl-3-$\alpha$-naphthyl-propionyl, 2-Carboxymethylcarbamoyl-3-$\alpha$-naphthyl-propionyl, Indolyl-2-carbonyl oder Cyclohepta[b]-pyrrolyl-5-carbonyl, A den bivalenten Rest der Aminosäure L-Histidin, $R_2$ Wasserstoff, $R_3$ Isobutyl oder Cyclohexylmethyl, $R_4$ Hydroxy, $R_5$ Isopropyl oder Cyclohexylmethyl und $R_6$ Niederalkylamino, z.B. Methyl-, Aethyl-, n-Propyl-, Isopropyl-, n-Butyl-, Isobutyl-, n-Pentyl oder Isopentylamino oder Amino-carboxy-alkylamino, worin die Substituenten nicht in 1-Stellung des Alkylrests, welcher bis zu 7 C-Atome enthält, stehen, z.B. 5-Amino-5-carboxy-pentylamino, bedeuten und die die Reste $R_3$ und $R_4$ tragenden C-Atome die S-Konfiguration aufweisen, ferner pharmazeutisch annehmbare Salze von diesen Verbindungen.

Die Erfindung betrifft in erster Linie auch Verbindungen der Formel I mit den Bezeichnungen

N-(N-Benzyl-indolyl-2-carbonyl)-His-Leu$\overset{C}{-}$Val-methylamid,

N-(3(R,S)-Benzyloxycarbonyl-4-α-naphthylbutyryl)-His-Leu$\overset{C}{-}$Val-methylamid,

N-Benzyloxycarbonyl-(p-benzyloxycarbonylamino-Phe)-His-Leu$\overset{C}{-}$Val-n-butylamid,

N-Pivaloyl-(p-benzyloxycarbonylamino-Phe)-His-Leu$\overset{C}{-}$Val-n-butylamid,

N-(Indolyl-2-carbonyl)-His-Leu$\overset{C}{-}$Val-4-[tris-(tert-butyldimethylsilyl-oxymethyl)-methylcarbamoyl]-butylamid,

N-(2-Aethylaminocarbonyloxy-4-phenyl-butyryl)-His-Cha$\overset{C}{-}$Val-n-butyl-amid,

und deren pharmazeutisch annehmbare Salze.

Ganz besonders betrifft die Erfindung Verbindungen der Formel I, worin $R_1$ 2(S)-Pivaloyloxy-3-phenyl-propionyl, 2(R)- und 2(S)-Dimethoxyphosphoryl-3-phenyl-propionyl, 2(R)- und 2(S)-Benzyl-5,5-dimethyl-4-oxo-hexanoyl, 2(R)- und 2(S)-Benzyl-4,4-dimethyl-3-oxo-pentanoyl, 2(R)- und 2(S)-tert-Butylcarbamoyl-3-$\alpha$-naphthyl-propionyloder 2(R)-und 2(S)-Aethoxycarbonyl-3-$\alpha$-naphthyl-propionyl, ferner Indolyl-2-carbonyl oder Cyclohepta[b]pyrrolyl-5-carbonyl, A den bivalenten Rest der Aminosäure L-Histidin, $R_2$ Wasserstoff, $R_3$ Cyclohexylmethyl, $R_4$ Hydroxy, $R_5$ Isopropyl und $R_6$ Niederalkylamino mit 1 bis 7 C-Atomen, z.B. Methyl-,

Aethyl-, n-Propyl-, Isopropyl-, n-Butyl-, Isobutyl-, n-Pentyl- oder Isopentylamino, bedeuten und die die Reste $R_3$, $R_4$ und $R_5$ tragenden C-Atome die S-Konfiguration aufweisen, ferner pharmazeutisch annehmbare Salze von diesen Verbindungen. Die Erfindung betrifft zuallererst die in den Beispielen erwähnten Verbindungen und deren pharmazeutisch annehmbare Salze, insbesondere

die Verbindung der Formel I, worin $R_1$ 2(S)-Pivaloyloxy-3-phenyl-propionyl, A den bivalenten Rest der Aminosäure L-Histidin, $R_2$ Wasserstoff, $R_3$ Cyclohexylmethyl, $R_4$ Hydroxy, $R_5$ Isopropyl und $R_6$ n-Butylamino bedeuten und die die Reste $R_3$, $R_4$ und $R_5$ tragenden C-Atome die S-Konfiguration aufweisen, und deren pharmazeutisch annehmbare Salze,

die Verbindungen der Formel I, worin $R_1$ 2(R,S)-, 2(R)- oder 2(S)-Dimethoxyphosphoryl-3-phenyl-propionyl, A den bivalenten Rest der Aminosäure L-Histidin, $R_2$ Wasserstoff, $R_3$ Cyclohexylmethyl, $R_4$ Hydroxy, $R_5$ Isopropyl und $R_6$ n-Butylamino bedeuten und die die Reste $R_3$, $R_4$ und $R_5$ tragenden C-Atome die S-Konfiguration aufweisen, und deren pharmazeutisch annehmbare Salze,

die Verbindungen der Formel I, worin $R_1$ 2(R,S)-, 2(R)- oder 2(S)-Benzyl-5,5-dimethyl-4-oxo-hexanoyl, A den bivalenten Rest der Aminosäure L-Histidin, $R_2$ Wasserstoff, $R_3$ Cyclohexylmethyl, $R_4$ Hydroxy, $R_5$ Isopropyl und $R_6$ n-Butylamino bedeuten und die die Reste $R_3$, $R_4$ und $R_5$ tragenden C-Atome die S-Konfiguration aufweisen, und deren pharmazeutisch annehmbare Salze,

die Verbindung der Formel I, worin $R_1$ 2(R,S)-Benzyl-4,4-dimethyl-3-oxo-pentanoyl, A den bivalenten Rest der Aminosäure L-Histidin, $R_2$ Wasserstoff, $R_3$ Cyclohexylmethyl, $R_4$ Hydroxy, $R_5$ Isopropyl und $R_6$ n-Butylamino bedeuten und die die Reste $R_3$, $R_4$ und $R_5$ tragenden C-Atome die S-Konfiguration aufweisen, und deren pharmazeutisch annehmbare Salze,

die Verbindung der Formel I, worin $R_1$ 2(R,S)-Aethoxycarbonyl-3-$\alpha$-naphthyl-propionyl, A den bivalenten Rest der Aminosäure L-Histidin, $R_2$ Wasserstoff, $R_3$ Cyclohexylmethyl, $R_4$ Hydroxy, $R_5$ Isopropyl und $R_6$ n-Butylamino bedeuten und die die Reste $R_3$, $R_4$ und $R_5$ tragenden C-Atome die S-Konfiguration aufweisen, und deren pharmazeutisch annehmbare Salze,

die Verbindung der Formel I, worin $R_1$ Cyclohepta[b]pyrrolyl-5-carbonyl, A den bivalenten Rest der Aminosäure L-Histidin, $R_2$ Wasserstoff, $R_3$ Cyclohexylmethyl, $R_4$ Hydroxy, $R_5$ Isopropyl und $R_6$ n-Butylamino bedeuten und die die Reste $R_3$, $R_4$ und $R_5$ tragenden C-Atome die S-Konfiguration aufweisen, und deren pharmazeutisch annehmbare Salze,

und die Verbindung der Formel I, worin $R_1$ 2(S)-Pivaloyloxy-3-phenyl-propionyl, A den bivalenten Rest der Aminosäure L-Histidin, $R_2$ Wasserstoff, $R_3$ Isobutyl, $R_4$ Hydroxy, $R_5$ Cyclohexylmethyl und $R_6$ n-Butylamino bedeuten und die die Reste $R_3$ und $R_4$ tragenden C-Atome die S-Konfiguration aufweisen, und deren pharmazeutisch annehmbare Salze.

Verfahren:

Die erfindungsgemässen Verbindungen der Formel I und Salze von solchen Verbindungen mit mindestens einer salzbildenden Gruppe werden nach an sich bekannten Verfahren erhalten, z.B. indem man

a) ein Fragment einer Verbindung der Formel I mit einer endständigen Carboxygruppe oder ein reaktionsfähiges Säurederivat dieses Fragments mit einem zur Verbindung der Formel I komplementären Fragment mit einer freien Aminogruppe oder einem reaktionsfähigen Derivat davon mit aktivierter Aminogruppe, wobei in den Reaktionskomponenten vorhandene freie funktionelle Gruppen mit Ausnahme der an der Reaktion teilnehmenden Gruppen gegebenenfalls in geschützter Form vorliegen, unter Bildung einer Amidbindung kondensiert, oder

b) die Ketogruppe in einer Verbindung der Formel

$$R_1 - A - \overset{\displaystyle R_2}{\overset{|}{N}} - \underset{\displaystyle R_3}{\underset{|}{CH}} - \overset{\displaystyle O}{\overset{\|}{C}} - CH_2 - \overset{\displaystyle R_5}{\overset{|}{CH}} - \overset{\displaystyle O}{\overset{\|}{C}} - R_6 \qquad (II),$$

worin die Substituenten die genannten Bedeutungen haben und freie funktionelle Gruppen mit Ausnahme der an der Reaktion teilnehmenden Ketogruppe gegebenenfalls in geschützter Form vorliegen, durch Umsetzung mit einem geeigneten Reduktionsmittel zu einer Hydroxygruppe reduziert, oder

c) eine Aldehyd-Verbindung der Formel

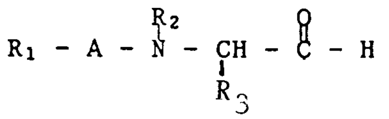 (III),

worin die Substituenten die genannten Bedeutungen haben und freie funktionelle Gruppen mit Ausnahme der Aldehydgruppe gegebenenfalls in geschützter Form vorliegen, mit einer Organometallverbindung der Formel

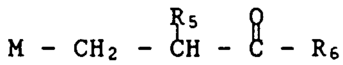 (IV),

worin die Substituenten die genannten Bedeutungen haben und M ein Metallradikal bedeutet, umsetzt und das gebildete Additionsprodukt hydrolysiert, oder

d) in einer Verbindung der Formel

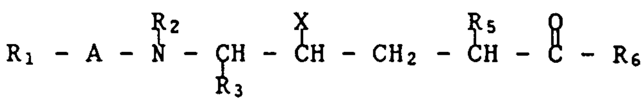 (V),

worin X eine nukleofuge Abgangsgruppe ist, die übrigen Substituenten die oben genannten Bedeutungen haben und freie funktionelle Gruppen gegebenenfalls in geschützter Form vorliegen, den Substituenten X mit einem den Substituenten $R_4$ in nukleophiler Form einführenden Reagens gegen $R_4$ austauscht, oder

e) in einer Verbindung der Formel

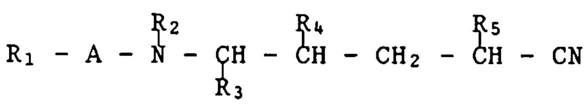 (VI),

worin die Substituenten die genannten Bedeutungen haben und vorhandene funktionelle Gruppen gegebenenfalls in geschützter Form vorliegen, die Cyanogruppe in eine N-substituierte Carboxamidogruppe $-(C=O)R_6$ überführt, oder

f) ein Epoxid der Formel

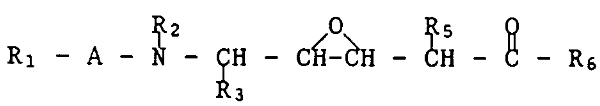 (VII),

worin die Substituenten die genannten Bedeutungen haben und freie funktionelle Gruppen gegebenenfalls in geschützter Form vorliegen, mit einem regio-selektiven Reduktionsmittel zum entsprechenden Alkohol reduziert, und gewünschtenfalls

g) in einer erhältlichen Verbindung vorhandene Schutzgruppen abspaltet und/oder gewünschtenfalls nach Ausführung eines der vorstehend genannten Verfahren a) - f) oder eines beliebigen anderen Verfahrens zur Herstellung einer Verbindung der Formel I eine erhältliche Verbindung der Formel I mit einer salzbildenden Gruppe in ihr Salz oder ein erhältliches Salz in die freie Verbindung oder in ein anderes Salz überführt und/oder gegebenenfalls erhältliche Isomerengemische auftrennt und/oder in einer erhältlichen Verbindung der Formel I die Konfiguration eines chiralen Kohlenstoffatoms umkehrt und/oder eine erfindungsgemässe Verbindung der Formel I in eine andere erfindungsgemässe Verbindung der Formel I umwandelt.

Die Erfindung betrifft auch die nach irgendeinem der oben genannten Verfahren erhältlichen anderen Verbindungen als Verbindungen der Formel I (Nebenprodukt), sowie Verbindungen der Formel I und ihre Salze, welche nach einem anderen Verfahren als einem der weiter vorn genannten hergestellt werden.

Verfahren a) (Herstellung einer Amidbindung):

Fragmente einer Verbindung der Formel I mit einer endständigen Carboxygruppe, welche mit einem zur Verbindung der Formel I komplementären Fragment unter Bildung einer Amidbindung kondensiert werden können, sind z.B. Verbindungen der Formeln $R_1$-OH, $R_1$-A-OH oder

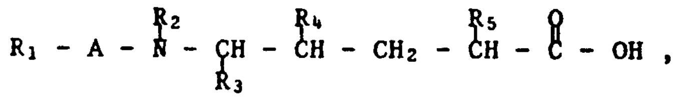

die von diesen Verbindungen abgeleiteten aktivierten Ester oder reaktionsfähigen Anhydride, ferner reaktionsfähige cyclische Amide. Die reaktionsfähigen Säurederivate können auch in situ gebildet werden.

Aktivierte Ester sind insbesondere am Verknüpfungskohlenstoffatom des veresternden Restes ungesättigte Ester, z.B. vom Vinylester-Typ, wie Vinylester (erhältlich z.B. durch Umesterung eines entsprechenden Esters mit Vinylacetat; Methode des aktivierten Vinylesters), Carbamoylvinylester (erhältlich z.B. durch Behandeln der entsprechenden Säure mit einem Isoxazoliumreagens; 1,2-Oxazolium- oder Woodward-Methode), oder 1-Niederalkoxyvinylester (erhältlich z.B. durch Behandeln der entsprechenden Säure mit einem Niederalkoxyacetylen; Aethoxyacetylen-Methode), oder Ester vom Amidinotyp, wie N,N'-disubstituierte Amidinoester (erhältlich z.B. durch Behandeln der entsprechenden Säure mit einem geeigneten N,N'-disubstituierten Carbodiimid, z.B. N,N'-Dicyclohexylcarbodiimid; Carbodiimid-Methode), oder N,N-disubstituierte Amidinoester (erhältlich z.B. durch Behandeln der entsprechenden Säure mit einem N,N-disubstituierten Cyanamid; Cyanamid-Methode), geeignete Arylester, insbesondere durch elektronenanziehende Substituenten geeignet substituierte Phenylester (erhältlich z.B. durch Behandeln der entsprechenden Säure mit einem geeignet substituierten Phenol, z.B. 4-Nitrophenol, 4-Methylsulfonylphenol,2,4,5-Trichlorphenol, 2,3,4,5,6-Pentachlorphenol oder 4-Phenyldiazophenol, in Gegenwart eines Kondensationsmittels, wie N,N'-Dicyclohexylcarbodiimid; Methode der aktivierten Arylester), Cyanmethylester (erhältlich z.B. durch Behandeln der entsprechenden Säure mit Chloracetonitril in Gegenwart einer Base; Cyanmethylester-Methode), Thioester, insbesondere gegebenenfalls, z.B. durch Nitro, substituierte Phenylthioester (erhältlich z.B. durch Behandeln der entsprechenden Säure mit gegebenenfalls, z.B. durch Nitro, substituierten Thiophenolen, u.a. mit Hilfe der Anhydrid- oder Carbodiimid-Methode; Methode der aktivierten Thiolester), oder insbesondere Amino- oder Amidoester (erhältlich z.B. durch Behandeln der entsprechenden Säure mit einer N-Hydroxyamino- bzw. N-Hydroxyamido-Verbindung, z.B. N-Hydroxysuccinimid, N-Hydroxypiperidin, N-Hydroxyphthalimid, N-Hydroxy-5-norbornen-2,3-dicarbonsäureimid, 1-Hydroxybenztriazol oder 3-Hydroxy-3,4-dihydro-1,2,3-benzotriazin-4-on, z.B. nach der Anhydrid-oder Carbodiimid-Methode; Methode der aktivierten N-Hydroxyester).

Anhydride von Säuren können symmetrische oder vorzugsweise gemischte Anhydride dieser Säuren sein, z.B. Anhydride mit anorganischen Säuren, wie Säurehalogenide, insbesondere Säurechloride (erhältlich z.B. durch Behandeln der entsprechenden Säure mit Thionylchlorid, Phosphorpentachlorid oder Oxalylchlorid; Säurechloridmethode), Azide (erhältlich z.B. aus einem entsprechenden Säureester über das entsprechende Hydrazid und dessen Behandlung mit salpetriger Säure; Azidmethode), Anhydride mit Kohlensäurehalbestern, z.B. Kohlensäureniederalkylhalbestern (erhältlich z.B. durch Behandeln der entsprechenden Säure mit Chlorameisensäureniederalkylestern oder mit einem 1-Niederalkoxycarbonyl-2-niederalkoxy-1,2-dihydrochinolin, z.B. 1-Niederalkoxycarbonyl-2-äthoxy-1,2-dihydrochinolin; Methode der gemischten O-Alkylkohlensäureanhydride), oder Anhydride mit dihalogenierter, insbesondere dichlorierter Phosphorsäure (erhältlich z.B. durch Behandeln der entsprechenden Säure mit Phosphoroxychlorid; Phosphoroxychloridmethode), Anhydride mit anderen Phosphorsäurederivaten (z.B. solchen, die man mit Phenyl-N-phenylphosphoramidochloridat erhalten kann) oder mit Phosphorigsäurederivaten, oder Anhydride mit organischen Säuren, wie gemischte Anhydride mit organischen Carbonsäuren (erhältlich z.B. durch Behandeln der entsprechenden Säure mit einem gegebenenfalls substituierten Niederalkan- oder Phenylniederalkancarbonsäurehalogenid, z.B. Phenylessigsäure-, Pivalinsäure- oder Trifluoressigsäurechlorid; Methode der gemischten Carbonsäureanhydride) oder mit organischen Sulfonsäuren (erhältlich z.B. durch Behandeln eines Salzes, wie eines Alkalimetallsalzes, der entsprechenden Säure mit einem geeigneten organischen Sulfonsäurehalogenid, wie Niederalkan- oder Aryl-, z.B. Methan- oder p-Toluolsulfonsäurechlorid; Methode der gemischten Sulfonsäureanhydride), sowie symmetrische Anhydride (erhältlich z.B. durch Kondensation der entsprechenden Säure in Gegenwart eines Carbodiimids oder von 1-Diäthylaminopropin; Methode der symmetrischen Anhyride).

Geeignete cyclische Amide sind insbesondere Amide mit fünfgliedrigen Diazacyclen aromatischen

Charakters, wie Amide mit Imidazolen, z.B. Imidazol (erhältlich z.B. durch Behandeln der entsprechenden Säure mit N,N'-Carbonyldiimidazol; Imidazol-Methode), oder Pyrazol, z.B. 3,5-Dimethylpyrazol (erhältlich z.B. über das Säurehydrazid durch Behandeln mit Acetylaceton; Pyrazolid-Methode).

Zur Verbindung der Formel I komplementäre Fragmente mit einer freien Aminogruppe sind z.B. je nach Bedeutung von $R_6$ ein primäres oder sekundäres Amin, ferner Verbindungen der Formeln

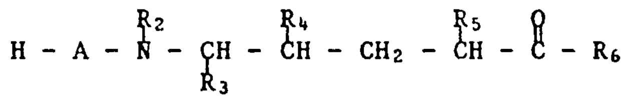

oder 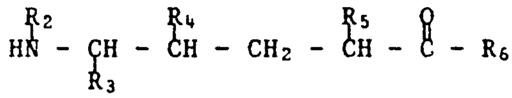 .

Die an der Reaktion teilnehmende Aminogruppe in einem zu einer Verbindung der Formel I komplementären Fragment liegt bevorzugt in freier Form vor, insbesondere wenn die damit reagierende Carboxygruppe in reaktionsfähiger Form vorliegt; sie kann aber auch selbst derivatisiert sein, z.B. durch Reaktion mit einem Phosphit, wie Diäthylchlorphosphit, 1,2-Phenylenchlorphosphit, Aethyldichlorphosphit, Aethylenchlorphosphit oder Tetraäthylpyrophosphit. Ein Derivat eines solchen komplementären Bruchstücks mit einer Aminogruppe ist z.B. auch ein Carbaminsäurehalogenid oder ein Isocyanat, wobei die an der Reaktion teilnehmende Aminogruppe durch Halogencarbonyl, z.B. Chlorcarbonyl, substituiert bzw. als Isocyanatgruppe abgewandelt ist, wobei im letzteren Falle nur Verbindungen der Formel I zugänglich sind, die am Stickstoffatom der durch die Reaktion gebildeten Amidgruppe ein Wasserstoffatom tragen.

Ist das komplementäre Fragment mit einer Aminogruppe ein durch Niederalkyl mono- oder disubstituiertes Amin oder Benzylamin, so stellt auch eine entsprechende Harnstoff-Verbindung ein reaktionsfähiges Derivat dar. Beispielsweise erhält man beim Erhitzen äquimolarer Mengen dieser Harnstoffverbindung und der Komponente mit freier Carboxygruppe entsprechende Verbindungen der Formel I.

Ist das komplementäre Fragment Dimethylamin, so ist auch Dimethylformamid ein reaktionsfähiges Derivat.

Funktionelle Gruppen in Ausgangsmaterialien, deren Umsetzung vermieden werden soll, insbesondere Carboxy-, Amino- und Hydroxygruppen, können durch geeignete Schutzgruppen (conventional protecting groups) geschützt sein, die üblicherweise bei der Synthese von Peptid-Verbindungen, aber auch von Cephalosporinen und Penicillinen verwendet werden. Diese Schutzgruppen können bereits in den Vorstufen vorhanden sein und sollen die betreffenden funktionellen Gruppen gegen unerwünschte Nebenreaktionen wie Acylierungen, Verätherungen, Veresterungen, Oxydationen, Solvolyse etc. schützen. Schutzgruppen können aber auch in den Endstoffen vorhanden sein. Verbindungen der Formel I mit geschützten funktionellen Gruppen können eine höhere metabolische Stabilität aufweisen als die entsprechenden Verbindungen mit freien funktionellen Gruppen.

Der Schutz von funktionellen Gruppen durch solche Schutzgruppen, die Schutzgruppen selbst, sowie ihre Abspaltungsreaktionen, sind beispielsweise in Standardwerken wie in J.F.W. McOmie, "Protective Groups in Organic Chemistry", Plenum Press, London und New York 1973, in Th. W. Greene, "Protective Groups in Organic Synthesis", Wiley, New York 1981, in "The Peptides"; Band 3 (Herausg. E. Gross und J. Meienhofer), Academic Press, London und New York 1981, sowie in "Methoden der organischen Chemie", Houben-Weyl, 4. Auflage, Bd. 15/I, Georg Thieme Verlag, Stuttgart 1974, beschrieben.

Eine Carboxygruppe ist z.B. als eine Estergruppe geschützt, die unter schonenden Bedingungen selektiv spaltbar ist. Eine in veresterter Form geschützte Carboxygruppe ist in erster Linie durch eine Niederalkylgruppe verestert, welche in 1-Stellung der Niederalkylgruppe verzweigt oder in 1- oder 2-Stellung der Niederalkylgruppe durch geeignete Substituenten substituiert ist.

Eine geschützte Carboxygruppe, welche durch eine Niederalkylgruppe verestert ist, die in 1-Stellung der Niederalkylgruppe verzweigt ist, ist beispielsweise tert-Niederalkoxycarbonyl, z.B. tert-Butoxycarbonyl, Arylmethoxycarbonyl mit einem oder zwei Arylresten, worin Aryl unsubstituiertes oder z.B. durch Niederalkyl, z.B. tert-Niederalkyl, wie tert-Butyl, Niederalkoxy, z.B. Methoxy, Hydroxy. Halogen, z.B. Chlor, und/oder Nitro mono-, di- oder trisubstituiertes Phenyl bedeutet, beispielsweise Benzyloxycarbonyl, durch die genannten Substituenten substituiertes Benzyloxycarbonyl, z.B. 4-Nitrobenzyloxycarbonyl oder 4-Methoxybenzyloxycarbonyl, Diphenylmethoxycarbonyl oder durch die genannten Substituenten substituiertes Diphenylmethoxycarbonyl, z.B. Di-(4-methoxyphenyl)-methoxycarbonyl.

Eine geschützte Carboxygruppe, welche durch eine Niederalkylgruppe verestert ist, welche in 1- oder

2-Stellung der Niederalkylgruppe durch geeignete Substituenten substituiert ist, ist beispielsweise 1-Niederalkoxyniederalkoxycarbonyl, z.B. Methoxymethoxycarbonyl, 1-Methoxyäthoxycarbonyl oder 1-Aethoxyäthoxycarbonyl, 1-Niederalkylthioniederalkoxycarbonyl, z.B. 1-Methylthiomethoxycarbonyl oder 1-Aethylthioäthoxycarbonyl, Aroylmethoxycarbonyl, z.B. Phenacyloxycarbonyl, 2-Halogenniederalkoxycarbonyl, z.B. 2,2,2-Trichloräthoxycarbonyl, 2-Bromäthoxycarbonyl oder 2-Jodäthoxycarbonyl, sowie 2-Triniederalkylsilylniederalkoxycarbonyl, z.B. 2-Trimethylsilyläthoxycarbonyl.

Eine Carboxygruppe kann auch als organische Silyloxycarbonylgruppe geschützt sein. Eine organische Silyloxycarbonylgruppe ist beispielsweise eine Triniederalkylsilyloxycarbonylgruppe, z.B. Trimethylsilyloxycarbonyl. Das Siliciumatom der Silyloxycarbonylgruppe kann auch durch zwei Niederalkylgruppen, z.B. Methylgruppen, und die Aminogruppe oder die Carboxygruppe eines zweiten Moleküls der Formel I substituiert sein. Verbindungen mit solchen Schutzgruppen lassen sich z.B. mit Dimethylchlorsilan als Silylierungsmittel herstellen.

Eine geschützte Carboxygruppe ist bevorzugt tert-Niederalkoxycarbonyl, z.B. tert-Butoxycarbonyl, Benzyloxycarbonyl, 4-Nitrobenzyloxycarbonyl oder Diphenylmethoxycarbonyl.

Eine Aminogruppe kann z.B. in Form einer Acylamino-, Arylmethylamino-, verätherten Mercaptoamino- oder Silylaminogruppe oder als Azidogruppe geschützt sein.

In einer entsprechenden Acylaminogruppe ist Acyl beispielsweise der Acylrest einer organischen Carbonsäure mit z.B. bis zu 18 Kohlenstoffatomen, insbesondere einer gegebenenfalls, z.B. durch Halogen oder Aryl, substituierten Niederalkancarbonsäure oder gegebenenfalls, z.B. durch Halogen, Niederalkoxy oder Nitro, substituierten Benzoesäure, oder bevorzugt eines Kohlensäurehalbesters. Solche Acylgruppen sind beispielsweise Niederalkanoyl, wie Formyl, Acetyl, Propionyl oder Pivaloyl, Halogenniederalkanoyl, z.B. 2-Halogenacetyl, wie 2-Chlor-, 2-Brom-, 2-Jod-, 2,2,2-Trifluor- oder 2,2,2-Trichloracetyl, gegebenenfalls z.B. durch Halogen, Niederalkoxy oder Nitro substituiertes Benzoyl, z.B. Benzoyl, 4-Chlorbenzoyl, 4-Methoxybenzoyl oder 4-Nitrobenzoyl, oder in 1-Stellung des Niederalkylrestes verzweigtes oder in 1- oder 2-Stellung geeignet substituiertes Niederalkoxycarbonyl, z.B. tert-Niederalkoxycarbonyl, wie tert-Butoxycarbonyl, Arylmethoxycarbonyl mit einem oder zwei Arylresten, die gegebenenfalls, z.B. durch Niederalkyl, z.B. tert-Niederalkyl, wie tert-Butyl, Niederalkoxy, wie Methoxy, Hydroxy, Halogen, wie Chlor, und/oder Nitro, mono- oder polysubstituiertes Phenyl darstellen, z.B. Benzyloxycarbonyl, 4-Nitrobenzyloxycarbonyl, Diphenylmethoxycarbonyl oder Di-(4-methoxyphenyl)-methoxycarbonyl, Aroylmethoxycarbonyl, z.B. Phenacyloxycarbonyl, 2-Halogen-niederalkoxycarbonyl, z.B. 2,2,2-Trichloräthoxycarbonyl, 2-Bromäthoxycarbonyl oder 2-Jodäthoxycarbonyl, 2-Triniederalkylsilylniederalkoxycarbonyl, z.B. 2-Trimethlysilyläthoxycarbonyl, oder 2-Triarylsilylniederalkoxycarbonyl, z.B. 2-Triphenylsilyläthoxycarbonyl.

Eine Arylmethylaminogruppe ist z.B. Mono-, Di- oder insbesondere Triphenylmethylamino, z.B. Benzyl-, Diphenylmethyl- oder Tritylamino.

In einer verätherten Mercaptoaminogruppe ist die verätherte Mercaptogruppe in erster Linie substituiertes Arylthio, z.B. 4-Nitrophenylthio.

Eine Silylaminogruppe ist beispielsweise eine Triniederalkylsilylaminogruppe, z.B. Trimethylsilylamino. Das Siliciumatom der Silylaminogruppe kann auch nur durch zwei Niederalkylgruppen, z.B. Methylgruppen, und die Aminogruppe oder Carboxylgruppe eines zweiten Moleküls der Formel I substituiert sein. Verbindungen mit solchen Schutzgruppen lassen sich z.B. mit Dimethylchlorsilan als Silylierungsmittel herstellen.

Bevorzugte Aminoschutzgruppen sind Acylreste von Kohlensäurehalbestern, insbesondere tert-Butoxycarbonyl, gegebenenfalls substituiertes Benzyloxycarbonyl, z.B. 4-Nitrobenzyloxycarbonyl, Diphenylmethoxycarbonyl, 2-Halogen-niederalkoxycarbonyl, z.B. 2,2,2-Trichloräthoxycarbonyl, ferner Trityl oder Formyl.

Eine Hydroxygruppe kann beispielsweise durch eine Acylgruppe, z.B. durch Halogen, z.B. Chlor, substituiertes Niederalkanoyl, z.B. 2,2-Dichloracetyl, oder insbesondere durch einen für geschützte Aminogruppen genannten Acylrest eines Kohlensäurehalbesters geschützt sein. Eine bevorzugte Hydroxyschutzgruppe ist beispielsweise 2,2,2-Trichloräthoxycarbonyl, 4-Nitrobenzyloxycarbonyl, Diphenylmethoxycarbonyl oder Trityl. Eine Hydroxygruppe kann ferner durch Triniederalkylsilyl, z.B. Trimethylsilyl oder Dimethyl-tert-butylsilyl, eine leicht abspaltbare Alkylgruppe, wie tert-Niederalkyl, z.B. tert-Butyl, einen oxa- oder einen thiaaliphatischen oder -cycloaliphatischen Kohlenwasserstoffrest, beispielsweise 1-Niederalkoxyniederalkyloder 1-Niederalkylthioniederalkyl, z.B. Methoxymethyl, 1-Methoxyäthyl, 1-Aethoxyäthyl, Methylthiomethyl, 1-Methylthioäthyl oder 1-Aethylthioäthyl, oder 2-Oxa- oder 2-Thiacycloalkyl mit 5-7 Ringatomen, z.B. 2-Tetrahydrofuryl oder 2-Tetrahydropyranyl, oder ein entsprechendes Thiaanaloges, sowie durch 1-Phenylniederalkyl, z.B. Benzyl, Diphenylmethyl oder Trityl, wobei die Phenylreste beispielsweise durch Halogen, z.B. Chlor, Niederalkoxy, z.B. Methoxy, und/oder Nitro substituiert sein können, geschützt sein.

Zwei benachbarte Hydroxylgruppen können beispielsweise durch eine vorzugsweise substituierte Methylengruppe geschützt sein, z.B. durch Niederalkyliden, z.B. Isopropyliden, Cycloalkyliden, z.B. Cyclohexyliden, oder Benzyliden.

Die Kondensation zur Herstellung der Amidbindung kann in an sich bekannter Weise durchgeführt werden, beispielsweise wie in Standardwerken, wie "Houben-Weyl, Methoden der organischen Chemie", 4. Auflage, Band 15/II, Georg Thieme Verlag, Stuttgart 1974, "The Peptides" (Herausg. E. Gross und J. Meienhofer), Band 1 und 2, Academic Press, London und New York, 1979/1980, oder M. Bodanszky, "Priciples of Peptide Synthesis", Springer-Verlag, Berlin 1984, beschrieben.

Die Kondensation kann in Gegenwart eines der üblichen Kondensationsmittel durchgeführt werden. Uebliche Kondensationsmittel sind z.B. Carbodiimide, beispielsweise Diäthyl-, Dipropyl-, N-Aethyl-N'-(3-dimethylaminopropyl)-carbodiimid oder insbesondere Dicyclohexylcarbodiimid, ferner geeignete Carbonylverbindungen, beispielsweise Carbonyldiimidazol, 1,2-Oxazoliumverbindungen, z.B. 2-Aethyl-5-phenyl-1,2-oxazolium-3'-sulfonat und 2-tert-Butyl-5-methylisoxazoliumperchlorat, oder eine geeignete Acylaminoverbindung, z.B. 2-Aethoxy-1-äthoxycarbonyl-1,2-dihydrochinolin, ferner aktivierte Phosphate, z.B. Diphenylphosphorylazid, Diäthylphosphorylcyanid oder Phenyl-N-phenylphosphoramidochloridat.

Gewünschtenfalls wird eine organische Base zugegeben, z.B. ein Triniederalkylamin mit voluminösen Resten, z.B. Aethyldiisopropylamin, oder eine heterocyclischen Base, z.B. Pyridin, 4-Dimethylaminopyridin oder bevorzugt N-Methylmorpholin.

Die Kondensation von Säureanhydriden mit Aminen kann z.B. in Gegenwart von anorganischen Carbonaten, z.B. Alkalimetallcarbonaten oder -hydrogencarbonaten, wie Natrium- oder Kaliumcarbonat oder -hydrogencarbonat (üblicherweise zusammen mit einem Sulfat), erfolgen.

Die Kondensation wird vorzugsweise in einem inerten, polaren, aprotischen, vorzugsweise wasserfreien, Lösungsmittel oder Lösungsmittelgemisch durchgeführt, beispielsweise in einem Carbonsäureamid, z.B. Formamid oder Dimethylformamid, einem halogenierten Kohlenwasserstoff, z.B. Methylenchlorid, Tetrachlorkohlenstoff oder Chlorbenzol, einem Keton, z.B. Aceton, cyclischen Aether, z.B. Tetrahydrofuran, einem Ester, z.B. Essigsäureäthylester, oder einem Nitril, z.B. Acetonitril, oder in Mischungen davon, gegebenenfalls bei erniedrigter oder erhöhter Temperatur, z.B. in einem Temperaturbereich von etwa -40 °C bis etwa +100 °C, bevorzugt von etwa -10 °C bis etwa +50 °C, und gegebenenfalls unter Inertgas-, z.B. Stickstoffatmosphäre.

Reaktionsfähige Säurederivate können auch in situ gebildet werden. So kann man z.B. N,N'-disubstituierte Amidinoester in situ bilden, indem man das Gemisch des Fragments mit freier Carboxygruppe und des komplementären Fragments mit einer Aminogruppe in Gegenwart eines geeigneten disubstituierten Carbodiimids, z.B. Dicyclohexylcarbodiimid, umsetzt. Ferner kann man Amino- oder Amidoester von solchen Säuren in Gegenwart der zu acylierenden Aminokomponente bilden, indem man das Gemisch der entsprechenden Säure- und Amino-Ausgangsstoffe in Gegenwart eines disubstituierten Carbodiimids, z.B. Dicyclohexylcarbodiimid, und eines N-Hydroxylamins oder N-Hydroxyamids, z.B. N-Hydroxybenztriazol, N-Hydroxysuccinimid oder N-Hydroxy-5-norbornen-2,3-dicarbonsäureimid, gegebenenfalls in Anwesenheit einer geeigneten Base, z.B. 4-Dimethylaminopyridin, N-Methylmorpholin oder Aethyldiisopropylamin, umsetzt.

Verfahren b) (Reduktion einer Ketogruppe):

In einem Ausgangsmaterial der Formel II sind funktionelle Gruppen mit Ausnahme der zu reduzierenden Ketogruppe gegebenenfalls durch eine der unter Verfahren a) genannten Schutzgruppen geschützt.

Zur Reduktion der Ketogruppe in einer Verbindung der Formel II eignen sich solche Reduktionsmittel, die unter den Reaktionsbedingungen des Verfahrens eine isolierte Ketogruppe selektiv oder schneller als die in Verbindungen der Formel I vorhandenen Amidgruppen reduzieren.

In erster Linie zu nennen sind geeignete Borhydride, wie Alkalimetallborhydride, insbesondere Natriumborhydrid, Lithiumborhydrid oder Natriumcyanborhydrid, oder geeignete Aluminiumhydride, wie Alkalimetallniederalkoxyaluminiumhydride mit voluminösen Resten, z.B. Lithium-tris-tert-butoxyaluminiumhydrid.

Die Reduktion kann auch mit Wasserstoff in Gegenwart geeigneter Schwermetallkatalysatoren, z.B. Raney-Nickel oder Platin- oder Palladiumkatalysatoren, z.B. Platin- oder Palladium-Aktivkohle, oder nach Meerwein-Ponndorf-Verley mit Hilfe von Aluminiumalkanolaten, bevorzugt Aluminium-2-propanolat oder -äthanolat, durchgeführt werden.

Die Reduktion kann vorzugsweise mit stöchiometrischen Mengen oder einem sinnvoll bemessenen Ueberschuss des Reduktionsmittels in einem inerten Lösungsmittel bei Temperaturen zwischen -80 °C und dem Siedepunkt des Lösungsmittels, vorzugsweise zwischen -20 °C und +100 °C, wenn nötig unter Schutzgas, z.B. Stickstoff oder Argon, durchgeführt werden. Ein Ueberschuss des Reduktionsmittels ist insbesondere dann nötig, wenn dieses auch mit dem Lösungsmittel, z.B. den Protonen eines protischen Lösungsmittels, reagiert.

Bei Verwendung von Natriumborhydrid eignen sich polare, protische Lösungsmittel, z.B. Methanol,

Aethanol oder Isopropanol; bei Verwendung der anderen Reduktionsmittel die unter Verfahren a) genannten polaren, aprotischen Lösungsmittel, z.B. Tetrahydrofuran.

Verfahren c) (Addition einer metallorganischen Verbindung):

In einem Ausgangsmaterial der Formel III sind funktionelle Gruppen mit Ausnahme der Aldehydgruppe gegebenenfalls durch die unter Verfahren a) genannten Schutzgruppen geschützt. Ebenso sind vorhandene funktionelle Gruppen in einer Verbindung der Formel IV geschützt.

In einer Verbindung der Formel IV ist ein Metallradikal -M z.B. -Li oder -MgHal, z.B. -MgCl, -MgBr oder -MgJ.

Die Umsetzung einer Verbindung der Formel III mit einer Verbindung der Formel IV erfolgt in üblicher Weise in einem wasserfreien, inerten, aprotischen Lösungsmittel, z.B. in einem Aether, wie Diäthyläther oder Tetrahydrofuran, oder einem Kohlenwasserstoff, wie Benzol oder Toluol, oder Gemischen davon, gegebenenfalls unter Kühlen, insbesondere nach Beginn der Reaktion, z.B. bis etwa -30 ° C, oder unter Erwärmen, z.B. bis zur Siedetemperatur des Reaktionsgemisches, gegebenenfalls in einer Inertgas-, z.B. Stickstoffatmosphäre. Eine bevorzugte Ausführungsform des Verfahrens ist die Umsetzung des Aldehyds der Formel III mit einem Ueberschuss der Lithiumverbindung der Formel IV.

Die Hydrolyse des Additionsprodukts erfolgt mit $H^+$-Ionen liefernden Lösungsmitteln, z.B. Wasser (Eis-Wasser-Gemisch) oder verdünnten, wässrigen Säuren, z.B. verdünnten Mineralsäuren, wie verdünnter, wässriger Schwefelsäure, oder verdünnten organischen Säuren, z.B. verdünnter, wässriger Essigsäure.

Die Umsetzung einer Verbindung der Formel III kann auch mit einer in situ hergestellten Verbindung der Formel IV erfolgen, welche man z.B. aus dem entsprechenden Halogenid, z.B. Chlorid, durch Umsetzung mit einem Metallierungsmittel, z.B. Magnesium, Lithium oder tert-Butyllithium, erhält.

Verfahren d) (nukleophile Substitution):

In einem Ausgangsmaterial der Formel V sind funktionelle Gruppen gegebenenfalls durch die unter Verfahren a) genannten Schutzgruppen geschützt.

In einer Verbindung der Formel V ist die nukleofuge Abgangsgruppe X insbesondere mit einer starken anorganischen oder organischen Säure verestertes Hydroxy, wie mit einer Mineralsäure, z.B. Halogenwasserstoffsäure, wie Chlor-, Brom- oder Jodwasserstoffsäure, ferner Schwefelsäure, oder Halogenschwefelsäure, z.B. Fluorschwefelsäure, oder mit einer starken organischen Sulfonsäure, wie einer gegebenenfalls, z.B. durch Halogen, wie Fluor, substituierten Niederalkansulfonsäure oder einer aromatischen Sulfonsäure, z.B. einer gegebenenfalls durch Niederalkyl, wie Methyl, Halogen, wie Brom und/oder Nitro substituierten Benzolsulfonsäure, z.B. einer Methansulfon-, Trifluormethansulfon- oder p-Toluolsulfonsäure, oder mit Stickstoffwasserstoffsäure verestertes Hydroxy.

Ein den Substituenten $R_4$ in nukleophiler Form einführendes Reagens ist eine Hydroxid-haltige Base, z.B. Natrium- oder Kaliumhydroxid.

Vorzugsweise werden die Reaktionsbedingungen so gewählt, dass die Reaktion im wesentlichen als nukleophile Substitution zweiter Ordnung ($S_N2$) abläuft. Die Reaktion mit einer Hydroxid-haltigen Base wird vorzugsweise in Wasser, dem gegebenenfalls als Lösungsvermittler ein organisches Lösungsmittel, z.B. Aethanol, Tetrahydrofuran oder Aceton, beigemischt ist. Die Substitutionsreaktion wird gegebenenfalls bei erniedrigter oder erhöhter Temperatur, z.B. in einem Temperaturbereich von etwa -40 ° C bis etwa +100 ° C, bevorzugt von etwa -10 ° C bis etwa +50 ° C, und gegebenenfalls unter Inertgas-, z.B. Stickstoffatmosphäre, durchgeführt.

Verfahren e) (Ueberführung einer Cyanogruppe in eine Amidgruppe):

In einem Ausgangsmaterial der Formel VI sind funktionelle Gruppen gegebenenfalls durch die unter a) genannten Schutzgruppen geschützt.

Die Ueberführung einer Verbindung der Formel VI in eine Verbindung der Formel I kann durch eine Ritter-Reaktion oder über Carbonsäureesterimid-Salze erfolgen.

Bei der Ritter-Reaktion setzt man die Nitrile in Gegenwart einer starken Säure, beispielsweise 85-90%iger Schwefelsäure, oder auch Polyphosphorsäure, Fluorwasserstoff, Ameisensäure, Bortrifluorid oder anderen Lewis-Säuren, nicht jedoch Aluminiumchlorid, mit Verbindungen um, die in dem sauren Medium Carbeniumionen bilden können, also z.B. mit Olefinen, wie Propylen, oder Alkoholen, wie Benzylalkohol, meist ohne Lösungsmittel oder beispielsweise in Eisessig.

In einer Variante der Ritter-Reaktion wird ein Nitril der Formel VI mit einem Olefin und Quecksilber(II)-nitrat umgesetzt und die Organoquecksilberverbindung anschliessend mit Natriumborhydrid zu einer N-substituierten Verbindung der Formel I reduziert.

Durch säurekatalysierte, bevorzugt Chlorwasserstoff-katalysierte, Anlagerung von Alkoholen an die Nitrile der Formel VI erhält man Carbonsäureesterimide, die durch thermische Umlagerung bei Temperaturen oberhalb von etwa 80 ° C Amide der Formel I ergeben.

Verfahren f) (Reduktion des Epoxids):

In einem Ausgangsmaterial der Formel VII sind funktionelle Gruppen gegebenenfalls durch die unter Verfahren a) genannten Schutzgruppen geschützt.

Es können solche Reduktionsmittel verwendet werden, die unter den Reaktionsbedingungen des Verfahrens die Epoxygruppe selektiv oder schneller als die vorhandenen Amidgruppen reduzieren und das Epoxid so öffnen, dass ein genügend und möglichst grosser Anteil der Reaktionsprodukte die neugebildete Hydroxygruppe in der der Formel I entsprechenden Position trägt. Beispiele für solche selektive Reduktionsmittel sind Lithiumborhydrid oder Natriumcyanborhydrid/Bortrifluoridätherat. Mit dem letztgenannten Reagens lässt sich die Reaktion z.B. so durchführen, dass man zu 1 Mol der Verbindung der Formel VII und einem Ueberschuss, z.B. 1,4-3 Mol, Natriumcyanborhydrid in Tetrahydrofuran bei erhöhter Temperatur, z.B. unter Rückfluss, eine Lösung von Bortrifluoridätherat, $BF_3 \cdot O(C_2H_5)_2$, in Tetrahydrofuran so zugibt, dass der pH-Wert der Reaktionslösung in der Nähe des Umschlagpunktes des ebenfalls zugegebenen Indikators Bromkresolgrün gehalten wird. Die Reduktion mit Lithiumborhydrid wird vorzugsweise in einem Aether, z.B. Tetrahydrofuran, 1,2-Dimethoxyäthan oder Diäthylenglykoldimethyläther, bei Temperaturen zwischen Raumtemperatur und Rückflusstemperatur durchgeführt.

Verfahren g) (Nachoperationen):

In einer erhältlichen Verbindung der Formel I, worin $R_1$, A, $R_2$, $R_3$, $R_4$, $R_5$ und $R_6$ die genannten Bedeutungen haben, kann man eine Carboxamidgruppe substituieren, eine in freier oder in reaktionsfähiger Form vorliegende Carboxygruppe verestern bzw. eine veresterte Carboxygruppe in eine Carboxy- oder eine Carboxamidgruppe überführen.

Die Substitution einer Carboxamidgruppe oder einer anderen Aminogruppe erfolgt z.B. durch Alkylierung.

Geeignete Mittel zur Alkylierung einer Carboxamidgruppe in einer Verbindung der Formel I sind z.B. Diazoverbindungen, z.B. Diazomethan. Man kann Diazomethan in einem inerten Lösungsmittel zersetzen, wobei das gebildete freie Methylen mit der Carboxamidgruppe in der Verbindung der Formel I reagiert. Die Zersetzung von Diazomethan erfolgt vorzugsweise katalytisch, z.B. in Gegenwart eines Edelmetalls in fein verteilter Form, z.B. Kupfer, oder eines Edelmetallsalzes, z.B. Kupfer(I)-chlorid oder Kupfer(II)-sulfat.

Weitere Alkylierungsmittel sind die in der Deutschen Offenlegungsschrift 2 331 133 genannte Alkylierungsmittel, z.B. Alkylhalogenide, Sulfosäureester, Meerweinsalze oder 1-substituierte 3-Aryltriazene. welche man unter den dort genannten Reaktionsbedingungen mit einer Verbindung der Formel I mit einer Carboxamidgruppe umsetzen kann.

Zur Veresterung einer Carboxygruppe in einer Verbindung der Formel I kann man die freie Säure verwenden oder die freie Säure in eines der unter Verfahren a) genannten reaktionsfähigen Derivate überführen und mit einem Alkohol umsetzen, oder man kann die freie Säure oder ein reaktionsfähiges Salz, z.B. das Cäsiumsalz, mit einem reaktionsfähigen Derivat eines Alkohols umsetzen. Beispielsweise kann man das Cäsiumsalz einer Carbonsäure mit dem Halogenid eines Alkohols umsetzen.

Die Veresterung einer Carboxygruppe kann mit den für die Substitution der Carboxamidgruppe oben genannten Alkylierungsmitteln und unter den gleichen Reaktionsbedingungen erfolgen, z.B. mit Diazomethan, Alkylhalogeniden, Sulfonsäureestern, Meerweinsalzen, 1-substituierten 3-Aryltriazenen etc.

Zur Ueberführung einer veresterten Carboxygruppe in einer Verbindung der Formel I in eine freie Carboxygruppe kann eine der unter Verfahren a), Abspaltung der Carboxyschutzgruppen, beschriebenen Methoden oder gewünschtenfalls eine alkalische Verseifung nach den im Organikum, 15. Auflage, VEB Deutscher Verlag der Wissenschaften, Berlin (Ost) 1976, genannten Reaktionsbedingungen angewendet werden.

In einer Verbindung der Formel I kann man eine veresterte Carboxygruppe durch Aminolyse mit Ammoniak oder einem primären oder sekundären Amin in eine gegebenenfalls substituierte Carboxamidgruppe überführen. Die Aminolyse kann nach den im Organikum, 15. Auflage, VEB Deutscher Verlag der Wissenschaften, Berlin (Ost) 1976, für solche Umsetzungen genannten Reaktionsbedingungen erfolgen.

In einer erhältlichen Verbindung der Formel I, worin die Substituenten die genannten Bedeutungen

haben und mindestens eine freie Hydroxygruppe vorhanden ist und die übrigen funktionellen Gruppen gegebenenfalls in geschützter Form vorliegen, kann man die freie Hydroxygruppe, z.B. die Hydroxygruppe $R_4$, veräthern oder verestern.

Die Verätherung dieser Hydroxygruppe kann mit den oben genannten Alkylierungsmitteln und unter den gleichen Reaktionsbedingungen erfolgen, z.B. mit Diazomethan, Alkylhalogeniden, Sulfonsäureestern, Meerweinsalzen, 1-substituierten 3-Aryltriazenen etc.

Die Veresterung der freien Hydroxygruppe kann mit den üblichen Acylierungsmitteln und den üblichen im Organikum angegebenen Reaktionsbedingungen erfolgen, z.B. mit Essigsäureanhydrid.

Die genannten Alkylierungsreaktionen, Verätherungen, Veresterungen etc. können statt im Endstoff auch in einem Ausgangsmaterial entsprechend durchgeführt werden.

In einer erhältlichen Verbindung der Formel I, worin eine oder mehrere funktionelle Gruppen geschützt sind, können diese Gruppen, z.B. Carboxy-, Amino- und/oder Hydroxygruppen, in an sich bekannter Weise, mittels Solvolyse, insbesondere Hydrolyse, gegebenenfalls enzymatische Hydrolyse, Alkoholyse oder Acidolyse, oder mittels Reduktion, insbesondere Hydrogenolyse, oder chemische Reduktion, gegebenenfalls stufenweise oder gleichzeitig, freigesetzt werden. Die Abspaltung der Schutzgruppen ist in den weiter vorn im Abschnitt "Schutzgruppen" genannten Standardwerken beschrieben.

Beispielsweise kann man geschütztes Carboxy, z.B. tert-Niederalkoxycarbonyl, in 2-Stellung durch eine organische Silylgruppe oder in 1-Stellung durch Niederalkoxy oder Niederalkylthio substituiertes Niederalkoxycarbonyl oder gegebenenfalls substituiertes Diphenylmethoxycarbonyl z.B. durch Behandeln mit einer geeigneten Säure, wie Ameisensäure oder Trifluoressigsäure, gegebenenfalls unter Zugabe einer nukleophilen Verbindung, wie Phenol oder Anisol, in freies Carboxy überführen. Gegebenenfalls substituiertes Benzyloxycarbonyl kann z.B. mittels Hydrogenolyse, d.h. durch Behandeln mit Wasserstoff in Gegenwart eines metallischen Hydrierkatalysators, wie eines Palladiumkatalysators, freigesetzt werden. Ferner kann man geeignet substituiertes Benzyloxycarbonyl, wie 4-Nitrobenzyloxycarbonyl, auch durch Reduktion, z.B. durch Behandeln mit einem Alkalimetall-, z.B. Natrium-dithionit, oder mit einem reduzierenden Metall, z.B. Zink, oder reduzierenden Metallsalz, wie einem Chrom-II-salz, z.B. Chrom-II-chlorid, üblicherweise in Gegenwart eines Wasserstoffabgebenden Mittels, das zusammen mit dem Metall nascierenden Wasserstoff zu erzeugen vermag, wie einer Säure, in erster Linie einer geeigneten Carbonsäure, wie einer gegebenenfalls, z.B. durch Hydroxy, substituierten Niederalkancarbonsäure, z.B. Essigsäure, Ameisensäure, Glycolsäure, Diphenylglycolsäure, Milchsäure, Mandelsaure, 4-Chlormandelsäure oder Weinsäure, oder eines Alkohols oder Thiols, wobei man vorzugsweise Wasser zugibt, in freies Carboxy überführen. Durch Behandeln mit einem reduzierenden Metall oder Metallsalz, wie oben beschrieben, kann man auch 2-Halogenniederalkoxycarbonyl (gegebenenfalls nach Umwandlung einer 2-Bromniederalkoxycarbonylgruppe in eine entsprechende 2-Jodniederalkoxycarbonylgruppe) oder Aroylmethoxycarbonyl in freies Carboxy umwandeln. Aroylmethoxycarbonyl kann ebenfalls durch Behandeln mit einem nukleophilen, vorzugsweise salzbildenden Reagens, wie Natriumthiophenolat oder Natriumjodid, gespalten werden. 2-Triniederalkylsilylniederalkoxycarbonyl kann auch durch Behandeln mit einem, das Fluoridanion liefernden Salz der Fluorwasserstoffsäure, wie einem Alkalimetallfluorid, z.B. Natrium- oder Kaliumfluorid, gegebenenfalls in Anwesenheit eines macrocyclischen Polyäthers ("Kronenäther"), oder mit einem Fluorid einer organischen quaternären Base, wie Tetraniederalkylammoniumfluorid oder Triniederalkylarylammoniumfluorid, z.B. Tetraäthylammoniumfluorid oder Tetrabutylammoniumfluorid, in Gegenwart eines aprotischen, polaren Lösungsmittels, wie Dimethylsulfoxid oder N,N-Dimethylacetamid, in freies Carboxy übergeführt werden. Mit einer organischen Silylgruppe, wie Triniederalkylsilyl, z.B. Trimethylsilyl, verestertes Carboxy kann in üblicher Weise solvolytisch, z.B. durch Behandeln mit Wasser, einem Alkohol oder Säure, oder ausserdem einem Fluorid, wie oben beschrieben, freigesetzt werden. Verestertes Carboxy kann auch enzymatisch gespalten werden, z.B. verestertes Arginin oder Lysin, wie Lysinmethylester, mittels Trypsin.

Eine geschützte Aminogruppe setzt man in an sich bekannter und je nach Art der Schutzgruppen in verschiedenartiger Weise, vorzugsweise mittels Solvolyse oder Reduktion, frei. 2-Halogenniederalkoxycarbonylamino (gegebenenfalls nach Umwandlung einer 2-Bromniederalkoxycarbonylaminogruppe in eine 2-Jodniederalkoxycarbonylaminogruppe), Aroylmethoxycarbonylamino oder 4-Nitrobenzyloxycarbonylamino kann z.B. durch Behandeln mit einem geeigneten Reduktionsmittel, wie Zink in Gegenwart einer geeigneten Carbonsäure, wie wässriger Essigsäure, gespalten werden. Aroylmethoxycarbonylamino kann auch durch Behandeln mit einem nukleophilen, vorzugsweise salzbildenden Reagenz, wie Natriumthiophenolat, und 4-Nitrobenzyloxycarbonylamino auch durch Behandeln mit einen Alkalimetall-, z.B. Natrium-dithionit, gespalten werden. Gegebenenfalls substituiertes Diphenylmethoxycarbonylamino, tert-Niederalkoxycarbonylamino oder 2-Triniederalkylsilylniederalkoxycarbonylamino kann durch Behandeln mit einer geeigneten Säure, z.B. Ameisen- oder Trifluoressigsäure, gegebenenfalls substituiertes Benzyloxycarbonylamino z.B. mittels Hydrogenolyse, d.h. durch Behandeln mit Wasserstoff in Gegenwart eines geeigneten Hydrierkatalysators, wie

eines Palladiumkatalysators, gegebenenfalls substituiertes Triarylmethylamino oder Formylamino z.B. durch Behandeln mit einer Säure, wie Mineralsäure, z.B. Chlorwasserstoffsäure, oder einer organischen Säure, z.B. Ameisen-, Essig- oder Trifluoressigsäure, gegebenenfalls in Gegenwart von Wasser, und eine mit einer organischen Silylgruppe geschützte Aminogruppe z.B. mittels Hydrolyse oder Alkoholyse freigesetzt werden. Eine durch 2-Halogenacetyl, z.B. 2-Chloracetyl, geschützte Aminogruppe kann durch Behandeln mit Thioharnstoff in Gegenwart einer Base, oder mit einem Thiolatsalz, wie einem Alkalimetallthiolat des Thioharnstoffs und anschliessende Solvolyse, wie Alkoholyse oder Hydrolyse, des entstandenen Kondensationsprodukts freigesetzt werden. Eine durch 2-Triniederalkylsilylniederalkoxycarbonyl geschützte Aminogruppe kann auch durch Behandeln mit einem Fluoridanionen liefernden Salz der Fluorwasserstoffsäure, wie oben im Zusammenhang mit der Freisetzung einer entsprechend geschützten Carboxygruppe angegeben, in die freie Aminogruppe überführt werden. Ebenso kann man direkt an ein Heteroatom, wie Stickstoff, gebundenes Silyl, wie Trimethylsilyl, mittels Fluoridionen abspalten.

In Form einer Azidogruppe geschütztes Amino wird z.B. durch Reduktion in freies Amino übergeführt, beispielsweise durch katalytische Hydrierung mit Wasserstoff in Gegenwart eines Hydrierkatalysators, wie Platinoxid, Palladium oder Raney-Nickel, oder auch durch Behandeln mit Zink in Gegenwart einer Säure, wie Essigsäure. Die katalytische Hydrierung wird vorzugsweise in einem inerten Lösungsmittel, wie einem halogenierten Kolenwasserstoff, z.B. Methylenchlorid, oder auch in Wasser oder einem Gemisch von Wasser und einem organischen Lösungsmittel, wie einem Alkohol oder Dioxan, bei etwa 20° C bis 25° C, oder auch unter Kühlen oder Erwärmen, durchgeführt.

Eine durch eine geeignete Acylgruppe, eine organische Silylgruppe oder durch gegebenenfalls substituiertes 1-Phenylniederalkyl geschützte Hydroxygruppe wird analog einer entsprechend geschützten Aminogruppe freigesetzt. Eine durch 2,2-Dichloracetyl geschützte Hydroxygruppe wird z.B. durch basische Hydrolyse, eine durch tert-Niederalkyl oder durch einen 2-oxa- oder 2-thia-aliphatischen oder -cycloaliphatischen Kohlenwasserstoffrest durch Acidolyse, z.B. durch Behandeln mit einer Mineralsäure oder einer starken Carbonsäure, z.B. Trifluoressigsäure, freigesetzt. Zwei Hydroxygruppen, die zusammen mittels einer vorzugsweise substituierten Methylengruppe, wie durch Niederalkyliden, z.B. Isopropyliden, Cycloalkyliden, z.B. Cyclohexyliden, oder Benzyliden, geschützt sind, können durch saure Solvolyse, besonders in Gegenwart einer Mineralsäure oder einer starken organischen Säure, freigesetzt werden.

Salze von Verbindungen der Formel I mit salzbildenden Gruppen können in an sich bekannter Weise hergestellt werden. So kann man Salze von Verbindungen der Formel I mit sauren Gruppen z.B. durch Behandeln mit Metallverbindungen, wie Alkalimetallsalzen von geeigneten organischen Carbonsäuren, z.B. dem Natriumsalz der 2-Aethylhexansäure, oder mit anorganischen Alkali- oder Erdalkalimetallsalzen, z.B. Natriumhydrogencarbonat, oder mit Ammoniak oder einem geeigneten organische Amin bilden, wobei man vorzugsweise stöchiometrische Mengen oder nur einen kleinen Ueberschuss des salzbildenden Mittels verwendet. Säureadditionssalze von Verbindungen der Formel I erhält man in üblicher Weise, z.B. durch Behandeln mit einer Säure oder einem geeigneten Anionenaustauscherreagenz. Innere Salze von Verbindungen der Formel I, welche z.B. eine freie Carboxygruppe und eine freie Aminogruppe enthalten, können z.B. durch Neutralisieren von Salzen, wie Säureadditionssalzen, auf den isoelektrischen Punkt, z.B. mit schwachen Basen, oder durch Behandeln mit Ionenaustauschern gebildet werden.

Salze können in üblicher Weise in die freien Verbindungen übergeführt werden: Metall- und Ammoniumsalze z.B. durch Behandeln mit geeigneten Säuren, Säureadditionssalze z.B. durch Behandeln mit einem geeigneten basischen Mittel.

Stereoisomerengemische, insbesondere Diastereomerengemische, können in an sich bekannter Weise, z.B. durch fraktionierte Kristallisation, Chromatographie etc., in die einzelnen Isomeren aufgetrennt werden.

Racemate können in an sich bekannter Weise, z.B. nach Ueberführung der optischen Antipoden in Diastereomere, beispielsweise durch Umsetzung mit optisch aktiven Säuren oder Basen, gespalten werden.

An einzelnen Chiralitätszentren in einer Verbindung der Formel I, z.B. dem CH-$R_4$-C-Atom, kann die Konfiguration gezielt umgekehrt werden. Beispielsweise kann man die Konfiguration am CH-$R_4$-Atom durch nukleophile Substitution zweiter Ordnung gemäss Verfahren d) nach Ueberführung der Gruppe $R_4$ in eine nucleofuge Abgangsgruppe X und Reaktion mit einem den gleichen Substituenten $R_4$ einführendes Reagens umkehren.

Die Erfindung betrifft auch diejenigen Ausführungsformen des Verfahrens, bei denen man von einer auf irgendeiner Stufe als Zwischenprodukt erhältlichen Verbindung ausgeht und die fehlenden Schritte durchführt oder man das Verfahren auf irgendeiner Stufe abbricht oder man eine nach dem erfindungsgemässen Verfahren erhältliche Verbindung unter den Verfahrensbedingungen erzeugt und in situ weiterverarbeitet.

Pharmazeutische Präparate:

Die pharmakologisch verwendbaren Verbindungen der vorliegenden Erfindung können z.B. zur Herstellung von pharmazeutischen Präparaten verwendet werden, welche eine wirksame Menge des Wirkstoffs zusammen oder im Gemisch mit einer signifikanten Menge von anorganischen oder organischen, festen oder flüssigen, pharmazeutisch verwendbaren Trägerstoffen enthalten.

Bei den erfindungsgemässen pharmazeutischen Präparaten handelt es sich um solche zur enteralen, wie nasalen, rektalen oder oralen, oder parenteralen, wie intramuskulären oder intravenösen, Verabreichung an Warmblüter (Menschen und Tiere), welche eine effektive Dosis des pharmakologischen Wirkstoffs allein oder zusammen mit einer signifikanten Menge eines pharmazeutisch anwendbaren Trägermateriale enthalten. Die Dosierung des Wirkstoffe hängt von der Warmblüter-Spezies, dem Körpergewicht, Alter und dem individuellen Zustand, der zu behandelnden Krankheit sowie von der Applikationsweise ab.

Die an Warmblüter, z.B. Menschen, von etwa 70 kg Körpergewicht, zu verabreichenden Dosismengen liegen zwischen etwa 3 mg und etwa 3 g, vorzugsweise zwischen etwa 10 mg und etwa 1 g, z.B. bei ungefähr 300 mg pro Person und Tag, verteilt auf vorzugsweise 1 bis 3 Einzeldosen, die z.B. gleich gross sein können. Ueblicherweise erhalten Kinder die halbe Dosis von Erwachsenen.

Die neuen pharmazeutischen Präparate enthalten von etwa 1 % bis etwa 95 %, vorzugsweise von etwa 20 % bis etwa 90 % des Wirkstoffes. Erfindungsgemässe pharmazeutische Präparate können z.B. in Dosiseinheitsform, wie Ampullen, Vials, Suppositorien, Dragées, Tabletten oder Kapseln, vorliegen.

Die pharmazeutischen Präparate der vorliegenden Erfindung werden in an sich bekannter Weise, z.B. mittels konventioneller Lösungs-, Lyophilisierungs-, Misch-, Granulier- oder Dragierverfahren, hergestellt.

Vorzugsweise verwendet man Lösungen des Wirkstoffe, daneben auch Suspensionen, und zwar insbesondere isotonische wässrige Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten und werden in an sich bekannter Weise, z.B. mittels konventioneller Lösungs- oder Lyophilisierungsverfahren, hergestellt. Die genannten Lösungen oder Suspensionen können viskositätserhöhende Stoffe, wie Natriumcarboxymethylcellulose, Carboxymethylcellulose, Dextran, Polyvinylpyrrolidon oder Gelatine, enthalten.

Suspensionen in Oel enthalten als ölige Komponente die für Injektionszwecke gebräuchlichen vegetabilen, synthetischen oder halbsynthetischen Oele. Als solche sind insbesondere flüssige Fettsäureester zu nennen, die als Säurekomponente eine langkettige Fettsäure mit 8-22, besonders 12-22, Kohlenstoffatomen, wie z.B. Laurinsäure, Tridecylsäure, Myristinsäure, Pentadecylsäure, Palmitinsäure, Margarinsäure, Stearinsäure, Arachinsäure, Behensäure oder entsprechende ungesättigte Säuren wie z.B. Oelsäure, Elaidinsäure, Erucasäure, Brasidinsäure oder Linolsäure, enthalten. Die Alkoholkomponente dieser Fettsäureester hat maximal 6 Kohlenstoffatome und ist ein ein- oder mehrwertiger, z.B. ein-, zwei- oder dreiwertiger Alkohol, z.B. Methanol, Aethanol, Propanol, Butanol oder Pentanol oder deren Isomere, vor allem aber Glycol oder Glyzerin. Als Fettsäureester sind daher beispielsweise zu nennen: Aethyloleat, Isopropylmyristat, Isopropylpalmitat, "Labrafil M 2735" (Polyoxyäthylenglyzerintrioleat der Firma Gattefossé, Paris), "Myglyol 812" (Triglyzerid gesättigter Fettsäuren der Kettenlänge $C_8$ bis $C_{12}$ der Firma Chemische Werke Witten/Ruhr, Deutschland), besonders aber vegetabile Oele wie Baumwollsaatöl, Mandelöl, Olivenöl, Ricinusöl, Sesamöl, Sojabohnenöl und vor allem Erdnussöl.

Die Herstellung der Injektionspräparate erfolgt in üblicher Weise unter sterilen Bedingungen, ebenso das Abfüllen in Ampullen oder Vialen sowie das Verschliessen der Behälter.

Pharmazeutische Präparate zur oralen Anwendung können erhalten werden, indem man den Wirkstoff mit festen Trägerstoffen kombiniert, ein erhaltenes Gemisch gegebenenfalls granuliert und das Gemisch bzw. Granulat, wenn erwünscht oder notwendig, nach Zugabe von geeigneten Hilfsstoffen, zu Tabletten oder Dragée-Kernen verarbeitet. Dabei kann man sie auch in Kunststoffträger einbauen, die die Wirkstoffe dosiert abgeben oder diffundieren lassen.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister unter Verwendung z.B. von Mais-, Weizen-, Reis- oder Kartoffelstärke, Gelatine, Traganth, Methylcellulose, Hydroxypropylmethylcellulose, Natriumcarboxymethylcellulose und/oder Polyvinylpyrrolidon, und/oder, wenn erwünscht, Sprengmittel, wie die obengenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyäthylenglykol. Dragée-Kerne werden mit geeigneten, gegebenenfalls magensaftresistenten Ueberzügen versehen, wobei man u.a. konzentrierte Zuckerlösungen, welche gegebenenfalls arabischen Gummi, Talk, Polyvinylpyrrolidon, Polyäthylenglykol und/oder Titandioxid enthalten, Lacklösungen in geeigneten organischen Lösungsmitteln oder Lösungsmittelgemischen oder, zur Herstellung von magensaftresistenten Ueberzügen, Lösungen von geeigneten Cellulosepräparaten, wie Aethylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Ausgangsmaterialien:

Die im folgenden genannten neuen Ausgangsmaterialien und/oder Zwischenprodukte, sowie Verfahren zu ihrer Herstellung, sind ebenfalls Gegenstand der vorliegenden Erfindung. Vorzugsweise werden solche Ausgangsstoffe verwendet und die Reaktionsbedingungen so gewählt, dass man zu den als besonders oder zuallererst betroffenen aufgeführten Verbindungen gelangt.

Die Ausgangsmaterialien zur Durchführung des Verfahrens a) können nach an sich bekannten Verfahren hergestellt werden, z.B. aus den betreffenden Aminosäuren durch Kondensation analog dem vorstehend beschriebenen Verfahren a).

Beispielsweise kann man eine Verbindung der Formel

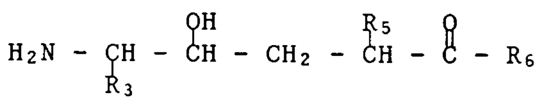

(VIII)

und Salze davon, worin $R_3$ Cyclohexylmethyl bedeutet und $R_5$ und $R_6$ die unter Formel I genannten Bedeutungen haben, herstellen, indem man eine Verbindung der Formel

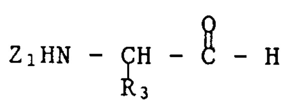

(IX),

worin $R_3$ die genannte Bedeutung hat und $Z_1$ eine Aminoschutzgruppe ist, mit einer Schwefel-Ylid-Verbindung in ein Epoxid der Formel

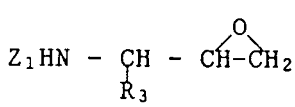

(X),

worin $R_3$ und $Z_1$ die genannten Bedeutungen haben, umwandelt, die erhältliche Verbindung (X) gegebenenfalls nach Trennung der Isomeren mit einem eine nukleofuge Abgangsgruppe X einführenden Reagens umsetzt, die erhältliche Verbindung der Formel

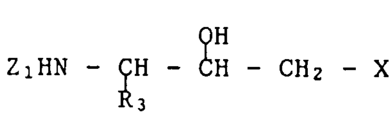

(XI),

worin $R_3$ und $Z_1$ die genannten Bedeutungen haben und X eine nukleofuge Abgangsgruppe ist, mit einer Carbonylverbindung der Formel

$R_a - (C = O) - R_b$ (XII),

worin jeder der Reste $R_a$ und $R_b$ unabhängig voneinander Wasserstoff, Niederalkyl, Aryl oder Arylniederalkyl, $R_a$ und $R_b$ zusammen gegebenenfalls überbrücktes Alkyliden mit 4 bis 12 Kohlenstoffatomen darstellen, oder einem reaktionsfähigen Derivat dieser Carbonylverbindung, in Gegenwart eines sauren Reagenses umsetzt, die erhältliche Verbindung der Formel

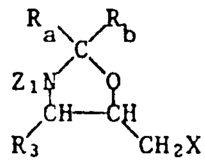 (XIII),

worin die Substituenten die genannten Bedeutungen haben, mit einem Carbonsäureestersalz der Formel

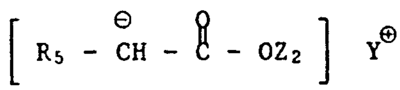 (XIV),

worin $R_5$ die unter Formel I genannte Bedeutung hat, $Z_2$ eine Carboxyschutzgruppe und $Y^{\oplus}$ ein Kation, z.B. ein Alkalimetall-, z.B. das Natrium- oder bevorzugt das Lithiumion, ist, umsetzt, und in einer erhältlichen Verbindung der Formel

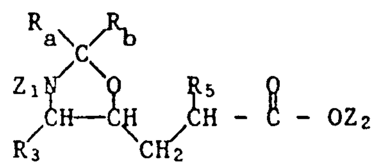 (XV),

worin die Sustituenten die genannten Bedeutungen haben, die Carboxyschutzgruppe $Z_2$ abspaltet und/oder ein erhältliches Isomerengemisch in die einzelnen Isomere auftrennt, die erhältliche Verbindung mit einer freien Carboxygruppe ($Z_2 = H$) mit einem Amin $R_6$-H amidiert, und in einer erhältlichen Verbindung der Formel

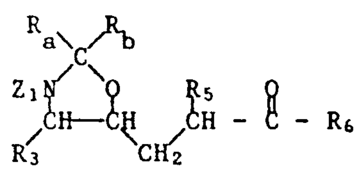 (XVI),

worin die Substituenten die genannten Bedeutungen haben, mit einem geeigneten Solvolysereagens den Ring öffnet und gegebenenfalls die Schutzgruppe $Z_1$ abspaltet.

Das Verfahren wird analog zu dem in der Europäischen Patentanmeldung 143 746 beschriebenen Verfahren durchgeführt.

Die gesamte Reaktionsfolge zur Herstellung der Zwischenprodukte der Formel VIII kann in situ oder nach Isolierung von einzelnen oder sämtlichen verfahrensgemäss erhältlichen Vorprodukten erfolgen.

Verbindungen der Formel II werden beispielsweise hergestellt, indem man eine Carbonsäure der Formel

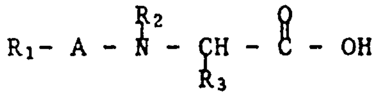 (XVII)

oder ein geeignetes funktionelles Derivat davon, worin die Substituenten die genannten Bedeutungen haben und freie funktionelle Gruppen mit Ausnahme der gegebenenfalls abgewandelten Carboxygruppe gegebenenfalls in geschützter Form vorliegen, mit einer Organometallverbindung der Formel IV, worin $R_5$ und $R_6$ die genannten Bedeutungen haben und M ein Metallradikal, z.B. -Li oder -MgHal, z.B. -MgCl, -MgBr oder -MgJ bedeutet, umsetzt und das gebildete Additionsprodukt solvolysiert.

Geeignete funktionelle Derivate von Carbonsäuren der Formel XVII sind beispielsweise das entsprechende Lithiumsalz der Carbonsäure, ein Carbonsäurehalogenid, z.B. Carbonsäurechlorid, ein Anhydrid, z.B. das symmetrische Carbonsäureanhydrid oder ein gemischtes Carbonsäureanhydrid mit einer sterisch gehinderten Carbonsäure, z.B. mit Pivalinsäure, oder ein Thioester, z.B. 2-Pyridylthioester.

Die Umsetzung einer Carbonsäure der Formel XVII oder eines geeigneten funktionellen Derivats davon mit einer Verbindung der Formel IV erfolgt in der üblichen Weise, z.B. nach den in Verfahren c) angegebenen Reaktionsbedingungen, gegebenenfalls jedoch unter Kühlung, z.B. bei Temperaturen von ca. -50 ° C bis ca. 0 ° C. In einer bevorzugten Ausführungsform des Verfahrens wird ein 2-Pyridylthioester der Carbonsäure der Formel XVII mit einer Brommagnesium-Verbindung der Formel IV umgesetzt.

Verbindungen der Formel III können nach an sich bekannten Verfahren hergestellt werden, beispielsweise indem man in einer Verbindung der Formel XVII, worin die Substituenten die genannten Bedeutungen haben und freie funktionelle Gruppen gegebenenfalls in geschützter Form vorliegen, die Carboxygruppe nach an sich bekannten Methoden, beispielsweise über den entsprechenden Methyl- oder Aethylester, über ein Imidazolid oder über ein N-Methoxy-N-methylamid, zur Aldehyd-Funktion reduziert.

Verbindungen der Formel IV können beispielsweise durch Umsetzung eines bekannten oder mit an sich bekannten Methoden herstellbaren Halogenids der Formel

$$Hal - CH_2 - \overset{R_5}{\overset{|}{CH}} - \overset{O}{\overset{\|}{C}} - R_6 \qquad (XVIII),$$

z.B. des Chlorids, mit einem Metallierungsmittel, z.B. Magnesium, Lithium oder tert-Butyllithium, hergestellt werden.

Verbindungen der Formel V werden beispielsweise hergestellt, indem man einen Aldehyd der Formel III mit einer Organometallverbindung der Formel IV gemäss Verfahren c) umsetzt und die gebildete Hydroxy-Verbindung der Formel I, gegebenenfalls nach Trennung der Isomeren, mit einer der Definition von X entsprechenden starken organische oder anorganischen Säure verestert, beispielsweise wie oben für die Herstellung der Verbindung der Formel XI aus dem entsprechenden Diol beschrieben.

Nitrile der Formeln VI werden beispielsweise hergestellt, indem man eine Verbindung der Formel

$$R_1 - A - \overset{R_2}{\overset{|}{N}} - \underset{R_3}{\underset{|}{CH}} - \overset{R_4}{\overset{|}{CH}} - CH_2 - \overset{R_5}{\overset{|}{CH}} - Hal \qquad (XIX),$$

worin die Substituenten die genannten Bedeutungen haben, mit einem Salz der Cyanwasserstoffsäure umsetzt.

Geeignete Salze der Cyanwasserstoffsäure sollten im gewählten inerten Lösungsmitel genügend löslich sein, damit eine Umsetzung erfolgen kann. Solche Salze sind z.B. Ammoniumcyanid, Alkalimetall- oder Erdalkalimetallcyanide, z.B. Natrium- oder Kaliumcyanid, oder Uebergangsmetallcyanide, z.B. Kupfercyanid. Letztere eignen sich aufgrund ihrer im Vergleich zu den Alkalimetallcyaniden geringeren Basizität.

Je nach der Art des eingesetzten Cyanids und des Lösungsmittels stellt sich ein Gleichgewicht zwischen der isomeren Nitril- und der Isonitrilform ein. Die Nitrilform wird bevorzugt gebildet, wenn z.B. die Umsetzung mit solchen Metallcyaniden erfolgt, deren Metallkationen ein niedrigeres Atomgewicht als das von Kupfer besitzen.

Geeignete inerte Lösungsmittel sind vor allem polare, aprotische Lösungsmittel, beispielsweise Carbonsäureamide, z.B. Dimethylformamid oder Dimethylacetamid, Nitrile, z.B. Acetonitril oder Propionitril, oder Diniederalkylsulfoxide, z.B. Dimethylsulfoxid.

Die Umsetzung erfolgt bei Raumtemperatur, bei erniedrigter oder bei erhöhter Temperatur, z.B. in einem Temperaturbereich von etwa -40 ° C bis etwa +100 ° C, bevorzugt von etwa -10 ° C bis etwa +50 ° C und gewünschtenfalls in einer Inertgas-, z.B. Stickstoffatmosphäre.

Epoxide der Formel VII werden z.B. hergestellt, indem man eine Verbindung der Formel IX mit einer Phosphoranylidenverbindung der Formel

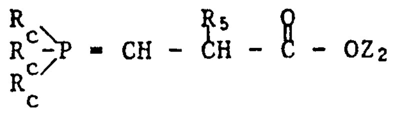 (XX),

worin $R_c$ einen gegebenenfalls substituierten Kohlenwasserstoffrest darstellt, $R_5$ die genannte Bedeutung hat und $Z_2$ eine Carboxyschutzgruppe ist, umsetzt und eine erhältliche Verbindung der Formel

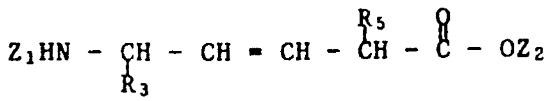 (XXI),

mit einem die Peroxogruppe enthaltenden Oxydationsmittel in ein Epoxid überführt und in einer erhältlichen Verbindung in beliebiger Reihenfolge der Reaktionsschritte die Schutzgruppen $Z_1$ und $Z_2$ abspaltet und durch die Gruppen $R_1$-A- und $R_6$ ersetzt.

$R_c$ ist bevorzugt Phenyl. Die Umsetzung einer Verbindung der Formel IX mit einer Phosphoranylidenverbindung der Formel XX erfolgt unter den für Wittig-Reaktionen bekannten und z.B. im Organikum beschriebenen Reaktionsbedingungen. Das dabei erhältliche Olefin der Formel XXI wird gegebenenfalls in situ mit dem Oxydationsmittel, z.B. Peressigsäure oder m-Chlorperbenzoesäure, umgesetzt. Die Abspaltung der Schutzgruppen $Z_1$ und $Z_2$ und die Einführung der Gruppen $R_1$-A- bzw. $R_6$ ist weiter vorn unter Verfahren a) beschrieben.

Die folgenden Beispiele dienen zur Illustration der Erfindung, schränken deren Umfang jedoch in keiner Weise ein.

Temperaturen werden in Celsiusgraden angegeben. Die $R_f$-Werte werden auf Kieselgeldünnschichtplatten in folgenden Lösungsmittelsystemen ermittelt:

| | | |
|---|---|---|
| N1 | Chloroform-Methanol | 9:1 |
| N2 | Chloroform-Methanol | 19:1 |
| N3 | Essigester-n-Hexan | 1:1 |
| N4 | Essigester-n-Hexan | 1:4 |
| N5 | Essigester-n-Hexan | 1:9 |
| N6 | Methylenchlorid-Aether | 4:1 |
| N7 | Methylenchlorid-Aether | 1:1 |
| N8 | Methylenchlorid-Methanol | 9:1 |

| | | |
|---|---|---|
| N9 | Methylenchlorid-Methanol | 6:1 |
| N10 | Methylenchlorid-Methanol | 20:1 |
| N11 | Methylenchlorid-Methanol | 10:1 |
| N12 | Methylenchlorid-Methanol | 5:1 |
| N13 | Essigester-n-Hexan | 4:1 |
| N14 | Essigester-n-Hexan | 2:1 |
| N15 | Essigester-n-Hexan | 1:2 |
| N16 | Methylenchlorid-Methanol-Wasser | 300:10:1 |
| N17 | Essigester-n-Hexan | 1:6 |
| N18 | Essigester-Methanol | 1:1 |
| N19 | Essigester-Methanol | 4:1 |
| N20 | Methylenchlorid-Methanol | 3:1 |
| N21 | Methylenchlorid-Methanol | 4:1 |
| N22 | Methylenchlorid-Methanol-Wasser | 40:10:1 |
| B1 | Chloroform-Methanol-Ammoniak konz. | 40:10:1 |
| B2 | Chloroform-Methanol-Ammoniak konz. | 350:50:1 |
| B3 | Methylenchlorid-Methanol-Ammoniak konz. | 1000:50:1 |
| B4 | Methylenchlorid-Methanol-Ammoniak konz. | 350:50:1 |
| B5 | Methylenchlorid-Methanol-Ammoniak konz. | 140:10:1 |
| B6 | Methylenchlorid-Methanol-Ammoniak konz. | 105:50:1 |
| B7 | Methylenchlorid-Methanol-Ammoniak konz. | 90:10:1 |
| B8 | Methylenchlorid-Methanol-Ammoniak konz. | 80:10:1 |
| B9 | Methylenchlorid-Methanol-Ammoniak konz. | 65:10:1 |
| B10 | Methylenchlorid-Methanol-Ammoniak konz. | 50:10:1 |
| B11 | Methylenchlorid-Methanol-Ammoniak konz. | 40:10:1 |
| B12 | Methylenchlorid-Methanol-Ammoniak konz. | 40:25:1 |
| B13 | Methylenchlorid-Methanol-Ammoniak konz. | 30:10:1 |
| B14 | Methylenchlorid-Methanol-Ammoniak konz. | 25:10:1 |
| B15 | Methylenchlorid-Methanol-Ammoniak konz. | 20:5:1 |
| B16 | Methylenchlorid-Methanol-Ammoniak konz. | 10:10:1 |
| B17 | Essigester-Pyridin-Eisessig-Wasser | 62:21:6:11 |
| B18 | n-Butanol-Pyridin-Eisessig-Wasser | 38:24:8:30 |
| B19 | Pyridin-n-Butanol-n-Amylalkohol-Methyläthylketon-Eisessig-Ameisensäure-Wasser | 25:20:15:10:3:3:25 |

| | | |
|---|---|---|
| B20 | n-Butanol-Pyridin-Ameisensäure-Wasser | 42:24:4:20 |
| B21 | n-Butanol-Pyridin-Eisessig-Wasser | 42:24:4:30 |
| B22 | n-Butanol-Pyridin-Ameisensäure-Wasser | 44:24:2:20 |
| B23 | Methylenchlorid-Methanol-Ammoniak konz. | 700:50:1 |
| B24 | Methylenchlorid-Methanol-Ammoniak konz. | 60:10:1 |
| B25 | Methylenchlorid-Methanol-Ammoniak konz. | 370:30:1 |
| B26 | Methylenchlorid-Methanol-Ammoniak konz. | 300:10:1 |
| B27 | Methylenchlorid-Methanol-Ammoniak konz. | 70:10:1 |
| B28 | Methylenchlorid-Methanol-Ammoniak konz. | 800:50:1 |
| B29 | Methylenchlorid-Methanol-Ammoniak konz. | 500:50:1 |
| B30 | Methylenchlorid-Methanol-Ammoniak konz. | 750:50:1 |
| B31 | Methylenchlorid-Methanol-Ammoniak konz. | 200:10:1 |
| B32 | Methylenchlorid-Methanol-Ammoniak konz. | 5:3:1 |
| B33 | Methylenchlorid-Methanol-Ammoniak konz. | 100:10:1 |
| S1 | Essigester-Pyridin-Eisessig-Wasser | 62:21:6:11 |
| S2 | Essigester-Methanol-Eisessig-Wasser | 67:10:12:23 |
| S3 | Chloroform-Methanol-Wasser-Eisessig | 300:108:20:2 |
| S4 | Chloroform-Methanol-Wasser-Eisessig | 150:54:10:1 |
| S5 | Chloroform-Methanol-Wasser-Eisessig | 140:80:20:1 |
| S6 | Chloroform-Methanol-Wasser-Eisessig | 180:20:2:1 |
| S7 | Chloroform-Methanol-Wasser-Eisessig | 110:94:26:1 |
| S8 | Chloroform-Methanol-Wasser-Eisessig | 80:20:3:3 |
| S9 | Chloroform-Methanol-Wasser-Eisessig | 70:30:3:3 |
| S10 | Methylenchlorid-Methanol-Wasser-Eisessig | 750:270:50:5 |
| S11 | n-Butanol-Eisessig-Wasser | 67:10:23 |
| S12 | Methylenchlorid-Methanol-Wasser-Eisessig | 160:30:3:9 |
| S13 | Essigester-n-Hexan-Eisessig | 40:60:1 |

Beispielsweise bedeutet die Abkürzung "$R_f$(N1)", dass der $R_f$-Wert im System N1 ermittelt wurde. Das Mengenverhältnis der Lösungsmittel zueinander ist in Volumenanteilen angegeben.

Die gleichen Abkürzungen werden für die Bezeichnung der Fliessmittel-Systeme bei der Flash-Chromatographie und der Mitteldruckchromatographie verwendet.

Abkürzungen für Aminosäuren und Aminosäurederivate:

| | |
|---|---|
| H-Ala-OH | L-Alanin |
| H-Arg-OH | L-Arginin |
| H-Cha-OH | L-Cyclohexylalanin |
| H-Gln-OH | L-Glutamin |
| H-Glu-OH | L-Glutaminsäure |
| H-Glu(OR)-OH | L-Glutaminsäure, worin die Carbonsäure-Funktion der Seitenkette mit dem Rest R verestert ist |
| H-Gly-OH | Glycin |
| H-His-OH | L-Histidin |

| | |
|---|---|
| H-(N-methyl-His)-OH | $N^{\alpha}$-Methyl-L-histidin |
| H-Ile-OH | L-Isoleucin |
| H-Leu-OH | L-Leucin |
| H-Lys-OH | L-Lysin |
| H-Lys(R)-OH | L-Lysin, worin die Amino-Funktion der Seitenkette mit dem Rest R substituiert ist |
| H-Nle-OH | L-Norleucin, (S)-$\alpha$-Aminohexansäure |
| H-Orn-OH | L-Ornithin, (S)-$\alpha$,$\delta$-Valeriansäure |
| H-Orn(R)-OH | L-Ornithin, worin die Amino-Funktion der Seitenkette mit dem Rest R substituiert ist. |
| H-Phe-OH | L-Phenylalanin |
| H-(N-methyl-Phe)-OH | N-Methyl-L-phenylalanin |
| H-(p-amino-Phe)-OH | L-p-Aminophenylalanin |
| H-Val-OH | L-Valin |

$-NH_2$ statt -OH: C-terminales (Carbonsäure)Amid
-OMe statt -OH: C-terminaler (Carbonsäure)Methylester
-NHMe statt -OH:C-terminales (Carbonsäure)Methylamid
Das Fragment mit der Bezeichnung

$$-Leu\overset{c}{-}Val-$$

bedeutet das zweiwertige Radikal von (2S,4S,5S)-5-Amino-2-isopropyl-4-hydroxy-7-methyloctansäure und hat die Formel

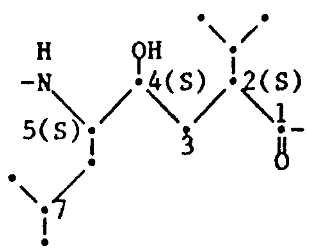

Das Fragment mit der Bezeichnung

$$-Leu\overset{cx}{-}Val-$$

leitet sich vom Fragment

$$-Leu\overset{c}{-}Val-$$

durch Ueberbrückung von NH und OH durch eine Isopropyliden-Gruppe ab und hat die Formel

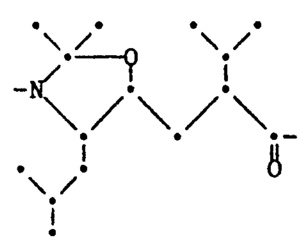

Das Fragment mit der Bezeichnung

$$-Cha\overset{c}{-}Val-$$

bedeutet das zweiwertige Radikal von (2S,4S,5S)-5-Amino-6-cyclohexyl-2-isopropyl-4-hydroxy-hexansäure und hat die Formel

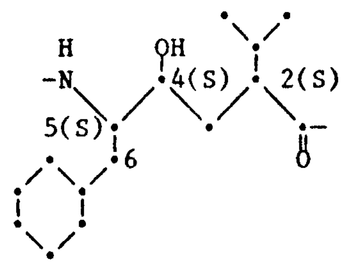

Das Fragment mit der Bezeichnung

$$-Cha\overset{cx}{—}Val-$$

leitet sich vom Fragment

$$-Cha\overset{c}{-}Val-$$

durch Ueberbrückung von NH und OH durch eine Ispropyliden-Gruppe ab.

Das Fragment mit der Bezeichnung

$$Gly(R_3)\overset{c}{-}Gly(R_5)$$

bedeutet das zweiwertige Radikal von 2-$R_5$-4(S)-hydroxy-5-amino-5-$R_3$-pentansäure mit (R)- oder (S)-Konfiguration an C-Atom 2 oder 5 und hat die Formel

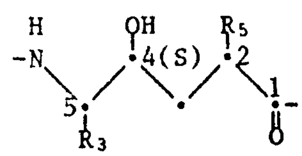

Das Fragment mit der Bezeichnung

$$-Gly(R_3)\overset{cx}{—}Gly(R_5)-$$

leitet sich vom Fragment

$$-Gly(R_3)\overset{c}{-}Gly(R_5)-$$

durch Ueberbrückung von NH und OH durch eine Isopropyliden-Gruppe ab.

<u>Weitere Abkürzungen:</u>

| | |
|---|---|
| abs. = | absolut (wasserfrei) |
| Ac = | Acetyl |
| BOC = | tert-Butoxycarbonyl |

| | |
|---|---|
| DCCI = | Dicyclohexylcarbodiimid |
| DCH = | Dicyclohexylharnstoff |
| DMF = | Dimethylformamid |
| DMSO = | Dimethylsulfoxid |
| HOBt = | 1-Hydroxybenzotriazol |
| Min. = | Minute(n) |
| Sdp. = | Siedepunkt |
| Smp. = | Schmelzpunkt |
| Std. = | Stunde(n) |
| THF = | Tetrahydrofuran |
| Z = | Benzyloxycarbonyl |

Beispiel 1:

**N-(Chinolyl-2-carbonyl)-Phe-Leu$\overset{C}{-}$Val-NHMe**

54 mg N-(Chinolyl-2-carbonyl)-L-phenylalanin, 41 mg

**H-Leu$\overset{C}{-}$Val-NHMe**

und 26 mg HOBt (Fluka purum, enthält 11-13 % Wasser) werden in 2,5 ml DMF auf 0 °C abgekühlt. Nach Zugabe von 45 mg DCCI wird während 15 Std. bei Eiskühlung und anschliessend während 2 Tagen bei Raumtemperatur gerührt. Der kristalline DCH wird abgenutscht und das Filtrat am Hochvakuum eingeengt. Der Rückstand wird in einem Gemisch von Methanol-Wasser-Eisessig (94:3:3) während 30 Min. bei 60 °C gerührt und anschliessend eingeengt. Der Rückstand wird mittels Flash-Chromatographie aufgetrennt (35 g Kieselgel 60, 40-63 µm, Fliessmittel-System N10). Die produkthaltigen Fraktionen werden eingedampft. Nach dem Trocknen im Hochvakuum wird die Titelverbindung als leicht gelbliches Pulver erhalten. $R_f$ (N10) = 0,33; $R_f$(N13) = 0,08.

Das Ausgangsmaterial

**H-Leu$\overset{C}{-}$Val-NHMe**

kann nach folgendem Reaktionsschema hergestellt werden:

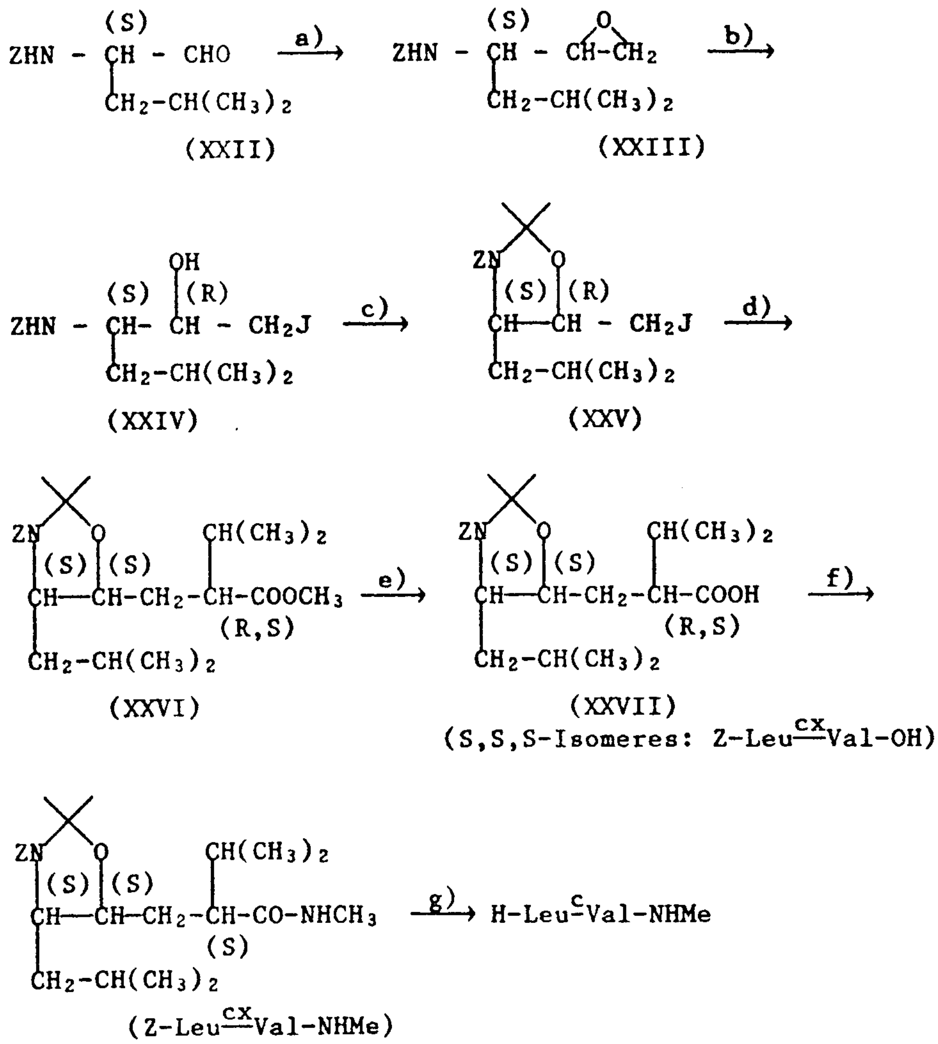


(XXII) (XXIII) (XXIV) (XXV) (XXVI) (XXVII)
(S,S,S-Isomeres: Z-Leu$^{cx}$Val-OH)
(Z-Leu$^{cx}$Val-NHMe)

a) 8,75 g Natriumhydriddispersion (55 % in Oel) werden in einem trockenen Sulfierkolben unter Argon durch dreimaliges Aufrühren in 85 ml Petroläther (Sdp. 40-60°) und anschliessendem Abdekantieren des Lösungsmittels vom Oel befreit. Nach Trocknen im Hochvakuum wird ein graues Pulver erhalten, das tropfenweise mit einer Lösung von 44,1 g Trimethylaulfoxoniumiodid in 180 ml Dimethylsulfoxid versetzt wird, wobei die Temperatur auf ca. 40° steigt. Die graue Suspension wird bei Raumtemperatur 1 Std. gerührt und anschliessend innerhalb von 50 Min. bei 10° mit einer Lösung von 43,4 g (S)-2-Benzyloxycarbonylamino-4-methyl-pentanal (XXII) (Herstellung: Helvetica Chimica Acta 66, 450 (1983)) in 180 ml DMSO versetzt. Die gelbe Suspension wird 15 Min. bei 10° und anschliessend 1,5 Std. bei Raumtemperatur gerührt. Die gelblich-trübe Lösung wird auf 500 g Eis gegossen. Die wässrige Lösung wird mit Aether extrahiert, die organische Phase mit Wasser gewaschen und nach Trocknen über Natriumsulfat eingedampft. Der ölige Rückstand wird mittels Flash-Chromatographie an 2 kg Kieselgel (40-63 µm) mit einem Gemisch von Methylenchlorid/n-Hexan/ Essigsäureäthylester (5:10:3) aufgetrennt. Die produkthaltigen Fraktionen werden vereinigt, eingedampft und im Hochvakuum getrocknet. Man erhält das Epoxid (XXIII) (Diastereomerengemisch, ca. 3:1) als leicht gelbliches Oel. $R_f$(B1) = 0,57; $R_f$-(N4) - 0,16.

b) 33,8 g der Verbindung (XXIII) werden in 255 ml Acetonitril aufgenommen und die erhaltene Lösung auf 0° abgekühlt. Nach Zugabe von 19,24 g Natriumjodid werden während 30 Min. tropfenweise bei 0° 16,27 ml Trimethylchlorsilan zugegeben. Das Gemisch wird 40 Min. bei 0-3° gerührt und anschliessend auf 250 ml eiskaltes Wasser gegossen. Das wässrige Gemisch wird mit Aether extrahiert und die organische Phase mit 10%iger wässriger Natriumthiosulfatlösung und gesättigter, wässriger Natriumchloridlösung gewaschen. Nach Trocknen über Natriumsulfat und Eindampfen erhält man ein öliges Gemisch des gewünschten Alkohols (XXIV) und des entsprechenden Trimethylsilyläthers (beides Diastereomerengemische). Der freie Alkohol wird mittels Flash-Chromatographie abgetrennt (2 kg Kieselgel 60,

40-63 µm, Laufmittel N 4). Nach Eindampfen der produkthaltigen, vereinigten Fraktionen erhält man den Alkohol (XXIV) als Oel. Nach Vereinigen und Eindampfen der Fraktionen, welche den Trimethylsilyläther enthalten, nimmt man den Rückstand in THF auf, versetzt mit einer Lösung von 2,67 g Tetrabutylammoniumfluorid in Wasser und rührt das so erhaltene Gemisch 3 Tage lang bei Raumtemperatur. Die Lösung wird anschliessend zwischen Wasser und Aether ausgeschüttelt. Die organische Phase wird mit Wasser gewaschen, über Natriumsulfat getrocknet und eingedampft. Nach Trocknen im Hochvakuum erhält man eine zusätzliche Menge des Diastereomerengemisches der Verbindung (XXIV) als Oel. Die gesamte Menge des Diastereomerengemisches (XXIV) wird zwecks weiterer Reinigung in zwei Teilen durch Mitteldruckchromatographie aufgetrennt (1,4 kg Lichroprep® Si 60, 25-40 µm, Laufmittel: Essigsäureäthylester/n-Hexan (1:8), Fraktionen von ca. 220 ml). Durch Kristallisation aus Petroläther (Sdp. 40-60°) lässt sich die stärker polare Komponente aus dem Rohmaterial und den Mischfraktionen teilweise anreichern (Smp. 104-106°). Die beiden Diastereomeren fallen in einem Verhältnis von ca. 3:1 an. Bei der weniger polaren, öligen Komponente handelt es sich um die gewünschte Verbindung (XXIV). $R_f$(N4) = 0,16; $R_f$(B1) = 0,54.

c) 66,6 g der Verbindung (XXIV) und 1,62 g p-Toluolsulfonsäuremonohydrat werden in 1500 ml 2,2-Dimethoxypropan 3 Std. lang unter Rückfluss erhitzt. Nach Abkühlen auf Raumtemperatur wird das Gemisch zwischen 1000 ml Aether und 500 ml gesättigter, wässriger Natriumhydrogencarbonatlösung ausgeschüttelt. Die organische Phase wird mit Wasser gewaschen, über Natriumsulfat getrocknet und eingedampft. Das halbkristalline Rohprodukt wird mittels Flash-Chromatographie gereinigt (2 kg Kieselgel 60, 40-63 µm, Laufmittel N 17, Fraktionen zu ca. 400 ml). Durch Eindampfen der vereinigten, produkthaltigen Fraktionen erhält man die Verbindung (XXV) als leicht gelbliche Kristalle. $R_f$(N4) = 0,48; $R_f$(N5) = 0,26.

d) 10,82 ml Diisopropylamin werden in 100 ml absolutem Tetrahydrofuran unter Argon gelöst und auf 0° abgekühlt. Anschliessend wird bei 0-5° das Gemisch 20 Min. lang tropfenweise mit einer 1,6 M Lösung von n-Butyllithium in Hexan versetzt und 15 Min. gerührt. Dann werden bei -70° bis -75° 10,1 ml Isovaleriansäuremethylester zugetropft und die Mischung 1,5 Std. bei -75° gerührt. Bei -60° bis -75° werden unter Rühren 190 ml Hexamethylphosphorsäuretriamid zugetropft. Die entstandene Suspension wird 10 Min. lang gerührt und schliesslich bei -70° bis -75° in 5 Min. tropfenweise mit einer Lösung von 30,0 g der Verbindung (XXV) in 50 ml Tetrahydrofuran versetzt. Das Reaktionsgemisch wird bei Raumtemperatur während 2,5 Std. gerührt und schliesslich auf ein Gemisch von 450 ml gesättigter, wässriger Ammoniumchloridlösung und 500 g Eis gegossen. Die wässrige Phase wird mit Aether extrahiert und die organische Phase mit Wasser gewaschen und über Natriumsulfat getrocknet. Nach Eindampfen erhält man das Diastereomerengemisch der Verbindung (XXVI) als gelbes Oel. $R_f$(N5) = 0,20; $R_f$(N4) = 0,40 (Werte für die weniger polare Komponente).

e) 10,7 g Kalium-tert-butylat werden in 80 ml Aether bei ca. 5° mit 1,07 ml Wasser versetzt. Die weisse Suspension wird noch 10 Min. im Eisbad gerührt und darauf mit 10,0 g der Verbindung (XXVI) (Diastereomerengemisch) in 80 ml Aether versetzt, wobei die Temperatur unterhalb 10° gehalten wird. Das Reaktionsgemisch wird nun während 18 Stunden bei Raumtemperatur gerührt und schliesslich auf 1600 ml gesättigte, wässrige Ammoniumchloridlösung gegossen. Die wässrige Phase wird mit Aether extrahiert und die organische Phase mit gesättigter, wässriger Natriumchloridlösung gewaschen, über Natriumsulfat getrocknet und eingedampft. Das ölige Rohprodukt wird durch Mitteldruckchromatographie aufgetrennt (740 g Lichroprep® Si 60, 25-40 µm, Laufmittel Essigsäureäthylester/n-Hexan 1:5).

Z-Leu$\overset{cx}{—}$Val-OH,

die weniger polare Komponente der Verbindung (XXVII) mit der gewünschten Konfiguration des an die Isopropylgruppe gebundenen C-Atoms (S-Konfiguration) wird als gelbes Oel erhalten. $R_f$ - (Methylenchlorid/Methanol/Wasser 500:10:1) = 0,13; $R_f$ (N6) = 0,58.

f)

Z-Leu$\overset{cx}{—}$Val-NHMe:

Eine Mischung aus 500 mg

Z-Leu$\overset{cx}{—}$Val-OH,

10 ml DMF, 245 mg HOBt und 330 mg DCCI wird 24 Std. bei 0° stehen gelassen. Die Mischung wird mit einem Ueberschuss an Methylamin versetzt und 2 Std. bei 0° und 2 Std. bei Raumtemperatur gerührt. Der kristallisierte DCH wird abfiltriert, das Filtrat eingeengt und am Hochvakuum getrocknet. Aus dem Rückstand wird durch Flash-Chromatographie (Fliessmittel-System N3) die Titelverbindung als farbloses Oel erhalte. $R_f$ (Methylenchlorid/Aether 2:1) = 0,45.

g)

H-Leu$\overset{c}{-}$Val-NHMe:

352 mg

Z-Leu$\overset{cx}{—}$Val-NHMe

werden in 30 ml Methanol-Wasser 9:1 in Gegenwart von 10 mg Palladium-Kohle (10% Pd) bei Normaldruck und Raumtemperatur bis zur Sättigung hydriert. Das Reaktionsgemisch wird filtriert und das Filtrat mit 75 ml Wasser bei Raumtemperatur gerührt. Nach dem Abdampfen des Lösungsmittels wird die Titelverbindung als farbloses Oel erhalten. $R_f$(B4) = 0,08; $R_f$(S4) = 0,27.

h) N-(Chinolyl-2-carbonyl)-L-phenylalanin: Zu einer Lösung von 0,50 g L-Phenylalanin in 3 ml 1N Natronlauge werden bei 0° während 10 Min. gleichzeitig 4,5 ml 1N Natronlauge und 0,78 g Chinolyl-2-carbonsäurechlorid (Chinaldinsäurechlorid, Smp. 91-94°, hergestellt nach der Vorschrift von D.L. Hammick & W.P. Dickinson, J.Chem.Soc. 1929, 214) in 10 ml Methylenchlorid zugetropft. Die Mischung wird 30 Min. bei Raumtemperatur gerührt, dann mit 1,5 ml 1N Salzsäure versetzt und mit Essigester extrahiert. Die Extrakte werden über Natriumsulfat getrocknet und eingeengt. Die Titelverbindung kristallisiert aus Methylenchlorid-Hexan-Gemisch als leicht rosa Pulver vom Smp. 160-162°. $R_f$(B11) = 0,25.

Beispiel 2:

N-(Chinolyl-2-carbonyl)-Phe-Leu$\overset{c}{-}$Val-7-tert-butoxycarbonyl-n-heptylamid

Analog Beispiel 1 wird die Titelverbindung ausgehend von 96 mg N-(Chinolyl-2-carbonyl)-L-phenylalanin (Beispiel 1h), 128 mg

H- Leu$\overset{c}{-}$Val-7-tert-butoxycarbonyl-n-heptylamid

in 5 ml DMF, 46 mg HOBt und 80 mg DCCI nach Reinigung durch Flash-Chromatographie (35 g Kieselgel 60, 40-63 µm, Fliessmittel System N3) erhalten. $R_f$(N10) = 0,31.

Die Ausgangsmaterialien werden folgendermassen hergestellt:

a)

H-Leu$\overset{c}{-}$Val-7-tert-butoxycarbonyl-heptylamid

wird durch Hydrierung von 213 mg

Z-Leu$\overset{cx}{—}$Val-7-tert-butoxycarbonyl-heptylamid

in Gegenwart von 40 mg Palladium-Kohle (10 %) analog Beispiel 1g erhalten. $R_f$(B2) = 0,13.

b)

**Z-Leu$\stackrel{cx}{-}$Val-7-tert-butoxycarbonyl-heptylamid**

wird analog Beispiel 1 ausgehend von 162 mg

**Z-Leu$\stackrel{cx}{-}$Val-OH**

und 103 mg 8-Amino-octansäure-tert-butylester nach Zugabe von 61 mg HOBt, 53 µl 4-Methylmorpholin und 107 mg DCCI erhalten. $R_f$(N4) = 0,20.

c) 8-Amino-octansäure-tert-butylester wird durch Hydrierung von 1,5 g 8-Benzyloxycarbonylamino-octansäure-tert-butylester in 15 ml Methanol nach Zugabe von 0,10 g Palladium-Kohle (10 %) analog Beispiel 1g als farbloses Oel erhalten. $R_f$(B3) = 0,08.

d) 8-Benzyloxycarbonylamino-octansäure-tert-butylester: In einem Autoklaven werden 2 g 8-Benzyloxycarbonylamino-octansäure in 12 ml Dioxan in Gegenwart von 1,2 ml Schwefelsäure mit 7,5 g Isobutylen umgesetzt und 48 Stunden bei Raumtemperatur stehengelassen. Die Reaktionsmischung wird mit Eis und 40 ml 1,8 N wässriger Ammoniaklösung versetzt. Das Reaktionsgemisch wird mit Aether extrahiert. Die organische Phase wird mit Wasser und gesättigter, wässriger Kochsalzlösung gewaschen, über Natriumsulfat getrocknet und im Vakuum eingeengt. Man erhält die Titelverbindung als gelbes, klares Oel. $R_f$(N8) = 0,44.

e) 8-Benzyloxycarbonylamino-octansäure: Zu 7 g 8-Amino-octansäure werden 25 ml 2H NaOH-Lösung gegeben. Anschliessend gibt man gleichzeitig bei ca. 0°-5° 30 Min. lang 17,1 g Chlorameisensäurebenzyl ester (50 % In Toluol) und 12,5 ml 4N NaOH-Lösung zu. Man beobachtet einen voluminösen weissen Niederschlag. Das Reaktionsgemisch wird mit 150 ml Aether und 60 ml Wasser versetzt. Die wässrige Phase wird mit Aether extrahiert, mit Eis versetzt und unter Rühren langsam mit verdünnter wässriger HCl-Lösung auf pH 2 gebracht. Die entstandene Suspension wird mit Essigester zweimal extrahiert. Man vereinigt die organischen Phasen, wäscht diese mit Wasser und gesättigter, wässriger Kochsalzlösung, trocknet über Natriumsulfat und dampft im Vakuum ein. Man erhält die Titelverbindung mit einem Schmelzpunkt von 63°-64°, $R_f$(N1) = 0,53.

Beispiel 3:

**N-(Chinolyl-2-carbonyl)-Phe-Leu$\stackrel{c}{-}$Val-7-carboxy-n-heptylamid**

124 mg

**N-(Chinolyl-2-carbonyl)-Phe-Leu$\stackrel{c}{-}$Val-7-tert-butoxycarbonyl-n-heptylamid**

(Beispiel 2) und 2,5 ml Trifluoressigsäure werden 10 Min. bei Raumtemperatur gerührt. Die Lösung wird eingeengt und der Rückstand durch Flash-Chromatographie (35 g Kieselgel 60, 40-63 µm, Fliessmittel-System B11) gereinigt. Die produkthaltigen Fraktionen werden eingedampft. Nach dem Trocknen bei 40° im Hochvakuum wird die Titelverbindung als amorphes Pulver erhalten. $R_f$(B11) = 0,30.

Beispiel 4:

**N-(Indolyl-2-carbonyl)-Nle-Leu$\stackrel{c}{-}$Val-n-butylamid**

56 mg Indol-2-carbonsäure, 140 mg

**H-Nle-Leu$\stackrel{c}{-}$Val-n-butylamid**

und 54 mg HOBt (Fluka purum, enthält 11-13 % Wasser) werden in 4 ml DMF auf 0 °C abgekühlt. Nach Zugabe von 95 mg DCCI wird während 24 Stunden bei Eiskühlung und anschliessend 2 Tage bei Raumtemperatur gerührt. Der kristalline DCH wird abgenutscht und das Filtrat am Hochvakuum eingeengt. Der Rückstand wird in 4 ml eines Gemisches aus Methanol-Wasser-Eisessig (94:3:3) während 30 Min. bei 60 °C gerührt und anschliessend eingeengt. Der Rückstand wird mittels Flash-Chromatographie aufgetrennt (100 g Kieselgel 60, 40-63 µm, Fliessmittel-System N 10). Die produkthaltigen Fraktionen werden eingedampft. Aus dem Rückstand wird durch Kristallisation aus Acetonitril die Titelverbindung als weisses Pulver erhalten. Smp. 243-244 °C; $R_f$(N 14) = 0,17.

Das Ausgangsmaterial wird folgendermassen hergestellt:

a)

H-Nle-Leu$\overset{c}{-}$Val-n-butylamid:

188 mg

Z-Nle-Leu$\overset{c}{-}$Val-n-butylamid

werden in 10 ml Methanol-Wasser 9:1 in Gegenwart von 30 mg Palladium-Kohle (10 % Pd) bei Normaldruck und Raumtemperatur bis zur Sättigung hydriert. Das Reaktionsgemisch wird filtriert. Nach dem Abdampfen des Lösungsmittels wird der Rückstand am Hochvakuum getrocknet und die Titelverbindung als farbloses Oel erhalten. $R_f$ (B4) = 0,41.

b)

Z-Nle-Leu$\overset{c}{-}$Val-n-butylamid:

Analog Beispiel 1 wird die Titelverbindung ausgehend von 138 mg Z-Nle-OH, 150 mg

H-Leu$\overset{c}{-}$Val-n-butylamid

in 6 ml DMF, 80 mg HOBt und 140 mg DCCI nach Reinigung durch Flash-Chromatographie (100 g Kieselgel 60, 40-63 µm, Fliessmittel N3) erhalten. $R_f$ (N3) = 0,15.

c)

H-Leu$\overset{c}{-}$Val-n-butylamid:

595 mg

Z-Leu$\overset{cx}{-}$Val-n-butylamid

werden in 40 ml Methanol-Wasser 9:1 in Gegenwart von 100 mg Palladium-Kohle (10 % Pd) bei Normaldruck und Raumtemperatur bis zur Sättigung hydriert. Das Reaktionsgemisch wird filtriert und das Filtrat mit 40 ml Wasser während 75 Min. bei Raumtemperatur gerührt. Nach dem Abdampfen des Lösungsmitteln wird die Titelverbindung als leicht gelblichen Oel erhalten. $R_f$(B11) = 0,51; $R_f$(54) = 0,42.

d)

Z-Leu$\overset{cx}{-}$Val-n-butylamid:

Eine Mischung aus 122 mg

Z-Leu$\overset{cx}{-}$Val-OH

(Beispiel 1e), 2 ml DMF, 60 mg HOBt und 80 mg DCCI wird 3 Tage bei 0°C stehen gelassen. Die Mischung wird unter Rühren mit 120 ml n-Butylamin versetzt und 2 Std. bei Eiskühlung und 24 Std. bei Raumtemperatur gerührt. Der kristalline DCH wird abgenutscht und das Filtrat am Hochvakuum eingeengt. Aus dem Rückstand wird durch Flash-Chromatographie (30 g Kieselgel 60, 40-63 µm, Fliessmittel N4) die Titelverbindung als leicht gelblichen Oel erhalten. $R_f$ (N3) = 0,52.

Beispiel 5: Analog Beispiel 4 werden hergestellt:

a)

N-(Indolyl-2-carbonyl)-Nle-Leu$\overset{c}{-}$Val-NHMe,

Flash-Chromatographie mit Fliessmittel N 10; $R_f$(B4) = 0,61: $R_f$(N8) = 0,46.
b)

N-(Indolyl-2-carbonyl)-Nle-Leu$\overset{c}{-}$Val-isopropylamid,

Flash-Chromatographie mit Fliessmittel N 14; $R_f$ (N 14) = 0,11; Smp. 241-243°.
c)

N-(Indolyl-2-carbonyl)-Nle-Leu$\overset{c}{-}$Val-n-propylamid,

Flash-Chromatographie mit Fliessmittel N 14; $R_f$ (N 14) = 0,14; Smp. 220-224°.
d)

N-(Indolyl-2-carbonyl)-Nle-Leu$\overset{c}{-}$Val-2-hydroxyäthylamid,

Flash-Chromatographie mit Fliessmittel N 10; $R_f$ (B 4) = 0,55; Smp. 241-242°.
e)

N-(Indolyl-2-carbonyl)-Nle-Leu$\overset{c}{-}$Val-benzylamid,

Flash-Chromatographie mit Fliessmittel N 3; $R_f$ (N 10) = 0,35; Smp. 231-234°.
f)

N-(Indolyl-2-carbonyl)-Nle-Leu$\overset{c}{-}$Val-2-pyridylmethylamid;

$R_f$ (B 5) = 0,23; $R_f$ (B7) = 0,46.
g)

N-(Indolyl-2-carbonyl)-Nle-Leu$\overset{c}{-}$Val-1-hydroxy-2(S)-butylamid;

$R_f$ (B 23) = 0,243.
h)

N-(Indolyl-2-carbonyl)-Nle-Leu$\overset{c}{-}$Val-äthylamid,

Flash-Chromatographie mit Fliessmittel N 14; $R_f$ (N 14) = 0,08; Smp. 236-238°.

i)

N-(Indolyl-2-carbonyl)-Nle-Leu$\overset{C}{-}$Val-2-(tert-butoxycarbonyl-amino)-äthylamid,

Flash-Chromatographie mit Fliessmittel N 10; $R_f$(N 10) = 0,20; Smp. 225-226°.

Beispiel 6:

N-(Indolyl-2-carbonyl)-Nle-Leu$\overset{C}{-}$Val-2-aminoäthylamid

30 mg

N-(Indolyl-2-carbonyl)-Nle-Leu$\overset{C}{-}$Val-tert-butoxycarbonylamino äthylamid

(Beispiel 5i) und 3 ml Trifluoressigsäure werden während 20 Min. bei Raumtemperatur gerührt. Die Reaktionslösung wird eingeengt und der Rückstand mittels Flash-Chromatographie (35 g Kieselgel 60, 40-63 µm, Fliessmittel-System B 8) aufgetrennt. Die produkthaltigen Fraktionen werden eingedampft. Aus dem Rückstand wird durch Lyophilisieren in 5 ml tert-Butanol die Titelverbindung als weisses Lyophilisat erhalten. $R_f$ (B 11) = 0,47.

Beispiel 7:

N-(Indolyl-2-carbonyl)-Phe-Leu$\overset{C}{-}$Val-2-(3-carbamoyl-4-hydroxyphenoxy)-äthylamid

25 mg 2-Indolcarbonsäure, 71 mg

H-Phe-Leu$\overset{C}{-}$Val-2-(3-carbamoyl-4-hydroxyphenoxy)-äthylamid,

23 mg HOBt und 40 mg DCCI werden analog Beispiel 1 umgesetzt. Nach Chromatographie im System B 7 erhält man die Titelverbindung als gelbliches Pulver. $R_f$ (B 7) = 0,27; $R_f$ (B 9) = 0,46. Die Ausgangsmaterialien werden folgendermassen hergestellt:

a)

H-Phe-Leu$\overset{C}{-}$Val-2-(3-carbamoyl-4-hydroxyphenoxy)-äthylamid

wird durch Hydrierung von 236 mg

Z-Phe-Leu$\overset{C}{-}$Val-2-(3-carbamoyl-4-hydroxyphenoxy)-äthylamid

in Gegenwart von 40 mg Palladium-Kohle (10 %) analog Beispiel 1 g erhalten. $R_f$ (N 11) = 0,23.

b)

Z-Phe-Leu$\overset{C}{-}$Val-2-(3-carbamoyl-4-hydroxyphenoxy)-äthylamid

wird analog Beispiel 1 ausgehend von 113 mg Z-L-Phenylalanin, 140 mg

H-Leu$\overset{c}{-}$Val-2-(3-carbamoyl-4-hydroxyphenoxy)-äthylamid

und 58 mg HOBt nach Zugabe von 99 mg DCCI erhalten. Das Produkt wird durch Flash-Chromatographie im System N 11 gereinigt. $R_f$ (N 11) = 0,39.

c)

H-Leu$\overset{c}{-}$Val-2-(3-carbamoyl-4-hydroxyphenoxy)-äthylamid

wird durch Hydrierung von 240 ml

Z-Leu$\overset{cx}{-}$Val-2-(3-carbamoyl-4-hydroxyphenoxy)-äthylamid

in Gegenwart von 40 mg Palladium-Kohle (10%) analog Beispiel 1 g erhalten. Reinigung durch Flash-Chromatographie im System B 11. $R_f$ (B 11) = 0,40; $R_f$ (B 7) = 0,15.

d)

Z-Leu$\overset{cx}{-}$Val-2-(3-carbamoyl-4-hydroxyphenoxy)-äthylamid

wird analog Beispiel 1 ausgehend von 147 mg

Z-Leu$\overset{cx}{-}$Val-OH

(Beispiel 1e), 107 mg 2-(3-Carbamoyl-4-hydroxyphenoxy)-äthylamin [J. Med. Chem. 27, 831 (1984)] und 61 mg HOBt nach Zugabe von 97 mg DCCI erhalten. Das Produkt wird duruch Flash-Chromatographie im System N 11 gereinigt. $R_f$ (N 11) = 0,38; $R_f$ (B 7) = 0,46.

Beispiel 8:

N-(Indolyl-2-carbonyl)-His-Leu$\overset{c}{-}$Val-n-butylamid

29 mg Indol-2-carbonsäure, 75 mg

H-His-Leu$\overset{c}{-}$Val-n-butylamid

und 27 mg HOBt (FLUKA purum, enthält 11-13 % Wasser) werden in 2 mi DMF auf 0 °C abgekühlt. Nach Zugabe von 48 mg DCCI wird während 24 Stunden bei Eiskühlung und anschliessend 2 Tage bei Raumtemperatur gerührt. Der kristalline DCH wird abgenutscht und das Filtrat am Hochvakuum eingeengt. Der Rückstand wird in 2 ml eines Gemisches aus Methanol-Wasser-Eisessig (94:3:3) während 60 Min. bei 60 °C gerührt und anschliessend eingeengt. Der Rückstand wird mittels Flash-Chromatographie aufgetrennt (100 g Kieselgel 60, 40-63 µm, Fliessmittel-System B23). Die produkthaltigen Fraktionen werden eingedampft. Aus dem Rückstand wird durch Lyophilisieren in 6 ml tert-Butanol die Titelverbindung als weisses Lyophilisat erhalten. $R_f$ (B4) = 0,40.

Das Ausgangsmaterial wird folgendermassen hergestellt:

a)

H-His-Leu$\overset{c}{-}$Val-n-butylamid:

98 mg

Z-His-Leu$^{C}$Val-n-butylamid

werden in 10 ml Methanol-Wasser 9:1 in Gegenwart von 20 mg Palladium-Kohle (10 % Pd) bei Normaldruck und Raumtemperatur bis zur Sättigung hydriert. Das Reaktionsgemisch wird filtriert. Nach dem Abdampfen des Lösungsmittels wird der Rückstand am Hockvakuum getrocknet und die Titelverbindung als leicht gelbliches Oel erhalten. $R_f$ (B 11) = 0,39.

b)

Z-His-Leu$^{C}$Val-n-butylamid:

Analog Beispiel 1 wird die Titelverbindung ausgehend von 72 mg Z-His-OH, 72 mg

H-Leu$^{C}$Val-n-butylamid

(Beispiel 4c) in 3 ml DMF, 38 mg HOBt und 68 mg DCCI nach Reinigung durch Flash-Chromatographie (100 g Kieselgel 60, 40-63 $\mu$m, Fliessmittel B 23) erhalten. $R_f$ (B 4) = 0,46.

Beispiel 9: Analog Beispiel 8 werden hergestellt:

a)

N-(Indolyl-2-carbonyl)-His-Leu$^{C}$Val-NHMe,

Flash-Chromatographie mit Fliessmittel B8; $R_f$(S4) = 0,60; $R_f$ (B4) = 0,27.

b)

N-(Indolyl-2-carbonyl)-His-Leu$^{C}$Val-benzylamid,

Flash-Chromatographie mit Fliessmittel B 23; $R_f$ (B 4) = 0,36.

c)

N-(Indolyl-2-carbonyl)-His-Leu$^{C}$Val-isoamylamid,

Flash-Chromatographie mit Fliessmittel B 23; $R_f$ (B 4) = 0,48.

d)

N-(Indolyl-2-carbonyl)-His-Leu$^{C}$Val-cyclopropylmethylamid,

Flash-Chromatographie mit Fliessmittel B 23; $R_f$ (B4) = 0,39.

Beispiel 10:

N-(N-Benzyl-indolyl-2-carbonyl)-His-Leu$^{C}$Val-NHMe

29 mg N-Benzyl-indol-2-carbonsäure, 40 mg

H-His-Leu$\overset{c}{-}$Val-NHMe,

18 mg HOBt und 28 mg DCCI werden in 1,5 ml DMF während 1,5 Tagen bei Raumtemperatur gerührt. Nach Abdampfen des Lösungsmittels im Hochvakuum wird der Rückstand in 4 ml eines Gemisches von Methanol-Wasser-Eisessig (94:3:3) bei 60° während 1 Std. verührt. Nach Eindampfen zur Trockne wird das Produkt nach Mitteldruckchromatographie (Lobar®-Fertigsäule Grösse B, 40-60 µm Korngrösse, Merck No. 10401, Laufmittel B8) als farbloses, amorphes Pulver erhalten. $R_f$ (B 24) = 0,4.

Die Ausgangsmaterialien werden folgendermassen hergestellt:

a) N-Benzyl-indol-2-carbonsäure wird durch Behandeln von 300 mg des entsprechenden Aethylesters (Herstellung siehe Synthesis 1984, 738) mit 2 ml 1N NaOH in 2 ml THF bei Raumtemperatur während 2 Tagen erhalten. Nach Zugabe von 2 ml 1N HCl wird zur Trockne eingedampft, zwischen Aether und Wasser ausgeschüttelt, die organische Phase getrocknet und das nach Eindampfen erhaltene Rohprodukt durch Mitteldruckchromatographie aufgetrennt (Lobar®-Säule Merck No. 10401, Laufmittel Essigester/n-Hexan 1:19). $R_f$(N4) = 0,03; $^1$H-NMR (TMS, DMSO-$d_6$): 11 ppm (1H), 7,7 (d,1H), 7,54 (d,1H), 7,34-7,0 (8H), 5,9 (s,2H).

b)

H-His-Leu$\overset{c}{-}$Val-NHMe

wird erhalten durch Hydrierung von 410 mg

Z-His-Leu$\overset{c}{-}$Val-NHMe

in Gegenwart von 50 mg Palladium-Kohle (10 %) in 20 ml Methanol-Wasser (95:5) unter Normaldruck und $CO_2$-Absorptionwährend 4 Std. bei Raumtemperatur. Nach Abfiltrieren des Katalysators und Eindampfen zur Trockne wird das Produkt nach Lyophilisation aus tert-Butanol als amorphes Pulver erhalten. $R_f$(B 24) = 0,09.

c)

Z-His-Leu$\overset{c}{-}$Val-NHMe:

326 mg Z-His-OH, 250 mg

H-Leu$\overset{c}{-}$Val-NHMe

und 172 mg HOBt werden in 7 ml DMF bei Eisbadtemperatur mit 274 mg DCCI versetzt. Es wird über Nacht bei 0° und anschliessend 24 Std. bei Raumtemperatur gerührt. Nach Abdampfen des Lösungsmittels wird der Rückstand in 5 ml eines Gemisches von Methanol-Wasser-Eisessig (94:3:3) bei 60° während 1 Stunde verrührt. Das Lösungsmittelgemisch wird im Vakuum abgedampft und das Produkt nach Mitteldruckchromatographie (1 Lobar®-Säule Grösse B, Merck No. 10401, Laufmittel B7) als amorphes Pulver erhalten. $R_f$ (B8) = 0,35.

Beispiel 11: Analog Beispiel 10 werden hergestellt:

a)

N-(2-[N-Benzyl-o,o'-dichloro-anilino]-phenylacetyl)-His-Leu$\overset{c}{-}$Val-NHMe,

$R_f$ (N 8) = 0,35.

b)

N-(Chinolyl-2-carbonyl)-His-Leu$\overset{c}{-}$Val-NHMe,

Flash-Chromatographie mit Fliessmittel B4; $R_f$ (B 4) = 0,31.
c)

N-Oxamoyl-His-Leu$\overset{c}{-}$Val-NHMe,

Flash-Chromatographie mit Fliessmittel B4; $R_f$ (B4) = 0,28; $R_f$ (S8) = 0,27.

Beispiel 12:

N-(3(R,S)-Benzyloxycarbonyl-4-α-naphthylbutyryl)-His-Leu$\overset{c}{-}$Val-NHMe

Ausgehend von 40,2 mg 2(R,S)-(α-Naphthylmethyl)-bernsteinsäure-1-benzylester und 44 mg

H-His-Leu$\overset{c}{-}$Val-NHMe

wird analog Beispiel 10 nach Flash-Chromatograhie (100 g Kieselgel 60, 40-63 µm, Fliessmittel-System B 25) die Titelverbindung als weisser Festkörper erhalten $R_f$ (B 4) = 0,30; $R_f$ (S12) = 0,40.

Das Ausgangsmaterial wird folgendermassen hergestellt:

a) 2(R,S)-(α-Naphthylmethyl)-bernsteinsäure-1-benzylester: Eine Lösung von 480,5 mg α-Naphthylmethyl-bernsteinsäureanhydrid (Herstellung siehe J. Chem. Soc. 1956, 355-358) und 216,3 mg Benzylalkohol in 5 ml Toluol wird 6 1/2 Std. gekocht und anschliessend eingedampft. Der Rückstand wird in 10 ml Diisopropyläther gelöst, mit 362 mg Dicyclohexylamin versetzt und über Nacht gerührt. Die ausgefallenen weissen Kristalle des Dicyclohexylammoniumsalzes werden abfiltriert und aus Diisopropyläther umkristallisiert, Smp. 120°-122°. Die Mutterlauge wird für die Herstellung des isomeren 4-Benzylesters (Beispiel 13a) eingesetzt. Das umkristallisierte Dicyclohexylammoniumsalz wird in 10 ml THF gelöst, mit 0,5 ml 1 N HCl angesäuert, filtriert und das Filtrat eingedampft, dann in 20 ml Toluol gelöst, filtriert und wieder eingedampft. Die Kristallisation des Rückstandes aus Cyclohexanon ergibt die Titelverbindung als weisse Nadeln, Smp. 122-124°; $R_f$ (S13) = 0,30.

Beispiel 13:

N-(3-Benzyloxycarbonyl-2(R,S)-α-naphthylmethyl-propionyl)-His-Leu$\overset{c}{-}$Val-NHMe

Ausgehend von 36 mg 2(R,S)-(α-Naphthylmethyl)-bernsteinsäure-4-benzylester und 38,5 mg

H-His-Leu$\overset{c}{-}$Val-NHMe

wird analog Beispiel 10 nach Flash-Chromatographie im System B 25 die Titelverbindung als weisser Festkörper erhalten. $R_f$ (B4) = 0,40; $R_f$ (S12) = 0,51.

Das Ausgangsmaterial wird folgendermassen hergestellt:

a) 2(R,S)-(α-Naphthylmethyl)-bernsteinsäure-4-benzylester: Die Mutterlauge des Dicyclohexylammoniumsalzes von Beispiel 12 a wird eingedampft, in 10 ml THF gelöst, mit 1,5 ml 1 N HCl angesäuert, filtriert und das Filtrat eingedampft. Der Rückstand wird in Aether gelöst, mit 1 N HCl und Sole gewaschen, über $Na_2SO_4$ getrocknet und eingedampft, wobei die Titelverbindung als farbloses Oel erhalten wird. $R_f$ (S 13) = 0,30.

Beispiel 14:

N-(Indolyl-2-carbonyl)-Leu-Leu$\overset{c}{-}$Val-NHMe

39 mg Indol-2-carbonsäure, 78 mg

H-Leu-Leu$\overset{c}{-}$Val-NHMe,

37 mg HOBt und 59 mg DCCI werden bei Raumtemperatur während 2 Tagen in 1,5 ml DMF gerührt. Nach Abdampfen des Lösungsmittels im Hochvakuum wird das Produkt durch Mitteldruckchromatographie (Lobar®-Säule Grösse B, Merck No. 10401, Laufmittel B 26) als amorphes Pulver gewonnen. $R_f$ (B 24) = 0,53.

Das Ausgangsmaterial wird folgendermassen hergestellt:

a)

H-Leu-Leu$\overset{c}{-}$Val-NHMe

wird erhalten durch Hydrierung von 115 mg

Z-Leu-Leu$\overset{c}{-}$Val-NHMe

in 10 ml Methanol-Wasser (95 : 5) in Gegenwart von 40 mg Palladium-Kohle (10 %) unter Normaldruck und $CO_2$-Absorption während 5 Stunden bei Raumtemperatur. Nach Abfiltrieren des Katalysators und Eindampfen zur Trockne wird das Produkt als amorphes Pulver erhalten. $R_f$ (B11) = 0,8.

b)

Z-Leu-Leu$\overset{c}{-}$Val-NHMe:

151 mg Z-Leu-OH (Dicyclohexylammonium-Salz),

75 mg H-Leu$\overset{c}{-}$Val-NHMe

und 52 mg HOBt werden bei Eisbadtemperatur in 3 ml DMF mit 82 mg DCCI versetzt. Er wird 8 Std. im Eisbad und 10 Std. bei Raumtemperatur gerührt. Nach Abdampfen des Lösungsmittels im Hochvakuum wird das Produkt nach Mitteldruckchromatographie (Lobar®-Säule, Merck No. 10401, Laufmittel B8) als amorphes Pulver gewonnen. $R_f$ (B 27) = 0,38.

Beispiel 15:

Analog Beispiel 14 werden hergestellt:

a) N-(Indolyl-2-carbonyl)-Cha-Leu$\overset{c}{-}$Val-NHMe, $R_f$ (B 24) = 0,56.

b) N-(Indolyl-2-carbonyl)-Gln-Leu$\overset{c}{-}$Val-NHMe, $R_f$ (B 9) = 0,23.

c) N-(N-[Indolyl-2-carbonyl]-L-threo-β-phenylseryl)-Leu$\overset{c}{-}$Val-NHMe, $R_f$(B 8) = 0,47.

Beispiel 16:

N-(Indolyl-2-carbonyl)-His-Cha$\overset{c}{-}$Val-NHMe

Eine Mischung aus 98,6 mg 37,7 mg Indol-2-carbonsäure, 35,8 mg HOBt,

H-His-Cha$\overset{c}{-}$Val-NHMe,

63,9 mg DCCI und 5,2 ml DMF wird 50 Std. bei Raumtemperatur gerührt. Der kristallisierte DCH wird abfiltriert und das Filtrat eingedampft. Das Rohprodukt wird mittels Flash-Chromatographie gereinigt (110 g Kieselgel 60, 40-63 µm, Laufmittel B4). Durch Eindampfen der vereinigten, produkthaltigen Fraktionen erhält man die Titelverbindung, $R_f$ (B 4) = 0,22.

Das Ausgangsmaterial wird folgendermassen hergestellt:

a)

H-His-Cha$\overset{c}{-}$Val-NHMe:

130 mg

Z-His-Cha$\overset{c}{-}$Val-NHMe

werden in 5 ml Methanol-Wasser 9:1 in Gegenwart von 20 mg Palladium-Kohle (10 % Pd) bei Normaldruck und Raumtemperatur bis zur Sättigung hydriert. Das Reaktionsgemisch wird filtriert und das Filtrat mit 5 ml Wasser bei Raumtemperatur gerührt. Nach dem Abdampfen des Lösungsmittels wird die Titelverbindung als farbloses Oel erhalten. $R_f$ (B 11) = 0,38.

b)

Z-His-Cha$\overset{c}{-}$Val-NHMe:

Eine Mischung aus 150,8 mg

H-Cha$\overset{c}{-}$Val-NHMe,

6 ml DMF, 81,1 mg HOBt, 153,3 mg Z-His-OH und 144,2 mg DCCI wird 48 Std. bei Raumtemperatur gerührt. Der DCH wird abfiltriert, das Filtrat eingeengt und am Hochvakuum getrocknet. Der Rückstand wird durch Flash-Chromatographie aufgetrennt (145 g Kieselgel 60, 40-63 µm, Laufmittel: B 23). Durch Eindampfen der vereinigten, produkthaltigen Fraktionen erhält man die Titelverbindung. $R_f$ (B 4) = 0,35; $R_f$ (S 4) = 0,65.

H-Cha$\overset{c}{-}$Val-NHMe

wird analog dem in Beispiel 1 gezeigten Schema folgendermassen hergestellt:

c) 2(S)-Benzyloxycarbonylamino-3-cyclohexyl-propionsäureäthylester: 243 g 2(S)-Benzyloxycarbonylamino-3-cyclohexyl-propionsäure (Herstellung: Helvetica Chimica Acta 57, 2131-(1974)) werden in 600 ml Toluol und 900 ml Aethanol vorgelegt. Das Reaktionsgemisch wird auf 0° gekühlt und 88,3 g Thionylchlorid innert 30 Min. zugetropft. Die Kühlung wird entfernt und die Mischung während 18 Std. gerührt. Das Reaktionsgemisch wird filtriert und das Filtrat eingeengt. Der Rückstand wird mittels Flash-Chromatographie (2 kg Kieselgel 60, 40-63 µm, Laufmittel N5) aufgetrennt. Die produkthaltigen Fraktionen werden vereinigt, eingedampft und im Hochvakuum getrocknet. Man erhält die Titelverbindung als leicht gelbliches Oel. $R_f$ (N 5) 0,2; $R_f$ (N15) 0,52.

d) 2(S)-Benzyloxycarbonylamino-3-cyclohexyl-propanal: 116,1 g 2(S)-Benzyloxycarbonylamino-3-cyclohexyl-propionsäureäthylester werden in 2,2 l Toluol vorgelegt und auf -65° abgekühlt. 836 ml Diisobutylaluminiumhydrid werden innert 30 Min. tropfenweise bei -65° zugegeben und das Gemisch 20 Min. nachgerührt. Dann werden bei -65° 84,2 ml Methanol innert 10 Min. zugetropft, anschliessend 825 ml wässrige Kalium-natrium-tartrat-Lösung ohne Kühlung. Das Reaktionsgemisch wird auf 3 l Kalium-natrium-tartrat-Lösung/Eis ausgetragen und mit 5 l Aether extrahiert. Die Aetherphase wird mit 2 l Wasser gewaschen, dann sofort in eine Lösung bestehend aus 106 g Semicarbazid-Hydrochlorid und 156,5 g Natriumacetat in 620 ml Wasser und 620 ml Aethanol gegossen. Das Reaktionsgemisch wird 1 Std. bei Raumtemperatur nachgerührt, dann im Scheidetrichter abgetrennt und die Wasserphase mit 2 x 1,5 l Aether extrahiert. Die organische Phase wird über Magnesiumsulfat getrocknet und eingedampft. Das Rohprodukt wird mittels Flash-Chromatographie gereinigt (2 kg Kieselgel 60, 40-63 µm, Laufmittel N3). Durch Eindampfen der vereinigten, produkthaltigen Fraktionen erhält man das Semicarbazon der Titelverbindung, $R_f$ (N8) = 0,51. 130 g dieses Semicarbazons werden in 1 l THF gelöst, mit 282 ml 37 % Formaldehydlösung und dann bei 10° mit 143 ml 0,5N HCl versetzt. Das Reaktionsgemisch wird 2 Std. bei Raumtemperatur gerührt, filtriert und das Filtrat mit 0,5 l Wasser, 0,5 l $NaHCO_3$ und 0,5 l Wasser gewaschen. Die Wasserphasen werden mit 600 ml Aether extrahiert. Die Aetherphasen werden über Magnesiumsulfat getrocknet und eingedampft. Der Rückstand wird mit 100 ml Toluol versetzt und eingedampft, wobei die Titelverbindung erhalten wird. Diese wird sofort weiterverarbeitet.

e) (1(S)-Benzyloxycarbonylamino-2-cyclohexyl-äthyl)-oxiran: 18,9 g Natriumhyddriddispersion (55 % in Oel) werden in einem trockenen Sulfierkolben unter Argon durch dreimaliges Aufrühren in 50 ml Petroläther (Sdp. 40-60°) und anschliessendes Abdekantieren des Lösungsmittels vom Oel befreit. Nach Trocknen im Hochvakuum wird ein graues Pulver erhalten, das in 500 ml THF vorgelegt und mit 55,6 g Trimethylsulfoxoniumiodid versetzt wird, wobei die Temperatur auf ca. 40° steigt. Die graue Suspension wird am Rückfluss 1 Std. gekocht und anschliessend innerhalb von 50 Min. bei -70° mit einer Lösung von 108,6 g 2(S)-Benzyloxycarbonylamino-3-cyclohexyl-propanal in 250 ml THF versetzt. Die gelbe Suspension wird 2 Std. bei 0° gerührt. Die gelblich-trübe Lösung wird auf 500 g Eis gegossen. Die wässrige Lösung wird mit 2,5 l Aether extrahiert, die organische Phase mit Wasser gewaschen und nach Trocknen über Natriumsulfat eingedampft. Der ölige Rückstand wird mittels Flash-Chromatographie (2,5 kg Kieselgel 60, 40-63 um, Laufmittel N4) aufgetrennt. Die produkthaltigen Fraktionen werden vereinigt, eingedampft und im Hochvakuum getrocknet. Man erhält die Titelverbindung (Diastereomerengemisch, ca. 4:1) als leicht gelbliches Oel. $R_f$(N 16) = 0,71; $R_f$(N4) = 0,16.

f) 3(S)-Benzyloxycarbonylamino-4-cyclohexyl-1-jod-butan-2(R,S)-ol: 42,3 g (1(S)-Benzyloxycarbonylamino-2-cyclohexyl-äthyl)-oxiranwerden in 200 ml Acetonitril aufgenommen und die erhaltene Lösung auf 0° abgekühlt. Nach Zugabe von 20,9 g Natriumjodid werden während 30 Min. tropfenweise bei 0° 17,7 ml Trimethylchlorsilan zugegeben. Das Gemisch wird 40 Min. bei 0-3° gerührt und anschliessend auf 700 ml eiskaltes Wasser gegossen. Das wässrige Gemisch wird mit Aether extrahiert und die organische Phase mit 750 ml 5 %-iger wässriger Natriumthiosulfatlösung und 750 ml gesättigter, wässriger Natriumchloridlösung gewaschen. Nach Trocknen über Natriumsulfat und Eindampfen erhält man ein öliges Gemisch der Titelverbindung, die direkt weiterverarbeitet wird.

g) 3-Benzyloxycarbonyl-4-(S)-cyclohexylmethyl-2,2-dimethyl-5(R)-jod-methyl-1,3-oxazolidin: 49,3 g der Verbindung Beispiel 16f) und 1,07 g p-Toluolsulfonsäuremonohydrat werden in 140 ml 2,2-Dimethoxypropan und 450 ml Methylenchlorid 3 Std. lang bei Raumtemperatur gerührt. Das Gemisch wird zwischen 1 l Methylenchlorid und 500 ml gesättigter, wässriger Natriumhydrogencarbonatlösung ausgeschüttelt. Die organsiche Phase wird mit Wasser gewaschen, über Natriumsulfat getrocknet und eingedampft. Das Rohprodukt wird mittels Flash-Chromatographie gereinigt (3 kg Kieselgel 60, 40-63 µm, Laufmittel N17). Durch Eindampfen der vereinigten, produkthaltigen Fraktionen erhält man die Titelverbindung als leicht gelbliches Oel. $R_f$ (N4) = 0,55; $R_f$ (N 17) = 0,46.

h) 2(R,S)-(3-Benzyloxycarbonyl-4-(S)-cyclohexylmethyl-2,2-dimethyl-1,3-oxazolidinyl-5-(S)-methyl)-3-methyl-buttersäuremethylester: 14,3 ml Diisopropylamin werden in 200 ml absolutem Tetrahydrofuran unter Argon gelöst und auf 0° abgekühlt. Anschliessend wird bei 0-5° das Gemisch 20 Min. lang tropfenweise mit 65,8 ml einer 1,6M Lösung von n-Butyllithium in Hexan versetzt und 20 Min. gerührt. Dann werden bei -70° bis -75° 13,3 ml Isovaleriansäuremethylester zugetropft und die Mischung 1,5 Std. bei -75° gerührt. Bei -60° bis -75° werden unter Rühren 320 ml Hexamethylphosphorsäuretriamid zugetropft. Die entstandene Suspension wird 10 Min. lang gerührt und schliesslich bei -70° bis -75° in 5 Min. tropfenweise mit einer Lösung von 43,4 g der Verbindung Beispiel 16g) in 110 ml Tetrahydrofuran versetzt. Das Reaktionsgemisch wird bei Raumtemperatur während 2,5 Std. gerührt und schliesslich auf ein Gemisch von 1 l gesättigter, wässriger Ammoniumchloridlösung und 500 g Eis gegossen. Die wässrige Phase wird mit 2 l Essigester extrahiert, die organische Phase mit Wasser gewaschen und über

Natriumsulfat getrocknet. Nach Eindampfen erhält man das Diastereomerengemisch der Titelverbindung als gelbes Oel. $R_f$ (N 4) = 0,36; $R_f$ (N5) = 0,21 (Werte für die weniger polare Komponente).

i) <u>2(R,S)-(3-Benzyloxycarbonyl-4(S)-cyclohexylmethyl-2,2-dimethyl-1,3-oxazolidinyl-5(S)-methyl)-3-methyl-buttersäure:</u> 16,5g Kalium-tert-butylat werden in 250 ml Aether bei ca. 5° mit 1,77 ml Wasser versetzt. Die weisse Suspension wird noch 10 Min. im Eisbad gerührt und darauf mit 35,8 g der Verbindung Beispiel 16h) (Diastereomerengemisch) in 250 ml Aether versetzt, wobei die Temperatur unterhalb 10° gehalten wird. Das Reaktionsgemisch wird nun während 18 Std. bei Raumtemperatur gerührt und schliesslich auf 500 ml gesättigte, wässrige Ammoniumchloridlösung gegossen. Die wässrige Phase wird mit Essigester extrahiert und die organische Phase mit gesättigter, wässriger Natriumchloridlösung gewaschen, über Natriumsulfat getrocknet und eingedampft. Das ölige Rohprodukt wird durch Flash-Chromatographie aufgetrennt (2,5 kg Kieselgel 60, 40-63 µm, Laufmittel N4).

Z-Cha$\overset{cx}{—}$Val-OH,

die weniger polare Komponente der Titelverbindung mit der gewünschten Konfiguration des an die Isopropylgruppe gebundenen C-Atoms (S-Konfiguration) wird als gelbes Oel erhalten. $R_f$ (N 16) = 0,20; $R_f$ (N 6) = 0,35.

j)

**Z-Cha$\overset{cx}{—}$Val-NHMe:**

Eine Mischung aus 311,9 mg

Z-Cha$\overset{cx}{—}$Val-OH,

6,4 ml DMF, 138,2 mg HOBt und 186,1 mg DCCI wird 24 Std. bei 0° stehen gelassen. Die Mischung wird mit einem Ueberschuss an Methylamin versetzt und 2 Std. bei 0° und 2 Std. bei Raumtemperatur gerührt. Der kristallisierte DCH wird abfiltriert, das Filtrat eingeengt und am Hochvakuum getrocknet. Aus dem Rückstand wird durch Flash-Chromatographie (Fliessmittel-System N3) die Titelverbindung als farbloses Oel erhalten. $R_f$ (N3) = 0,45.

k)

**H-Cha$\overset{c}{-}$Val-NHMe:**

243 mg

Z-Cha$\overset{cx}{—}$Val-NHMe

werden in 10 ml Methanol-Wasser 9:1 in Gegenwart von 50 mg Palladium-Kohle (10 % Pd) bei Normaldruck und Raumtemperatur bis zur Sättigung hydriert. Das Reaktionsgemisch wird filtriert und das Filtrat mit 10 ml Wasser bei Raumtemperatur gerührt. Nach dem Abdampfen des Lösungsmittels wird die Titelverbindung als farbloses Oel erhalten. $R_f$ (B 4) = 0,11; $R_f$ (S 4) = 0,31.

Beispiel 17:

**N-(2(R,S)-Acetoxy-3-α-naphthyl-propionyl)-His-Leu$\overset{c}{-}$Val-n-butylamid**

Eine Lösung von 21,4 mg 2(R,S)-Acetoxy-3-α-naphthyl-propionsäure, 35 mg

H-His-Leu$\overset{c}{-}$Val-n-butylamid

und 12,7 mg HOBt in 1,5 ml abs. DMF wird bei 0° unter Rühren mit 22,2 mg DCCI versetzt. Das Reaktionsgemisch wird 12 Std. bei 0° und 48 Std. bei Raumtemperatur weitergerührt und dann filtriert. Das Filtrat wird bei 40° eingedampft und der Rückstand in 2 ml eines Gemisches aus Methanol-Wasser-Eisessig (94:3:3) während 60 Min. bei 60° gehalten. Danach wird die Lösung am Rotationsverdampfer eingedampft, am Hochvakuum kurz getrocknet und der so erhaltene Rückstand auf Kieselgel mit Lösungsmittel-System B28 chromatographiert, wobei die Titelverbindung als weisse Kristalle anfällt. $R_f$ (B 4) = 0,415.

Das Ausgangsmaterial wird folgendermassen hergestellt:

a) 2(R,S)-Acetoxy-3-α-naphthyl-propionsäure: Ein Gemisch aus 0,21 g 2-Hydroxy-3-α-naphthyl-propionsäure und 0,2 ml Acetylchlorid wird bei Raumtemperatur 40 Min. gerührt und dann am Rotationsverdampfer unterhalb 50° eingedampft. Die zurückbleibende Titelverbindung wird über Pottasche bei 40° 16 Std. am Hochvakuum getrocknet. $R_f$ (N 18) = 0,63.

b) 2-Hydroxy-3-α-naphthyl-propionsäure: Eine Lösung von 1,4 g 2-Brom-3-α-naphthyl-propionsäure und 1,4 g Calciumcarbonat in 14 ml Wasser wird 10 Std. am Rückfluss gekocht, abgekühlt, mit 2 N Salzsäure auf pH 2 gestellt und mit Essigester extrahiert. Die organischen Phasen werden mit Natriumsulfat getrocknet und eingedampft. Aus dem Rückstand erhält man durch Chromatographie auf Kieselgel mit Fliessmittel N21 die kristalline Titelverbindung. $R_f$ (N 18) = 0,5.

c) 2-Brom-3-α-naphthyl-propionsäure: Eine Lösung von 10 g α-Naphthylamin in 100 ml Aceton wird unter Rühren bei Raumtemperatur tropfenweise mit 39,2 ml konz. HBr und anschliessend bei 0-5° mit 17,4 ml einer 4N wässrigen Lösung von Natriumnitrit versetzt. Anschliessend werden 70 ml Acrylsäure rasch zugetropft und 0,4 g Kupfer(I)bromid zugegeben. Das Reaktionsgemisch wird bei 60° mit ca. 30 g Natriumacetat auf pH 3 gestellt, noch 90 Min. bei 70° weitergerührt und dann am Rotationsverdampfer eingedampft. Der Rückstand wird in Essigester gelöst und viermal mit Natriumbicarbonatlösung extrahiert. Die Bicarbonatauszüge werden mit Salzsäure sauer gestellt und mit Essigester extrahiert. Die organischen Phasen werden mit Wasser und Sole gewaschen, über Natriumsulfat getrocknet und eingedampft. Die Chromatographie des Rückstandes mit Toluol-Essigester (9:1) auf Kieselgel ergibt die Titelverbindung als bräunliche Kristalle. $R_f$ (N19) = 0,25.

Beispiel 18:

N-[4-(2-Benzofuranyl)-2(R,S)-benzyl-4-oxo-butyryl]-His-Leu$\overset{c}{-}$Val-n-butylamid

Analog Beispiel 17 wird aus 50 mg 4-(2-Benzofuranyl)-2(R,S)-benzyl-4-oxo-buttersäure, 50 mg

H-His-Leu$\overset{c}{-}$Val-n-butylamid,

25 mg HOBt und 36 mg DCCI die Titelverbindung erhalten und durch Flash-Chromatographie mit Lösungsmittel-System B7 gereinigt. $R_f$ (B7) = 0,30.

Das Ausgangsmaterial wird folgendermassen hergestellt:

a) 4-(2-Benzofuranyl)-2(R,S)-benzyl-4-oxo-buttersäure: 2,0 g (Benzofuranyl-2-carbonylmethyl)-benzylmalonsäure-diäthylester werden in 10 ml Aethanol gelöst und nacheinander bei Raumtemperatur mit 10 ml Wasser und 7,4 ml 2N Natronlauge versetzt. Nach 16 Stunden Rühren bei Raumtemperatur wird mit 1N HCl auf pH 3 angesäuert und mit Methylenchlorid extrahiert. Die rohe Disäure mit Smp. 154° kristallisiert beim Einengen. Diese wird in einem Rundkolben während 10 Min. bei einer Temperatur von 170° decarboxyliert. Nach Abkühlen der Schmelze wird aus Essigester/Hexan kristallisiert, wobei die Titelverbindung vom Smp. 170-171° rein anfällt. $R_f$ (N12) = 0,63.

b) (Benzofuranyl-2-carbonylmethyl)-benzyl-malonsäure-diäthylester: 5,0 g Benzylmalonsäure-diäthylester werden bei Raumtemperatur zu einer Suspension von 870 mg Natriumhydrid in 25 ml DMF getropft. Nach 20 Min. werden 4,78 g Brommethyl-2-benzofuranyl-keton (hergestellt nach Liebigs Ann. Chem. 312, 332 (1900)) gelöst in 25 ml DMF zugetropft. Nach 2 Std. Rühren bei Raumtemperatur wird auf Eis

und 1N HCl gegossen und mit Methylenchlorid extrahiert. Das Rohprodukt wird durch Flash-Chromatographie an 260 g Kieselgel mit Methylenchlorid als Laufmittel gereinigt, wobei die Titelverbindung als Oel erhalten wird. $R_f$ ($CH_2Cl_2$) = 0,23.

Beispiel 19:

**N-(4-Cyano-2(R,S)-α-naphthylmethyl-butyryl)-His-Leu$\overset{C}{-}$Val-n-butylamid**

Analog Beispiel 17 wird aus 21,0 mg 4-Cyano-2(R,S)-α-naphthylmethyl-buttersäure, 35,0 mg

**H-His-Leu$\overset{C}{-}$Val-n-butylamid,**

12,7 mg HOBt und 22,2 mg DCCI die Titelverbindung erhalten und durch Flash-Chromatographie mit Lösungsmittel-System B3 gereinigt. $R_f$ (B4) = 0,46 (langsameres Diastomeres).

Das Ausgangsmaterial wird folgendermassen hergestellt:

a) 4-Cyano-2(R,S)-α-naphthylmethyl-buttersäure: Zu einer Suspension von 0,48 g Natriumhydriddispersion in 25 ml DMF werden 2,13 g 2-Cyanäthyl-malonsäure-diäthylester zugegeben. Das Reaktionsgemisch wird 2 Std. bei 80° gerührt und dann bei Raumtemperatur mit 1,77 g α-Chlormethylnaphthalin in 5 ml DMF versetzt. Das Gemisch wird 16 Std. bei 50° weitergerührt und dann eingedampft. Der Rückstand wird in Essigester gelöst, mit 0,1 N Salzsäure und Wasser gewaschen, über Natriumsulfat getrocknet und eingedampft, wobei 2-Cyanäthyl-α-naphthylmethyl-malonsäurediäthylester zurückbleibt.

7,2 g dieses Zwischenproduktes werden in 36 ml DMSO, 360 µl Wasser und 1,73 g Lithiumchlorid 3 Std. bei 180° gerührt. Das Reaktionsgemisch wird am Hochvakuum eingedampft, der Rückstand in Essigester gelöst, mit Wasser und Sole gewaschen, über Natriumsulfat getrocknet und eingedampft, wobei 4-Cyano-2(R,S)-α-naphthylmethylbuttersäure-äthylester erhalten wird.

Zur Verseifung werden 0,45 g dieses Esters in 7 ml Methanol und 1,8 ml 1N Natronlauge 16 Std. bei 35° gerührt. Danach wird das Reaktionsgemisch eingedampft, der Rückstand in Wasser gelöst, mit Essigester gewaschen, mit 2N Schwefelsäure angesäuert und mit Essigester extrahiert. Aus diesen Essigesterextrakten erhält man nach Waschen mit Wasser, Trocknen über Natriumsulfat und Eindampfen die Titelverbindung als gelbes Oel.

Beispiel 20:

**N-(2(R)- und 2(S)-Cyanmethyl-3-α-naphthyl-propionyl)-His-Leu$\overset{C}{-}$Val-n-butylamid**

Analog Beispiel 17 wird aus 19,9 mg 2(R,S)-Cyanmethyl-3-α-naphthyl-propionsäure, 35,0 mg

**H-His-Leu$\overset{C}{-}$Val-n-butylamid,**

12,7 mg HOBt und 22,2 mg DCCI die Titelverbindung als Diastereomerengemisch erhalten und durch Flash-Chlromatographie mit Lösungsmittel-System B28 getrennt. $R_f$ (B4) = 0,46 (schnelleres Diastereomeres); $R_f$ (B4) = 0,44 (langsameres Diastereomeres).

Das Ausgangsmaterial wird folgendermassen hergestellt.

a) 2(R,S)-Cyanmethyl-3-α-naphthyl-propionsäure: Zu einer gerührten Suspension von 0,44 g Natriumhydriddispersion in 50 ml DMF werden 3,0 g α-Naphthylmethyl-malonsäure-diäthylester und nach 30 Min. 0,6 ml Chloracetonitril zugegeben. Das Reaktionsgemisch wird 1 Std. bei 50° gerührt und dann am Hochvakuum eingedampft. Der Rückstand wird in Essigester gelöst, mit 0,1 N Salzsäure und Wasser gewaschen, über Natriumsulfat getrocknet, eingedampft und durch Chromatographie auf Silicagel mit

Toluol gereinigt, wobei Cyanmethyl-(α-naphthylmethyl)-malonsäure-diäthylester erhalten wird.

Analog Beispiel 19a) wird dieser Malonester hydrolysiert und decarboxyliert, der anfallende 2(R,S)-Cyanmethyl-3-α-naphthyl-propionsäure-äthylester durch Chromatographie mit Lösungsmittel-System N4 gereinigt und mit Natronlauge in Methanol verseift, wobei die Titelverbindung als gelbliches Oel erhalten wird.

Beispiel 21:

**N-Benzyloxycarbonyl-(p-benzyloxycarbonylamino-Phe)-His-Leu$\overset{C}{-}$Val-n-butylamid**

Analog Beispiel 17 wird aus 58 mg N-Benzyloxycarbonyl-p-benzyloxycarbonylamino-L-phenylalanin, 50 mg

**H-His-Leu$\overset{C}{-}$Val-n-butylamid,**

20 mg HOBt und 32 mg DCCI die Titelverbindung hergestellt und durch Flash-Chromatographie mit Lösungsmittel-System B31 gereinigt. $R_f$ (B8) = 0,51.

Das Ausgangsmaterial wird folgendermassen hergestellt:

a) N-Benzyloxycarbonyl-p-benzyloxycarbonylamino-L-phenylalanin: 1,01 g p-Amino-L-phenylalanin (Bachem AG, Bubendorf) wird als Natriumsalz in 6 ml $H_2O$ aufgenommen. Darauf werden bei Eisbadkühlung gleichzeitig 3,35 ml Chlorameisensäure-benzylester (50 % in Toluol) und 5 ml 2N NaOH zugetropft und während 20 Min. nachgerührt. Die weisse Suspension wird mit 2N HCl auf pH 1 gestellt, mit Essigester extrahiert und die organische Phase über Natriumsulfat getrocknet und eingedampft. Der Rückstand wird durch Flash-Chromatographie mit Lösungsmittel-System B11 gereinigt. Das Eluat wird mit 2N HCl angesäuert, mit Essigester extrahiert, die Extrakte getrocknet und eingedampft, wobei die Titelverbindung als weisse Kristalle erhalten wird. $R_f$ (B11) = 0,22.

Beispiel 22:

**N-Pivaloyl-(p-benzyloxycarbonylamino-Phe)-His-Leu$\overset{C}{-}$Val-n-butylamid**

Analog Beispiel 17 wird aus 52 mg N-Pivaloyl-p-benzyloxycarbonylamino-L-phenylalanin, 50 mg

**H-His-Leu$\overset{C}{-}$Val-n-butylamid,**

20 mg HOBt und 32 mg DCCI die Titelverbindung hergestellt und durch Flash-Chromatographie mit Lösungsmittel-System B31 gereinigt. $R_f$ (B8) = 0,41.

Das Ausgangsmaterial wird folgendermassen hergestellt:

a) N-Pivaloyl-p-benzyloxycarbonylamino-L-phenylalanin: 2,22 g N-Pivaloyl-p-amino-L-phenylalanin werden in 4 ml 2H NaOH und 5 ml $H_2O$ vorgelegt. Unter Eisbadkühlung werden gleichzeitig 1,3 ml Chlorameisensäure-benzylester und 2,1 ml 4N NaOH zugetropft. Die Suspension wird 30 Min. nachgerührt und dann mit konz. HCl auf pH 1 gestellt. Es wird mit Essigester extrahiert, mit Wasser gewaschen, getrocknet und das Lösungsmittel im Vakuum abgedampft, wobei beige Kristalle erhalten werden. $^1$H-NMR (DMSO-$d_6$, TMS): 1,0 ppm (s, 9H); 2,9-3,0 (dd, 2H); 4,4 (m, 1H); 5,15 (s, 2H); 5,72 (s, 2H); 7,1 (d, 1H); 7,3-7,5 (m, 5H); 14,6 (s, 1H).

b) N-Pivaloyl-p-amino-L-phenylalanin: 2,73 g N-Pivaloyl-p-nitro-L-phenylalanin in 50 ml Methanol werden in Gegenwart von 270 mg Palladium-Kohle (10 % Pd) 20 Min. bei Normaldruck und Raumtemperatur hydriert. Der Katalysator wird abfiltriert und das Filtrat zur Trockne eingedampft, wobei die Titelverbindung als rosarotes Pulver zurückbleibt. $^1$H-NMR (DMSO-$d_6$, TMS): 1,05 ppm (s, 9H); 2,8-2,95 (m, 2H); 4,2-4,5 (m, 1H); 6,45 (d, 2H); 6,9 (d, 2H); 7,25 (d, 1H).
c) N-pivaloyl-p-nitro-L-phenylalanin: 2,0 g p-Nitro-L-phenylalanin (Bachem AG, Bubendorf) werden in 5 ml 2N NaOH und 2 ml THF gelöst und bei 10-15° gleichzeitig mit 1,2 ml Pivaloylchlorid in 1,3 ml Toluol und 2,4 ml 4N NaOH versetzt. Es wird 1 Std. bei Raumtemperatur nachgerührt und dann mit konz. HCl auf pH 1 gestellt. Die wässrige Phase wird mit Essigester extrahiert und die organische Phase getrocknet und eingedampft, wobei die Titelverbindung als gelbliches Pulver erhalten wird. $R_f$ (N20) = 0,23.

Beispiel 23: Analog Beispiel 17 werden hergestellt:

a) N-(2(R)- und

**2(S)-Hydroxy-3-α-naphthyl-propionyl)-His-Leu$\overset{C}{-}$Val-n-butylamid,**

Trennung der Diastereomeren durch Flash-Chromatographie mit Lösungsmittel-System B30; $R_f$ (B4) = 0,43 (langsameres Diastereomeres) und $R_f$ (B4) = 0,49 (schnelleres Diastereomeres).
b)

**N-(3-α-Naphthyl-propionyl)-His-Leu$\overset{C}{-}$Val-n-butylamid,**

Flash-Chromatographie mit Fliessmittel B28; $R_f$ (B4) = 0,41.
c)

**N-(Chinolyl-2-carbonyl)-His-Leu$\overset{C}{-}$Val-n-butylamid,**

Mitteldruck-Chromatographie an Lobar®-Fertigsäule mit Fliessmittel B31; $R_f$ (B8) = 0,64.
d)

**N-(β-Naphthylcarbonyl)-His-Leu$\overset{C}{-}$Val-n-butylamid,**

Flash-Chromatographie mit Fliessmittel B31; $R_f$ (B8) = 0,60.
e)

**N-(α-Naphthoxyacetyl)-His-Leu$\overset{C}{-}$Val-n-butylamid,**

Flash-Chromatographie mit Fliessmittel B23; $R_f$ (B4) = 0,49.
f)

**N-(2(R,S)-Benzyl-5,5-dimethyl-4-oxo-hexanoyl)-His-Leu$\overset{C}{-}$Val-n- butylamind:**

$R_f$ (B7) = 0,33. Das Ausgangsmaterial ist in Beispiel 25a beschrieben.

Beispiel 24:

**N-(2(R,S)-Acetoxy-3-α-naphthyl-propionyl)-His-Cha$\overset{C}{-}$Val-n-butylamid**

Analog Beispiel 17 wird aus 30 mg 2(R,S)-Acetoxy-3-α-naphthyl-propionsäure, 54 mg

H-His-Cha$\overset{c}{-}$Val-n-butylamid,

17,8 mg HOBt und 31 mg DCCI die Titelverbindung erhalten und durch Flash-Chromatographie mit Lösungsmittel-System B28 gereinigt. $R_f$ (B4) = 0,5.

Das Ausgangsmaterial wird folgendermassen hergestellt:

a)

H-His-Cha$\overset{c}{-}$Val-n-butylamid

wird durch Hydrierung von 1,6 g

Z-His-Cha$\overset{c}{-}$Val-n-butylamid

in Gegenwart von 200 mg Palladium-Kohle (10 %) analog Beispiel 16a erhalten. $R_f$ (B4) = 0,05; $R_f$ (S4) = 0,16.

b)

Z-His-Cha$\overset{c}{-}$Val-n-butylamid

wird ausgehend von 1,75 g Z-His-OH, 1,97 g

H-Cha$\overset{c}{-}$Val-n-butylamid,

930 mg HOBt und 1,62 g DCCI analog Beispiel 16b erhalten und durch Flash-Chromatographie mit Lösungsmittel-System B4 gereinigt. Smp. 208-210°. $R_f$ (B4) = 0,49; $R_f$ (S4) = 0,62.

c)

H-Cha$\overset{c}{-}$Val-n-butylamid

wird durch Hydrierung von 4,2 g

Z-Cha$\overset{cx}{-}$Val-n-butylamid

in Gegenwart von 500 mg Palladium-Kohle (10 %) analog Beispiel 16k erhalten. $R_f$ (B4) = 0,25.

d)

Z-Cha$\overset{cx}{-}$Val-n-butylamid

wird ausgehend von 4,01 g

Z-Cha$\overset{cx}{-}$Val-OH,

2,68 g n-Butylamin, 1,80 g HOBt und 2,41 g DCCI analog Beispiel 16j erhalten und durch Flash-Chromatographie mit Lösungsmittel-System N4 gereinigt, $R_f$ (N3) = 0,61.

Beispiel 25:

N-(2(R,S)-, 2(R)- und 2(S)-Benzyl-5,5-dimethyl-4-oxo-hexanoyl)-His-Cha$\stackrel{C}{-}$Val-n-butylamid

Analog Beispiel 24 wird aus 40 mg 2(R,S)-, 2(R)- bzw. 2(S)-Benzyl-5,5-dimethyl-4-oxo-hexansäure, 50 mg

H-His-Cha$\stackrel{C}{-}$Val-n-butylamid,

25 mg HOBt und 36 mg DCCI die entsprechende Titelverbindung erhalten und durch Flash-Chromatographie mit Lösungsmittel-System B5 gereinigt. $R_f$ (B5) = 0,24; $R_f$ (B7) = 0,35 (beide Diastereomere).

Das Ausgangsmaterialien werden folgendermassen hergestellt:

a) 2(R,S)-Benzyl-5,5-dimethyl-4-oxo-hexansäure: Analog Beispiel 18a und 18b wird ausgehend von 5,0 g Benzylmalonsäure-diäthylester, 870 mg Natriumhydrid und 3,58 g Brommethyl-tert-butyl-keton in DMF die Titelverbindung erhalten. Smp. 94-95° $R_f$ (N11) = 0,56.

b) 2(R)- und 2(S)-Benzyl-5,5-dimethyl-4-oxo-hexansäure: 400 mg des Racemats aus Beispiel 25a, 370 mg 2(S)-Amino-3-phenylpropanol (L-Phenylalaninol), 272 mg HOBt und 500 mg DCCI werden analog Beispiel 1 umgesetzt. Die Diastereomeren werden durch Flash-Chromatographie mit Lösungsmittelsystem N3 getrennt. $R_f$ (N3) = 0,23 (schnelleres Diastereomeres) und $R_f$ (N3) = 0,17 (langsameres Diastereomeres). Durch Verkochen mit Essigsäure/2N HCl 1:1 während 10 Std. bei 80° wird der Phenylalaninol-Rest wieder abgespalten, und die Titelverbindungen werden als reine Enantiomere isoliert.

Beispiel 26:

N-(2(R,S)-Aethoxycarbonyl-3-α-naphthyl-propionyl)-His-Cha$\stackrel{C}{-}$Val-n-butylamid

Analog Beispiel 24 wird aus 42 mg α-Naphthylmethyl-malonsäure-monoäthylester, 60 mg

H-His-Cha$\stackrel{C}{-}$Val-n-butylamid,

26 mg HOBt und 40 mg DCCI die Titelverbindung erhalten und durch Flash-Chromatographie mit Lösungsmittel-System B5 gereinigt. $R_f$ (B5) = 0,22; $R_f$ (B7) = 0,36.

Das Ausgangsmaterial wird folgendermassen hergestellt:

a) α-Naphthylmethyl-malonsäure-monoäthylester: 5,00 g α-Naphthylmethyl-malonsäure-diäthylester (J.Am.Chem.Soc. 62, 2335 (1940)) gelöst in 50 ml Wasser/Aethanol 1:1 werden mit 16,5 ml 1N NaOH versetzt. Nach 30 Min. bei Raumtemperatur ist die Verseifung beendet. Das Reaktionsgemisch wird mit 16,5 ml 1N HCl versetzt und mit Methylenchlorid extrahiert. Die Extrakte werden eingedampft und der Rückstand durch Flash-Chromatographie mit Lösungsmittel-System N11 gereinigt. $R_f$ (N11) = 0,35; $R_f$ - (N7) = 0,25.

Beispiel 27:

N-(Cyclohepta[b]pyrrolyl-5-carbonyl)-His-Cha$\stackrel{C}{-}$Val-n-butylamid

Analog Beispiel 24 wird aus 37 mg Cyclohepta[b]pyrrol-5-carbonsäure, 60 mg

**H-His-Cha$\overset{C}{-}$Val-n-butylamid,**

32 mg HOBt und 48 mg DCCI die Titelverbindung erhalten und durch Flash-Chromatographie mit Lösungsmittel-System B7 gereinigt. $R_f$ (B7) = 0,35.

Das Ausgangsmaterial wird folgendermassen hergestellt:

a) Cyclohepta[b]pyrrol-5-carbonsäure: 260 mg Cyclohepta[b]pyrrol-5-carbonsäurenitril werden in 16 ml eines 1:1-Gemisches von Aethanol und 10N Natronlauge während 24 Std. mit Rückfluss gekocht. Danach wird mit konz. Salzsäure angesäuert, mit Methylenchlorid extrahiert und die Extrakte eingedampft. $R_f$ (B11) = 0,18; $R_f$ (N12) = 0,55.

b) Cyclohepta[b]pyrrol-5-carbonsäurenitril: 800 mg Cyclohepta[b]pyrrol (hergestellt nach Helv. Chim. Acta 67, 1647 (1984)) werden portionenweise bei 0° zu einer Lösung von 1,1 ml Oxalylchlorid in 60 ml DMF gegeben. Es wird noch 30 Min. bei Raumtemperatur nachgerührt, anschliessend mit 60 ml gesättigter Natriumacetat-Lösung versetzt und mit konz. Ammoniak auf pH 9 gestellt. Der gebildete Cyclohepta[b]pyrrol-5-carbaldehyd wird mit Methylenchlorid extrahiert und durch Flash-Chromatographie mit Lösungsmittel-System N6 gereinigt. $R_f$ (N6) = 0,46.

300 mg dieses Aldehyds werden mit 640 mg Hydroxylamin und 1,5 g Natriumacetat in 20 ml Methanol bei Raumtemperatur kräftig gerührt. Das Reaktionsgemisch wird auf Wasser gegossen und das gebildete Cyclohpeta[b]pyrrol-5-carbaldoxim mit Methylenchlorid extrahiert. $R_f$ (N6) = 0,35.

340 mg diesem Oxims werden in 20 ml Methylenchlorid bei Raumtemperatur mit 371 mg N,N'-Carbonyl-diimidazol versetzt. Nach 1 Std. wird das Reaktionsgemisch direkt auf 65 g Kieselgel aufgetragen und unter leichtem Ueberdruck die Titelverbindung mit Lösungsmittel-System B6 eluiert. $R_f$ (B6) = 0,70.

Beispiel 28:

**N-[2(R,S)-(2-Dimethylaminoäthyl-carbamoyl)-3-α-naphthyl-propionyl]-His-Cha$\overset{C}{-}$Val-n-butylamid**

Analog Beispiel 24 wird aus 49 mg 2(R,S)-(2-Dimethylaminoäthylcarbamoyl)-3-α-naphthyl-propionsäure, 60 mg

**H-His-Cha$\overset{C}{-}$Val-n-butylamid,**

26 mg HOBt und 40 mg DCCI die Titelverbindung erhalten und durch Flash-Chromatographie mit Lösungsmittel-System B9 gereinigt. $R_f$ (B9) = 0,27; $R_f$ (B7) = 0,13.

Das Ausgangsmaterial wird folgendermassen hergestellt:

a) 2(R,S)-(2-Dimethylaminoäthyl-carbamoyl)-3-α-naphthyl-propionsaure: Analog Beispiel 1 wird aus 1,00 g α-Naphthylmethyl-malonsäure-monoäthylester (Beispiel 26a), 0,80 ml 2-Dimethylaminoäthylamin, 0,67 g HOBt und 1,14 g DCCI 2(R,S)-(2-Dimethylaminoäthyl-carbamoyl)-3-α-naphthyl-propionsäureäthylester hergestellt. $R_f$ (B5) = 0,33; $R_f$(B7) = 0,46.

Der Aethylester wird analog Beispiel 26a mit Natronlauge in wässrigem Aethanol verseift. Die Titelverbindung kristallisiert aus Wasser bei 4° als dünne, farblose Kristalle vom Smp. 102°. $R_f$ (B11) = 0,08.

Beispiel 29:

**N-[2(R,S)-(2-Hydroxy-1(S)-methyl-äthylcarbamoyl)-3-α-naphthyl-propionyl]-His-Cha$\overset{C}{-}$Val-n-butylamid**

Analog Beispiel 24 wird aus 47 mg 2(R,S)-(2-Hydroxy-1(S)-methyl-äthylcarbamoyl)-3-α-naphthyl-propionsäure, 60 mg

H-His-Cha$\stackrel{c}{-}$Val-n-butylamid,

26 mg HOBt und 40 mg DCCI die Titelverbindung erhalten und durch Flash-Chromatographie mit Lösungsmittel-System B7 gereinigt. $R_f$ (B7) = 0,22; $R_f$ (B9) = 0,31.

Das Ausgangsmaterial wird folgendermassen hergestellt:

a) 2(R,S)-(2-Hydroxy-1(S)-methyl-äthylcarbamoyl)-3-α-naphthyl-propionsäure wird ausgehend von 2,00 g α-Naphthylmethyl-malonsäure-monoäthylester (Beispiel 26a), 0,69 g 2(S)-Amino-1-propanol, 1,46 g HOBt und 2,27 g DCCI analog Beispiel 28a hergestellt. $R_f$ (B11) = 0,18; $R_f$ (S10) = 0,52.

Beispiel 30:

N-[2(R,S)-(2,2-Dimethoxy-äthylcarbamoyl)-3-α-naphthyl-propionyl]-His-Cha$\stackrel{c}{-}$Val-n-butylamid

Analog Beispiel 24 wird aus 51 mg 2(R,S)-(2,2-Dimethoxy-äthylcarbamoyl)-3-α-naphthyl-propionsäure, 60 mg

H-His-Cha$\stackrel{c}{-}$Val-n-butylamid,

26 mg HOBt und 40 mg DCCI die Titelverbindung erhalten und durch Flash-Chromatographie mit Lösungsmittel-System B7 gereinigt. $R_f$ (B7) = 0,35; $R_f$ (B5) = 0,20.

Das Ausgangsmaterial wird folgendermassen hergestellt:

a) 2(R,S)-(2,2-Dimethoxy-äthylcarbamoyl)-3-α-naphthyl-propionsäure wird ausgehend von 1,00 g α-Naphthylmethyl-malonsäure-monoäthylester (Beispiel 26a), 0,48 ml Aminoacetaldehyd-dimethylacetal, 0,73 g HOBt und 1,14 g DCCI analog Beispiel 28a hergestellt. $R_f$ (B11) = 0,28; $R_f$ (S10) = 0,65.

Beispiel 31:

N-[2(R,S)-(tert-Butoxycarbonyl-methylcarbamoyl)-3-α-naphthyl-propionyl]-His-Cha$\stackrel{c}{-}$Val-n-butylamid

Analog Beispiel 24 wird aus 92 mg 2(R,S)-(tert-Butoxycarbonyl-methylcarbamoyl)-3-α-naphthyl-propionsäure, 100 mg

H-His-Cha$\stackrel{c}{-}$Val-n-butylamid,

43 mg HOBt und 67 mg DCCI die Titelverbindung hergestellt und durch Flash-Chromatographie mit Lösungsmittel-System B5 gereinigt. $R_f$ (B5) = 0,21; $R_f$ (B7) = 0,34.

Das Ausgangsmaterial wird folgendermassen hergestellt:

a) 2(R,S)-(tert-Butoxycarbonyl-methylcarbamoyl)-3-α-naphthyl-propionsäure wird ausgehend von 1,00 g α-Naphthylmethyl-malonsäure-monoäthylester (Beispiel 26a), 1,73 g Glycin-tert-butylesterdibenzolsulfimidsalz, 0,73 g HOBt und 1,14 g DCCI analog Beispiel 28a hergestellt und durch Flash-Chromatographie mit Lösungsmittel-System N12 gereinigt. $R_f$ (N12) = 0,38.

Beispiel 32:

N-[2(R,S)-Carboxymethylcarbamoyl-3-α-naphthyl-propionyl]-His-Cha$\overset{C}{-}$Val-n-butylamid

90 mg

N-[2(R,S)-(tert-Butoxycarbonyl-methylcarbamoyl)-3-α-naphthyl-propionyl]-His-Cha$\overset{C}{-}$Val-n-butylamid

(Beispiel 31) werden in 0,5 ml Trifluoressigsäure gelöst unnd 1 Std. bei Raumtemperatur stehen gelassen. Anschliessend wird zur Trockne eingedampft und das Produkt durch Flash-Chromatographie mit Lösungsmittel-System B11 gereinigt. $R_f$ (B11) = 0,34; $R_f$ (B9) = 0,21.

Beispiel 33:

N-(5,5-Dimethyl-2(R,S)-α-naphthylmethyl-4-oxo-hexanoyl)-His-Cha$\overset{C}{-}$Val-n-butylamid

Analog Beispiel 24 wird aus 46 mg 5,5-Dimethyl-2(R,S)-(α-naphthylmethyl)-4-oxo-hexansäure, 60 mg

H-His-Cha$\overset{C}{-}$Val-n-butylamid,

26 mg HOBt und 40 mg DCCI die Titelverbindung erhalten und durch Flash-Chromatographie mit Lösungsmittel-System B5 gereinigt. $R_f$ (B5) = 0,21; $R_f$ (B7) = 0,37.

Das Ausgangsmaterial wird folgendermassen hergestellt:

a) 5,5-Dimethyl-2(R,S)-α-naphthylmethyl-4-oxo-hexansäure: Analog Beispiel 18a und 18b wird ausgehend von 5,0 g α-Naphthylmethyl-malonsäure-diäthylester (J.Am.Chem.Soc. 62, 2335 (1940)), 730 mg Natriumhydrid und 2,9 g Brommethyl-tert-butyl-keton in DMF und anschliessender Verseifung und Decarboxylierung die Titelverbindung erhalten. Smp. 93-93,5° $R_f$ (N11) = 0,50.

Beispiel 34:

N-(Aethoxycarbonyl-α-naphthoxy-acetyl)-His-Cha$\overset{C}{-}$Val-n-butylamid

Analog Beispiel 24 wird aus 29,6 mg α-Naphthoxy-malonsäure-monoäthylester, 50,0 mg

H-His-Cha$\overset{C}{-}$Val-n-butylamid,

16,5 mg HOBt und 28,8 mg DCCI die Titelverbindung hergestellt und durch Flash-Chromatographie mit Lösungsmittel-System B3 gereinigt. $R_f$ (B4) = 0,54.

Das Ausgangsmaterial wird folgendermassen hergestellt:

a) α-Naphthoxy-malonsäure-monoäthylester: 2,0 g α-Naphthoxy-malonsäure-diäthylester werden in 5 ml absolutem Aethanol gelöst und unter Rühren mit der Lösung von 0,41 g Kaliumhydroxid in 5 ml abs. Aethanol versetzt. Das Reaktionsgemisch wird 16 Std. bei Raumtemperatur gerührt und dann zur Trockne eingedampft. Der Rückstand wird in Wasser gelöst, mit Aether gewaschen, mit 5N Salzsäure angesäuert und mit Essigester extrahiert. Aus diesen Essigesterextrakten erhält man nach Waschen mit

Sole, Trocknen über Natriumsulfat und Eindampfen die Titelverbindung als hellbraunes Oel.

Beispiel 35:

N-(4-Cyano-2(R,S)-α-naphthoxy-butyryl)-His-Cha$\overset{C}{-}$Val-n-butylamid

Analog Beispiel 24 wird aus 27,6 mg 4-Cyano-2(R,S)-α-naphthoxy-buttersäure, 50,0 mg

H-His-Cha$\overset{C}{-}$Val-n-butylamid,

16,5 mg HOBt und 28,8 mg DCCI die Titelverbindung hergestellt und durch Flash-Chromatographie mit Lösungsmittel-System B28 gereinigt. $R_f$ (B4) = 0,52.

Das Ausgangsmaterial wird folgendermassen hergestellt:

a) 4-Cyano-2(R,S)-α-naphthoxy-buttersäure: Eine Mischung von 5,0 g α-Naphthoxy-malonsäure-diäthylester und 1,09 ml Acrylnitril wird unter Rühren bei 0° mit 0,11 ml einer 50%igen wässrigen KOH-Lösung versetzt. Das Reaktionsgemisch wird 5 Tage bei Raumtemperatur gerührt und dann in Essigester gelöst. Die Essigesterlösung wird mit 2N Natriumbicarbonatlösung, 2N Salzsäure und Sole gewaschen, über Natriumsulfat getrocknet und eingedampft, wobei (2-Cyano-äthyl)-α-naphthoxy-malonsäure-diäthylester zurückbleibt.

7,84 g dieses Malonesters werden in 39,2 ml DMSO, 398 µl Wasser und 1,87 g Lithiumchlorid 3 Std. bei 180° gerührt. Das Reaktionsgemisch wird am Hochvakuum eingedampft, der Rückstand in Essigester gelöst und mit Wasser extrahiert. Die Wasserextrakte werden mit 2N Salzsäure auf pH 2 gestellt und mit Essigester extrahiert. Die Essigesterextrakte werden mit Wasser und Sole gewaschen, über Natriumsulfat getrocknet und eingedampft, wobei die Titelverbindung als hellbraunes Oel erhalten wird.

Beispiel 36:

N-(4-tert-Butoxycarbonyl-2(R,S)-α-naphthoxy-butyryl)-His-Cha$\overset{C}{-}$Val-n-butylamid

Analog Beispiel 24 wird aus 35,7 mg 4-tert-Butoxycarbonyl-2(R,S)-α-naphthoxy-buttersäure, 50,0 mg

H-His-Cha$\overset{C}{-}$Val-n-butylamid,

16,5 mg HOBt und 28,8 mg DCCI die Titelverbindung hergestellt und durch Flash-Chromatographie mit Lösungsmittel-System B28 gereinigt. $R_f$ (B4) = 0,33.

Das Ausgangsmaterial wird folgendermassen hergestellt:

a) 4-tert-Butoxycarbonyl-2(R,S)-α-naphthoxy-buttersäure: Zu einer Lösung von 7,6 g Natriumhydriddispersion in 300 ml DMF wird langsam eine Lösung von 25 g 1-Naphthol in 50 ml DMF zugetropft. Die Suspension wird 30 Min. gerührt und danach tropfenweise mit 41,5 g Brommalonsaure-diäthylester in 50 ml DMF versetzt. Das Reaktionsgemisch wird 16 Std. bei Raumtemperatur weitergerührt und dann am Hochvakuum eingedampft. Der Rückstand wird in Essigester gelöst und mit 2N Natronlauge, Wasser und Sole gewaschen, über Natriumsulfat getrocknet und eingedampft. Der Rückstand wird bei 145-170°/2,6 Pa destilliert und ergibt α-Naphthoxy-malonsäure-diäthylester als orangefarbenes Oel.

Eine Lösung von 3,0 g dieses Malonsäureesters und 1,45 ml Acrylsäure-tert-butylester in 5 ml Acetonitril wird mit 1,49 ml 1,8-Diazabicyclo[5.4.0]undec-7-en versetzt und 24 Std. bei Raumtemperatur gerührt. Das Reaktionsgemisch wird mit 25 ml Wasser versetzt, mit ca. 12 ml 2N Salzsäure auf pH 2 gestellt und mit Aether extrahiert. Die Aetherphasen werden mit Sole und Wasser gewaschen, über Natriumsulfat getrocknet und eingedampft, wobei (2-tert-Butoxycarbonyläthyl)-α-naphthoxy-malonsäure-

diäthylester als hellbraunes Oel zurückbleibt.

Dieser Ester wird analog Beispiel 35a mit Lithiumchlorid und Wasser in DMSO bei 180° hydrolysiert und decarboxyliert. Die rohe Säure wird durch Chromatographie mit Lösungsmittel-System B4 gereinigt und ergibt die Titelverbindung als weissen Schaum.

Beispiel 37:

N-(2(R,S)-Aethylaminocarbonyloxy-3-α-naphthyl-propionyl)-His-Cha$\overset{C}{-}$Val-n-butylamid

Analog Beispiel 24 wird aus 15,5 mg 2(R,S)-Aethylaminocarbonyloxy-3-α-naphthyl-propionsäure, 25,0 mg

H-His-Cha$\overset{C}{-}$Val-n-butylamid,

8,3 mg HOBt und 14,4 mg DCCI die Titelverbindung hergestellt und durch Flash-Chromatographie mit Lösungsmittel-System B28 gereinigt. $R_f$ (B29) = 0,25.

Das Ausgangsmaterial wird folgendermassen hergestellt:

a) 2(R,S)-Aethylaminocarbonyloxy-3-α-naphthyl-propionsäure: Eine Lösung von 50 mg 2-Hydroxy-3-α-naphthyl-propionsäure (Beispiel 17b) in 2 ml DMF wird mit 54 µl Aethylisocyanat versetzt. Das Reaktionsgemisch wird 3 Std. bei 100° gerührt und dann am Rotationsverdampfer unter Hochvakuum eingedampft. Der Rückstand wird in Essigester gelöst, mit 0,1 N Salzsäure, Wasser und Sole gewaschen, über Natriumsulfat getrocknet, eingedampft und durch Chromatographie auf Silicagel mit Lösungsmittel-System B11 gereinigt. Gelbes Oel, $R_f$ (B11) = 0,27.

Beispiel 38:

N-[2(R,S)-(2-Benzyloxycarbonylamino-2-methyl-propionyloxy)-3-α-naphthyl-propionyl]-His-Cha$\overset{C}{-}$Val-n-butylamid

Analog Beispiel 24 wird aus 47,0 mg 2(R,S)-(2-Benzyloxycarbonylamino-2-methyl-propionyloxy)-3-α-naphthyl-propionsäure, 50,0 mg

H-His-Cha$\overset{C}{-}$Val-n-butylamid,

16,5 mg HOBt und 28,2 mg DCCI die Titelverbindung hergestellt und durch Flash-Chromatographie mit Lösungsmittel-System B30 gereinigt. $R_f$ (B29) = 0,38.

Das Ausgangsmaterial wird folgendermassen hergestellt:

a) 2(R,S)-(2-Benzyloxycarbonylamino-2-methyl-propionyloxy)-3-α-naphthyl-propionsäure: Zu einer gerührten Suspension von 0,14 g Natriumhydrid (55 % in Mineralöl) in 10 ml DMF wird bei Raumtemperatur eine Lösung von 0,77 g α-Benzyloxycarbonylamino-isobuttersäure in 5 ml DMF und nach 30 Min. 1 g 2-Brom-3-α-naphthyl-propionsäure-äthylester in 5 ml DMF zugetropft. Das Reaktionsgemisch wird 16 Std. bei Raumtemperatur gerührt und dann am Hochvakuum eingedampft. Der Rückstand wird in Essigester gelöst, mit Wasser und Sole gewaschen, über Natriumsulfat getrocknet und eingedampft. Durch Chromatographie des Rückstandes auf Silicagel mit Toluol/Essigester (95:5) erhält man den Aethylester der Titelverbindung.

Zur Verseifung werden 230 mg des Esters in 5 ml THF/Wasser (9:1) gelöst und bei 0° mit 20,8 mg $LiOH \cdot H_2O$ versetzt. Das Reaktionsgemisch wird 20 Std. bei 0° gerührt und dann dreimal mit Essigester extrahiert. Die Essigesterphasen werden mit Wasser und Sole gewaschen, über Natriumsulfat getrocknet

und eingedampft. Aus dem Rückstand erhält man nach Chromatographie auf Silicagel mit Lösungsmittel-System B11 die Titelverbindung als gelbes Oel, $R_f$ (B11) = 0,34.

Beispiel 39:

N-(3-Phenyl-2(S)-pivaloyloxy-propionyl)-His-Cha$^{C}$-Val-n-butylamid

Analog Beispiel 24 wird aus 30 mg 3-Phenyl-2(S)-pivaloyloxy-propionsäure, 50 mg

H-His-Cha$^{C}$-Val-n-butylamid,

18 mg HOBt und 29 mg DCCI die Titelverbindung hergestellt und durch Mitteldruck-Chromatographie über eine Lobar®-Fertigsäule mit Methylenchlorid-Methanol-Ammoniak konz. 150:10:1 gereinigt. $R_f$ (B8) = 0,45.

Das Ausgangsmaterial wird folgendermassen hergestellt:

a) 3-Phenyl-2(S)-pivaloyloxy-propionsäure: 3,01 g 3-Phenyl-2(S)-pivaloyloxy-propionsäure-benzylester werden in 35 ml Methanol in Gegenwart von 300 mg Palladium-Kohle (10 %) während 30 Min. bei Raumtemperatur unter Normaldruck hydriert. Der Katalysator wird abfiltriert und das Filtrat im Vakuum eingedampft. Man erhält die Titelverbindung als klares farbloses Oel. $R_f$ (N21) = 0,74. $^1$H-NMR (DMSO-$d_6$): 12,5 (1H); 7,25 (s,5 H); 5,1 (dxd,1H); 3,15 (m,2H); 1,1 ppm (s,9H).

b) 3-Phenyl-2(S)-pivaloyloxy-propionsäure-benzylester: 0,24 ml Pivalinsäurechlorid in 2 ml Methylenchlorid werden zu einer auf 0°C gekühlten Lösung von 250 mg 3-Phenyl-2(S)-hydroxy-propionsäure-benzylester (L-β-Phenylmilchsäure-benzylester) und 0,408 ml Triäthylamin in 5 ml Methylenchlorid getropft. Das Gemisch wird 30 Std. bei Raumtemperatur gerührt und anschliessend auf Eis gegossen. Die organische Phase wird mit 1N Salzsäure und Wasser gewaschen und über Natriumsulfat getrocknet. Das Lösungsmittel wird im Vakuum abgedampft und der Rückstand durch Mitteldruck-Chromatographie (Lobar®-Fertigsäule Merck, Grösse B, Laufmittel Petroläther/Aether 11:1) gereinigt. Die Titelverbindung wird als farbloses Oel erhalten. $R_f$ (N4) = 0,52.

c) 3-Phenyl-2(S)-hydroxypropionsäure-benzylester: 23 ml einer 20 % wässrigen Lösung von Cäsiumcarbonat (pH 7) werden bei Raumtemperatur zu einer Lösung von 4,0 g L-β-Phenylmilchsäure (3-Phenyl-2-(S)-hydroxypropionsäure) in 65 ml Methanol und 6,5 ml Wasser zugetropft. Das Lösungsmittel wird abgedampft und der Rückstand 24 Std. bei Raumtemperatur im Hochvakuum getrocknet. Das getrocknete Cäsiumsalz wird in 37 ml wasserfreiem DMF aufgenommen, auf 0°C gekühlt und tropfenweise mit 3,15 ml Benzylbromid versetzt. Die entstandene weisse Suspension wird 24 Std. bei Raumtemperatur gerührt und anschliessend filtriert. Das Filtrat wird im Hochvakuum eingedampft, der Rückstand in Aether aufgenommen, mit Wasser und gesättigter wässriger $NaHCO_3$-Lösung gewaschen und über Natriumsulfat getrocknet. Nach Abdampfen des Lösungsmittels im Vakuum erhält man die Titelverbindung als gelbliches Oel. $R_f$ (N4) = 0,2. $^1$H-NMR (DMSO-$d_6$): 7,3 (s,5 H); 7,2 (s,5 H); 5,6 (d,1H); 5,15 (s,2 H); 4,4 (m,1 H), 3,0 ppm (m,2 H).

Beispiel 40:

N-(2(R,S)-, 2(R)- und 2(S)-Dimethoxyphosphoryl-3-phenyl-propionyl)-His-Cha$^{C}$-Val-n-butylamid

Analog Beispiel 24 wird aus 31 mg 2(R,S)-Dimethoxyphosphoryl-3-phenyl-propionsäure, 50 mg

H-His-Cha$^{C}$-Val-n-butylamid,

18 mg HOBt und 29 mg DCCI die Titelverbindung als Diastereomerengemisch hergestellt und durch

Mitteldruck-Chromatographie (Lobar®-Fertigsäule, Fliessmittel Methylenchlorid-Methanol-Ammoniak konz. 120:10:1) gereinigt. $R_f$ (B8) = 0,46 (schnelleres Diastereomeres); $R_f$ (B8) = 0,41 (langsameres Diastereomeres). Die Diastereomeren können chromatographisch getrennt werden.

Das Ausgangsmaterial wird folgendermassen hergestellt:

a) 2(R,S)-Dimethoxyphosphoryl-3-phenyl-propionsäure: 6,66 g 2-Dimethoxyphosphoryl-3-phenyl-propionsäure-methylester (α-Benzylphosphonoessigsäure-trimethylester) werden in 20 ml MeOH vorgelegt und bei Raumtemperatur mit 8 ml $H_2O$ und 12,14 ml 2N KOH versetzt und während 90 Min. gerührt. Darauf wird mit 12,14 ml 2N HCl neutralisiert, auf 20 ml eingedampft, mit Essigester extrahiert, getrocknet und wieder eingedampft. $^1$H-NMR (DMSO-$d_6$): 2,8-3,2 ppm (m,2 H); 3,6 (s, 3H); 3,75 (s, 3H); 7,2 (s, 5H).

b) α-Benzyl-phosphonoessigsäure-trimethylester: 2,4 g NaH (50 % in Oel) werden in 250 ml Dimethoxyäthan vorgelegt und unter Eisbadkühlung mit 10 g Phosphonoessigsäure-trimethylester in 40 ml Dimethoxyäthan versetzt. Es wird 90 Min. bei Raumtemperatur gerührt und sodann mit 6,52 ml Benzylbromid in 40 ml Dimethoxyäthan versetzt. Nach 4,5 Std. Rühren bei Raumtemperatur wird das Reaktionsgemisch auf 100 ml einer 2N wässrigen Lösung von $NaH_2PO_4$ und Eis gegossen, mit Aether extrahiert, die organische Phase getrocknet und eingedampft. Das Rohprodukt wird durch Mitteldruck-Chromatographie (1400 g Lichoprep® Si 60, 25-40 μm, Laufmittel Essigester-n-Hexan 2:1 und reiner Essigester) gereinigt. $^1$H-NMR (DMSO-$d_6$): 2,9-3,4 ppm (m, 2H); 3,60 (s, 3H); 3,61 (s, 3H); 3,85 (s, 3H); 7,22 (s, 5H).

## Beispiel 41:

### Ammonium-N-(2(R,S)-methoxyoxidophosphoryl-3-phenyl-propionyl)-His-Cha$^C$Val-n-butylamid und Diammonium-N-(3-phenyl-2(R,S)-phosphonato-propionyl)-His-Cha$^C$Val-n-butylamid

43 mg

N-(2(R,S)-Dimethoxyphosphoryl-3-phenyl-propionyl)-His-Cha$^C$Val-n-butylamid

(Beispiel 40) werden in 1 ml Chloroform bei Raumtemperatur mit 45 μl Trimethylbromsilan versetzt und während 30 Std. stehen gelassen. Das Reaktionsgemisch wird eingedampft, mit 1 ml 1N Salzsäure versetzt, 1 Std. bei Raumtemperatur gerührt und wieder eingedampft. Der Rückstand wird mit Flash-Chromatographie (Laufmittel Methylenchlorid-Methanol-Ammoniak konz., vorerst 10:5:1, dann 5:3:1) getrennt.

Methylester-ammoniumsalz: $R_f$ (B32) = 0,38 (schnelleres Diastereomeres); $R_f$ (B32) = 0,33 (langsameres Diastereomeres). Diammoniumsalz: $R_f$ (B32) = 0,32 (schnelleres Diastereomeres); $R_f$ (B32) = 0,26 (langsameres Diastereomeres).

## Beispiel 42:

### N-(2(R,S)-Benzyl-3-diäthoxyphosphoryl-propionyl-His-Cha$^C$Val-n-butylamid

Analog Beispiel 24 wird aus 93 mg 2(R,S)-Benzyl-3-diäthoxyphosphoryl-propionsäure, 130 mg

H-His-Cha$^C$Val-n-butylamid,

47 mg HOBt und 75 mg DCCI die Titelverbindung hergestellt und durch Flash-Chromatographie mit Lösungsmittel-System B5 gereinigt. $R_f$ (B8) = 0,37 (schnelleres Diastereomeres); $R_f$ (B8) = 0,32 (langsameres Diastereomeres).

Das Ausgangsmaterial wird folgendermassen hergestellt:

a) 2(R,S)-Benzyl-3-diäthoxyphosphoryl-propionsäure: 745 mg 2-Benzyl-3-diäthoxyphosphoryl-propionsäure-äthylester werden in 5 ml Aethanol und 4 ml Wasser vorgelegt, mit 1,13 ml 2N KOH versetzt und 4 Std. bei Raumtemperatur gerührt, dann mit 1,13 ml 2N HCl neutralisiert und eingedampft. Der Rückstand wird durch Flash-Chromatographie mit Lösungsmittel-System N10 gereinigt. $R_f$ (N8) = 0,46. $^1$H-NMR (DMSO-$d_6$): 1,2 ppm (t, 6H); 1,7 (m, 1H); 2,05 (m, 1H); 2,7-3,0 (m, 3H); 3,95 (q, 4H); 7,15-7,35 (m, 5H).

b) 2-Benzyl-3-diäthoxyphosphoryl-propionsäure-äthylester: 30 ml Aethanol werden bei Raumtemperatur mit 4 ml einer 1 % Natriumäthanolat-Lösung, 1,0 g α-Benzylacrylsäureäthylester und 0,68 ml Diäthylphosphit (Phosphorigsäurediäthylester) in 5 ml Aethanol versetzt. Das Gemisch wird während 24 Std. bei Raumtemperatur gerührt, dann mit 200 mg $NaH_2PO_4$ in 1 ml Wasser versetzt und eingedampft. Es wird zwischen Aether und Wasser verteilt, die organische Phase getrocknet, eingedampft und durch Flash-Chromatographie mit Methylenchlorid-Aether 7:1 gereinigt. $^1$H-NMR (DMSO-$d_6$): 1,05 ppm (t, 3H); 1,18 (t, 6H); 1,8-2,2 (m, 2H); 2,9 (m, 3H); 3,95 (m, 6H); 7,2 (m, 5H).

c) α-Benzylacrylsäure-äthylester: 20 g Benzylmalonsäurediäthylester in 40 ml Aethanol werden bei Raumtemperatur mit 4,0 g KOH in 50 ml Aethanol versetzt, über Nacht bei Raumtemperatur gerührt, eingedampft, mit 7,1 ml Wasser versetzt und im Eisbad mit 6,3 ml konz. Salzsäure angesäuert, Es wird zwischen Wasser und Aether verteilt, die organsiche Phase getrocknet und der Aether abdestilliert. Der Rückstand wird mit 12,9 ml Pyridin, 0,61 g Piperidin und 1,78 g Paraformaldehyd versetzt. Das Gemisch wird im Oelbad (130°) während 90 Min. erhitzt, abgekühlt, mit 220 ml Wasser versetzt und dreimal mit 75 ml n-Hexan extrahiert. Die vereinigten organischen Phasen werden mit Wasser, 1N HCl, Wasser, ges. $NaHCO_3$-Lösung und Sole gewaschen. Die Titelverbindung wird durch Destillation gewonnen. $^1$H-NMR (DMSO-$d_6$): 1,2 ppm (t, 3H); 3,6 (d, 2H); 4,1 (q, 2H); 5,6 (m, 1H); 6,15 (m, 1H); 7,25 (m, 5H).

Beispiel 43:

**N-(3-Benzyloxycarbonyl-2(R,S)-α-naphthoxy-propionyl)-His-Cha$\overset{C}{-}$Val-n-butylamid**

Analog Beispiel 24 wird aus 135 mg 3-Benzyloxycarbonyl-2(R,S)-α-naphthoxy-propionsäure, 181 mg

**H-His-Cha$\overset{C}{-}$Val-n-butylamid,**

60 mg HOBt und 105 mg DCCI die Titelverbindung hergestellt und durch Flash-Chromatographie mit Lösungsmittel-System B23 gereinigt. $R_f$ (B23) = 0,34.

Das Ausgangsmaterial wird folgendermassen hergestellt:

a) 3-Benzyloxycarbonyl-2-(R,S)-α-naphthoxy-propionsäure: 1,04 g α-Naphthoxy-bernsteinsäure in 20 ml DMF werden bei 0° mit 0,35 g Natriumhydrid (55 % in Oel) versetzt, 4 Std. bei Raumtemperatur gerührt, dann mit 1,08 ml Benzylbromid versetzt und 20 Std. bei 45° gerührt. Das Reaktionsgemisch wird eingedampft, am Hochvakuum getrocknet, in 1N Salzsäure aufgenommen, und mit Essigester extrahiert. Die organische Phase wird über $MgSO_4$ getrocknet und eingedampft. Der Rückstand wird durch Flash-Chromatographie mit Essigester-n-Hexan-Methanol 20:10:1 gereinigt, wobei die Titelverbindung als beiges Oel erhalten wird. $R_f$ (S13) = 0,48.

b) α-Naphthoxybernsteinsäure: 23 g α-Naphthol in 700 ml DMF und 200 ml THF werden unter Rühren bei 0° portionenweise mit 20,2 g Natriumhydrid (55 % in Oel) versetzt. Nach 15 Min. bei 10° werden 29,6 g Brombernsteinsäure in 100 ml DMF in 30 Min. zugetropft. Das Reaktionsgemisch wird 2 Std bei Raumtemperatur und 15 Std. bei 100° gerührt, wobei THF abdestilliert. Dann wird am Hochvakuum eingeengt und zwischen 1N Salzsäure und Essigester verteilt. Die organische Phase wird getrocknet, eingedampft und durch Flash-Chromatographie mit Lösungsmittel-System B10 gereinigt. Durch Kristallisation aus Essigester-n-Hexan 1:2 wird die Titelverbindung als beiges Pulver vom Smp. 161-162° erhalten. $R_f$ (S10) = 0,17.

Beispiel 44:

**N-(4-Carbamoyl-2(R)- und 2(S)-α-naphthoxy-butyryl)-His-Cha$\overset{C}{-}$Val-n-butylamid**

Analog Beispiel 24 wird aus 14,8 mg 4-Carbamoyl-2(R,S)-α-naphthoxy-buttersäure, 25,0 mg

**H-His-Cha$\overset{C}{-}$Val-n-butylamid,**

8,3 mg HOBt und 14,4 mg DCCI die Titelverbindung als Diastereomerengemisch hergestellt und durch Flash-Chromatographie mit Lösungsmittel-System B29 aufgetrennt. $R_f$ (B4) = 0,35 (schnelleres Diastereomeres) und $R_f$ (B4) = 0,25 (langsameres Diastereomeres).

Das Ausgangsmaterial wird folgendermassen hergestellt:

a) 4-Carbamoyl-2(R,S)-α-naphthoxy-buttersäure: Eine Lösung von 3 g α-Naphthoxy-malonsäurediäthylester (Beispiel 36a) und 0,71 g Acrylamid in 5 ml Acetonitril wird mit 1,49 ml 1,8-Diazabicyclo[5.4.0]-undec-7-en versetzt und 24 Std. bei Raumtemperatur gerührt. Das Reaktionsgemisch wird mit 25 ml Wasser versetzt, mit 2N Salzsäure auf pH 2 gestellt und mit Aether extrahiert. Die Aetherphasen werden mit Wasser und Sole gewaschen, über Natriumsulfat getrocknet und eingedampft, wobei (2-Carbamoyl-äthyl)-α-naphthoxy-malonsäurediäthylester zurückbleibt.

Dieser Ester wird analog Beispiel 35a mit Lithiumchlorid und Wasser in DMSO bei 180° hydrolysiert und decarboxyliert. Die rohe Säure wird durch Chromatographie mit Lösungsmittel-System B11 gereinigt und ergibt die reine Titelverbindung.

Beispiel 45: Analog Beispiel 24 werden hergestellt:

a)

**N-(2(R)- und 2(S)-Cyanmethyl-3-α-naphthyl-propionyl)-His-Cha$\overset{C}{-}$Val-n-butylamid;**

Trennung durch Flash-Chromatographie mit Fliessmittel B28; $R_f$ (B4) = 0,43 (schnelleres Diastereomeres) und $R_f$ (B4) = 0,38 (langsameres Diastereomeres).

b)

**N-(Indolyl-2-carbonyl)-His-Cha$\overset{C}{-}$Val-n-butylamid;**

Flash-Chromatographie mit Fliessmittel B23; $R_f$ (B4) = 0,43; $R_f$ (B4) = 0,59.

c)

**N-Phenoxyacetyl-His-Cha$\overset{C}{-}$Val-n-butylamid;**

Flash-Chromatographie mit Fliessmittel B23; $R_f$ (B4) = 0,48; $R_f$ (S4) = 0,62.

d)

**N-(β-Naphthylcarbonyl)-His-Cha$\overset{C}{-}$Val-n-butylamid;**

Flash-Chromatographie mit Fliessmittel B23; $R_f$ (B4) = 0,44; $R_f$ (S4) = 0,67.

e)

N-(α-Naphthoxyacetyl)-His-Cha$\overset{C}{-}$Val-n-butylamid;

Flash-Chromatographie mit Fliessmittel B23; $R_f$ (B4) = 0,52.
f)

N-(3-Benzyloxycarbonyl-2(R,S)-α-naphthylmethyl-propionyl)-His-Cha$\overset{C}{-}$Val-n-butylamid;

Flash-Chromatographie mit Fliessmittel B3; $R_f$ (B23) = 0,21; $R_f$ (56) = 0,23.
g)

N-(2(R,S)-Benzyl-4-oxo-pentanoyl)-His-Cha$\overset{C}{-}$Val-n-butylamid;

$R_f$ (B7) = 0,43.
h)

N-(2(R,S)-Benzyl-4,4-dimethyl-3-oxo-pentanoyl)-His-Cha$\overset{C}{-}$Val-n-butylamid;

$R_f$ (B7) = 0,35.
i)

N-(5-Dimethylamino-2(R)- und 2(S)-α-naphthylmethyl-pentanoyl)-His-Cha$\overset{C}{-}$Val-n-butylamid;

$R_f$ (B11) = 0,63 (schnelleres Diastereomeres) und $R_f$ (B11) = 0,18 (langsameres Diastereomeres).
j)

N-(2(R,S)-Benzyl-5,5-dimethyl-4-oxo-hexanoyl)-His-Cha$\overset{C}{-}$Val-methylamid;

$R_f$ (B7) = 0,30.
k)

N-(2-(R,S)-tert-Butylcarbamoyl-3-α-naphthyl-propionyl)-His-Cha$\overset{C}{-}$Val-n-butylamid;

$R_f$ (B5) = 0,20; $R_f$ (B7) = 0,30.

Beispiel 46:

N-(3-Carboxy-2(R)- und 2(S)-α-naphthylmethyl-propionyl)-His-Cha$\overset{C}{-}$Val-n-butylamid

60 mg

**N-(3-Benzyloxycarbonyl-2(R,S)-α-naphthylmethyl-propionyl)-His-Cha$\overset{C}{-}$Val-n-butylamid**

(Beispiel 45f) werden in 6 ml Methanol gelöst und in Gegenwart von 100 mg Palladium-Kohle (10 % Pd) bis zur Sättigung hydriert. Der Rückstand wird durch Chromatographie an 30 g Kieselgel mit Methylenchlorid-Methanol-Wasser (vorerst 110:10:1, dann 50:10:1) aufgetrennt. $R_f$ (S10) = 0,66 und $R_f$ (N22) = 0,37 (schnelleres Diastereomeres); $R_f$ (S10) = 0,48 und $R_f$ (N22) = 0,17 (langsameres Diastereomeres).

Beispiel 47:

**N-(Chinolyl-2-carbonyl)-N-methyl-His-Cha$\overset{C}{-}$Val-n-butylamid**

50 mg

**H-Cha$\overset{C}{-}$Val-n-butylamid (Beispiel 24c), 55 mg $N^{\alpha}$-(Chinolyl-2-carbonyl)-$N^{\alpha}$-methyl-L-histidin**

und 26 mg HOBt werden in 2 ml DMF im Eisbad mit 41 mg DCCI versetzt. Es wird 6 Std. im Eisbad und 3 Tage bei Raumtemperatur gerührt. Nach Abdampfen des Lösungsmittels im Hochvakuum wird der Rückstand in 5 ml eines Gemisches von Methanol-Wasser-Eisessig (94:3:3) während 60 Min. auf 60 °C erwärmt und wieder eingedampft. Der Rückstand wird durch Mitteldruck-Chromatographie über eine Lobar®-Fertigsäule, Grösse B, mit Lösungsmittel-System B26 gereinigt und aus tert-Butanol durch Lyophilisation isoliert. $R_f$ (B8) = 0,36.

Das Ausgangsmaterial wird folgendermassen hergestellt:

a) $N^{\alpha}$-(Chinolyl-2-carbonyl)-$N^{\alpha}$-methyl-L-histidin: 321 mg $N^{\alpha}$-(Chinolyl-2-carbonyl)-$N^{\alpha}$-methyl-L-histidin-methylester werden in 3 ml THF gelöst und bei Raumtemperatur mit 1,42 ml 1N NaOH versetzt. Nach 1,5 Std. wird mit 1,42 ml 1N HCl neutralisiert und zur Trockne eingedampft. $R_f$ (B8) = 0,02.

b) $N^{\alpha}$-(Chinolyl-2-carbonyl)-$N^{\alpha}$-methyl-L-histidin-methylester: 400 mg $N^{\alpha}$-Methyl-L-histidin-methylester-dihydrochlorid, 298 mg Chinolin-2-carbonsäure, 263 mg HOBt, 0,531 ml Aethyldiisopropylamin und 418 mg DCCI werden in 2 ml DMF während 5 Tagen bei Raumtemperatur gerührt und dann eingedampft. Der Rückstand wird 1 Std. in einem Gemisch von Methanol-Wasser-Eisessig (94:3:3) bei 60° verrührt, wieder eingedampft und durch Mitteldruck-Chromatographie über eine Lobar®-Fertigsäule mit Lösungsmittel-System B31 gereinigt. $R_f$ (B8) = 0,63.

c) $N^{\alpha}$-Methyl-L-histidin-methylester-dihydrochlorid: 2 g $N^{\alpha}$-Methyl-L-histidin-hydrochlorid (Bachem AG, Bubendorf) werden in 30 ml Methanol gelöst und bei Eisbadkühlung mit 1,2 ml $SOCl_2$ versetzt. Es wird über Nacht bei Raumtemperatur gerührt und anschliessend zur Trockne eingedampft. $^1$H-NMR (DMSO-$d_6$, TMS): 2,65 ppm (s, 3H); 3,45 (dd, 2H); 3,7 (s, 3H); 4,4 (m, 1H); 7,55 (s, H); 9,1 (s, 1H).

Beispiel 48:

**N-Methyl-N-(3-phenyl-2(S)-pivaloyloxy-propionyl)-His-Cha$\overset{C}{-}$Val-n-butylamid**

Analog Beispiel 47 wird aus 68 mg $N^{\alpha}$-Methyl-$N^{\alpha}$-(3-phenyl-2(S)-pivaloyloxy-propionyl)-L-histidin, 50 mg

H-Cha$\overset{c}{-}$Val-n-butylamid,

26 mg HOBt und 41 mg DCCI die Titelverbindung hergestellt und durch Mitteldruck-Chromatographie über eine Lobar®-Fertigsäule mit Lösungsmittel-System B26 gereinigt. $R_f$ (B8) = 0,29.

Das Ausgangsmaterial wird folgendermassen hergestellt:

a) N$^\alpha$-Methyl-N$^\alpha$-(3-phenyl-2(S)-pivaloyloxy-propionyl)-L-histidin: Analog Beispiel 47a wird der entsprechende Methylester mit Natronlauge verseift. $R_f$ (B8) = 0,01.

b) N$^\alpha$-Methyl-N$^\alpha$-(3-phenyl-2(S)-pivaloyloxy-propionyl)-L-histidin-methylester: Analog Beispiel 47b wird aus N$^\alpha$-Methyl-L-histidin-methylester-dihydrochlorid und 3-Phenyl-2(S)-pivaloyloxy-propionsäure (Beispiel 39a) die Titelverbindung hergestellt und durch Flash-Chromatographie mit Fliessmittel N10, dann Mitteldruck-Chromatographie (Lichroprep® Si60, 25-40 µm, Fliessmittel B33) gereinigt. $R_f$ (B8) = 0,59.

Beispiel 49:

N-(Indolyl-2-carbonyl)-N-methyl-His-Cha$\overset{c}{-}$Val-n-butylamid-hydrochlorid

Analog Beispiel 47 wird aus 53 mg N$^\alpha$-(Indolyl-2-carbonyl)-N$^\alpha$-methyl-L-histidin, 50 mg

His-Cha$\overset{c}{-}$Val-n-butylamid,

26 mg HOBt und 41 mg DCCI die Titelverbindung hergestellt, durch Mitteldruck-Chromatographie (Lichroprep® Si60, 25-40 µm, Fliessmittel B33) gereinigt und aus tert-Butanol lyophilisiert. $R_f$ (B8) = 0,57.

Das Ausgangsmaterial N$^\alpha$-(Indolyl-2-carbonyl)-N$^{\alpha\ \text{-methyl-L-histidin}}$ wird analog Beispiel 47a hergestellt.

Beispiel 50:

N-(Naphthalin-1,8-dicarbonyl)-Phe-Cha$\overset{c}{-}$Val-n-butylamid

Analog Beispiel 1 wird ausgehend von 110 mg Naphthalin-1,8-dicarbonyl-L-phenylalanin (Egypt.J.Chem. 24, 127 (1981)), 80 mg

H-Cha$\overset{c}{-}$Val-n-butylamid

(Beispiel 24c), 49 mg HOBt und 81 mg DCCI die Titelverbindung erhalten und durch Flash-Chromatographie mit Lösungsmittel-System N6 gereinigt. $R_f$ (N7) = 0,41; $R_f$ (N6) = 0,17.

Beispiel 51:

N-(Indolyl-2-carbonyl)-Phe-Cha$\overset{c}{-}$Val-n-butylamid

Eine Mischung aus 39 mg Indol-2-carbonsäure, 114 mg

H-Phe-Cha$\overset{c}{-}$Val-n-butylamid,

37 mg HOBt und 66 mg DCCI in 6 ml DMF wird 24 Std. bei Raumtemperatur gerührt. Der kristallisierte DCH wird abfiltriert und das Filtrat eingedampft. Das Rohprodukt wird mittels Flash-Chromatographie gereinigt (100 g Kieselgel 60, 40-63 $\mu$m, Laufmittel N6). Durch Eindampfen der vereinigten, produkthaltigen Fraktionen erhält man die Titelverbindung. $R_f$ (B3) =0,46; $R_f$ (S9) = 0,89.

Das Ausgangsmaterial wird folgendermassen hergestellt:

a)

**H-Phe-Cha$\overset{C}{-}$Val-n-butylamid:**

144 mg

**Z-Phe-Cha$\overset{C}{-}$Val-n-butylamid**

werden in 5 ml Methanol-Wasser 9:1 in Gegenwart von 20 mg Palladium-Kohle (10 % Pd) bei Normaldruck und Raumtemperatur bis zur Sättigung hydriert. Das Reaktionsgemisch wird filtriert und das Filtrat mit 5 ml Wasser bei Raumtemperatur gerührt. Nach dem Abdampfen der Lösungsmittel wird die Titelverbindung als farbloses Oel erhalten. $R_f$ (B2) = 0,62; $R_f$ (S9) = 0,85.

b)

**Z-Phe-Cha$\overset{C}{-}$Val-n-butylamid:**

Eine Mischung aus 150 mg Z-Phe-OH, 164 mg

**H-Cha$\overset{C}{-}$Val-n-butylamid,**

76 mg HOBt und 134 mg DCCI in 6 ml DMF wird 48 Std. bei Raumtemperatur gerührt. Der DCH wird abfiltriert, das Filtrat eingeengt und am Hochvakuum getrocknet. Der Rückstand wird durch Flash-Chromatographie aufgetrennt (185 g Kieselgel 60, 40-63 $\mu$m, Laufmittel N6). Durch Eindampfen der vereinigten, produkthaltigen Fraktionen erhält man die Titelverbindung. $R_f$ (B4) = 0,80; $R_f$ (N7) = 0,37.

Beispiel 52:

**N-(Indolyl-2-carbonyl)-Nle-Cha$\overset{C}{-}$Val-n-butylamid**

Analog Beispiel 51 wird aus 40 mg Indol-2-carbonsäure, 110 mg

**H-Nle-Cha$\overset{C}{-}$Val-n-butylamid,**

38 mg HOBt und 68 mg DCCI in 6ml DMF die Titelverbindung erhalten und durch Flash-Chromatographie mit Lösungsmittel-System N7 gereinigt. $R_f$ (B2) = 0,38; $R_f$ (S9) = 0,89.

Das Ausgangsmaterial wird folgendermassen hergestellt:

a)

**H-Nle-Cha$\overset{C}{-}$Val-n-butylamid**

wird analog Beispiel 51a durch Hydrierung von 144 mg

**Z-Nle-Cha$\overset{C}{-}$Val-n-butylamid**

in 5 ml Methanol-Wasser 9:1 in Gegenwart von 20 mg Palladium-Kohle (10 % Pd) erhalten. $R_f$ (B2) = 0,57; $R_f$ (S9) = 0,69.

b)

**Z-Nle-Cha$\overset{C}{-}$Val-n-butylamid**

wird analog Beispiel 51b ausgehend von 133 mg Z-Nle-OH, 163 mg

**H-Cha$\overset{C}{-}$Val-n-butylamid,**

77 mg HOBt und 134 mg DCCI in 6 ml DMF hergestellt. $R_f$ (B2) = 0,80; $R_f$ (N7) = 0,31.

Beispiel 53:

**N-(Indolyl-2-carbonyl)-His-Cha$\overset{C}{-}$Val-3-dimethyl-aminopropylamid**

Analog Beispiel 17 wird aus 15 mg Indol-2-carbonsäure, 43 mg

**H-His-Cha$\overset{C}{-}$Val-3-dimethylaminopropylamid,**

14 mg HOBt und 25 mg DCCI in 2 ml DHF die Titelverbindung erhalten und durch Flash-Chromatographie mit Lösungsmittel-System B11 gereinigt. $R_f$ (B1) = 0,32; $R_f$ (S9) = 0,12.

Das Ausgangsmaterial wird folgendermassen hergestellt:

a)

**H-His-Cha$\overset{C}{-}$Val-3-dimethylaminopropylamid**

wird durch Hydrierung von 57 mg

**Z-His-Cha$\overset{C}{-}$Val-3-dimethylaminopropylamid**

in 18 ml Methanol-Wasser 9:1 in Gegenwart von 15 mg Palladium-Kohle (10 % Pd) analog Beispiel 16a hergestellt. $R_f$ (B1) = 0,085.

b)

**Z-His-Cha$\overset{C}{-}$Val-3-dimethylaminopropylamid**

wird ausgehend von 136 mg Z-His-OH, 167 mg

**H-Cha$\overset{C}{-}$Val-3-dimethylaminopropylamid,**

72 mg HOBt und 128 mg DCCI in 6 ml DMF analog Beispiel 16b erhalten. $R_f$ (B1) = 0,32; $R_f$ (S9) = 0,14.

c)

H-Cha$\overset{c}{-}$Val-3-dimethylaminopropylamid: 247 mg Z-Cha$\overset{cx}{-}$Val-3-dimethylaminopropylamid

werden in 10 ml Methanol-Wasser 9:1 in Gegenwart von 40 mg Palladium-Kohle (10 % Pd) bei Normaldruck und Raumtemperatur bis zur Sättigung hydriert. Das Reaktionsgemisch wird filtriert und das Filtrat mit 10 ml Wasser bei Raumtemperatur gerührt. Nach dem Abdampfen der Lösungsmittel wird die Titelverbindung als farbloses Oel erhalten. $R_f$ (B1) = 0,13.

d)

Z-Cha$\overset{cx}{-}$Val-3-dimethylaminopropylamid:

Eine Mischung aus 357 mg

Z-Cha$\overset{cx}{-}$Val-OH

(Beispiel 16i), 8,5 ml DMF, 153 mg HOBt und 206 mg DCCI wird 24 Std. bei 0° stehen gelassen. Die Mischung wird mit 337 mg Dimethyl-3-amino-propylamin versetzt und 2 Std. bei 0° und 39 Std. bei Raumtemperatur gerührt. Der kristallisierte DCH wird abfiltriert, das Filtrat eingeengt und am Hochvakuum getrocknet. Aus dem Rückstand wird durch Flash-Chromatographie (Fliessmittel-System B4) die Titelverbindung als farbloses Oel erhalten. $R_f$ (B4) = 0,32; $R_f$ (N12) = 0,24.

Beispiel 54:

N-(Indolyl-2-carbonyl)-His-Cha$\overset{c}{-}$Val-2-morpholinoäthylamid

Analog Beispiel 17 wird aus 26 mg Indol-2-carbonsäure, 83 mg

H-His-Cha$\overset{c}{-}$Val-2-morpholinoäthylamid,

25 mg HOBt und 43 mg DCCI in 4 ml DMF die Titelverbindung erhalten und durch Flash-Chromatographie mit Fliessmittel-System B4 gereinigt. $R_f$ (B2) = 0,19; $R_f$ (S9) = 0,41.

Das Ausgangsmaterial wird folgendermassen hergestellt:

a)

H-His-Cha$\overset{c}{-}$Val-2-morpholinoäthylamid

wird durch Hydrierung von 104 mg

Z-His-Cha$\overset{c}{-}$Val-2-morpholinoäthylamid

in 10 ml Methanol-Wasser 9:1 in Gegenwart von 40 mg Palladium-Kohle (10 % Pd) analog Beispiel 16a hergestellt. $R_f$ (B1) = 0,43; $R_f$ (S9) = 0,13.

b)

Z-His-Cha$\overset{c}{-}$Val-2-morpholinoäthylamid

wird ausgehend von 174 mg Z-His-OH, 230 mg

**H-Cha$\overset{c}{-}$Val-2-morpholinoäthylamid,**

92 mg HOBt und 163 mg DCCI in 8 ml DMF analog Beispiel 16b erhalten. $R_f$ (B2) = 0,28; $R_f$ (S9) = 0,39.

c)

**H-Cha$\overset{c}{-}$Val-2-morpholinoäthylamid**

wird durch Hydrierung von 340 mg

**Z-Cha$\overset{cx}{-}$Val-2-morpholinoäthylamid**

in 10 ml Methanol-Wasser 9:1 in Gegenwart von 50 mg Palladium-Kohle (10 % Pd) analog Beispiel 53c hergestellt. $R_f$ (B1) = 0,48; $R_f$ (S9) = 0,21.

d)

**Z-Cha$\overset{cx}{-}$Val-2-morpholinoäthylamid**

wird analog Beispiel 53d aus 267 mg

**Z-Cha$\overset{cx}{-}$Val-OH,**

98 mg 4-(2-Aminoäthyl)-morpholin, 115 mg HOBt und 155 mg DCCI in 7 ml DMF hergestellt. $R_f$ (N1) = 0,68; $R_f$ (N6) = 0,10.

Beispiel 55:

**N-(Indolyl-2-carbonyl)-His-Cha$\overset{c}{-}$Val-(5-tert-butoxycarbonylamino-5-methoxycarbonyl-pentyl)-amid**

Analog Beispiel 17 wird aus 31 mg Indol-2-carbonsäure, 124 mg

**H-His-Cha$\overset{c}{-}$Val-(5-tert-butoxycarbonylamino-5-methoxycarbonyl-pentyl)-amid,**

29 mg HOBt und 52 mg DCCI in 3 ml DMF die Titelverbindung erhalten und durch Flash-Chromatographie mit Lösungsmittel-System B23 gereinigt. $R_f$ (B4) = 0,42; $R_f$ (S9) = 0,71.

Das Ausgangsmaterial wird folgendermassen hergestellt:

a)

**H-His-Cha$\overset{c}{-}$Val-(5-tert-butoxycarbonylamino-5-methoxycarbonyl-pentyl)-amid**

wird durch Hydrierung von 143 mg

Z-His-Cha$\overset{c}{-}$Val-(5-tert-butoxycarbonylamino-5-methoxycarbonyl-pentyl)-amid

in 10 ml Methanol-Wasser 9:1 in Gegenwart Von 25 mg Palladium-Kohle (10 % Pd) analog Beispiel 16a hergestellt. $R_f$ (B4) = 0,10; $R_f$ (B11) = 0,66.

b)

Z-His-Cha$\overset{c}{-}$Val-(5-tert-butoxycarbonylamino-5-methoxycarbonyl-pentyl)-amid

wird ausgehend von 197 mg Z-His-OH, 349 mg

H-Cha$\overset{c}{-}$Val-(5-tert-butoxycarbonylamino-5-methoxycarbonyl-pentyl)-amid,

104 mg HOBt und 183 mg DCCI in 11 ml DMF analog Beispiel 16b erhalten. $R_f$ (B4) = 0,59.

c)

H-Cha$\overset{c}{-}$Val-(5-tert-butoxycarbonylamino-5-methoxycarbonyl-pentyl)-amid

wird durch Hydrierung von 469 mg

Z-Cha$\overset{cx}{-}$Val-(5-tert-butoxycarbonylamino-5-methoxycarbonyl-pentyl)-amid

in 7 ml Methanol-Wasser 9:1 in Gegenwart von 100 mg Palladium-Kohle (10 % Pd) analog Beispiel 53c hergestellt. $R_f$ (B4) = 0,16.

d)

Z-Cha$\overset{cx}{-}$Val-(5-tert-butoxycarbonylamino-5-methoxycarbonyl-pentyl)-amid

wird analog Beispiel 53d aus 446 mg

Z-Cha$\overset{cx}{-}$Val-OH,

320 mg $N^{\alpha}$-tert-butoxycarbonyl-L-lysin-methylester,

153 mg HOBt und 278 mg DCCI in 14 ml DMF hergestellt. $R_f$ (N3) = 0,48; $R_f$ (N10) = 0,63.

Beispiel 56:

N-(Indolyl-2-carbonyl)-His-Cha$\overset{C}{-}$Val-(5-amino-5-carboxy-pentyl)-amid-hydrochlorid

102 mg

N-(Indolyl-2-carbonyl)-His-Cha$\overset{C}{-}$Val-(5-tert-butoxycarbonyl-amino-5-methoxycarbonyl-pentyl)-amid,

1,9 ml 0,1 H NaOH und 1 ml Wasser werden in 5 ml Methanol bei Raumtemperatur 16 Std. gerührt. Das Reaktionsgemisch wird mit 1,9 ml 0,1 N HCl neutralisiert, eingedampft und mit Essigester extrahiert. Die Extrakte werden eingedampft, mit 1,5 ml Trifluoressigsäure versetzt und 20 Min. bei Raumtemperatur stehen gelassen. Die Trifluoressigsäure wird abgedampft und das Rohprodukt durch Flash-Chromatographie (160 g Kieselgel 40-63 µm, Laufmittel S4) gereinigt. Der Rückstand wird in 1N HCl aufgenommen, vollständig eingedampft und aus tert-Butanol lyophilisiert, wobei die Titelverbindung als hellbeiges Pulver zurückbleibt. $R_f$ (B32) = 0,76.

Beispiel 57:

N-(α-Naphthoxyacetyl)-His-Cha$\overset{C}{-}$Val-2-hydroxyäthylamid

Analog Beispiel 53 wird ausgehend von 59 mg α-Naphthoxy-essigsäure, 146 mg

H-His-Cha$\overset{C}{-}$Val-2-hydroxyäthylamid,

45 mg HOBt und 78 mg DCCI die Titelverbindung hergestellt und durch Flash-Chromatographie mit Lösungsmittel-System B4 gereinigt. $R_f$ (B4) = 0,30.

Beispiel 58:

N-(3-Phenyl-2(S)-pivaloyloxy-propionyl)-His-Leu$\overset{C}{-}$Gly(R- und S-cyclohexylmethyl)-n-butylamid

Analog Beispiel 17 werden aus 40 mg 3-Phenyl-2(S)-pivaloyloxypropionsäure (Beispiel 39a), 70 mg

H-His-Leu$\overset{C}{-}$Gly(R- und S-cyclohexylmethyl)-n-butylamid,

25 mg HOBt und 39 mg DCCI die Titelverbindungen hergestellt, durch Mitteldruck-Chromatographie über eine Lobar®-Fertigsäule mit Lösungsmittelsystem B31 gereinigt und durch Lyophilisierung aus tert-Butanol isoliert. $R_f$ (B8) = 0,64 (Diastereomeres A) und $R_f$ (B8) = 0,55 (Diastereomeres B).

Das Ausgangsmaterial wird folgendermassen hergestellt:

a)

H-His-Leu$\overset{C}{-}$Gly(R- und S-cyclohexylmethyl)-n-butylamid

werden durch Hydrierung von

**Z-His-Leu$\overset{c}{-}$Gly(R- und S-cyclohexylmethyl)-n-butylamid**

analog Beispiel 16a erhalten. $R_f$ (B8) = 0,17 (Diastereomeres A) und $R_f$ (B8) = 0,11 (Diastereomeres B).
b)

**Z-His-Leu$\overset{c}{-}$Gly(R- und S-cyclohexylmethyl)-n-butylamid**

werden ausgehend von Z-His-OH und

**H-Leu$\overset{c}{-}$Gly(R- und S-cyclohexylmethyl)-n-butylamid**

analog Beispiel 16b erhalten und durch Flash-Chromatographie mit Fliessmittel N10 gereinigt. $R_f$ (N8) = 0,33 (Diastereomeres A) und $R_f$ (N8) = 0,43 (Diastereomeres B).
c)

**H-Leu$\overset{c}{-}$Gly(R- und S-cyclohexylmethyl)-n-butylamid**

werden durch Hydrierung von

**Z-Leu$\overset{cx}{-}$Gly(R- und S-cyclohexylmethyl)-n-butylamid**

analog Beispiel 16k erhalten. $R_f$ (B10) = 0,64 (Diastereomeres A) und $R_f$ (B10) - 0,61 (Diastereomeres B).
d)

**Z-Leu$\overset{cx}{-}$Gly(R- und S-cyclohexylmethyl)-n-butylamid**

werden aus

**Z-Leu$\overset{cx}{-}$Gly(R- und S-cyclohexylmethyl)-OH**

und n-Butylamin analog Beispiel 16j erhalten und durch Flash-Chromatographie mit Fliessmittel N4 aufgetrennt. $R_f$ (N3) = 0,65 (Diastereomeres A) und $R_f$ (N3) = 0,59 (Diastereomeres B).
e)

**Z-Leu$\overset{cx}{-}$Gly(R- und S-cyclohexylmethyl)-OH**

(3-[3-Benzyloxycarbonyl-2,2-dimethyl-4(S)-isobutyl-1,3-oxazolidin-5(S)-yl]-2(R,S)-cyclohexylmethyl-propionsäure) wird analog Beispiel 16i durch Hydrolyse des entsprechenden Methylesters hergestellt. $R_f$ (N4) = 0,05 (Diastereomerengemisch).
f) 3-(3-Benzyloxycarbonyl-2,2-dimethyl-4(S)-isobutyl-1,3-oxazolidin-5(S)-yl)-2(R,S)-cyclohexylmethyl-propionsäure-methylester wird analog Beispiel 1d aus der Verbindung (XXV) und 3-Cyclohexylpropionsäure-methylester hergestellt und durch Mitteldruck-Chromatographie über eine Lobar®-Fertigsäule mit n-Hexan-Essigester 19:1 gereinigt. $R_f$ (N5) = 0,14 und 0,17.
g) 3-Cyclohexylpropionsäure-methylester: 20 g Cyclohexyl-propionsäure werden in 200 ml 5N HCl in Methanol über Nacht bei Raumtemperatur stehen gelassen. Das Lösungsmittel wird abgedampft, der Rückstand in Aether aufgenommen, mit gesättigter $NaHCO_3$-Lösung und Sole gewaschen und getrocknet. Destillation bei 121-125°/2,0-2,5$\cdot 10^{-2}$ bar ergibt die Titelverbindung.

Beispiel 59:

**N-(3-Phenyl-2(S)-pivaloyloxy-propionyl)-His-Leu$\stackrel{c}{-}$Gly-(R- und S-α-decahydronaphthyl)-n-butylamid**

Analog Beispiel 17 verden aus 29 mg 3-Phenyl-2(S)-pivaloyloxy-propionsäure(Beispiel 39a), 55 mg

**H-His-Leu$\stackrel{c}{-}$Gly(R- und S-α-decahydronaphthyl)-n-butylamid,**

18 mg HOBt und 28 mg DCCI die Titelverbindungen hergestellt und durch Mitteldruck-Chromatographie über eine Lobar®-Fertigsäule mit Lösungsmittelsystem B31 gereinigt. $R_f$ (B8) = 0,53 (Diastereomeres A) und $R_f$ (B8) = 0,45 (Diastereomeres B).

Die Ausgangsmaterialien werden folgendermassen hergestellt:

a)

**H-His-Leu$\stackrel{c}{-}$Gly(R- und S-α-decahydronaphthyl)-n-butylamid**

werden durch Hydrierung von

**Z-His-Leu$\stackrel{c}{-}$Gly(R- und S-α-decahydronaphthyl)-n-butylamid**

analog Beispiel 16a erhalten. $R_f$ (B8) = 0 (beide Diastereomere).

b)

**Z-His-Leu$\stackrel{c}{-}$Gly(R- und S-α-decahydronaphthyl)-n-butylamid**

werden ausgehend Von Z-His-OH und

**H-Leu$\stackrel{c}{-}$Gly(R- und S-α-decahydronaphthyl)-n-butylamid**

analog Beispiel 16b erhalten und durch Mitteldruck-Chromatographie mit Fliessmittel B31 gereinigt. $R_f$ - (B8) = 0,59 (Diastereomeres A) und $R_f$ (B8) = 0,48 (Diastereomeres B).

c)

**H-Leu$\stackrel{c}{-}$Gly(R- und S-α-decahydronaphthyl)-n-butylamid**

werden durch Hydrierung von

**Z-Leu$\stackrel{cx}{-}$Gly(R- und S-α-decahydronaphthyl)-n-butylamid**

n-butylamid analog Beispiel 16k erhalten und durch Mitteldruck-Chromatographie mit Fliessmittel N8 gefolgt von reinem Methanol gereinigt. $R_f$ (B8) = 0 (beide Diastereomere).

d)

**Z-Leu$\stackrel{cx}{-}$Gly(R- und S-α-decahydronaphthyl)-n-butylamid**

werden aus

**Z-Leu$^{cx}$Gly(R,S-α-decahydronaphthyl)-OH**

und n-Butylamin analog Beispiel 16j erhalten und durch Flash-Chromatographie mit Fliessmittel N4 aufgetrennt. $R_f$ (N3) = 0,68 (Diastereomeres A) und $R_f$ (N3) = 0,61 (Diastereomeres B).

e)

**Z-Leu$^{cx}$Gly(R,S-α-decahydronaphthyl)-OH**

(3-[3-Benzyloxycarbonyl-2,2-dimethyl-4(S)-isobutyl-1,3-oxazolidin-5(S)-yl]-2(R,S)-α-decahydronaphthyl-propionsäure) wird analog Beispiel 16i durch Hydrolyse des entsprechenden Methylesters hergestellt und durch Mitteldruck-Chromatographie mit n-Hexan-Essigester 19:1 gereinigt. $R_f$ (N4) = 0,13 und 0,18.

f) 3-[3-Benzyloxycarbonyl-2,2-dimethyl-4(S)-isobutyl-1,3-oxazolidin-5(S)-yl]-2(R,S)-α-decahydronaphthyl-propionsäure-methylester wird analog Beispiel 1d aus der Verbindung (XXV) und α-Decahydronaphthyl-essigsäure-methylester hergestellt und durch Mitteldruck-Chromatographie mit n-Hexan-Essigester 19:1 gereinigt.

g) α-Decahydronaphthyl-essigsäuremethylester wird durch Veresterung von α-Decahydronaphthyl-essigsäure analog Beispiel 58g hergestellt. $^1$H-NMR (DMSO-$d_6$, TMS): 3,58 ppm (s, 3H) und andere.

h) α-Decahydronaphthyl-essigsäure: 25 g α-Naphthylessigsäure werden in 120 ml Wasser und 134 ml 1N Natronlauge gelöst und in Gegenwart von 1,3 g Platin-Rhodium-Katalysator ($PtO_2 \cdot H_2O$-$Rh_2O_3$ = 54:46) während 31 Std. bei Raumtemperatur und Normaldruck hyydriert. Der Katalysator wird abfiltriert, das Filtrat mit konz. Salzsäure auf pH 1 angesäuert und mit Aether extrahiert. Durch Trocknen und Eindampfen des Aetherextrakts wird die Titelverbindung erhalten.

Beispiel 60:

**N-(3-Phenyl-2(S)-pivaloyloxy-propionyl)-His-Leu$^{c}$Gly-(R- und S-dimethylamino)-n-butylamid**

Analog Beispiel 17 werden aus 20 mg 3-Phenyl-2(S)-pivaloyloxy-propionsäure(Beispiel 39a),

**27 mg H-His-Leu$^{c}$Gly(R- und S-dimethylamino)-n-butylamid,**

12 mg HOBt und 20 mg DCCI die Titelverbindungen hergestellt, durch Mitteldruck-Chromatographie über eine Lobar®-Fertigsäule mit Lösungsmittelsystem B5 gereinigt und durch Lyophilisierung aus tert-Butanol isoliert. $R_f$ (B8) = 0,36 (Diastereomeres A) und $R_f$ (B8) = 0,30 (Diastereomeres B). Die Ausgangamaterialien werden folgendermassen hergestellt:

a)

**H-His-Leu$^{c}$Gly(R- und S-dimethylamino)-n-butylamid**

werden durch Hydrierung von

**Z-His-Leu$^{c}$Gly(R- und S-dimethylamino)-n-butylamid**

analog Beispiel 16a erhalten. $R_f$ (B8) = 0,01 (beide Diastereomere).

b)

**Z-His-Leu$^{c}$Gly(R- und S-dimethylamino)-n-butylamid**

werden ausgehend von 2-His-OH und

**H-Leu$\stackrel{c}{-}$Gly(R- und S-dimethylamino)-n-butylamid**

analog Beispiel 16b erhalten und durch Mitteldruck-Chromatographie mit Fliessmittel B33 gereinigt. $R_f$ - (B8) = 0,36 (Diastereomeres A) und $R_f$ (B8) = 0,15 (Diastereomeres B).

c)

**H-Leu$\stackrel{c}{-}$Gly(R- und S-dimethylamino)-n-butylamid**

werden durch Hydrierung von

**Z-Leu$\stackrel{cx}{-}$Gly(R,S-dimethylamino)-n-butylamid**

analog Beispiel 16k und Trennung durch Mitteldruck-Chromatographie mit Fliessmittel B33 erhalten. $R_f$ - (B8) = 0,28 (Diastereomeres A) und $R_f$ (B8) = 0,19 (Diastereomeres B).

d)

**Z-Leu$\stackrel{c}{-}$Gly(R,S-dimethylamino)-n-butylamid wird aus Z-Leu$\stackrel{cx}{-}$Gly-(R,S-dimethylamino)-OH**

und n-Butylamin analog Beispiel 16j erhalten und durch Flash-Chromatographie mit Fliessmittel Methylenchlorid-Methanol-Ammoniak konz. 500:10:1 gereinigt. $R_f$ (B31) = 0,91 und 0,95.

e)

**Z-Leu$\stackrel{c}{-}$Gly(R,S-dimethylamino)-OH**

(3-[3-Benzyloxycarbonyl-2,2-dimethyl-4(S)-isobutyl-1,3-oxazolidin-5(S)-yl]-2(R,S)-dimethylamino-propionsäure) wird analog Beispiel 16j durch Hydrolyse des entsprechenden Aethylesters hergestellt und durch Mitteldruck-Chromatographie mit Fliessmittel N8 gereinigt. $R_f$ (N3) = 0,21 und 0,10.

f) 3-(3-Benzyloxycarbonyl-2,2-dimethyl-4(S)-isobutyl-1,3-oxazolidin-5(S)-yl)-2(R,S)-dimethylamino-propionsäure-äthylester wird analog Beispiel 1d aus der Verbindung (XXV) und Dimethylamino-essigsäureäthylester hergestellt und durch Mitteldruck-Chromatographie mit Fliessmittel N3 gereinigt. $R_f$ (N3) = 0,52 und 0,35.

Beispiel 61:

**N-(Indolyl-2-carbonyl)-His-Leu$\stackrel{c}{-}$Val-4-[tris-(tert-butyldimethylsilyloxymethyl)-methylcarbamoyl]-butylamid**

21 mg Indol-2-carbonsäure, 20 mg HOBt und 23 µl Aethyldiisopropylamin werden in 2 ml DMF auf 0 °C abgekühlt. Nach Zugabe von 116 mg

**H-His-Leu$\stackrel{c}{-}$Val-4-[tris-(tert-butyldimethylsilyloxymethyl)-methylcarbamoyl]-butylamid**

und 34 mg DCCI wird 24 Std. bei Eiskühlung und anschliessend während 2 Tagen bei Raumtemperatur gerührt. Der kristalline DCH wird abgenutscht und das Filtrat am Hochvakuum eingeengt. Der Rückstand wird durch Flash-Chromatographie mit Lösungsmittel-System B23 gereinigt. Durch Lyophilisieren in 20 ml tert-Butanol wird die Titelverbindung als leicht gelbliches Lyophilisat erhalten. $R_f$ (B4) = 0,49.

Das Ausgangsmaterial wird folgendermassen hergestellt:

a)

H-His-Leu$^{C}$Val-4-[tris-(tert-butyldimethylsilyloxymethyl)-methylcarbamoyl]-butylamid

wird aus der entsprechenden N-Benzyloxycarbonyl-Verbindung durch Hydrierung analog Beispiel 4a erhalten. $R_f$ (B4) = 0,11.

b)

Z-His-Leu$^{C}$Val-4-[tris-(tert-butyldimethylsilyloxymethyl)-methylcarbamoyl]-butylamid:

Analog Beispiel 61 wird aus 58 mg Z-His-OH, 155 mg

H-Leu$^{C}$Val-4-[tris-(tert-butyldimethylsilyloxymethyl)-methylcarbamoyl]-butylamid

(hergestellt nach EP-A 143 746), 35 µl Aethyldiisopropylamin, 31 mg HOBt und 54 mg DCCI in 4 ml DMF die Titelverbindung hergestellt und durch Flash-Chromatographie mit Fliessmittel B23 gereinigt. $R_f$ (B4) = 0,42.

Beispiel 62:

N-(Indolyl-2-carbonyl)-His-Leu$^{C}$Val-4-[tris-(hydroxymethyl)-methylcarbamoyl]-butylamid

36 mg

N-(Indolyl-2-carbonyl)-His-Leu$^{C}$Val-4-[tris-(tert-butyldimethylsilyloxymethyl)-methylcarbamoyl]-butylamid

und 2 ml Essigsäure-Wasser 2:1 werden während 2 Std. bei Raumtemperatur gerührt und anschliessend eingedampft. Der Rückstand wird durch Flash-Chromatographie mit Lösungmsittel-System B11 gereinigt und ergibt die Titelverbindung als weisses Pulver. $R_f$ (B11) = 0,27.

Beispiel 63:

N-(1-Benzyl-indolyl-3-carbonyl)-His-Leu$^{C}$Val-2-picolylamid

Analog Beispiel 4 wird die Titelverbindung ausgehend von 30 mg N-Benzyl-indol-3-carbonsäure, 45 mg

H-His-Leu$\overset{C}{-}$Val-2-picolylamid,

19 mg HOBt und 30 mg DCCI erhalten und durch Flash-Chromatographie mit Lösungsmittel-System B7 gereinigt. $R_f$ (BJ) = 0,31; $R_f$ (B9) = 0,43.

Die Ausgangsmaterialien werden folgendermassen hergestellt:

a)

H-His-Leu$\overset{C}{-}$Val-2-picolylamid

wird durch Hydrierung von 430 mg

Z-His-Leu$\overset{C}{-}$Val-2-picolylamid

in Gegenwart von 50 mg Palladium-Kohle (10 %) analog Beispiel 1 g erhalten. $R_f$ (B11) = 0,18; $R_f$ (B7) = 0,12.

b)

Z-His-Leu$\overset{C}{-}$Val-2-picolylamid

wird analog Beispiel 1 ausgehend von 225 mg Z-His-OH, 350 mg

H-Leu$\overset{C}{-}$Val-2-picolylamid,

130 mg HOBt und 218 mg DCCI erhalten und durch Flash-Chromatographie mit Lösungsmittel-System B7 gereinigt. $R_f$ (B7) = 0,30; $R_f$ (B11) = 0,63.

c)

H-Leu$\overset{C}{-}$Val-2-picolylamid

wird durch Hydrierung von 1,69 g

Z-Leu$\overset{CX}{—}$Val-2-picolylamid

in Gegenwart von 170 mg Palladium-Kohle (10 %) analog Beispiel 1g erhalten. $R_f$ (B7) = 0,25.

d)

Z-Leu$\overset{CX}{—}$Val-2-picolylamid

wird analog Beispiel 1 ausgehend von 1,50 g

Z-Leu$\overset{CX}{—}$Val-OH,

0,76 ml 2-Aminomethylpyridin, 680 mg HOBt und 1,070 g DCCI erhalten und durch Flash-Chromatographie mit Lösungsmittel-System N7 gereinigt. $R_f$ (N7) = 0,32.

Beispiel 64: Analog Beispiel 8 werden hergestellt:

a)

**N-(Indolyl-2-carbonyl)-His-Leu$\overset{C}{-}$Val-n-pentylamid;**

Flash-Chromatographie mit Fliessmittel B4; $R_f$ (B4) = 0,42.

b)

**N-(Indolyl-2-carbonyl)-His-Leu$\overset{C}{-}$Val-cyclohexylmethylamid;**

Flash-Chromatographie mit Fliessmittel B23; $R_f$ (B4) = 0,34.

c)

**N-(Indolyl-2-carbonyl)-His-Leu$\overset{C}{-}$Val-5-hydroxypentylamid;**

Flash-Chromatographie mit Fliessmittel B4; $R_f$ (B4) = 0,18.

d)

**N-(α-Naphthoxyacetyl)-His-Leu$\overset{C}{-}$Val-2-hydroxyäthylamid;**

Flash-Chromatographie mit Fliessmittel B4; $R_f$ (B4) = 0,27.

Beispiel 65: Analog Beispiel 4 werden hergestellt:

a)

**N-(α-Naphthoxyacetyl)-Phe-Leu$\overset{C}{-}$Val-n-butylamid;**

Flash-Chromatographie mit Fliessmittel N10; $R_f$ (N10) = 0,15.

b)

**N-Methyl-N-(α-naphthoxyacetyl)-Phe-Leu$\overset{C}{-}$Val-n-butylamid;**

Flash-Chromatographie mit Fliessmittel N7; $R_f$ (N7) = 0,26.

Beispiel 66: Nach einem der vorstehenden Beispiele können hergestellt werden:

N-[2-(2-Amino-2-methyl-propionyloxy)-3-α-naphthyl-propionyl]-His-Cha$\overset{C}{-}$Val-n-butylamid;

N-(2-Acetoacetoxy-3-α-naphthyl-propionyl)-His-Cha$\overset{C}{-}$Val-n-butylamid;

N-(2-Cyano-3-α-naphthyl-propionyl)-His-Cha$\overset{C}{-}$Val-n-butylamid;

N-(2-Acetyl-3-α-naphthyl-propionyl)-His-Cha$\overset{C}{-}$Val-n-butylamid;

N-(2-Acetonyl-3-α-naphthyl-propionyl)-His-Cha$\overset{C}{-}$Val-n-butylamid;

N-(5-Dimethylamino-2-α-naphthoxy-pentanoyl)-His-Cha$\overset{C}{-}$Val-n-butylamid;

N-(4-Carboxy-2-α-naphthoxy-butyryl)-His-Cha$\overset{C}{-}$Val-n-butylamid;

N-(2-α-Naphthoxy-4-oxo-pentanoyl)-His-Cha$\overset{C}{-}$Val-n-butylamid;

N-(Cyano-α-naphthoxy-acetyl)-His-Cha$\overset{C}{-}$Val-n-butylamid;

N-(3-Phenyl-2-pivaloyloxy-propionyl)-His-Cha$\overset{C}{-}$Gly(dimethylamino)-n-butylamid;

N-(2-Benzyl-5,5-dimethyl-4-oxo-hexanoyl)-His-Cha$\overset{C}{-}$Gly(dimethylamino)-n-butylamid;

N-(3-Phenyl-2-pivaloyloxy-propionyl)-His-Cha$\overset{C}{-}$Val-2-(4-imidazolyl-äthylamid;

N-(2-Benzyl-5,5-dimethyl-4-oxo-hexanoyl)-His-Cha$\overset{C}{-}$Val-2-(4-imidazolyl)-äthylamid;

N-(3-Phenyl-2-pivaloyloxy-propionyl)-His-Cha$\overset{C}{-}$Val-2-[2-(4-imidazolyl)-äthylamino]-äthylamid;

N-(2-Benzyl-5,5-dimethyl-4-oxo-hexanoyl)-His-Cha$\overset{C}{-}$Val-2-[2-(4-imidazolyl)-äthylamino]-äthylamid;

N-(2-Benzyl-5,5-dimethyl-4-oxo-hexanoyl)-His-Cha$\overset{C}{-}$Val-4-carboxy-butylamid;

N-[2-(2-Dimethylaminoäthylcarbamoyl)-3-phenyl-propionyl]-His-Cha$\overset{C}{-}$Val-4-carboxybutylamid;

N-(2-Dimethoxyphosphoryl-3-phenyl-propionyl)-His-Cha$\overset{C}{-}$Val-4-carboxybutylamid;

N-[2-(5-Amino-5-carboxy-pentylcarbamoyl]-3-phenyl-propionyl]-His-Cha$\overset{C}{-}$Val-n-butylamid;

N-(2-Dimethylaminomethyl-3-α-naphthyl-propionyl)-His-Cha$\overset{C}{-}$Val-4-carboxybutylamid;

N-(2-Benzyl-5-dimethylamino-4-oxo-pentanoyl)-His-Cha$\overset{C}{-}$Val-methylamid;

N-(2-Acetoxy-4-phenyl-butyryl)-His-Cha$\overset{C}{-}$Val-n-butylamid;

N-(2-Cyanomethyl-4-phenyl-butyryl)-His-Cha$\overset{C}{-}$Val-n-butylamid;

N-(2-Aethylaminocarbonyloxy-4-phenyl-butyryl)-His-Cha$\overset{C}{-}$Val-n-butylamid;

N-(3-α-Naphthyl-2-neopentyloxy-propionyl)-His-Cha$\overset{C}{-}$Val-n-butylamid;

N-(2-Benzyl-5,5-dimethyl-4-oxo-hexanoyl)-His-Cha$\overset{C}{-}$Gly(cyclohexylmethyl)-n-butylamid;

N-[4,4-Dimethyl-3-oxo-2-(2-phenyläthyl)-pentanoyl]-His-Cha$\overset{C}{-}$Val-n-butylamid;

N-(2-Dimethoxyphosphoryl-4-phenyl-butyryl)-His-Cha$\overset{C}{-}$Val-n-butylamid;

N-(2-Diäthoxyphosphorylmethyl-4-phenyl-butyryl)-His-Cha$\overset{C}{-}$Val-n-butylamid;

N-[5,5-Dimethyl-4-oxo-2-(2-phenyläthyl)-hexanoyl]-His-Cha$\overset{C}{-}$Val-n-butylamid;

N-(2-tert-Butylcarbamoyl-4-phenyl-butyryl)-His-Cha$\overset{C}{-}$Val-n-butylamid;

N-(2-tert-Butylcarbamoyl-3-phenyl-propionyl)-His-Cha$\overset{C}{-}$Val-n-butylamid;

N-(2-tert-Butylcarbamoyl-3-α-naphthyl-propionyl)-His-Cha$\overset{C}{-}$Val-methylamid;

N-(2-Benzyl-5,5-dimethyl-4-oxo-hexanoyl)-His-Cha$\overset{C}{-}$Val-methylamid;

N-(2-Benzyl-5,5-dimethyl-4-oxo-hexanoyl)-His-Leu$\overset{C}{-}$Gly(cyclohexylmethyl)-n-butylamid;

N-(2-Benzyl-4,4-dimethyl-3-oxo-pentanoyl)-His-Leu$\overset{c}{-}$Gly(cyclohexylmethyl)-n-butylamid;
N-(2-tert-Butylcarbamoyl-3-phenyl-propionyl)-His-Leu$\overset{c}{-}$Gly(cyclohexylmethyl)-n-butylamid.

Beispiel 67: Gelatine-Lösung

Eine sterilfiltrierte wässrige Lösung von

N-(2(R,S)-Benzyl-5,5-dimethyl-4-oxo-hexanoyl)-His-Cha$\overset{c}{-}$Val-n-butylamid

wird unter Erwärmen mit einer sterilen Gelatinelösung, welche als Konservierungsmittel Phenol enthält, unter aseptischen Bedingungen so vermischt, dass 1,0 ml Lösung folgende Zusammensetzung hat:

| | |
|---|---|
| N-(2(R,S)-Benzyl-5,5-dimethyl-4-oxo-hexanoyl)-His-Cha$\overset{c}{-}$Val-n-butylamid | 3 mg |
| Gelatine | 150,0 mg |
| Phenol | 4,7 mg |
| dest. Wasser bis zu | 1,0 ml |

Die Mischung wird unter aseptischen Bedingungen in Vialen zu 1,0 ml abgefüllt.

Beispiel 68: Sterile Trockensubstanz zur Injektion

N-(2(R,S)-Benzyl-5,5-dimethyl-4-oxo-hexanoyl)-His-Cha$\overset{c}{-}$Val-n-butylamid

Man löst 5 mg in 1 ml einer wässrigen Lösung mit 20 mg Mannit. Die Lösung wird sterilfiltriert und unter aseptischen Bedingungen in eine 2 ml Ampulle gefüllt, tiefgekühlt und lyophilisiert. Vor dem Gebrauch wird das Lyophilisat in 1 ml destilliertem Wasser oder 1 ml physiologischer Salzlösung gelöst. Die Lösung wird intramuskulär oder intravenös angewendet. Diese Formulierung kann auch in Doppelkammerspritzampullen abgefüllt werden.

Beispiel 69: Nasenspray

In einer Mischung von 3,5 ml Myglyol 812® und 0,08 g Benzylalkohol werden 500 mg fein gemahlenes (<5,0 μm)

N-(2(R,S)-Benzyl-5,5-dimethyl-4-oxo-hexanoyl)-His-Cha$\overset{c}{-}$Val-n-butylamid

suspendiert. Diese Suspension wird in einen Behälter mit Dosierventil eingefüllt. Es werden 5,0 g Freon

12® unter Druck durch das Ventil in den Behälter abgefüllt. Durch Schütteln wird das "Freon" in der Myglyol-Benzylalkoholmischung gelöst. Dieser Spraybehälter enthält ca. 100 Einzeldosen, die einzeln appliziert werden können.

## Patentansprüche

## Patentansprüche für folgende Vertragsstaaten : BE, CH, DE, FR, GB, IT, LU, NL, SE

**1.** Verbindungen der Formel

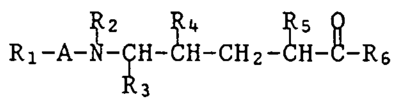

(I),

worin $R_1$ $C_1$-$C_7$-Alkanoyl, Phenoxy-$C_1$-$C_7$-alkanoyl, Naphthoxy-$C_1$-$C_7$-alkanoyl, $\alpha$-Naphthoxy-carboxy-$C_1$-$C_7$-alkanoyl, $\alpha$-Naphthoxy-$C_1$-$C_7$-alkoxycarbonyl-$C_1$-$C_7$-alkanoyl, $\alpha$-Naphthoxy-benzyloxycarbonyl-$C_1$-$C_7$-alkanoyl, $\alpha$-Naphthoxy-carbamoyl-$C_1$-$C_7$-alkanoyl, $\alpha$-Naphthoxy-cyano-$C_1$-$C_7$-alkanoyl, $\alpha$-Naphthoxy-di-$C_1$-$C_7$-alkylamino-$C_1$-$C_7$-alkanoyl, $\alpha$-Naphthoxy-oxo-$C_1$-$C_7$-alkanoyl, Phenyl-, $\alpha$-Naphthyl- oder $\beta$-Naphthyl-$C_1$-$C_7$-alkanoyl, worin $C_1$-$C_7$-Alkanoyl unsubstituiert oder durch Hydroxy, $C_1$-$C_7$-Alkoxy, $C_1$-$C_7$-Alkanoyloxy, Carboxy, $C_1$-$C_7$-Alkoxycarbonyl, Carbamoyl, $C_1$-$C_1$-Alkylcarbamoyl, Di-$C_1$-$C_7$-alkyl-carbamoyl, Cyano, Phosphono, Di-$C_1$-$C_7$-alkoxyphosphoryl, Benzofuranyl und/oder Oxo substituiert sein kann und gegebenenfalls verzweigt ist, über eine Carbonylgruppe -CO- gebundenes 2-Phenyläthyl, 3-Phenyl-2-propyl, 4-Phenyl-3-butyl, 2-$\alpha$-Naphthyläthyl, 3-$\alpha$-Naphthyl-2-propyl oder 4-$\alpha$-Naphthyl-3-butyl, das jeweils in 1-Stellung durch Acyloxy in Form von Aethylaminocarbonyloxy, 2-Benzyloxycarbonylamino-2-methyl-propionyloxy, 2-Amino-2-methyl-propionyloxy oder Acetoacetoxy, durch verestertes Carboxy in Form von Benzyloxycarbonyl, durch substituiertes Carbamoyl in Form von Carboxymethylcarbamoyl, tert-Butoxycarbonylmethylcarbamoyl, 2-Dimethylaminoäthylcarbamoyl, 3-Hydroxy-2-propylcarbamoyl, 2,2-Dimethoxyäthylcarbamoyl oder 5-Amino-5-carboxypentylcarbamoyl oder durch verestertes Phosphono in Form von Hydroxymethoxyphosphoryl substituiert ist, 3-Phenyl-2-äthoxy-hydroxyphosphoryl-propionyl, 3-Phenyl- oder 3-$\alpha$-Naphthyl-2-dimethylaminomethyl-propionyl, 4-Phenyl- oder 4-$\alpha$-Naphthyl-3-benzyloxycarbonyl-butyryl, 2-Benzyl- oder 2-$\alpha$-Naphthylmethyl-4,4-dimethyl-3-oxo-pentanoyl, 2-Benzyl- oder 2-$\alpha$-Naphthylmethyl-5-dimethylamino-pentanoyl, 2-Benzyl- oder 2-$\alpha$-Naphthylmethyl-5-dimethylamino-4-oxo-pentanoyl, 2-Benzyl- oder 2-$\alpha$-Naphthylmethyl-5,5-dimethyl-4-oxo-hexanoyl, $\alpha$,p-Diamino-phenylacetyl, $\alpha$,p-Dibenzyloxycarbonylamino-phenylacetyl, $\alpha$-Pivaloylamino-p-benzyloxycarbonylamino-phenylacetyl, 2-(o,o-Dichloroanilino)-phenylacetyl, 2-(o,o-Dichloro-N-benzylanilino)-phenylacetyl, $\alpha$- oder $\beta$-Naphthylcarbonyl, 1,8-Naphthalindicarbonyl, Pyrrolylcarbonyl, Cyclohepta[b]pyrrolyl-5-carbonyl, Indolylcarbonyl, 1-Benzyl-indolyl-3-carbonyl, 4,5,6,7-Tetrahydroindolyl-2-carbonyl, Chinolylcarbonyl oder Oxamoyl,

A den bivalenten Rest der Aminosäuren Leucin, Norleucin, Phenylalanin, N-Methyl-phenylalanin, $\beta$-Phenylserin, Cyclohexylalanin, Glutamin, Histidin oder N-Methyl-histidin, $R_2$ Wasserstoff, $R_3$ Isobutyl oder Cyclohexylmethyl, $R_4$ Hydroxy, $R_5$ Isopropyl, Cyclohexylmethyl, $\alpha$-Decahydronaphthyl oder Dimethylamino, und

$R_6$ $C_1$-$C_7$-Alkylamino, Di-$C_1$-$C_7$-alkylamino, Hydroxy-$C_1$-$C_7$-alkylamino, 2-Phenoxyäthylamino, 2-(3-Carbamoyl-4-hydroxyphenoxy)-äthylamino, Carboxy-alkylamino oder Amino-carboxy-alkylamino, worin der Carboxyrest nicht in 1-Stellung des Alkylrests, welcher bis zu 10 C-Atome enthält, steht, $C_1$-$C_7$-Alkoxycarbonyl-alkylamino oder ($C_1$-$C_7$-Alkanoylamino-, $C_1$-$C_7$-Alkoxycarbonylamino- oder Benzyloxycarbonylamino-)-$C_1$-$C_7$-alkoxycarbonyl-alkylamino, worin der Carbonylrest nicht in 1-Stellung des Alkylrests, welcher bis zu 10 C-Atome enthält, steht, 4-Tris-(hydroxymethyl)-methylcarbamoyl-n-butylamino, Amino-$C_1$-$C_7$-alkylamino, Di-$C_1$-$C_7$-alkylamino-$C_1$-$C_7$-alkylamino, $C_1$-$C_7$-Alkoxycarbonylamino-$C_1$-$C_7$-alkylamino, Morpholino-$C_1$-$C_7$-alkylamino, Cycloalkyl-$C_1$-$C_7$-alkylamino mit 4-10 C-Atomen, Benzylamino, Pyridyl-$C_1$-$C_7$-alkylamino, Imidazolyl-$C_1$-$C_7$-alkylamino oder 2-(2-[4-Imidazolyl]-äthylamino)-äthylamino darstellt, sowie pharmazeutisch annehmbare Salze von diesen Verbindungen mit salzbildenden Gruppen, mit Ausnahme der Verbindung der Formel I, worin $R_1$ $\alpha$-Naphthoxyacetyl, A den bivalenten Rest der Aminosäure Histidin, $R_2$ Wasserstoff, $R_3$ Isobutyl, $R_4$ Hydroxy, $R_5$ Isopropyl und $R_6$ Cyclohexylmethylamino bedeuten.

2. Verbindungen der in Anspruch 1 gezeigten Formel I, worin $R_1$ Phenoxy-$C_1$-$C_7$-alkanoyl, Naphthoxy-$C_1$-$C_7$-alkanoyl, Phenyl-$C_1$-$C_7$-alkanoyl, worin $C_1$-$C_7$-Alkanoyl unsubstituiert oder substituiert durch $C_1$-$C_7$-Alkanoyloxy, Carboxy, $C_1$-$C_7$-Alkoxycarbonyl, Carbamoyl, $C_1$-$C_7$-Alkylcarbamoyl, Cyano, Di-$C_1$-$C_7$-alkoxyphosphoryl oder $C_1$-$C_7$-Alkyl und Oxo oder Benzofuranyl und Oxo substituiert sein kann und gegebenenfalls verzweigt ist, Naphthyl-$C_1$-$C_7$-alkanoyl, worin $C_1$-$C_7$-Alkanoyl unsubstituiert oder durch $C_1$-$C_7$-Alkanoyloxy, Carboxy, $C_1$-$C_7$-Alkoxycarbonyl, Carbamoyl, Cyano oder $C_1$-$C_7$-Alkyl und Oxo substituiert sein kann und gegebenenfalls verzweigt ist, 2-tert-Butylcarbamoyl-3-$\alpha$-naphthyl-propionyl, 2-Carboxymethylcarbamoyl-3-$\alpha$-naphthyl-propionyl, Indolyl-2-carbonyl oder Cyclohepta[b]pyrrolyl-5-carbonyl, A den bivalenten Rest der Aminosäure L-Histidin, $R_2$ Wasserstoff, $R_3$ Isobutyl oder Cyclohexylmethyl, $R_4$ Hydroxy, $R_5$ Isopropyl oder Cyclohexylmethyl und $R_6$ $C_1$-$C_7$-Alkylamino oder Amino-carboxy-alkylamino, worin die Substituenten nicht in 1-Stellung des Alkylrests stehen, welcher bis zu 7 C-Atome enthält, bedeuten und die die Reste $R_3$ und $R_4$ tragenden C-Atome die S-Konfiguration aufweisen, ferner pharmazeutisch annehmbare Salze von diesen Verbindungen.

3. Verbindungen gemäss Anspruch 1 oder 2 der Formel I, worin $R_1$ 2(S)-Pivaloyloxy-3-phenyl-propionyl, 2(R)- und 2(S)-Dimethoxyphosphoryl-3-phenyl-propionyl, 2(R)- und 2(S)-Benzyl-5,5-dimethyl-4-oxo-hexanoyl, 2(R)- und 2(S)-Benzyl-4,4-dimethyl-3-oxo-pentanoyl, 2(R)-und 2(S)-tert-Butylcarbamoyl-3-$\alpha$-naphthyl-propionyl oder 2(R)-und 2(S)-Aethoxycarbonyl-3-$\alpha$-naphthyl-propionyl, Indolyl-2-carbonyl oder Cyclohepta[b]pyrrolyl-5-carbonyl, A den bivalenten Rest der Aminosäure L-Histidin, $R_2$ Wasserstoff, $R_3$ Cyclohexylmethyl, $R_4$ Hydroxy, $R_5$ Isopropyl und $R_6$ $C_1$-$C_7$-Alkylamino bedeuten und die die Reste $R_3$, $R_4$ und $R_5$ tragenden C-Atome die S-Konfiguration aufweisen, ferner pharmazeutisch annehmbare Salze von diesen Verbindungen.

4. Die Verbindung gemäss Anspruch 1, 2 oder 3 der Formel I, worin $R_1$ 2(S)-Pivaloyloxy-3-phenyl-propionyl, A den bivalenten Rest der Aminosäure L-Histidin, $R_2$ Wasserstoff, $R_3$ Cyclohexylmethyl, $R_4$ Hydroxy, $R_5$ Isopropyl und $R_6$ n-Butylamino bedeuten und die die Reste $R_3$, $R_4$ und $R_5$ tragenden C-Atome die S-Konfiguration aufweisen, und deren pharmazeutisch annehmbare Salze.

5. Verbindungen gemäss Anspruch 1, 2 oder 3 der Formel I, worin $R_1$ 2(R,S)-, 2(R)- oder 2(S)-Dimethoxyphosphoryl-3-phenyl-propionyl, A den bivalenten Rest der Aminosäure L-Histidin, $R_2$ Wasserstoff, $R_3$ Cyclohexylmethyl, $R_4$ Hydroxy, $R_5$ Isopropyl und $R_6$ n-Butylamino bedeuten und die die Reste $R_3$, $R_4$ und $R_5$ tragenden C-Atome die S-Konfiguration aufweisen, und deren pharmazeutisch annehmbare Salze.

6. Verbindungen gemäss Anspruch 1, 2 oder 3 der Formel I, worin $R_1$ 2(R,S)-, 2(R)-oder 2(S)-Benzyl-5,5-dimethyl-4-oxo-hexanoyl, A den bivalenten Rest der Aminosäure L-Histidin, $R_2$ Wasserstoff, $R_3$ Cyclohexylmethyl, $R_4$ Hydroxy, $R_5$ Isopropyl und $R_6$ n-Butylamino bedeuten und die die Reste $R_3$, $R_4$ und $R_5$ tragenden C-Atome die S-Konfiguration aufweisen, und deren pharmazeutisch annehmbare Salze.

7. Die Verbindung gemäss Anspruch 1, 2 oder 3 der Formel I, worin $R_1$ 2(R,S)-Benzyl-4,4-dimethyl-3-oxo-pentanoyl, A den bivalenten Rest der Aminosäure L-Histidin, $R_2$ Wasserstoff, $R_3$ Cyclohexylmethyl, $R_4$ Hydroxy, $R_5$ Isopropyl und $R_6$ n-Butylamino bedeuten und die die Reste $R_3$, $R_4$ und $R_5$ tragenden C-Atome die S-Konfiguration aufweisen, und deren pharmazeutisch annehmbare Salze.

8. Die Verbindung gemäss Anspruch 1, 2 oder 3 der Formel I, worin $R_1$ 2(R,S)-Aethoxycarbonyl-3-$\alpha$-naphthyl-propionyl, A den bivalenten Rest der Aminosäure L-Histidin, $R_2$ Wasserstoff, $R_3$ Cyclohexylmethyl, $R_4$ Hydroxy, $R_5$ Isopropyl und $R_6$ n-Butylamino bedeuten und die die Reste $R_3$, $R_4$ und $R_5$ tragenden C-Atome die S-Konfiguration aufweisen, und deren pharmazeutisch annehmbare Salze.

9. Die Verbindung gemäss Anspruch 1, 2 oder 3 der Formel I, worin $R_1$ Cyclohepta[b]pyrrolyl-5-carbonyl, A den bivalenten Rest der Aminosäure L-Histidin, $R_2$ Wasserstoff, $R_3$ Cyclohexylmethyl, $R_4$ Hydroxy, $R_5$ Isopropyl und $R_6$ n-Butylamino bedeuten und die die Reste $R_3$, $R_4$ und $R_5$ tragenden C-Atome die S-Konfiguration aufweisen, und deren pharmazeutisch annehmbare Salze.

10. Die Verbindung gemäss Anspruch 1, 2 oder 3 der Formel I, worin $R_1$ 2(S)-Pivaloyloxy-3-phenyl-propionyl, A den bivalenten Rest der Aminosäure L-Histidin, $R_2$ Wasserstoff, $R_3$ Isobutyl, $R_4$ Hydroxy, $R_5$ Cyclohexylmethyl und $R_6$ n-Butylamino bedeuten und die die Reste $R_3$ und $R_4$ tragenden C-Atome die S-Konfiguration aufweisen, und deren pharmazeutisch annehmbare Salze.

**11.** Die Verbindungen der in Anspruch 1 gezeigten Formel I mit den Bezeichnungen

N-(N-Benzyl-indolyl-2-carbonyl)-His-Leu$\overset{C}{-}$Val-methylamid,

N-(3(R,S)-Benzyloxycarbonyl-4-α-naphthylbutyryl)-His-Leu$\overset{C}{-}$Val-methylamid,

N-Benzyloxycarbonyl-(p-benzyloxycarbonylamino-Phe)-His-Leu$\overset{C}{-}$Val-n-butylamid,

N-Pivaloyl-(p-benzyloxycarbonylamino-Phe)-His-Leu$\overset{C}{-}$Val-n-butylamid,

N-(Indolyl-2-carbonyl)-His-Leu$\overset{C}{-}$Val-4-[tris-(tert-butyldimethylsilyloxymethyl)-methylcarbamoyl]-butylamid,

N-(2-Aethylaminocarbonyloxy-4-phenyl-butyryl)-His-Cha$\overset{C}{-}$Val-n-butylamid,

und deren pharmazeutisch annehmbare Salze.

**12.** Pharmazeutische Präparate enthaltend Verbindungen gemäss Anspruch 1, 2, 3 oder 11 der Formel I oder pharmazeutisch verwendbare Salze von diesen Verbindungen mit salzbildenden Gruppen zusammen mit einem pharmazeutischen Trägermaterial.

**13.** Verwendung von Verbindungen gemäss Anspruch 1, 2, 3 oder 11 der Formel I oder von pharmazeutisch verwendbaren Salzen von Verbindungen der Formel I zur Herstellung eines Arzneimittels zur Behandlung von Bluthochdruck und Herzinsuffizienz.

**14.** Verfahren Zur Herstellung von Verbindungen der Formel I, worin die Substituenten die in Anspruch 1, 2, 3 oder 11 genannten Bedeutungen haben, dadurch gekennzeichnet, dass man

a) ein Fragment einer Verbindung der Formel I mit einer endständigen Carboxygruppe oder ein reaktionsfähiges Säurederivat dieses Fragments mit einem zur Verbindung der Formel I komplementären Fragment mit einer freien Aminogruppe oder einem reaktionsfähigen Derivat davon mit aktivierter Aminogruppe, wobei in den Reaktionskomponenten vorhandene freie funktionelle Gruppen mit Ausnahme der an der Reaktion teilnehmenden Gruppen gegebenenfalls in geschützter Form vorliegen, unter Bildung einer Amidbindung kondensiert, oder

b) die Ketogruppe in einer Verbindung der Formel

$$R_1 - A - \overset{R_2}{\overset{|}{N}} - \underset{R_3}{\underset{|}{CH}} - \overset{O}{\overset{\|}{C}} - CH_2 - \overset{R_5}{\overset{|}{CH}} - \overset{O}{\overset{\|}{C}} - R_6 \qquad (II),$$

worin die Substituenten die genannten Bedeutungen haben und freie funktionelle Gruppen mit Ausnahme der an der Reaktion teilnehmenden Ketogruppe gegebenenfalls in geschützter Form vorliegen, durch Umsetzung mit einem geeigneten Reduktionsmittel zu einer Hydroxygruppe reduziert, oder

c) eine Aldehyd-Verbindung der Formel

$$R_1 - A - \overset{R_2}{\overset{|}{N}} - \underset{R_3}{\underset{|}{CH}} - \overset{O}{\overset{\|}{C}} - H \qquad (III),$$

worin die Substituenten die genannten Bedeutungen haben und freie funktionelle Gruppen mit Ausnahme der Aldehydgruppe gegebenenfalls in geschützter Form vorliegen, mit einer Organometallverbindung der Formel

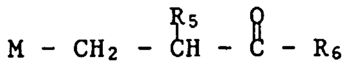 (IV),

worin die Substituenten die genannten Bedeutungen haben und M ein Metallradikal bedeutet, umsetzt und das gebildete Additionsprodukt hydrolysiert, oder

d) in einer Verbindung der Formel

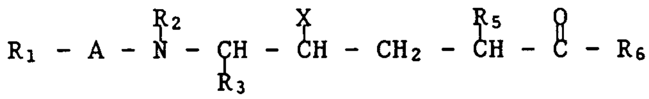 (V),

worin X eine nukleofuge Abgangsgruppe ist, die übrigen Substituenten die oben genannten Bedeutungen haben und freie funktionelle Gruppen gegebenenfalls in geschützter Form vorliegen, den Substituenten X mit einem den Substituenten $R_4$ in nukleophiler Form einführenden Reagens gegen $R_4$ austauscht, oder

e) in einer Verbindung der Formel

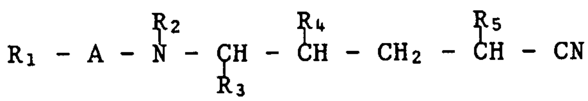 (VI),

worin die Substituenten die genannten Bedeutungen haben und vorhandene funktionelle Gruppen gegebenenfalls in geschützter Form vorliegen, die Cyanogruppe in eine N-substituierte Carboxamidogruppe $-(C=O)R_6$ überführt, oder

f) ein Epoxid der Formel

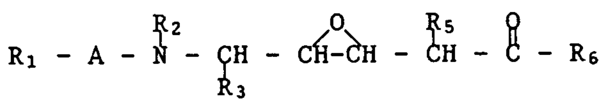 (VII),

worin die Substituenten die genannten Bedeutungen haben und freie funktionelle Gruppen gegebenenfalls in geschützter Form vorliegen, mit einem regio-selektiven Reduktionsmittel zum entsprechenden Alkohol reduziert, und gewünschtenfalls

g) in einer erhältlichen Verbindung vorhandene Schutzgruppen abspaltet und/oder gewünschtenfalls nach Ausführung eines der vorstehend genannten Verfahren a) - f) oder eines beliebigen anderen Verfahrens zur Herstellung einer Verbindung der Formel I eine erhältliche Verbindung der Formel I mit einer salzbildenden Gruppe in ihr Salz oder ein erhältliches Salz in die freie Verbindung oder in ein anderes Salz überführt und/oder gegebenenfalls erhältliche Isomerengemische auftrennt und/oder in einer erhältlichen Verbindung der Formel I die Konfiguration eines chiralen Kohlenstoffatoms umkehrt und/oder eine erfindungsgemässe Verbindung der Formel I in eine andere erfindungsgemässe Verbindung der Formel I umwandelt.

**15.** Verbindungen der Formel

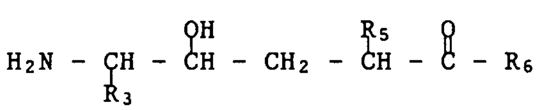 (VIII),

worin $R_3$ Cyclohexylmethyl bedeutet und $R_5$ und $R_6$ die in Anspruch 1, 2 oder 3 genannten Bedeutungen haben, und Salze davon.

**16.** Verfahren zur Herstellung von Verbindungen der Formel VIII gemäss Anspruch 15, worin die Substituenten die in Anspruch 15 genannten Bedeutungen haben, dadurch gekennzeichnet, dass man eine Verbindung der Formel

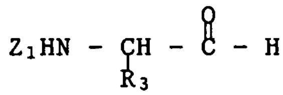

(IX),

worin $R_3$ die genannte Bedeutung hat und $Z_1$ eine Aminoschutzgruppe ist, mit einer Schwefel-Ylid-Verbindung in ein Epoxid der Formel

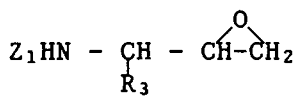

(X),

worin $R_3$ und $Z_1$ die genannten Bedeutungen haben, umwandelt, die erhältliche Verbindung (X) gegebenenfalls nach Trennung der Isomeren mit einem eine nukleofuge Abgangsgruppe X einführenden Reagens umsetzt, die erhältliche Verbindung der Formel

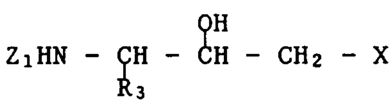

(XI),

worin $R_3$ und $Z_1$ die genannten Bedeutungen haben und X eine nukleofuge Abgangsgruppe ist, mit einer Carbonylverbindung der Formel

$R_a - (C = O) - R_b$ (XII),

worin jeder der Reste $R_a$ und $R_b$ unabhängig voneinander Wasserstoff, Niederalkyl, Aryl oder Arylniederalkyl, $R_a$ und $R_b$ zusammen gegebenenfalls überbrücktes Alkyliden mit 4 bis 12 Kohlenstoffatomen darstellen, oder einem reaktionsfähigen Derivat dieser Carbonylverbindung, in Gegenwart eines sauren Reagenses umsetzt, die erhältliche Verbindung der Formel

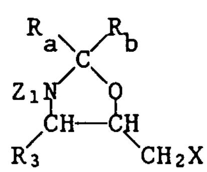

(XIII),

worin die Substituenten die genannten Bedeutungen haben, mit einem Carbonsäureestersalz der Formel

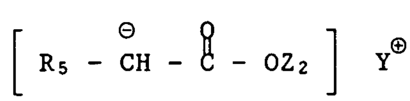

(XIV),

worin $R_5$ die unter Formel I genannte Bedeutung hat, $Z_2$ eine Carboxyschutzgruppe und $Y^{\oplus}$ ein Kation, z.B. ein Alkalimetall-, z.B. das Natrium- oder bevorzugt das Lithiumion, ist, umsetzt, und in einer erhältlichen Verbindung der Formel

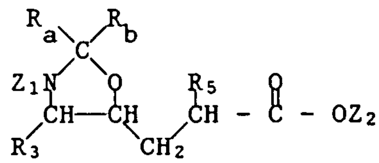

(XV),

worin die Sustituenten die genannten Bedeutungen haben, die Carboxyschutzgruppe $Z_2$ abspaltet und/oder ein erhältliches Isomerengemisch in die einzelnen Isomere auftrennt, die erhältliche Verbindung mit einer freien Carboxygruppe ($Z_2 = H$) mit einem Amin $R_6$-H amidiert, und in einer erhältlichen Verbindung der Formel

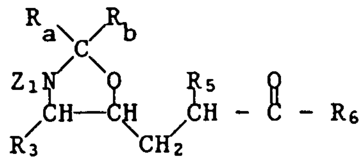

(XVI),

worin die Substituenten die genannten Bedeutungen haben, mit einem geeigneten Solvolysereagens den Ring öffnet und gegebenenfalls die Schutzgruppe $Z_1$ abspaltet.

**Patentansprüche für folgenden Vertragsstaat : AT**

**1.** Verfahren zur Herstellung einer Verbindung der Formel

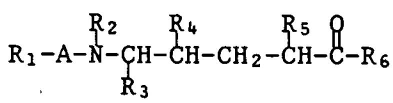

(I),

worin $R_1$ $C_1$-$C_7$-Alkanoyl, Phenoxy-$C_1$-$C_7$-alkanoyl, Naphthoxy-$C_1$-$C_7$-alkanoyl, $\alpha$-Naphthoxy-carboxy-$C_1$-$C_7$-alkanoyl, $\alpha$-Naphthoxy-$C_1$-$C_7$-alkoxycarbonyl-$C_1$-$C_7$-alkanoyl, $\alpha$-Naphthoxy-benzyloxycarbonyl-$C_1$-$C_7$-alkanoyl, $\alpha$-Naphthoxy-carbamoyl-$C_1$-$C_7$-alkanoyl, $\alpha$-Naphthoxy-cyano-$C_1$-$C_7$-alkanoyl, $\alpha$-Naphthoxy-di-$C_1$-$C_7$-alkylamino-$C_1$-$C_7$-alkanoyl, $\alpha$-Naphthoxy-oxo-$C_1$-$C_7$-alkanoyl, Phenyl-, $\alpha$-Naphthyl- oder $\beta$-Naphthyl-$C_1$-$C_7$-alkanoyl, worin $C_1$-$C_7$-Alkanoyl unsubstituiert oder durch Hydroxy, $C_1$-$C_7$-Alkoxy, $C_1$-$C_7$-Alkanoyloxy, Carboxy, $C_1$-$C_7$-Alkoxycarbonyl, Carbamoyl, $C_1$-$C_7$-Alkylcarbamoyl, Di-$C_1$-$C_7$-alkyl-carbamoyl, Cyano, Phosphono, Di-$C_1$-$C_7$-alkoxy-phosphoryl, Benzofuranyl und/oder Oxo substituiert sein kann und gegebenenfalls verzweigt ist, über eine Carbonylgruppe -CO- gebundenes 2-Phenyläthyl, 3-Phenyl-2-propyl, 4-Phenyl-3-butyl, 2-$\alpha$-Naphthyläthyl, 3-$\alpha$-Naphthyl-2-propyl oder 4-$\alpha$-Naphthyl-3-butyl, das jeweils in 1-Stellung durch Acyloxy in Form von Aethylaminocarbonyloxy, 2-Benzyloxycarbonylamino-2-methyl-propionyloxy, 2-Amino-2-methyl-propionyloxy oder Acetoacetoxy, durch verestertes Carboxy in Form von Benzyloxycarbonyl, durch substituiertes Carbamoyl in Form von Carboxymethylcarbamoyl, tert-Butoxycarbonylmethylcarbamoyl, 2-Dimethylaminoäthylcarbamoyl, 3-Hydroxy-2-propylcarbamoyl, 2,2-Dimethoxyäthylcarbamoyl oder 5-Amino-5-carboxypentylcarbamoyl oder durch verestertes Phosphono in Form von Hydroxymethoxyphosphoryl substituiert ist, 3-Phenyl-2-äthoxy-hydroxyphosphoryl-propionyl, 3-Phenyl- oder 3-$\alpha$-Naphthyl-2-dimethylaminomethyl-propionyl, 4-Phenyl- oder 4-$\alpha$-Naphthyl-3-benzyloxycarbonyl-butyryl, 2-Benzyl- oder 2-$\alpha$-Naphthylmethyl-4,4-dimethyl-3-oxo-pentanoyl, 2-Benzyl- oder 2-$\alpha$-Naphthylmethyl-5-dimethylaminopentanoyl, 2-Benzyl-oder 2-$\alpha$-Naphthylmethyl-5-dimethylamino-4-oxo-pentanoyl, 2-Benzyl- oder 2-$\alpha$-Naphthylmethyl-5,5-dimethyl-4-oxo-hexanoyl, $\alpha$,p-Diamino-phenylacetyl, $\alpha$,p-Dibenzyloxycarbonylamino-phenylacetyl, $\alpha$-Pivaloylamino-p-benzyloxycarbonylamino-phenylacetyl, 2-(o,o-Dichloroanilino)-phenylacetyl, 2-(o,o-Dichloro-N-benzylanilino)-phenylacetyl, $\alpha$- oder $\beta$-Naphthylcarbonyl, 1,8-Naphthalindicarbonyl, Pyrrolylcarbonyl, Cyclohepta[b]pyrrolyl-5-carbonyl, Indolylcarbonyl, 1-Benzyl-indolyl-3-carbonyl, 4,5,6,7-Tetrahydroindolyl-2-carbonyl, Chinolylcarbonyl oder Oxamoyl,

A den bivalenten Rest der Aminosäuren Leucin, Norleucin, Phenylalanin, N-Methyl-phenylalanin, $\beta$-

Phenylserin, Cyclohexylalanin, Glutamin, Histidin oder N-Methyl-histidin, $R_2$ Wasserstoff, $R_3$ Isobutyl oder Cyclohexylmethyl, $R_4$ Hydroxy, $R_5$ Isopropyl, Cyclohexylmethyl, $\alpha$-Decahydronaphthyl oder Dimethylamino, und

$R_6$ $C_1$-$C_7$-Alkylamino, Di-$C_1$-$C_7$-alkylamino, Hydroxy-$C_1$-$C_7$-alkylamino, 2-Phenoxyäthylamino, 2-(3-Carbamoyl-4-hydroxyphenoxy)-äthylamino. Carboxy-alkylamino oder Amino-carboxy-alkylamino, worin der Carboxyrest nicht in 1-Stellung des Alkylrests, welcher bis zu 10 C-Atome enthält, steht, $C_1$-$C_7$-Alkoxycarbonyl-alkylamino oder ($C_1$-$C_7$-Alkanoylamino-, $C_1$-$C_7$-Alkoxycarbonylamino- oder Benzyloxycarbonylamino-)-$C_1$-$C_7$-alkoxycarbonyl-alkylamino, worin der Carbonylrest nicht in 1-Stellung des Alkylrests, welcher bis zu 10 C-Atome enthält, steht, 4-Tris-(hydroxymethyl)-methylcarbamoyl-n-butylamino. Amino-$C_1$-$C_7$-alkylamino, Di-$C_1$-$C_7$-alkylamino-$C_1$-$C_7$-alkylamino, $C_1$-$C_7$-Alkoxycarbonylamino-$C_1$-$C_7$-alkylamino, Morpholino-$C_1$-$C_7$-alkylamino, Cycloalkyl-$C_1$-$C_7$-alkylamino mit 4-10 C-Atomen, Benzylamino, Pyridyl-$C_1$-$C_7$-alkylamino, Imidazolyl-$C_1$-$C_7$-alkylamino oder 2-(2-[4-Imidazolyl]-äthylamino)-äthylamino darstellt, ferner von Salzen einer solchen Verbindung mit salzbildenden Gruppen,

mit Ausnahme der Verbindung der Formel I, worin $R_1$ $\alpha$-Naphthoxyacetyl, A den bivalenten Rest der Aminosäure Histidin, $R_2$ Wasserstoff, $R_3$ Isobutyl, $R_4$ Hydroxy, $R_5$ Isopropyl und $R_6$ Cyclohexylmethylamino bedeuten, dadurch gekennzeichnet, dass man

a) ein Fragment einer Verbindung der Formel I mit einer endständigen Carboxygruppe oder ein reaktionsfähiges Säurederivat dieses Fragments mit einem zur Verbindung der Formel I komplementären Fragment mit einer freien Aminogruppe oder einem reaktionsfähigen Derivat davon mit aktivierter Aminogruppe, wobei in den Reaktionskomponenten vorhandene freie funktionelle Gruppen mit Ausnahme der an der Reaktion teilnehmenden Gruppen gegebenenfalls in geschützter Form vorliegen, unter Bildung einer Amidbindung kondensiert, oder

b) die Ketogruppe in einer Verbindung der Formel

$$R_1 - A - \overset{R_2}{\overset{|}{N}} - \underset{R_3}{\underset{|}{CH}} - \overset{O}{\overset{\|}{C}} - CH_2 - \overset{R_5}{\overset{|}{CH}} - \overset{O}{\overset{\|}{C}} - R_6 \qquad (II),$$

worin die Substituenten die genannten Bedeutungen haben und freie funktionelle Gruppen mit Ausnahme der an der Reaktion teilnehmenden Ketogruppe gegebenenfalls in geschützter Form vorliegen, durch Umsetzung mit einem geeigneten Reduktionsmittel zu einer Hydroxygruppe reduziert, oder

c) eine Aldehyd-Verbindung der Formel

$$R_1 - A - \overset{R_2}{\overset{|}{N}} - \underset{R_3}{\underset{|}{CH}} - \overset{O}{\overset{\|}{C}} - H \qquad (III),$$

worin die Substituenten die genannten Bedeutungen haben und freie funktionelle Gruppen mit Ausnahme der Aldehydgruppe gegebenenfalls in geschützter Form vorliegen, mit einer Organometallverbindung der Formel

$$M - CH_2 - \overset{R_5}{\overset{|}{CH}} - \overset{O}{\overset{\|}{C}} - R_6 \qquad (IV),$$

worin die Substituenten die genannten Bedeutungen haben und M ein Metallradikal bedeutet, umsetzt und das gebildete Additionsprodukt hydrolysiert, oder

d) in einer Verbindung der Formel

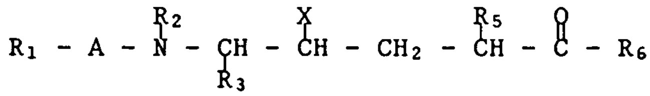 (V),

worin X eine nukleofuge Abgangsgruppe ist, die übrigen Substituenten die oben genannten Bedeutungen haben und freie funktionelle Gruppen gegebenenfalls in geschützter Form vorliegen, den Substituenten X mit einem den Substituenten $R_4$ in nukleophiler Form einführenden Reagens gegen $R_4$ austauscht, oder

e) in einer Verbindung der Formel

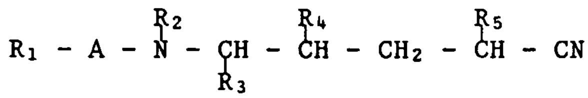 (VI),

worin die Substituenten die genannten Bedeutungen haben und vorhandene funktionelle Gruppen gegebenenfalls in geschützter Form vorliegen, die Cyanogruppe in eine N-substituierte Carboxamidogruppe $-(C=O)R_6$ überführt, oder

f) ein Epoxid der Formel

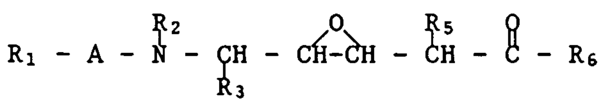 (VII),

worin die Substituenten die genannten Bedeutungen haben und freie funktionelle Gruppen gegebenenfalls in geschützter Form vorliegen, mit einem regio-selektiven Reduktionsmittel zum entsprechenden Alkohol reduziert, und gewünschtenfalls

g) in einer erhältlichen Verbindung vorhandene Schutzgruppen abspaltet und/oder gewünschtenfalls nach Ausführung eines der vorstehend genannten Verfahren a) - f) oder eines beliebigen anderen Verfahrens zur Herstellung einer Verbindung der Formel I eine erhältliche Verbindung der Formel I mit einer salzbildenden Gruppe in ihr Salz oder ein erhältliches Salz in die freie Verbindung oder in ein anderes Salz überführt und/oder gegebenenfalls erhältliche Isomerengemische auftrennt und/oder in einer erhältlichen Verbindung der Formel I die Konfiguration eines chiralen Kohlenstoffatoms umkehrt und/oder eine erfindungsgemässe Verbindung der Formel I in eine andere erfindungsgemässe Verbindung der Formel I umwandelt.

**2.** Verfahren zur Herstellung einer Verbindung der in Anspruch 1 gezeigten Formel I, worin $R_1$ Phenoxy-$C_1$-$C_7$-alkanoyl, Naphthoxy-$C_1$-$C_7$-alkanoyl, Phenyl-$C_1$-$C_7$-alkanoyl, worin $C_1$-$C_7$-Alkanoyl unsubstituiert oder substituiert durch $C_1$-$C_7$-Alkanoyloxy, Carboxy, $C_1$-$C_7$-Alkoxycarbonyl, Carbamoyl, $C_1$-$C_7$-Alkylcarbamoyl, Cyano, Di-$C_1$-$C_7$-alkoxyphosphoryl oder $C_1$-$C_7$-Alkyl und Oxo oder Benzofuranyl und Oxo substituiert sein kann und gegebenenfalls verzweigt ist, Naphthyl-$C_1$-$C_7$-alkanoyl, worin $C_1$-$C_7$-Alkanoyl unsubstituiert oder durch $C_1$-$C_7$-Alkanoyloxy, Carboxy, $C_1$-$C_7$-Alkoxycarbonyl, Carbamoyl, Cyano oder $C_1$-$C_7$-Alkyl und Oxo substituiert sein kann und gegebenenfalls verzweigt ist, 2-tert-Butylcarbamoyl-3-$\alpha$-naphthyl-propionyl, 2-Carboxymethylcarbamoyl-3-$\alpha$-naphthyl-propionyl, Indolyl-2-carbonyl oder Cyclohepta[b]pyrrolyl-5-carbonyl, A den bivalenten Rest der Aminosäure L-Histidin, $R_2$ Wasserstoff, $R_3$ Isobutyl oder Cyclohexylmethyl, $R_4$ Hydroxy, $R_5$ Isopropyl oder Cyclohexylmethyl und $R_6$ $C_1$-$C_7$-Alkylamino oder Amino-carboxy-alkylamino, worin die Substituenten nicht in 1-Stellung des Alkylrests stehen, welcher bis zu 7 C-Atome enthält, bedeuten und die die Reste $R_3$ und $R_4$ tragenden C-Atome die S-Konfiguration aufweisen, ferner von Salzen einer solchen Verbindung mit salzbildenden Gruppen, dadurch gekennzeichnet, dass man

a) ein Fragment einer Verbindung der Formel I mit einer endständigen Carboxygruppe oder ein reaktionsfähiges Säurederivat dieses Fragments mit einem zur Verbindung der Formel I komplementären Fragment mit einer freien Aminogruppe oder einem reaktionsfähigen Derivat davon mit aktivierter Aminogruppe, wobei in den Reaktionskomponenten vorhandene freie funktionelle Gruppen mit Ausnahme der an der Reaktion teilnehmenden Gruppen gegebenenfalls in geschützter Form

vorliegen, unter Bildung einer Amidbindung kondensiert, oder

b) die Ketogruppe in einer Verbindung der Formel

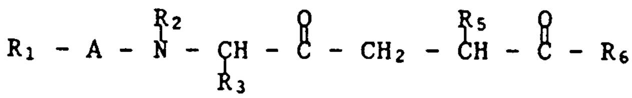 (II),

worin die Substituenten die genannten Bedeutungen haben und freie funktionelle Gruppen mit Ausnahme der an der Reaktion teilnehmenden Ketogruppe gegebenenfalls in geschützter Form vorliegen, durch Umsetzung mit einem geeigneten Reduktionsmittel zu einer Hydroxygruppe reduziert, oder

c) eine Aldehyd-Verbindung der Formel

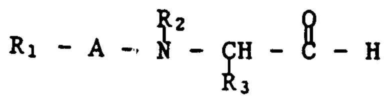 (III),

worin die Substituenten die genannten Bedeutungen haben und freie funktionelle Gruppen mit Ausnahme der Aldehydgruppe gegebenenfalls in geschützter Form vorliegen, mit einer Organometallverbindung der Formel

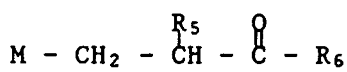 (IV),

worin die Substituenten die genannten Bedeutungen haben und M ein Metallradikal bedeutet, umsetzt und das gebildete Additionsprodukt hydrolysiert, oder

d) in einer Verbindung der Formel

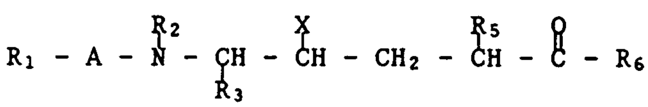 (V),

worin X eine nukleofuge Abgangsgruppe ist, die übrigen Substituenten die oben genannten Bedeutungen haben und freie funktionelle Gruppen gegebenenfalls in geschützter Form vorliegen, den Substituenten X mit einem den Substituenten $R_4$ in nukleophiler Form einführenden Reagens gegen $R_4$ austauscht, oder

e) in einer Verbindung der Formel

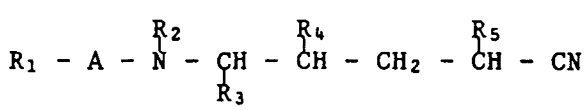 (VI),

worin die Substituenten die genannten Bedeutungen haben und vorhandene funktionelle Gruppen gegebenenfalls in geschützter Form vorliegen, die Cyanogruppe in eine N-substituierte Carboxamidogruppe $-(C=O)R_6$ überführt, oder

f) ein Epoxid der Formel

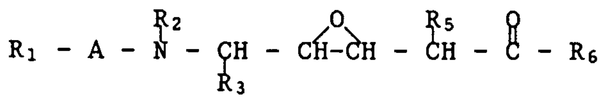 (VII),

worin die Substituenten die genannten Bedeutungen haben und freie funktionelle Gruppen gegebenenfalls in geschützter Form vorliegen, mit einem regio-selektiven Reduktionsmittel zum entsprechenden Alkohol reduziert, und gewünschtenfalls
g) in einer erhältlichen Verbindung vorhandene Schutzgruppen abspaltet und/oder gewünschtenfalls nach Ausführung eines der vorstehend genannten Verfahren a) - f) oder eines beliebigen anderen Verfahrens zur Herstellung einer Verbindung der Formel I eine erhältliche Verbindung der Formel I mit einer salzbildenden Gruppe in ihr Salz oder ein erhältliches Salz in die freie Verbindung oder in ein anderes Salz überführt und/oder gegebenenfalls erhältliche Isomerengemische auftrennt und/oder in einer erhältlichen Verbindung der Formel I die Konfiguration eines chiralen Kohlenstoffatoms umkehrt und/oder eine erfindungsgemässe Verbindung der Formel I in eine andere erfindungsgemässe Verbindung der Formel I umwandelt.

**3.** Verfahren zur Herstellung einer Verbindung der in Anspruch 1 gezeigten Formel I, worin
a) $R_1$ N-Benzyl-indolyl-2-carbonyl, A den bivalenten Rest der Aminosäure Histidin, $R_2$ Wasserstoff, $R_3$ Isobutyl, $R_4$ Hydroxy, $R_5$ Isopropyl und $R_6$ Methylamino bedeuten, oder
b) $R_1$ 3-(R,S)-Benzyloxycarbonyl-4-α-naphthylbutyryl, A den bivalenten Rest der Aminosäure Histidin, $R_2$ Wasserstoff, $R_3$ Isobutyl, $R_4$ Hydroxy, $R_5$ Isopropyl und $R_6$ Methylamino bedeuten, oder
c) $R_1$ N-Benzyloxycarbonyl-(p-benzyloxycarbonylamino-Phe), A den bivalenten Rest der Aminosäure Histidin, $R_2$ Wasserstoff, $R_3$ Isobutyl, $R_4$ Hydroxy, $R_5$ Isopropyl und $R_6$ n-Butylamino bedeuten, oder
d) $R_1$ N-Pivaloyl-(p-benzyloxycarbonylamino-Phe), A den bivalenten Rest der Aminosäure Histidin, $R_2$ Wasserstoff, $R_3$ Isobutyl, $R_4$ Hydroxy, $R_5$ Isopropyl und $R_6$ n-Butylamino bedeuten, oder
e) $R_1$ Indolyl-2-carbonyl, A den bivalenten Rest der Aminosäure Histidin, $R_2$ Wasserstoff, $R_3$ Isobutyl, $R_4$ Hydroxy, $R_5$ Isopropyl und $R_6$ 4-[tris-tert-butyl-dimethylsilyloxymethyl)-methylcarbamoyl]butylamino bedeuten, oder
f) $R_1$ 2-Aethylaminocarbonyloxy-4-phenyl-butyryl, A den bivalenten Rest der Aminosäure Histidin, $R_2$ Wasserstoff, $R_3$ Cyclohexylmethyl, $R_4$ Hydroxy, $R_5$ Isopropyl und $R_6$ n-Butylamino bedeuten, ferner von Salzen einer solchen Verbindung mit salzbildenden Gruppen, dadurch gekennzeichnet, dass man

a) ein Fragment einer Verbindung der Formel I mit einer endständigen Carboxygruppe oder ein reaktionsfähiges Säurederivat dieses Fragments mit einem zur Verbindung der Formel I komplementären Fragment mit einer freien Aminogruppe oder einem reaktionsfähigen Derivat davon mit aktivierter Aminogruppe, wobei in den Reaktionskomponenten vorhandene freie funktionelle Gruppen mit Ausnahme der an der Reaktion teilnehmenden Gruppen gegebenenfalls in geschützter Form vorliegen, unter Bildung einer Amidbindung kondensiert, oder
b) die Ketogruppe in einer Verbindung der Formel

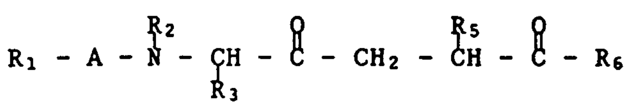 (II),

worin die Substituenten die genannten Bedeutungen haben und freie funktionelle Gruppen mit Ausnahme der an der Reaktion teilnehmenden Ketogruppe gegebenenfalls in geschützter Form vorliegen, durch Umsetzung mit einem geeigneten Reduktionsmittel zu einer Hydroxygruppe reduziert, oder
c) eine Aldehyd-Verbindung der Formel

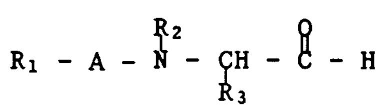 (III),

worin die Substituenten die genannten Bedeutungen haben und freie funktionelle Gruppen mit Ausnahme der Aldehydgruppe gegebenenfalls in geschützter Form vorliegen, mit einer Organometallverbindung der Formel

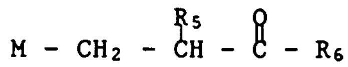 (IV),

worin die Substituenten die genannten Bedeutungen haben und M ein Metallradikal bedeutet, umsetzt und das gebildete Additionsprodukt hydrolysiert, oder
d) in einer Verbindung der Formel

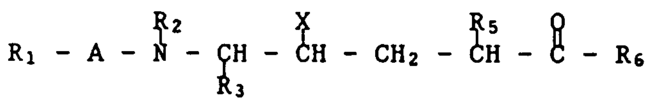 (V),

worin X eine nukleofuge Abgangsgruppe ist, die übrigen Substituenten die oben genannten Bedeutungen haben und freie funktionelle Gruppen gegebenenfalls in geschützter Form vorliegen, den Substituenten X mit einem den Substituenten $R_4$ in nukleophiler Form einführenden Reagens gegen $R_4$ austauscht, oder
e) in einer Verbindung der Formel

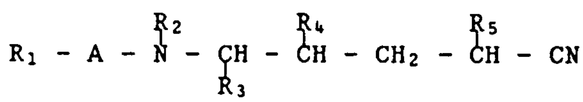 (VI),

worin die Substituenten die genannten Bedeutungen haben und vorhandene funktionelle Gruppen gegebenenfalls in geschützter Form vorliegen, die Cyanogruppe in eine N-substituierte Carboxamidogruppe $-(C=O)R_6$ überführt, oder
f) ein Epoxid der Formel

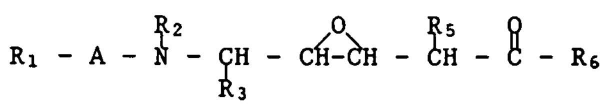 (VII),

worin die Substituenten die genannten Bedeutungen haben und freie funktionelle Gruppen gegebenenfalls in geschützter Form vorliegen, mit einem regio-selektiven Reduktionsmittel zum entsprechenden Alkohol reduziert, und gewünschtenfalls
g) in einer erhältlichen Verbindung vorhandene Schutzgruppen abspaltet und/oder gewünschtenfalls nach Ausführung eines der vorstehend genannten Verfahren a) - f) oder eines beliebigen anderen Verfahrens zur Herstellung einer Verbindung der Formel I eine erhältliche Verbindung der Formel I mit einer salzbildenden Gruppe in ihr Salz oder ein erhältliches Salz in die freie Verbindung oder in ein anderes Salz überführt und/oder gegebenenfalls erhältliche Isomerengemische auftrennt und/oder in einer erhältlichen Verbindung der Formel I die Konfiguration eines chiralen Kohlenstoffatoms umkehrt und/oder eine erfindungsgemässe Verbindung der Formel I in eine andere erfindungsgemässe Verbindung der Formel I umwandelt.

**4.** Verfahren zur Herstellung einer Verbindung der Formel I gemäss einem der Ansprüche 1 oder 2, dadurch gekennzeichnet, dass man ein Fragment einer Verbindung der Formel I mit einer endständigen Carboxygruppe oder ein reaktionsfähiges Säurederivat dieses Fragments mit einem zur Verbindung der Formel I komplementären Fragment mit einer freien Aminogruppe oder einem reaktionsfähigen Derivat davon mit aktivierter Aminogruppe, wobei in den Reaktionskomponenten vorhandene freie

funktionelle Gruppen mit Ausnahme der an der Reaktion teilnehmenden Gruppen gegebenenfalls in geschützter Form vorliegen, unter Bildung einer Amidbindung kondensiert und gewünschtenfalls in einer erhältlichen Verbindung vorhandene Schutzgruppen durch Behandlung mit Säure oder mit Base oder durch Hydrogenolyse abspaltet und/oder eine erhältliche Verbindung der Formel I mit einer salzbildenden Gruppe in ihr Salz oder ein erhältliches salz in die freie Verbindung oder in ein anderes Salz überführt und/oder gegebenenfalls erhältliche Isomerengemische auftrennt und/oder eine erfindungsgemässe Verbindung der Formel I in eine andere erfindungsgemässe Verbindung der Formel I umwandelt.

**5.** Verfahren zur Herstellung einer Verbindung der Formel I gemäss Anspruch 3, dadurch gekennzeichnet, dass man ein Fragment einer Verbindung der Formel I mit einer endständigen Carboxygruppe oder ein reaktionsfähiges Säurederivat dieses Fragments mit einem zur Verbindung der Formel I komplementären Fragment mit einer freien Aminogruppe oder einem reaktionsfähigen Derivat davon mit aktivierter Aminogruppe, wobei in den Reaktionskomponenten vorhandene freie funktionelle Gruppen mit Ausnahme der an der Reaktion teilnehmenden Gruppen gegebenenfalls in geschützter Form vorliegen, unter Bildung einer Amidbindung kondensiert und gewünschtenfalls in einer erhältlichen Verbindung vorhandene Schutzgruppen durch Behandlung mit Säure oder mit Base oder durch Hydrogenolyse abspaltet und/oder eine erhältliche Verbindung der Formel I mit einer salzbildenden Gruppe in ihr Salz oder ein erhältliches Salz in die freie Verbindung oder in ein anderes Salz überführt und/oder gegebenenfalls erhältliche Isomerengemische auftrennt und/oder eine erfindungsgemässe Verbindung der Formel I in eine andere erfindungsgemässe Verbindung der Formel I umwandelt.

**6.** Verfahren zur Herstellung einer Verbindung der Formel I gemäss einem der Ansprüche 1 oder 2, dadurch gekennzeichnet, dass man ein Fragment einer Verbindung der Formel I mit einer endständigen Carboxygruppe oder einen davon abgeleiteten aktivierten Ester, ein davon abgeleitetes reaktionsfähiges Anhydrid oder ein davon abgeleitetes reaktionsfähiges cyclisches Amid mit einem zur Verbindung der Formel I komplementären Fragment mit einer freien Aminogruppe, wobei in den Reaktionskomponenten vorhandene freie funktionelle Gruppen mit Ausnahme der an der Reaktion teilnehmenden Gruppen gegebenenfalls in geschützter Form vorliegen, in Gegenwart eines Kondensationsmittels ausgewählt aus einem Carbodiimid, Carbonyldiimidazol, 1,2-Oxazoliumverbindungen, einem 1,2-Dihydrochinolin oder einem aktivierten Phosphat, und gewünschtenfalls in Gegenwart einer organischen Base oder von Alkalimetallcarbonaten in einem inerten, polaren, aprotischen Lösungsmittel in einem Temperaturbereich von -40 °C bis +100 °C kondensiert, wobei aktivierte Ester vom Amino- oder Amidoester-Typ gewünschtenfalls in situ durch Zugabe einer N-Hydroxyamino- oder N-Hydroxyamido-Verbindung gebildet werden, und gewünschtenfalls in einer erhältlichen Verbindung vorhandene Schutzgruppen durch Behandlung mit Säure oder mit Base oder durch Hydrogenolyse abspaltet und/oder eine erhältliche Verbindung der Formel I mit einer salzbildenden Gruppe in ihr Salz oder ein erhältliches Salz in die freie Verbindung oder in ein anderes Salz überführt und/oder gegebenenfalls erhältliche Isomerengemische auftrennt und/oder eine erfindungsgemässe Verbindung der Formel I in eine andere erfindungsgemässe Verbindung der Formel I umwandelt.

**7.** Verfahren zur Herstellung einer Verbindung der Formel I gemäss Anspruch 3, dadurch gekennzeichnet, dass man ein Fragment einer Verbindung der Formel I mit einer endständigen Carboxygruppe oder einen davon abgeleiteten aktivierten Ester, ein davon abgeleitetes reaktionsfähiges Anhydrid oder ein davon abgeleitetes reaktionsfähiges cyclisches Amid mit einem zur Verbindung der Formel I komplementären Fragment mit einer freien Aminogruppe, wobei in den Reaktionskomponenten vorhandene freie funktionelle Gruppen mit Ausnahme der an der Reaktion teilnehmenden Gruppen gegebenenfalls in geschützter Form vorliegen, in Gegenwart eines Kondensationsmittels ausgewählt aus einem Carbodiimid, Carbonyldiimidazol, 1,2-Oxazoliumverbindungen, einem 1,2-Dihydrochinolin oder einem aktivierten Phosphat, und gewünschtenfalls in Gegenwart einer organischen Base oder von Alkalimetallcarbonaten in einem inerten, polaren, aprotischen Lösungsmittel in einem Temperaturbereich von -40 °C bis +100 °C kondensiert, wobei aktivierte Ester vom Amino- oder Amidoester-Typ gewünschtenfalls in situ durch Zugabe einer N-Hydroxyamino- oder N-Hydroxyamido-Verbindung gebildet werden, und gewünschtenfalls in einer erhältlichen Verbindung vorhandene Schutzgruppen durch Behandlung mit Säure oder mit Base oder durch Hydrogenolyse abspaltet und/oder eine erhältliche Verbindung der Formel I mit einer salzbildenden Gruppe in ihr Salz oder ein erhältliches Salz in die freie Verbindung oder in ein anderes Salz überführt und/oder gegebenenfalls erhältliche Isomerengemische auftrennt und/oder eine erfindungsgemässe Verbindung der Formel I in eine andere erfindungsgemässe Verbindung der Formel I umwandelt.

**8.** Verfahren gemäss einem der Ansprüche 1, 2, 4 oder 6, dadurch gekennzeichnet, dass man solche Ausgangsstoffe wählt, dass eine Verbindung der Formel I, worin $R_1$ 2(S)-Pivaloyloxy-3-phenylpropionyl, 2(R)- und 2(S)-Dimethoxyphosphoryl-3-phenyl-propionyl, 2(R)- und 2(S)-Benzyl-5,5-dimethyl-4-oxo-hexanoyl, 2(R)- und 2(S)-Benzyl-4,4-dimethyl-3-oxo-pentanoyl, 2(R)-und 2(S)-tert-Butylcarbamoyl-3-α-naphthyl-propionyl oder 2(R)-und 2(S)-Aethoxycarbonyl-3-α-naphthyl-propionyl, Indolyl-2-carbonyl oder Cyclohepta[b]pyrrolyl-5-carbonyl, A den bivalenten Rest der Aminosäure L-Histidin, $R_2$ Wasserstoff, $R_3$ Cyclohexylmethyl, $R_4$ Hydroxy, $R_5$ Isopropyl und $R_6$ $C_1$-$C_7$-Alkylamino bedeuten und die die Reste $R_3$, $R_4$ und $R_5$ tragenden C-Atome die S-Konfiguration aufweisen, ferner deren pharmazeutisch annehmbare Salze gebildet werden.

**9.** Verfahren gemäss einem der Ansprüche 1, 2, 4, 6 oder 8, dadurch gekennzeichnet, dass man solche Ausgangsstoffe wählt, dass die Verbindung der Formel I, worin $R_1$ 2(S)-Pivaloyloxy-3-phenyl-propionyl, A den bivalenten Rest der Aminosäure L-Histidin, $R_2$ Wasserstoff, $R_3$ Cyclohexylmethyl, $R_4$ Hydroxy, $R_5$ Isopropyl und $R_6$ n-Butylamino bedeuten und die die Reste $R_3$, $R_4$ und $R_5$ tragenden C-Atome die S-Konfiguration aufweisen, und deren pharmazeutisch annehmbare Salze gebildet werden.

**10.** Verfahren gemäss einem der Ansprüche 1, 2, 4, 6 oder 8, dadurch gekennzeichnet, dass man solche Ausgangsstoffe wählt, dass eine der Verbindungen der Formel I, worin $R_1$ 2(R,S)-, 2(R)- oder 2(S)-Dimethoxyphosphoryl-3-phenyl-propionyl, A den bivalenten Rest der Aminosäure L-Histidin, $R_2$ Wasserstoff, $R_3$ Cyclohexylmethyl, $R_4$ Hydroxy, $R_5$ Isopropyl und $R_6$ n-Butylamino bedeuten und die die Reste $R_3$, $R_4$ und $R_5$ tragenden C-Atome die S-Konfiguration aufweisen, und deren pharmazeutisch annehmbare Salze gebildet werden.

**11.** Verfahren gemäss einem der Ansprüche 1, 2, 4, 6 oder 8, dadurch gekennzeichnet, dass man solche Ausgangsstoffe wählt, dass eine der Verbindungen der Formel I, worin $R_1$ 2(R,S)-, 2(R)- oder 2(S)-Benzyl-5,5-dimethyl-4-oxo-hexanoyl, A den bivalenten Rest der Aminosäure L-Histidin, $R_2$ Wasserstoff, $R_3$ Cyclohexylmethyl, $R_4$ Hydroxy, $R_5$ Isopropyl und $R_6$ n-Butylamino bedeuten und die die Reste $R_3$, $R_4$ und $R_5$ tragenden C-Atome die S-Konfiguration aufweisen, und deren pharmazeutisch annehmbare Salze gebildet werden.

**12.** Verfahren gemäss einem der Ansprüche 1, 2, 4, 6 oder 8, dadurch gekennzeichnet, dass man solche Ausgangsstoffe wählt, dass die Verbindung der Formel I, worin $R_1$ 2(R,S)-Benzyl-4,4-dimethyl-3-oxo-pentanoyl, A den bivalenten Rest der Aminosäure L-Histidin, $R_2$ Wasserstoff, $R_3$ Cyclohexylmethyl, $R_4$ Hydroxy, $R_5$ Isopropyl und $R_6$ n-Butylamino bedeuten und die die Reste $R_3$, $R_4$ und $R_5$ tragenden C-Atome die S-Konfiguration aufweisen, und deren pharmazeutisch annehmbare Salze gebildet werden.

**13.** Verfahren gemäss einem der Ansprüche 1, 2, 4, 6 oder 8, dadurch gekennzeichnet, dass man solche Ausgangsstoffe wählt, dass die Verbindung der Formel I, worin $R_1$ 2(R,S)-Aethoxycarbonyl-3-α-naphthyl-propionyl, A den bivalenten Rest der Aminosäure L-Histidin, $R_2$ Wasserstoff, $R_3$ Cyclohexylmethyl, $R_4$ Hydroxy, $R_5$ Isopropyl und $R_6$ n-Butylamino bedeuten und die die Reste $R_3$, $R_4$ und $R_5$ tragenden C-Atome die S-Konfiguration aufweisen, und deren pharmazeutisch annehmbare Salze gebildet werden.

**14.** Verfahren gemäss einem der Ansprüche 1, 2, 4, 6 oder 8, dadurch gekennzeichnet, dass man solche Ausgangsstoffe wählt, dass die Verbindung der Formel I, worin $R_1$ Cyclohepta[b]pyrrolyl-5-carbonyl, A den bivalenten Rest der Aminosäure L-Histidin, $R_2$ Wasserstoff, $R_3$ Cyclohexylmethyl, $R_4$ Hydroxy, $R_5$ Isopropyl und $R_6$ n-Butylamino bedeuten und die die Reste $R_3$, $R_4$ und $R_5$ tragenden C-Atome die S-Konfiguration aufweisen, und deren pharmazeutisch annehmbare Salze gebildet werden.

**15.** Verfahren gemäss einem der Ansprüche 1, 2, 4, 6 oder 8, dadurch gekennzeichnet, dass man solche Ausgangsstoffe wählt, dass die Verbindung der Formel I, worin $R_1$ 2(S)-Pivaloyloxy-3-phenyl-propionyl, A den bivalenten Rest der Aminosäure L-Histidin, $R_2$ Wasserstoff, $R_3$ Isobutyl, $R_4$ Hydroxy, $R_5$ Cyclohexylmethyl und $R_6$ n-Butylamino bedeuten und die die Reste $R_3$ und $R_4$ tragenden C-Atome die S-Konfiguration aufweisen, und deren pharmazeutisch annehmbare Salze gebildet werden.

**16.** Verfahren zur Herstellung von pharmazeutischen Präparaten, dadurch gekennzeichnet, dass man eine

gemäss einem in einem der Ansprüche 1-15 genannten Verfahren hergestellte Verbindung der Formel I oder pharmazeutisch verwendbare Salze einer solchen Verbindung der Formel I, die salzbildende Gruppen aufweist, mit einem pharmazeutischen Trägermaterial mischt.

**17.** Verfahren zur Herstellung einer Verbindung der Formel

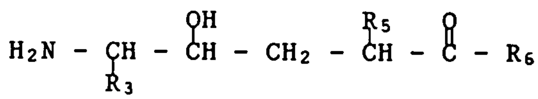

(VIII),

worin $R_3$ Cyclohexylmethyl bedeutet und $R_5$ und $R_6$ die in Anspruch 1 oder 2 genannten Bedeutungen haben, und von deren Salzen, dadurch gekennzeichnet, dass man eine Verbindung der Formel

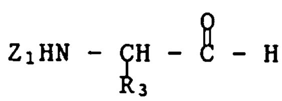

(IX),

worin $R_3$ die genannte Bedeutung hat und $Z_1$ eine Aminoschutzgruppe ist, mit einer Schwefel-Ylid-Verbindung in ein Epoxid der Formel

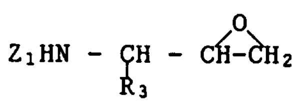

(X),

worin $R_3$ und $Z_1$ die genannten Bedeutungen haben, umwandelt, die erhältliche Verbindung (X) gegebenenfalls nach Trennung der Isomeren mit einem eine nukleofuge Abgangsgruppe X einführenden Reagens umsetzt, die erhältliche Verbindung der Formel

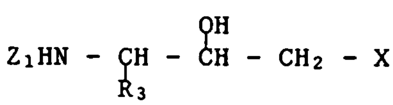

(XI),

worin $R_3$ und $Z_1$ die genannten Bedeutungen haben und X eine nukleofuge Abgangsgruppe ist, mit einer Carbonylverbindung der Formel

$R_a - (C = O) - R_b$ (XII),

worin jeder der Reste $R_a$ und $R_b$ unabhängig voneinander Wasserstoff, Niederalkyl, Aryl oder Arylniederalkyl, $R_a$ und $R_b$ zusammen gegebenenfalls überbrücktes Alkyliden mit 4 bis 12 Kohlenstoffatomen darstellen, oder einem reaktionsfähigen Derivat dieser Carbonylverbindung, in Gegenwart eines sauren Reagenses umsetzt, die erhältliche Verbindung der Formel

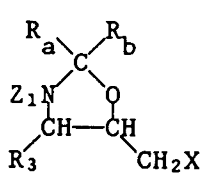

(XIII),

worin die Substituenten die genannten Bedeutungen haben, mit einem Carbonsäureestersalz der Formel

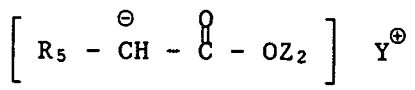 (XIV),

worin $R_5$ die unter Formel I genannte Bedeutung hat, $Z_2$ eine Carboxyschutzgruppe und $Y^{\oplus}$ ein Kation, z.B. ein Alkalimetall-, z.B. das Natrium- oder bevorzugt das Lithiumion, ist, umsetzt, und in einer erhältlichen Verbindung der Formel

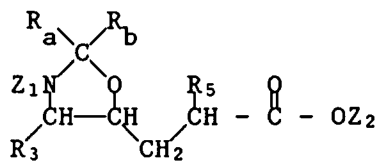 (XV),

worin die Sustituenten die genannten Bedeutungen haben, die Carboxyschutzgruppe $Z_2$ abspaltet und/oder ein erhältliches Isomerengemisch in die einzelnen Isomere auftrennt, die erhältliche Verbindung mit einer freien Carboxygruppe ($Z_2$ = H) mit einem Amin $R_6$-H amidiert, und in einer erhältlichen Verbindung der Formel

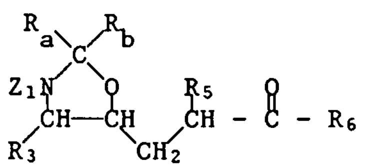 (XVI),

worin die Substituenten die genannten Bedeutungen haben, mit einem geeigneten Solvolysereagens den Ring öffnet und gegebenenfalls die Schutzgruppe $Z_1$ abspaltet.

## Claims

### Claims for the following Contracting States : BE, CH, DE, FR, GB, IT, LI, LU, NL, SE

**1.** A compound of the formula

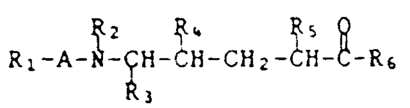 (I)

wherein $R_1$ is $C_1$-$C_7$alkanoyl, phenoxy-$C_1$-$C_7$alkanoyl, naphthoxy-$C_1$-$C_7$alkanoyl, $\alpha$-naphthoxy-carboxy-$C_1$-$C_7$alkanoyl, $\alpha$-naphthoxy-$C_1$-$C_7$alkoxycarbonyl-$C_1$-$C_7$alkanoyl, $\alpha$-naphthoxy-benzyloxycarbonyl-$C_1$-$C_7$alkanoyl, $\alpha$-naphthoxycarbamoyl-$C_1$-$C_7$alkanoyl, $\alpha$-naphthoxy-cyano-$C_1$-$C_7$alkanoyl, $\alpha$-naphthoxy-di-$C_1$-$C_7$alkylamino-$C_1$-$C_7$alkanoyl, $\alpha$-naphthoxy-oxo-$C_1$-$C_7$alkanoyl, phenyl-, $\alpha$-naphthyl- or $\beta$-naphthyl-$C_1$-$C_7$alkanoyl, wherein $C_1$-$C_7$alkanoyl may be unsubstituted or substituted by hydroxy, $C_1$-$C_7$alkoxy, $C_1$-$C_7$alkanoyloxy, carboxy, $C_1$-$C_7$alkoxycarbonyl, carbamoyl, $C_1$-$C_7$alkylcarbamoyl, di-$C_1$-$C_7$alkylcarbamoyl, cyano, phosphono, di-$C_1$-$C_7$alkoxyphosphoryl, benzofuranyl and/or by oxo and is straight-chain or branched; 2-phenylethyl, 3-phenyl-2-propyl, 4-phenyl-3-butyl, 2-$\alpha$-naphthylethyl, 3-$\alpha$-naphthyl-2-propyl or 4-$\alpha$-naphthyl-3-butyleach of which is bonded via a carbonyl group -CO-and is substituted in the 1-position by acyloxy in the form of ethylaminocarbonyloxy, 2-benzyloxycarbonylamino-2-methyl-propionyloxy, 2-amino-2-methyl-propionyloxy or acetoacetoxy, by esterified carboxy in the form of benzyloxycarbonyl, by substituted carbamoyl in the form of carboxymethylcarbamoyl, tert-butoxycarbonylmethylcarbamoyl, 2-dimethylaminoethylcarbamoyl, 3-hydroxy-2-propylcarbamoyl, 2,2-dimethox-

yethylcarbamoyl or 5-amino-5-carboxypentylcarbamoyl, or by esterified phosphono in the form of hydroxymethoxyphosphoryl; 3-phenyl-2-ethoxyhydroxyphosphoryl-propionyl, 3-phenyl- or 3-$\alpha$-naphthyl-2-dimethylaminomethyl-propionyl, 4-phenyl- or 4-$\alpha$-naphthyl-3-benzyloxycarbonyl-butyryl, 2-benzyl- or 2-$\alpha$-naphthylmethyl-4,4-dimethyl-3-oxo-pentanoyl, 2-benzyl- or 2-$\alpha$-naphthylmethyl-5-dimethylamino-pentanoyl, 2-benzyl- or 2-$\alpha$-naphthylmethyl-5-dimethylamino-4-oxo-pentanoyl, 2-benzyl- or 2-$\alpha$-naphthylmethyl-5,5-dimethyl-4-oxo-hexanoyl, $\alpha$,p-diamino-phenylacetyl, $\alpha$,p-dibenzyloxycarbonylamino-phenylacetyl, $\alpha$-pivaloylamino-p-benzyloxycarbonylamino-phenylacetyl, 2-(o,o-dichloroanilino)-phenylacetyl, 2-(o,o-dichloro-N-benzylanilino)-phenylacetyl, $\alpha$- or $\beta$-naphthylcarbonyl, 1,8-naphthalenedicarbonyl, pyrrolylcarbonyl, cyclohepta[b]pyrrolyl-5-carbonyl, indolyl-carbonyl, 1-benzyl-indolyl-3-carbonyl, 4,5,6,7-tetrahydroindolyl-2-carbonyl, quinolylcarbonyl or oxamoyl,

A is the bivalent residue of the amino acids leucine, norleucine, phenylalanine, N-methyl-phenylalanine, $\beta$-phenylserine, cyclohexylalanine, glutamine, histidine or N-methyl-histidine, $R_2$ is hydrogen, $R_3$ is isobutyl or cyclohexylmethyl, $R_4$ is hydroxy, $R_5$ is isopropyl, cyclohexylmethyl, $\alpha$-decahydronaphthyl or dimethylamino, and

$R_6$ is $C_1$-$C_7$alkylamino, di-$C_1$-$C_7$alkylamino, hydroxy-$C_1$-$C_7$alkylamino, 2-phenoxyethylamino, 2-(3-carbamoyl-4-hydroxyphenoxy)-ethylamino, carboxy-alkylamino or aminocarboxy-alkylamino wherein the carboxy radical is not in the 1 position of the alkyl radical, which contains up to 10 carbon atoms, $C_1$-$C_7$alkoxycarbonyl-alkylamino or ($C_1$-$C_7$alkanoylamino-, $C_1$-$C_7$alkoxycarbonylamino- or benzyloxycarbonylamino-)$C_1$-$C_7$alkoxycarbonyl-alxylamino, wherein the carbonyl radical is not in the 1-position of the alkyl radical, which contains up to 10 carbon atoms, 4-tris-(hydroxymethyl)-methylcarbamoyl-n-butylamino, amino-$C_1$-$C_7$alkylamino, di-$C_1$-$C_7$alkylamino-$C_1$-$C_7$alkylamino, $C_1$-$C_7$alkoxycarbonylamino-$C_1$-$C_7$alkylamino, morpholino-$C_1$-$C_7$alkylamino, cycloalkyl-$C_1$-$C_7$alkylamino having from 4 to 10 carbon atoms, benzylamino, pyridyl-$C_1$-$C_7$alkylamino, imidazolyl-$C_1$-$C_7$alkylamino or 2-(2-[4-imidazolyl]-ethylamino)-ethylamino,

or a pharmaceutically acceptable salt of such a compound having salt-forming groups, with the exception of the compound of formula I wherein $R_1$ is $\alpha$-naphthoxyacetyl, A is the bivalent residue of the amino acid histidine, $R_2$ is hydrogen, $R_3$ is isobutyl, $R_4$ is hydroxy, $R_5$ is isopropyl and $R_6$ is cyclohexylmethylamino.

**2.** A compound of formula I shown in claim 1, wherein $R_1$ is phenoxy-$C_1$-$C_7$alkanoyl, naphthoxy-$C_1$-$C_7$alkanoyl, phenyl-$C_1$-$C_7$alkanoyl, wherein $C_1$-$C_7$alkanoyl may be unsubstituted or substituted by $C_1$-$C_7$alkanoyloxy, carboxy, $C_1$-$C_7$alkoxycarbonyl, carbamoyl, $C_1$-$C_7$alkylcarbamoyl, cyano, di-$C_1$-$C_7$alkoxyphosphoryl or $C_1$-$C_7$alkyl and oxo, or by benzofuranyl and oxo, and is straight-chain or branched; naphthyl-$C_1$-$C_7$alkanoyl wherein $C_1$-$C_7$alkanoyl may be unsubstituted or substituted by $C_1$-$C_7$-alkanoyloxy, carboxy, $C_1$-$C_7$alkoxycarbonyl, carbamoyl, cyano or $C_1$-$C_7$alkyl and oxo, and is straight-chain or branched; 2-tert-butylcarbamoyl-3-$\alpha$-naphthyl-propionyl, 2-carboxymethylcarbamoyl-3-$\alpha$-naphthyl-propionyl, indolyl-2-carbonyl or cyclohepta[b]pyrrolyl-5-carbonyl, A is the bivalent residue of the amino acid L-histidine, $R_2$ is hydrogen, $R_3$ is isobutyl or cyclohexylmethyl, $R_4$ is hydroxy, $R_5$ is isopropyl or cyclohexylmethyl, and $R_6$ is $C_1$-$C_7$alkylamino or amino-carboxy-alkylamino wherein the substituents are not in the 1 position of the alkyl radical, which contains up to 7 carbon atoms, and the carbon atoms carrying the radicals $R_3$ and $R_4$ have the S-configuration, or a pharmaceutically acceptable salt of such a compound.

**3.** A compound according to either claim 1 or claim 2 of formula I, wherein $R_1$ is 2(S)-pivaloyloxy-3-phenyl-propionyl, 2(R)- and 2(S)-dimethoxyphosphoryl-3-phenyl-propionyl, 2(R)- and 2(S)-benzyl-5,5-dimethyl-4-oxo-hexanoyl, 2(R)- and 2(S)-benzyl-4,4-dimethyl-3-oxo-pentanoyl, 2(R)- and 2(S)-tert-butylcarbamoyl-3-$\alpha$-naphthyl-propionylor 2(R)- and 2(S)-ethoxycarbonyl-3-$\alpha$-naphthyl-propionyl, indolyl-2-carbonyl or cyclohepta[b]pyrrolyl-5-carbonyl, A is the bivalent residue of the amino acid L-histidine, $R_2$ is hydrogen, $R_3$ is cyclohexylmethyl, $R_4$ is hydroxy, $R_5$ is isopropyl and $R_6$ is $C_1$-$C_7$alkylamino, and the carbon atoms carrying the radicals $R_3$, $R_4$ and $R_5$ have the S-configuration, or a pharmaceutically acceptable salt of such a compound.

**4.** A compound according to claim 1, 2 or 3 of formula I, wherein $R_1$ is 2(S)-pivaloyloxy-3-phenyl-propionyl, A is the bivalent residue of the amino acid L-histidine, $R_2$ is hydrogen, $R_3$ is cyclohexylmethyl, $R_4$ is hydroxy, $R_5$ is isopropyl and $R_6$ is n-butylamino, and the carbon atoms carrying the

radicals $R_3$, $R_4$ and $R_5$ have the S-configuration, or a pharmaceutically acceptable salt thereof.

**5.** A compound according to claim 1, 2 or 3 of formula I, wherein $R_1$ is 2(R,S)-, 2(R)- or 2(S)-dimethoxyphosphoryl-3-phenyl-propionyl, A is the bivalent residue of the amino acid L-histidine, $R_2$ is hydrogen, $R_3$ is cyclohexylmethyl, $R_4$ is hydroxy, $R_5$ is isopropyl and $R_6$ is n-butylamino, and the carbon atoms carrying the radicals $R_3$, $R_4$ and $R_5$ have the S-configuration, or a pharmaceutically acceptable salt thereof.

**6.** A compound according to claim 1, 2 or 3 of formula I, wherein $R_1$ is 2(R,S)-, 2(R)- or 2(S)-benzyl-5,5-dimethyl-4-oxo-hexanoyl, A is the bivalent residue of the amino acid L-histidine, $R_2$ is hydrogen, $R_3$ is cyclohexylmethyl, $R_4$ is hydroxy, $R_5$ is isopropyl and $R_6$ is n-butylamino, and the carbon atoms carrying the radicals $R_3$, $R_4$ and $R_5$ have the S-configuration, or a pharmaceutically acceptable salt thereof.

**7.** A compound according to claim 1, 2 or 3 of formula I, wherein $R_1$ is 2(R,S)-benzyl-4,4-dimethyl-3-oxo-pentanoyl, A is the bivalent residue of the amino acid L-histidine, $R_2$ is hydrogen, $R_3$ is cyclohexylmethyl, $R_4$ is hydroxy, $R_5$ is isopropyl and $R_6$ is n-butylamino, and the carbon atoms carrying the radicals $R_3$, $R_4$ and $R_5$ have the S-configuration, or a pharmaceutically acceptable salt thereof.

**8.** A compound according to claim 1, 2 or 3 of formula I, wherein $R_1$ is 2(R,S)-ethoxycarbonyl-3-α-naphthyl-propionyl, A is the bivalent residue of the amino acid L-histidine, $R_2$ is hydrogen, $R_3$ is cyclohexylmethyl, $R_4$ is hydroxy, $R_5$ is isopropyl and $R_6$ is n-butylamino, and the carbon atoms carrying the radicals $R_3$, $R_4$ and $R_5$ have the S-configuration, or a pharmaceutically acceptable salt thereof.

**9.** A compound according to claim 1, 2 or 3 of formula I, wherein $R_1$ is cyclohepta[b]pyrrolyl-5-carbonyl, A is the bivalent residue of the amino acid L-histidine, $R_2$ is hydrogen, $R_3$ is cyclohexylmethyl, $R_4$ is hydroxy, $R_5$ is isopropyl and $R_6$ is n-butylamino, and the carbon atoms carrying the radicals $R_3$, $R_4$ and $R_5$ have the S-configuration, or a pharmaceutically acceptable salt thereof.

**10.** A compound according to claim 1, 2 or 3 of formula I, wherein $R_1$ is 2(S)-pivaloyloxy-3-phenyl-propionyl, A is the bivalent residue of the amino acid L-histidine, $R_2$ is hydrogen, $R_3$ is isobutyl, $R_4$ is hydroxy, $R_5$ is cyclohexylmethyl and $R_6$ is n-butylamino, and the carbon atoms carrying the radicals $R_3$ and $R_4$ have the S-configuration, or a pharmaceutically acceptable salt thereof.

**11.** The compounds of formula I shown in claim 1 with the names

N-(N-benzyl-indolyl-2-carbonyl)-His-Leu$\overset{C}{-}$Val-methylamide,
N-(3(R,S)-benzyloxycarbonyl-4-α-naphthylbutyryl)-His-Leu$\overset{C}{-}$Val-methylamide,
N-benzyloxycarbonyl-(p-benzyloxycarbonylamino-Phe)-His-Leu$\overset{C}{-}$Val-n-butylamide,
N-pivaloyl-(p-benzyloxycarbonylamino-Phe)-His-Leu$\overset{C}{-}$Val-n-butylamide,
N-(indolyl-2-carbonyl)-His-Leu$\overset{C}{-}$Val-4-[tris-(tert-butyl-dimethylsilyloxymethyl)-methylcarbamoyl]-butylamide,
N-(2-ethylaminocarbonyloxy-4-phenyl-butyryl)-His-Cha$\overset{C}{-}$Val-n-butylamide,

and the pharmaceutically acceptable salts thereof.

**12.** A pharmaceutical composition comprising a compound according to claim 1, 2, 3 or 11 of formula I, or a pharmaceutically acceptable salt of such a compound having salt-forming groups, together with a pharmaceutical carrier.

**13.** The use of a compound according to claim 1, 2, 3 or 11 of formula I or of a pharmaceutically acceptable salt of such a compound of formula I for the preparation of a medicament for the treatment of hypertension and cardiac insufficiency.

**14.** A process for the preparation of compounds of formula I wherein the substituents have the meanings given in claim 1, 2, 3 or 11, which comprises

a) condensing a fragment of a compound of formula I having a terminal carboxy group or a reactive acid derivative of that fragment with a fragment that is complementary to the compound of formula I and has a free amino group, or with a reactive derivative thereof having an activated amino group, to form an amide bond, any free functional groups present in the reactants, with the exception of the groups participating in the reaction, optionally being in protected form, or

b) reducing the keto group in a compound of the formula

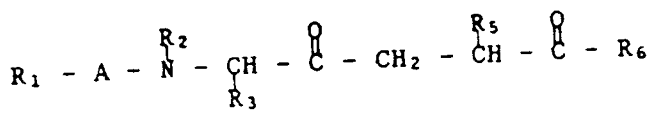

(II)

wherein the substituents have the meanings mentioned and free functional groups, with the exception of the keto group participating in the reaction, are optionally in protected form, to a hydroxy group by reaction with a suitable reducing agent, or

c) reacting an aldehyde compound of the formula

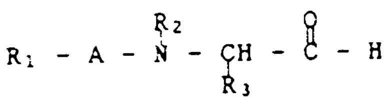

(III)

wherein the substituents have the meanings mentioned and free functional groups, with the exception of the aldehyde group, are optionally in protected form, with an organometal compound of the formula

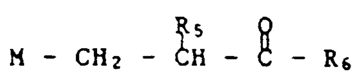

(IV)

wherein the substituents have the meanings mentioned and M is a metal radical, and hydrolysing the resulting addition product, or

d) in a compound of the formula

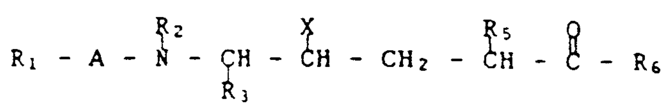

(V)

wherein X is a nucleofugal leaving group, the other substituents have the meanings mentioned above and free functional groups are optionally in protected form, exchanging the substituent X for $R_4$ with a reagent that introduces the substituent $R_4$ in nucleophilic form, or

e) in a compound of the formula

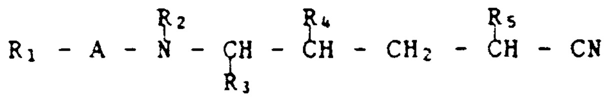

(VI)

wherein the substituents have the meanings mentioned and any functional groups present are optionally in protected form, converting the cyano group into an N-substituted carboxamido group $-(C=O)R_6$, or

f) reducing an epoxide of the formula

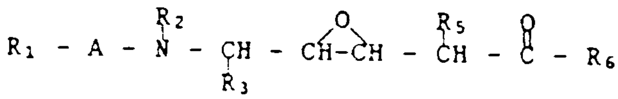

(VII)

wherein the substituents have the meanings mentioned and free functional groups are optionally in protected form, to the corresponding alcohol with a regioselective reducing agent and, if desired,

g) removing any protecting groups present in an obtainable compound and/or, if desired, after carrying out one of the processes a) to f) mentioned above or any other process for the preparation of a compound of formula I, converting an obtainable compound of formula I having a salt-forming group into its salt or an obtainable salt into the free compound or into a different salt and/or optionally separating obtainable isomeric mixtures and/or, in an obtainable compound of formula I, reversing the configuration of a chiral carbon atom, and/or converting a compound of formula I according to the invention into a different compound of formula I according to the invention.

**15.** A compound of the formula

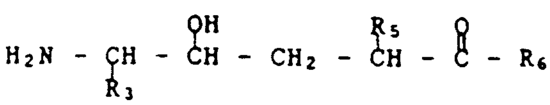

(VIII)

wherein $R_3$ is cyclohexylmethyl and $R_5$ and $R_6$ have the meanings mentioned in claim 1, 2 or 3, or a salt thereof.

**16.** A process for the preparation of a compound of formula VIII according to claim 15, wherein the substituents have the meanings mentioned in claim 15, which comprises converting a compound of the formula

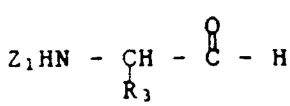

(IX)

wherein $R_3$ has the meaning mentioned and $Z_1$ is an amino-protecting group, with a sulfur-ylide compound into an epoxide of the formula

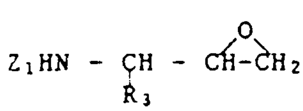

(X)

wherein $R_3$ and $Z_1$ have the meanings mentioned, reacting the obtainable compound (X), optionally after separation of the isomers, with a reagent that introduces a nucleofugal leaving group X, reacting

the obtainable compound of the formula

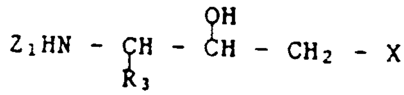

(XI)

wherein $R_3$ and $Z_1$ have the meanings mentioned and X is a nucleofugal leaving group, with a carbonyl compound of the formula

$R_a$ - (C = O) $R_b$ (XII)

wherein each of the radicals $R_a$ and $R_b$, independently of the other, is hydrogen, lower alkyl, aryl or aryl-lower alkyl, or $R_a$ and $R_b$ together are optionally bridged alkylidene having from 4 to 12 carbon atoms, or with a reactive derivative of that carbonyl compound, in the presence of an acidic reagent, reacting the obtainable compound of the formula

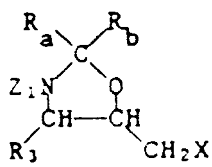

(XIII)

wherein the substituents have the meanings mentioned, with a carboxylic acid ester salt of the formula

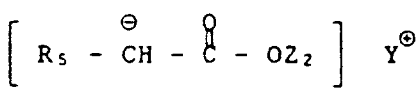

(XIV)

wherein $R_5$ has the meaning mentioned under formula I, $Z_2$ is a carboxy-protecting group and $Y^{\oplus}$ is a cation, for example an alkali metal, for example the sodium or preferably the lithium ion, and in an obtainable compound of the formula

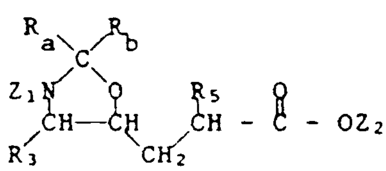

(XV)

wherein the substituents have the meanings mentioned, removing the carboxy-protecting group $Z_2$ and/or separating an obtainable isomeric mixture into the individual isomers, amidating the obtainable compound having a free carboxy group ($Z_2$ = H) with an amine $R_6$-H, and in an obtainable compound of the formula

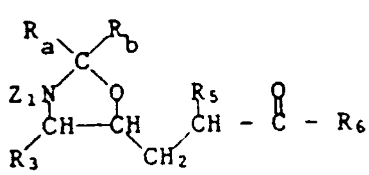

(XVI)

wherein the substituents have the meanings mentioned, opening the ring with a suitable solvolysis

reagent and optionally removing the protecting group $Z_1$.

**Claims for the following Contracting State : AT**

**1.** A process for the preparation of a compound of the formula

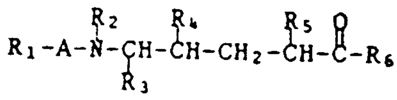

(I)

wherein $R_1$ is $C_1$-$C_7$alkanoyl, phenoxy-$C_1$-$C_7$alkanoyl, naphthoxy-$C_1$-$C_7$alkanoyl, α-naphthoxy-carboxy-$C_1$-$C_7$alkanoyl, α-naphthoxy-$C_1$-$C_7$alkoxycarbonyl-$C_1$-$C_7$alkanoyl, α-naphthoxy-benzyloxycarbonyl-$C_1$-$C_7$alkanoyl, α-naphthoxy-carbamoyl-$C_1$-$C_7$alkanoyl, α-naphthoxy-cyano-$C_1$-$C_7$alkanoyl, α-naphthoxy-di-$C_1$-$C_7$alkylamino-$C_1$-$C_7$alkanoyl, α-naphthoxy-oxo-$C_1$-$C_7$alkanoyl, phenyl-, α-naphthyl- or β-naphthyl-$C_1$-$C_7$alkanoyl, wherein $C_1$-$C_7$alkanoyl may be unsubstituted or substituted by hydroxy, $C_1$-$C_7$alkoxy, $C_1$-$C_7$alkanoyloxy, carboxy, $C_1$-$C_7$alkoxycarbonyl, carbamoyl, $C_1$-$C_7$alkylcarbamoyl, di-$C_1$-$C_7$alkylcarbamoyl, cyano, phosphono, di-$C_1$-$C_7$alkoxyphosphoryl, benzofuranyl and/or by oxo and is straight-chain or branched; 2-phenylethyl, 3-phenyl-2-propyl, 4-phenyl-3-butyl, 2-α-naphthylethyl, 3-α-naphthyl-2-propyl or 4-α-naphthyl-3-butyleach of which is bonded via a carbonyl group -CO-and is substituted in the 1-position by acyloxy in the form of ethylaminocarbonyloxy, 2-benzyloxycarbonylamino-2-methyl-propionyloxy, 2-amino-2-methyl-propionyloxy or acetoacetoxy, by esterified carboxy in the form of benzyloxycarbonyl, by substituted carbamoyl in the form of carboxymethylcarbamoyl, tert-butoxycarbonylmethylcarbamoyl, 2-dimethylaminoethylcarbamoyl, 3-hydroxy-2-propylcarbamoyl, 2,2-dimethoxyethylcarbamoyl or 5-amino-5-carboxypentylcarbamoyl, or by esterified phosphono in the form of hydroxymethoxyphosphoryl; 3-phenyl-2-ethoxy-hydroxyphosphoryl-propionyl, 3-phenyl- or 3-α-naphthyl-2-dimethylaminomethyl-propionyl, 4-phenyl- or 4-α-naphthyl-3-benzyloxycarbonyl-butyryl, 2-benzyl- or 2-α-naphthylmethyl-4,4-dimethyl-3-oxo-pentanoyl, 2-benzyl- or 2-α-naphthylmethyl-5-dimethylamino-pentanoyl, 2-benzyl- or 2-α-naphthylmethyl-5-dimethylamino-4-oxo-pentanoyl, 2-benzyl- or 2-α-naphthylmethyl-5,5-dimethyl-4-oxo-hexanoyl, α,p-diamino-phenylacetyl, α,p-dibenzyloxycarbonylamino-phenylacetyl, α-pivaloylamino-p-benzyloxycarbonylamino-phenylacetyl, 2-(o,o-dichloroanilino)-phenylacetyl, 2-(o,o-dichloro-N-benzylanilino)-phenylacetyl, α- or β-naphthylcarbonyl, 1,8-naphthalenedicarbonyl, pyrrolylcarbonyl, cyclohepta[b]pyrrolyl-5-carbonyl, indolylcarbonyl, 1-benzyl-indolyl-3-carbonyl, 4,5,6,7-tetrahydroindolyl-2-carbonyl, quinolylcarbonyl or oxamoyl,

A is the bivalent residue of the amino acids leucine, norleucine, phenylalanine, N-methyl-phenylalanine, β-phenylserine, cyclohexylalanine, glutamine, histidine or N-methyl-histidine, $R_2$ is hydrogen, $R_3$ is isobutyl or cyclohexylmethyl, $R_4$ is hydroxy, $R_5$ is isopropyl, cyclohexylmethyl, α-decahydronaphthyl or dimethylamino, and

$R_6$ is $C_1$-$C_7$alkylamino, di-$C_1$-$C_7$alkylamino, hydroxy-$C_1$-$C_7$alkylamino, 2-phenoxyethylamino, 2-(3-carbamoyl-4-hydroxyphenoxy)-ethylamino, carboxy-alkylamino or aminocarboxy-alkylamino wherein the carboxy radical is not in the 1 position of the alkyl radical, which contains up to 10 carbon atoms, $C_1$-$C_7$alkoxycarbonyl-alkylamino or ($C_1$-$C_7$alkanoylamino-, $C_1$-$C_7$alkoxycarbonylamino- or benzyloxycarbonylamino-)$C_1$-$C_7$alkoxycarbonyl-alkylamino, wherein the carbonyl radical is not in the 1-position of the alkyl radical, which contains up to 10 carbon atoms, 4-tris-(hydroxymethyl)-methylcarbamoyl-n-butylamino, amino$C_1$-$C_7$alkylamino, di-$C_1$-$C_7$alkylamino-$C_1$-$C_7$alkylamino, $C_1$-$C_7$alkoxycarbonylamino-$C_1$-$C_7$alkylamino, morpholino-$C_1$-$C_7$alkylamino, cycloalkyl-$C_1$-$C_7$alkylamino having from 4 to 10 carbon atoms, benzylamino, pyridyl-$C_1$-$C_7$alkylamino, imidazolyl-$C_1$-$C_7$alkylamino or 2-(2-[4-imidazolyl]-ethylamino)-ethylamino,

or a salt of such a compound having salt-forming groups, with the exception of the compound of formula I wherein $R_1$ is α-naphthoxyacetyl, A is the bivalent residue of the amino acid histidine, $R_2$ is hydrogen, $R_3$ is isobutyl, $R_4$ is hydroxy, $R_5$ is isopropyl and $R_6$ is cyclohexylmethylamino, which comprises

a) condensing a fragment of a compound of formula I having a terminal carboxy group or a reactive acid derivative of that fragment with a fragment that is complementary to the compound of formula I

and has a free amino group, or with a reactive derivative thereof having an activated amino group, to form an amide bond, any free functional groups present in the reactants, with the exception of the groups participating in the reaction, optionally being in protected form, or

b) reducing the keto group in a compound of the formula

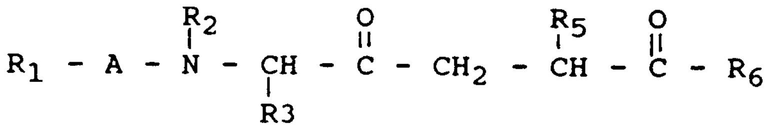

(II)

wherein the substituents have the meanings mentioned and free functional groups, with the exception of the keto group participating in the reaction, are optionally in protected form, to a hydroxy group by reaction with a suitable reducing agent, or

c) reacting an aldehyde compound of the formula

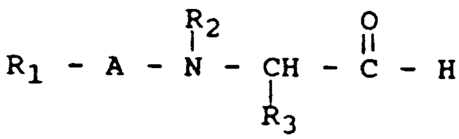

(III)

wherein the substituents have the meanings mentioned and free functional groups, with the exception of the aldehyde group, are optionally in protected form, with an organometal compound of the formula

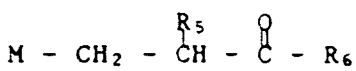

(IV)

wherein the substituents have the meanings mentioned and M is a metal radical, and hydrolysing the resulting addition product, or

d) in a compound of the formula

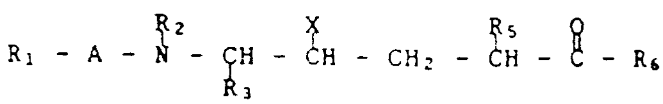

(V)

wherein X is a nucleofugal leaving group, the other substituents have the meanings mentioned above and free functional groups are optionally in protected form, exchanging the substituent X for $R_4$ with a reagent that introduces the substituent $R_4$ in nucleophilic form, or

e) in a compound of the formula

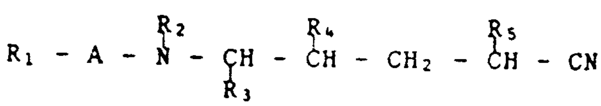

(VI)

wherein the substituents have the meanings mentioned and any functional groups present are optionally in protected form, converting the cyano group into an N-substituted carboxamido group $-(C=O)R_6$, or

f) reducing an epoxide of the formula

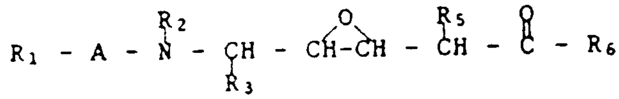

(VII)

wherein the substituents have the meanings mentioned and free functional groups are optionally in protected form, to the corresponding alcohol with a regioselective reducing agent and, if desired,
g) removing any protecting groups present in an obtainable compound and/or, if desired, after carrying out one of the processes a) to f) mentioned above or any other process for the preparation of a compound of formula I, converting an obtainable compound of formula I having a salt-forming group into its salt or an obtainable salt into the free compound or into a different salt and/or optionally separating obtainable isomeric mixtures and/or, in an obtainable compound of formula I, reversing the configuration of a chiral carbon atom, and/or converting a compound of formula I according to the invention into a different compound of formula I according to the invention.

**2.** A process for the preparation of a compound of formula I shown in claim 1, wherein $R_1$ is phenoxy-$C_1$-$C_7$alkanoyl, naphthoxy-$C_1$-$C_7$alkanoyl, phenyl-$C_1$-$C_7$alkanoyl, wherein $C_1$-$C_7$alkanoyl may be unsubstituted or substituted by $C_1$-$C_7$alkanoyloxy, carboxy, $C_1$-$C_7$alkoxycarbonyl, carbamoyl, $C_1$-$C_7$alkylcarbamoyl, cyano, di-$C_1$-$C_7$alkoxyphosphoryl or $C_1$-$C_7$alkyl and oxo, or by benzofuranyl and oxo, and is straight-chain or branched; naphthyl-$C_1$-$C_7$alkanoyl wherein $C_1$-$C_7$alkanoyl may be unsubstituted or substituted by $C_1$-$C_7$alkanoyloxy, carboxy, $C_1$-$C_7$alkoxycarbonyl, carbamoyl, cyano or $C_1$-$C_7$alkyl and oxo, and is straight-chain or branched; 2-tert-butylcarbamoyl-3-$\alpha$-naphthyl-propionyl, 2-carboxymethylcarbamoyl-3-$\alpha$-naphthyl-propionyl, indolyl-2-carbonyl or cyclohepta[b]pyrrolyl-5-carbonyl, A is the bivalent residue of the amino acid L-histidine, $R_2$ is hydrogen, $R_3$ is isobutyl or cyclohexylmethyl, $R_4$ is hydroxy, $R_5$ is isopropyl or cyclohexylmethyl, and $R_6$ is $C_1$-$C_7$alkylamino or aminocarboxy-alkylamino wherein the substituents are not in the 1 position of the alkyl radical, which contains up to 7 carbon atoms, and the carbon atoms carrying the radicals $R_3$ and $R_4$ have the S-configuration, or a salt of such a compound having salt-forming groups, which comprises
a) condensing a fragment of a compound of formula I having a terminal carboxy group or a reactive acid derivative of that fragment with a fragment that is complementary to the compound of formula I and has a free amino group, or with a reactive derivative thereof having an activated amino group, to form an amide bond, any free functional groups present in the reactants, with the exception of the groups participating in the reaction, optionally being in protected form, or
b) reducing the keto group in a compound of the formula

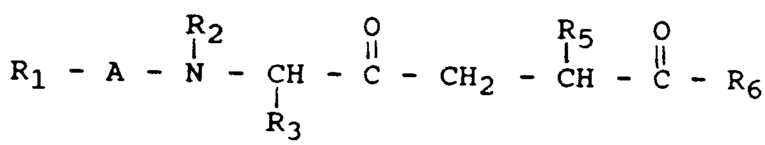

(II)

wherein the substituents have the meanings mentioned and free functional groups, with the exception of the keto group participating in the reaction, are optionally in protected form, to a hydroxy group by reaction with a suitable reducing agent, or
c) reacting an aldehyde compound of the formula

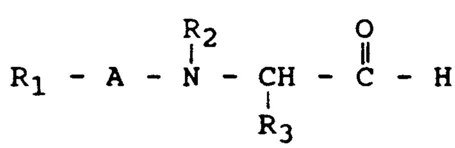

(III)

wherein the substituents have the meanings mentioned and free functional groups, with the exception of the aldehyde group, are optionally in protected form, with an organometal compound of the formula

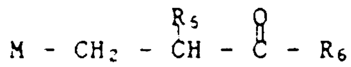

(IV)

wherein the substituents have the meanings mentioned and M is a metal radical, and hydrolysing the resulting addition product, or

d) in a compound of the formula

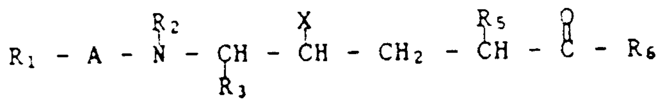

(V)

wherein X is a nucleofugal leaving group, the other substituents have the meanings mentioned above and free functional groups are optionally in protected form, exchanging the substituent X for $R_4$ with a reagent that introduces the substituent $R_4$ in nucleophilic form, or

e) in a compound of the formula

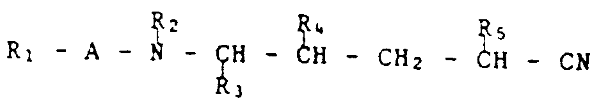

(VI)

wherein the substituents have the meanings mentioned and any functional groups present are optionally in protected form, converting the cyano group into an N-substituted carboxamido group $-(C=O)R_6$, or

f) reducing an epoxide of the formula

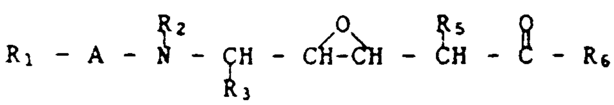

(VII)

wherein the substituents have the meanings mentioned and free functional groups are optionally in protected form, to the corresponding alcohol with a regioselective reducing agent and, if desired,

g) removing any protecting groups present in an obtainable compound and/or, if desired, after carrying out one of the processes a) to f) mentioned above or any other process for the preparation of a compound of formula I, converting an obtainable compound of formula I having a salt-forming group into its salt or an obtainable salt into the free compound or into a different salt and/or optionally separating obtainable isomeric mixtures and/or, in an obtainable compound of formula I, reversing the configuration of a chiral carbon atom, and/or converting a compound of formula I according to the invention into a different compound of formula I according to the invention.

**3.** A process for the preparation of a compound of formula I shown in claim 1, wherein

a) $R_1$ is N-benzyl-indolyl-2-carbonyl, A is the bivalent residue of the amino acid histidine, $R_2$ is hydrogen, $R_3$ is isobutyl, $R_4$ is hydroxy, $R_5$ is isopropyl and $R_6$ is methylamino, or

b) $R_1$ is 3-(R,S)-benzyloxycarbonyl-4-α-naphthylbutyryl, A is the bivalent residue of the amino acid histidine, $R_2$ is hydrogen, $R_3$ is isobutyl, $R_4$ is hydroxy, $R_5$ is isopropyl and $R_6$ is methylamino, or

c) $R_1$ is N-benzyloxycarbonyl-(p-benzyloxycarbonylamino-Phe), A is the bivalent residue of the amino acid histidine, $R_2$ is hydrogen, $R_3$ is isobutyl, $R_4$ is hydroxy, $R_5$ is isopropyl and $R_6$ is n-butylamino, or

d) $R_1$ is N-pivaloyl-(p-benzyloxycarbonylamino-Phe), A is the bivalent residue of the amino acid histidine, $R_2$ is hydrogen, $R_3$ is isobutyl, $R_4$ is hydroxy, $R_5$ is isopropyl and $R_6$ is n-butylamino, or

e) $R_1$ is indolyl-2-carbonyl, A is the bivalent residue of the amino acid histidine, $R_2$ is hydrogen, $R_3$ is isobutyl, $R_4$ is hydroxy, $R_5$ is isopropyl and $R_6$ is 4-[tris-tert-butyl-dimethylsilyloxymethyl)-methylcarbamoyl]-butylamino, or

f) $R_1$ is 2-ethylaminocarbonyloxy-4-phenyl-butyryl, A is the bivalent residue of the amino acid histidine, $R_2$ is hydrogen, $R_3$ is cyclohexylmethyl, $R_4$ is hydroxy, $R_5$ is isopropyl and $R_6$ is n-butylamino,

or a salt of such a compound having salt-forming groups, which comprises

a) condensing a fragment of a compound of formula I having a terminal carboxy group or a reactive acid derivative of that fragment with a fragment that is complementary to the compound of formula I and has a free amino group, or with a reactive derivative thereof having an activated amino group, to form an amide bond, any free functional groups present in the reactants, with the exception of the groups participating in the reaction, optionally being in protected form, or

b) reducing the keto group in a compound of the formula

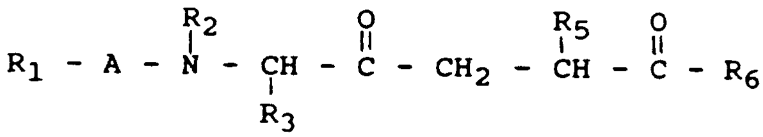

(II)

wherein the substituents have the meanings mentioned and free functional groups, with the exception of the keto group participating in the reaction, are optionally in protected form, to a hydroxy group by reaction with a suitable reducing agent, or

c) reacting an aldehyde compound of the formula

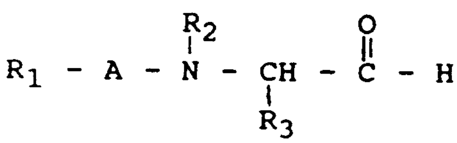

(III)

wherein the substituents have the meanings mentioned and free functional groups, with the exception of the aldehyde group, are optionally in protected form, with an organometal compound of the formula

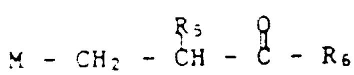

(IV)

wherein the substituents have the meanings mentioned and M is a metal radical, and hydrolysing the resulting addition product, or

d) in a compound of the formula

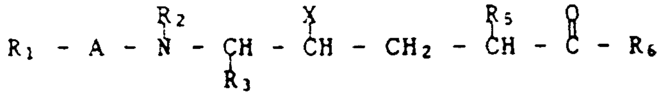

(V)

wherein X is a nucleofugal leaving group, the other substituents have the meanings mentioned above and free functional groups are optionally in protected form, exchanging the substituent X for $R_4$ with a reagent that introduces the substituent $R_4$ in nucleophilic form, or

e) in a compound of the formula

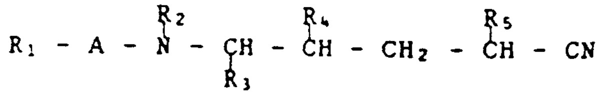 (VI)

wherein the substituents have the meanings mentioned and any functional groups present are optionally in protected form, converting the cyano group into an N-substituted carboxamido group $-(C=O)R_6$, or

f) reducing an epoxide of the formula

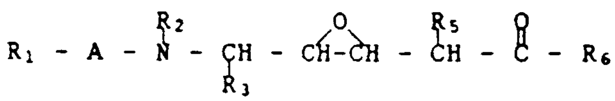 (VII)

wherein the substituents have the meanings mentioned and free functional groups are optionally in protected form, to the corresponding alcohol with a regioselective reducing agent and, if desired,

g) removing any protecting groups present in an obtainable compound and/or, if desired, after carrying out one of the processes a) to f) mentioned above or any other process for the preparation of a compound of formula I, converting an obtainable compound of formula I having a salt-forming group into its salt or an obtainable salt into the free compound or into a different salt and/or optionally separating obtainable isomeric mixtures and/or, in an obtainable compound of formula I, reversing the configuration of a chiral carbon atom, and/or converting a compound of formula I according to the invention into a different compound of formula I according to the invention.

**4.** A process for the preparation of a compound of formula I according to either claim 1 or claim 2, which comprises condensing a fragment of a compound of formula I having a terminal carboxy group or a reactive acid derivative of that fragment with a fragment that is complementary to the compound of formula I and has a free amino group, or with a reactive derivative thereof having an activated amino group, to form an amide bond, any free functional groups present in the reactants, with the exception of the groups participating in the reaction, optionally being in protected form and, if desired, removing any protecting groups present in an obtainable compound by treatment with an acid or base or by hydrogenolysis, and/or converting an obtainable compound of formula I having a salt-forming group into its salt or an obtainable salt into the free compound or into a different salt, and/or optionally separating obtainable isomeric mixtures, and/or converting a compound of formula I according to the invention into a different compound of formula I according to the invention.

**5.** A process for the preparation of a compound of formula I according to claim 3, which comprises condensing a fragment of a compound of formula I having a terminal carboxy group or a reactive acid derivative of that fragment with a fragment that is complementary to the compound of formula I and has a free amino group, or with a reactive derivative thereof having an activated amino group, to form an amide bond, any free functional groups present in the reactants, with the exception of the groups participating in the reaction, optionally being in protected form and, if desired, removing any protecting groups present in an obtainable compound by treatment with an acid or base or by hydrogenolysis, and/or converting an obtainable compound of formula I having a salt-forming group into its salt or an obtainable salt into the free compound or into a different salt, and/or optionally separating obtainable isomeric mixtures, and/or converting a compound of formula I according to the invention into a different compound of formula I according to the invention.

**6.** A process for the preparation of a compound of formula I according to either claim 1 or claim 2, which comprises condensing a fragment of a compound of formula I having a terminal carboxy group or an activated ester derived therefrom, a reactive anhydride derived therefrom or a reactive cyclic amide derived therefrom, with a fragment that is complementary to the compound of formula I and has a free amino group, any free functional groups present in the reactants, with the exception of the groups

participating in the reaction, optionally being in protected form, in the presence of a condensing agent selected from a carbodiimide, carbonyldiimidazole, 1,2-oxazolium compounds, a 1,2-dihydroquinoline or an activated phosphate, and, if desired, in the presence of an organic base or of alkali metal carbonates in an inert polar aprotic solvent in a temperature range of from -40 ° C to +100 ° C, activated esters of the amino or amido ester type if desired being formed in situ by the addition of an N-hydroxyamino or N-hydroxyamido compound, and, if desired, removing any protecting groups present in an obtainable compound by treatment with an acid or base or by hydrogenolysis, and/or converting an obtainable compound of formula I having a salt-forming group into its salt or an obtainable salt into the free compound or into a different salt, and/or optionally separating obtainable isomeric mixtures, and/or converting a compound of formula I according to the invention into a different compound of formula I according to the invention.

**7.** A process for the preparation of a compound of formula I according to claim 3, which comprises condensing a fragment of a compound of formula I having a terminal carboxy group or an activated ester derived therefrom, a reactive anhydride derived therefrom or a reactive cyclic amide derived therefrom, with a fragment that is complementary to the compound of formula I and has a free amino group, any free functional groups present in the reactants, with the exception of the groups participating in the reaction, optionally being in protected form, in the presence of a condensing agent selected from a carbodiimide, carbonyldiimidazole, 1,2-oxazolium compounds, a 1,2-dihydroquinoline or an activated phosphate, and, if desired, in the presence of an organic base or of alkali metal carbonates in an inert polar aprotic solvent in a temperature range of from -40 ° C to +100 ° C, activated esters of the amino or amido ester type if desired being formed in situ by the addition of an N-hydroxyamino or N-hydroxyamido compound, and, if desired, removing any protecting groups present in an obtainable compound by treatment with an acid or base or by hydrogenolysis, and/or converting an obtainable compound of formula I having a salt-forming group into its salt or an obtainable salt into the free compound or into a different salt, and/or optionally separating obtainable isomeric mixtures, and/or converting a compound of formula I according to the invention into a different compound of formula I according to the invention.

**8.** A process according to any one of claims 1, 2, 4 and 6, which comprises selecting such starting materials that a compound of formula I wherein $R_1$ is 2(S)-pivaloyloxy-3-phenyl-propionyl, 2(R)- and 2-(S)-dimethoxyphosphoryl-3-phenyl-propionyl, 2(R)- and 2(S)-benzyl-5,5-dimethyl-4-oxo-hexanoyl, 2(R)- and 2(S)-benzyl-4,4-dimethyl-3-oxo-pentanoyl, 2(R)- and 2(S)-tert-butylcarbamoyl-3-$\alpha$-naphthyl-propionylor 2(R)- and 2(S)-ethoxycarbonyl-3-$\alpha$-naphthyl-propionyl, indolyl-2-carbonyl or cyclohepta[b]-pyrrolyl-5-carbonyl, A is the bivalent residue of the amino acid L-histidine, $R_2$ is hydrogen, $R_3$ is cyclohexylmethyl, $R_4$ is hydroxy, $R_5$ is isopropyl and $R_6$ is $C_1$-$C_7$alkylamino, and the carbon atoms carrying the radicals $R_3$, $R_4$ and $R_5$ have the S-configuration, or a pharmaceutically acceptable salt thereof, is formed.

**9.** A process according to any one of claims 1, 2, 4, 6 and 8, which comprises selecting such starting materials that the compound of formula I wherein $R_1$ is 2(S)-pivaloyloxy-3-phenyl-propionyl, A is the bivalent residue of the amino acid L-histidine, $R_2$ is hydrogen, $R_3$ is cyclohexylmethyl, $R_4$ is hydroxy, $R_5$ is isopropyl and $R_6$ is n-butylamino, and the carbon atoms carrying the radicals $R_3$, $R_4$ and $R_5$ have the S-configuration, or a pharmaceutically acceptable salt thereof, is formed.

**10.** A process according to any one of claims 1, 2, 4, 6 and 8, which comprises selecting such starting materials that a compound of formula I wherein $R_1$ is 2(R,S)-, 2(R)-or 2(S)-dimethoxyphosphoryl-3-phenyl-propionyl, A is the bivalent residue of the amino acid L-histidine, $R_2$ is hydrogen, $R_3$ is cyclohexylmethyl, $R_4$ is hydroxy, $R_5$ is isopropyl and $R_6$ is n-butylamino, and the carbon atoms carrying the radicals $R_3$, $R_4$ and $R_5$ have the S-configuration, or a pharmaceutically acceptable salt thereof, is formed.

**11.** A process according to any one of claims 1, 2, 4, 6 and 8, which comprises selecting such starting materials that a compound of formula I wherein $R_1$ is 2(R,S)-, 2(R)-or 2(S)-benzyl-5,5-dimethyl-4-oxo-hexanoyl, A is the bivalent residue of the amino acid L-histidine, $R_2$ is hydrogen, $R_3$ is cyclohexylmethyl, $R_4$ is hydroxy, $R_5$ is isopropyl and $R_6$ is n-butylamino, and the carbon atoms carrying the radicals $R_3$, $R_4$ and $R_5$ have the S-configuration, or a pharmaceutically acceptable salt thereof, is formed.

**12.** A process according to any one of claims 1, 2, 4, 6 and 8, which comprises selecting such starting materials that the compound of formula I wherein $R_1$ is 2(R,S)-benzyl-4,4-dimethyl-3-oxo-pentanoyl, A is the bivalent residue of the amino acid L-histidine, $R_2$ is hydrogen, $R_3$ is cyclohexylmethyl, $R_4$ is hydroxy, $R_5$ is isopropyl and $R_6$ is n-butylamino, and the carbon atoms carrying the radicals $R_3$, $R_4$ and $R_5$ have the S-configuration, or a pharmaceutically acceptable salt thereof, is formed.

**13.** A process according to any one of claims 1, 2, 4, 6 and 8, which comprises selecting such starting materials that the compound of formula I wherein $R_1$ is 2(R,S)-ethoxycarbonyl-3-$\alpha$-naphthyl-propionyl, A is the bivalent residue of the amino acid L-histidine, $R_2$ is hydrogen, $R_3$ is cyclohexylmethyl, $R_4$ is hydroxy, $R_5$ is isopropyl and $R_6$ is n-butylamino, and the carbon atoms carrying the radicals $R_3$, $R_4$ and $R_5$ have the S-configuration, or a pharmaceutically acceptable salt thereof, is formed.

**14.** A process according to any one of claims 1, 2, 4, 6 and 8, which comprises selecting such starting materials that the compound of formula I wherein $R_1$ is cyclohepta[b]pyrrolyl-5-carbonyl, A is the bivalent residue of the amino acid L-histidine, $R_2$ is hydrogen, $R_3$ is cyclohexylmethyl, $R_4$ is hydroxy, $R_5$ is isopropyl and $R_6$ is n-butylamino, and the carbon atoms carrying the radicals $R_3$, $R_4$ and $R_5$ have the S-configuration, or a pharmaceutically acceptable salt thereof, is formed.

**15.** A process according to any one of claims 1, 2, 4, 6 and 8, which comprises selecting such starting materials that the compound of formula I wherein $R_1$ is 2(S)-pivaloyloxy-3-phenyl-propionyl, A is the bivalent residue of the amino acid L-histidine, $R_2$ is hydrogen, $R_3$ is isobutyl, $R_4$ is hydroxy, $R_5$ is cyclohexylmethyl and $R_6$ is n-butylamino, and the carbon atoms carrying the radicals $R_3$ and $R_4$ have the S-configuration, or a pharmaceutically acceptable salt thereof, is formed.

**16.** A process for the preparation of pharmaceutical compositions which comprises mixing a compound of formula I prepared according to a process defined in any one of claims 1 to 15, or a pharmaceutically acceptable salt of such a compound of formula I having salt-forming groups, with a pharmaceutical carrier.

**17.** A process for the preparation of a compound of the formula

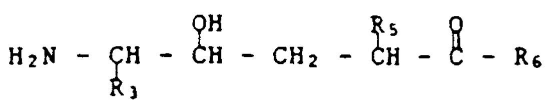

(VIII)

wherein $R_3$ is cyclohexylmethyl and $R_5$ and $R_6$ have the meanings mentioned in claim 1 or 2, or a salt thereof, which comprises converting a compound of the formula

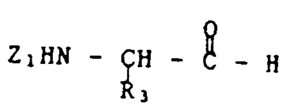

(IX)

wherein $R_3$ has the meaning mentioned and $Z_1$ is an amino-protecting group, with a sulfur-ylide compound into an epoxide of the formula

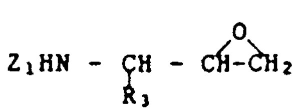

(X)

wherein $R_3$ and $Z_1$ have the meanings mentioned, reacting the obtainable compound (X), optionally after separation of the isomers, with a reagent that introduces a nucleofugal leaving group X, reacting the obtainable compound of the formula

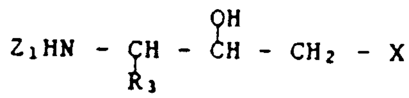

(XI)

wherein $R_3$ and $Z_1$ have the meanings mentioned and X is a nucleofugal leaving group, with a carbonyl compound of the formula

$R_a$ - (C = O) -$R_b$ (XII)

wherein each of the radicals $R_a$ and $R_b$, independently of the other, is hydrogen, lower alkyl, aryl or aryl-lower alkyl, or $R_a$ and $R_b$ together are optionally bridged alkylidene having from 4 to 12 carbon atoms, or with a reactive derivative of that carbonyl compound, in the presence of an acidic reagent, reacting the obtainable compound of the formula

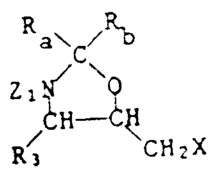

(XIII)

wherein the substituents have the meanings mentioned, with a carboxylic acid ester salt of the formula

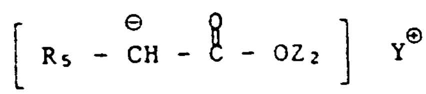

(XIV)

wherein $R_5$ has the meaning mentioned under formula I, $Z_2$ is a carboxy-protecting group and $Y^{\oplus}$ is a cation, for example an alkali metal, for example the sodium or preferably the lithium ion, and in an obtainable compound of the formula

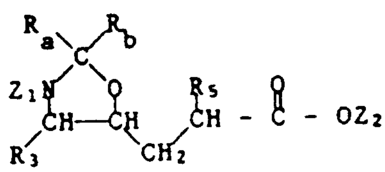

(XV)

wherein the substituents have the meanings mentioned, removing the carboxy-protecting group $Z_2$ and/or separating an obtainable isomeric mixture into the individual isomers, amidating the obtainable compound having a free carboxy group ($Z_2$ = H) with an amine $R_6$-H, and in an obtainable compound of the formula

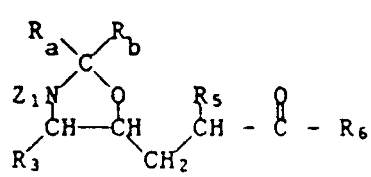

(XVI)

wherein the substituents have the meanings mentioned, opening the ring with a suitable solvolysis reagent and optionally removing the protecting group $Z_1$.

**Revendications**

**Revendications pour les Etats contractants suivants : BE, CH, DE, FR, GB, IT, LI, LU, NL, SE**

**1.** Composés de formule

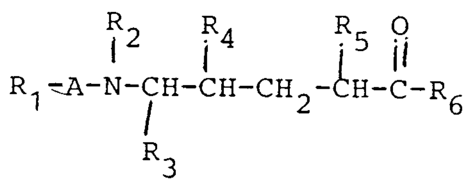

(I),

dans laquelle $R_1$ représente un groupe alcanoyle en C 1-C 7, phénoxy-alcanoyle en C 1-C 7, naphtoxy-alcanoyle en C 1-C 7, alpha-naphtoxy-carboxy-alcanoyle en C 1-C 7, alpha-naphtoxy-(alcoxy en C 1-C 7)-carbonyl-alcanoyle en C 1-C 7, alpha-naphtoxy-benzyloxycarbonyl-alcanoyle en C 1-C 7, alpha-naphtoxy-carbamoyl-alcanoyle en C 1-C 7, alpha-naphtoxy-cyano-alcanoyle en C 1-C 7, alpha-naphtoxy-di-(alkyle en C 1-C 7)-amino-alcanoyle en C 1-C 7, alpha-naphtoxy-oxo-alcanoyle en C 1-C 7, phényl-, alpha-naphtyl- ou bêta-naphtyl-alcanoyle en C 1-C 7 dans lesquels la partie alcanoyle en C 1-C 7 est non substituée ou peut être substituée par des groupes hydroxy, alcoxy en C 1-C 7, alcanoyloxy en C 1-C 7, carboxy, (alcoxy en C 1-C 7)-carbonyle, carbamoyle, (alkyle en C 1 - C 7)-carbamoyle, di-(alkyle en C 1-C 7)-carbamoyle, cyano-, phosphono, di-(alcoxy en C 1-C 7)-phosphoryle, benzofurannyle et/ou oxo et éventuellement ramifiée, un groupe 2-phényléthyle, 3-phényl-2-propyle, 4-phényl-3-butyle, 2-alpha-naphtyléthyle, 3-alpha-naphtyl-2-propyle ou 4-alpha-naphtyl-3-butyle relié par l'intermédiaire d'un groupe carbonyle -CO- et qui est substitué en position 1 par un groupe acyloxy sous la forme d'un groupe éthylaminocarbonyloxy, 2-benzyloxycarbonylamino-2-méthylpropionyloxy, 2-amino-2-méthyl-propionyloxy ou acétoacétoxy, par un groupe carboxy estérifié sous la forme d'un groupe benzyloxycarbonyle, par un groupe carbamoyle substitué sous la forme d'un groupe carboxy-méthylcarbamoyle, tert-butoxycarbonylméthylcarbamoyle, 2-diméthylaminoéthylcarbamoyle, 3-hydroxy-2-propylcarbamoyle, 2,2-diméthoxyéthylcarbamoyle ou 5-amino-5-carboxypentylcarbamoyle, ou par un groupe phosphono estérifié sous la forme d'un groupe hydroxyméthoxyphosphoryle, un groupe 3-phényl-2-éthoxy-hydroxy-phosphoryl-propionyle, 3-phényl- ou 3-alpha-naphtyl-2-diméthylamino-méthyl-propionyle, 4-phényl-ou 4-alpha-naphtyl-3-benzyloxycarbonyl-butyryle, 2-benzyl-ou 2-alpha-naphtyl-méthyl-4,4-diméthyl-3-oxo-pentanoyle, 2-benzyl- ou 2-alpha-naphtylméthyl-5-diméthylaminopentanoyle, 2-benzyl- ou 2-alpha-naphtylméthyl-5-diméthylamino-4-oxo-pentanoyle, 2-benzyl- ou 2-alpha-naphtylméthyl-5,5-diméthyl-4-oxo-hexanoyle, alpha,p-diaminophénylacétyle, alpha,p-dibenzyloxycarbonylamino-phénylacétyle, alpha-pivaloylamino-p-benzyloxycarbonylaminophénylacétyle, 2-(o,o'-dichloranilino)-phénylacétyle, 2-(o,o-dichloro-N-benzylanilino)-phénylacétyle, alpha-ou bêta-naphtylcarbonyle, 1,8-naphtalène-dicarbonyle, pyrrolylcarbonyle, cyclohepta[b]pyrrolyl-5-carbonyle, indolylcarbonyle, 1-benzylindolyl-3-carbonyle, 4,5,6,7-tétrahydroindolyl-2-carbonyle, quinoléylcarbonyle ou oxamoyle,

A représente le radical bivalent des aminoacides leucine, norleucine, phénylalanine, N-méthyl-phénylalanine, bêta-phénylsérine, cyclohexylalanine, glutamine, histidine, ou N-méthylhistidine, $R_2$ représente l'hydrogène, $R_3$ représente un groupe isobutyle ou cyclohexylméthyle, $R_4$ représente un groupe hydroxy, $R_5$ représente un groupe isopropyle, cyclohexylméthyle, alpha-décahydronaphtyle ou diméthylamino, et

$R_6$ représente un groupe alkylamino en C 1-C 7, di-(alkyle en C 1-C 7)-amino, hydroxy-alkylamino en C 1-C 7, 2-phénoxyéthylamino, 2-(3-carbamoyl-4-hydroxyphénoxy)-éthylamino, carboxy-alkylamino ou amino-carboxy-alkylamino dans lesquels le groupe carboxy ne peut se trouver en position 1 de la partie alkyle, laquelle contient jusqu'à 10 atomes de carbone, (alcoxy en C 1-C 7)-carbonyl-alkylamino ou (-(alcanoyle en C 1-C 7)-amino-, (alcoxy en C 1-C 7)-carbonylamino- ou benzyloxycarbonylamino-)-(alcoxy en C 1-C 7)-carbonyl-alkylamino dans lesquels le groupe carbonyle ne peut se trouver en position 1 de la partie alkyle, laquelle contient jusqu'à 10 atomes de carbone, 4-tris-(hydroxyméthyl)-méthylcarbamoyl-n-butylamino, amino-(alkyle en C 1-C 7)-amino, di-(alkyle en C 1-C 7)-amino-(alkyle en C 1-C 7)-amino, (alcoxy en C 1-C 7)-carbonylamino-(alkyle en C 1-C 7)-amino, morpholino-(alkyle en C 1-C 7)-amino, cycloalkyl-(alkyle en C 1-C 7)-amino en C 4-C 10, benzylamino, pyridyl-(alkyle en C 1-C 7)-amino, imidazolyl-(alkyle en C 1-C 7)-amino ou 2-(2-[4-imidazolyl)-éthylamino]-éthylamino,

et les sels acceptables pour l'usage pharmaceutique de ceux de ces composés qui contiennent des

groupes salifiables, à l'exception du composé de formule I dans laquelle $R_1$ représente un groupe alpha-naphtoxyacétyle, A le radical bivalent de l'amino-acide histidine, $R_2$ l'hydrogène, $R_3$ un groupe isobutyle, $R_4$ un groupe hydroxy, $R_5$ un groupe isopropyle et $R_6$ un groupe cyclohexylméthylamino.

**2.** Composés de formule I de la revendication 1, dans laquelle $R_1$ représente un groupe phénoxy-alcanoyle en C 1-C 7, naphtoxy-alcanoyle en C 1-C 7, phénylalcanoyle en C 1-C 7 dans lesquels la partie alcanoyle en C 1-C 7 est non substituée ou peut être substituée par des groupes alcanoyloxy en C 1-C 7, carboxy, (alcoxy en C 1-C 7)-carbonyle, carbamoyle, (alkyle en C 1-C 7)-carbamoyle, cyano, di-(alcoxy en C 1-C 7)-phosphoryle ou alkyle en C 1-C 7 et oxo ou benzofurannyle et oxo et éventuellement ramifiée, un groupe naphtyl-alcanoyle en C 1-C 7 dans lequel la partie alcanoyle en C 1-C 7 est non substituée ou peut être substituée par des groupes alcanoyloxy en C 1-C 7, carboxy, (alcoxy en C 1-C 7)-carbonyle, carbamoyle, cyano ou alkyle en C 1-C 7 et oxo et éventuellement ramifiée, un groupe 2-tert-butylcarbamoyl-3-alpha-naphtyl-propionyle, 2-carboxy-méthyl-carbamoyl-3-alpha-naphtyl-propionyle, indolyl-2-carbonyle ou cyclohepta[b]pyrrolyl-5-carbonyle. A représente le radical bivalent de l'aminoacide L-histidine, $R_2$ l'hydrogène, $R_3$ un groupe isobutyle ou cyclohexylméthyle, $R_4$ un groupe hydroxy, $R_5$ un groupe isopropyle ou cyclohexylméthyle et $R_6$ un groupe alkylamino en C 1-C 7 ou amino-carboxy-alkylamino dans lesquels les substituants ne peuvent se trouver dans la position 1 du groupe alkyle, lequel contient jusqu'à 7 atomes de carbone, et les atomes de carbone portant les substituants $R_3$ et $R_4$ sont en configuration S, ainsi que les sels de ces composés acceptables pour l'usage pharmaceutique.

**3.** Composés de formule I selon revendication 1 ou 2, dans lesquels $R_1$ représente un groupe 2(S)-pivaloyloxy-3-phényl-propionyle, 2(R)- et 2(S)-diméthoxy-phosphoryl-3-phényl-propionyle, 2(R)- et 2(S)-benzyl-5,5-diméthyl-4-oxo-hexanoyle, 2(R)- et 2(S)-benzyl-4,4-diméthyl-3-oxo-pentanoyle, 2(R)- et 2(S)-tert-butylcarbamoyl-3-alpha-naphtyl-propionyle ou 2(R)- et 2(S)-éthoxy-carbonyl-3-alpha-naphtyl-propionyle, indolyl-2-carbonyle ou cyclohepta[b]pyrrolyl-5-carbonyle, A représente le radical bivalent de l'aminoacide L-histidine, $R_2$ l'hydrogène, $R_3$ un groupe cyclohexylméthyle, $R_4$ un groupe hydroxy, $R_5$ un groupe isopropyle et $R_6$ un groupe alkylamino en C 1-C 7, et les atomes de carbone portant les substituants $R_3$, $R_4$ et $R_5$ sont en configuration S, ainsi que les sels acceptables pour l'usage pharmaceutique de ces composés.

**4.** Le composé de formule I selon revendication 1, 2 ou 3, pour lequel $R_1$ représente un groupe 2(S)-pivaloyloxy-3-phényl-propionyle, A le radical bivalent de l'aminoacide L-histidine, $R_2$ l'hydrogène, $R_3$ un groupe cyclohexylméthyle, $R_4$ un groupe hydroxy, $R_5$ un groupe isopropyle et $R_6$ un groupe n-butylamino et les atomes de carbone portant les substituants $R_3$, $R_4$ et $R_5$ sont en configuration S, et les sels acceptables pour l'usage pharmaceutique de ce composé.

**5.** Composés selon revendication 1, 2 ou 3 de formule I dans laquelle $R_1$ représente un groupe 2(R,S)-, 2-(R)- ou 2(S)-diméthoxyphosphoryl-3-phényl-propionyle, A le radical bivalent de l'aminoacide L-histidine, $R_2$ l'hydrogène, $R_3$ un groupe cyclohexylméthyle, $R_4$ un groupe hydroxy, $R_5$ un groupe isopropyle et $R_6$ un groupe n-butylamino, et les atomes de carbone portant les substituants $R_3$, $R_4$ et $R_5$ sont en configuration S, et leurs sels acceptables pour l'usage pharmaceutique.

**6.** Composés selon revendication 1, 2 ou 3, de formule I dans laquelle $R_1$ représente un groupe 2(R,S)-, 2(R)- ou 2(S)-benzyl-5,5-diméthyl-4-oxo-hexanoyle, A le radical bivalent de l'aminoacide L-histidine, $R_2$ l'hydrogène, $R_3$ un groupe cyclohexylméthyle, $R_4$ un groupe hydroxy, $R_5$ un groupe isopropyle et $R_6$ un groupe n-butylamino, et les atomes de carbone portant les substituants $R_3$, $R_4$ et $R_5$ sont en configuration S, et leurs sels acceptables pour l'usage pharmaceutique.

**7.** Le composé selon revendication 1, 2 ou 3, de formule I dans laquelle $R_1$ représente le groupe 2(R,S)-benzyl-4,4-diméthyl-3-oxo-pentanoyle, A le radical bivalent de l'aminoacide L-histidine, $R_2$ l'hydrogène, $R_3$ un groupe cyclohexylméthyle, $R_4$ un groupe hydroxy, $R_5$ un groupe isopropyle et $R_6$ un groupe n-butylamino, et les atomes de carbone portant les substituants $R_3$, $R_4$ et $R_5$ sont en configuration S, et ses sels acceptables pour l'usage pharmaceutique.

**8.** Le composé selon revendication 1, 2 ou 3, de formule I dans laquelle $R_1$ représente le groupe 2(R,S)-éthoxycarbonyl-3-alpha-naphtyl-propionyle, A le radical bivalent de l'aminoacide L-histidine, $R_2$ l'hydrogène, $R_3$ un groupe cyclohexylméthyle, $R_4$ un groupe hydroxy, $R_5$ un groupe isopropyle et $R_6$ un

groupe n-butylamino, et les atomes de carbone portant les substituants $R_3$, $R_4$ et $R_5$ sont en configuration S, et ses sels acceptables pour l'usage pharmaceutique.

**9.** Le composé selon revendication 1, 2 ou 3, de formule I dans laquelle $R_1$ représente un groupe cyclohepta[b]pyrrolyl-5-carbonyle, A le radical bivalent de l'aminoacide L-histidine, $R_2$ l'hydrogène, $R_3$ un groupe cyclohexylméthyle, $R_4$ un groupe hydroxy, $R_5$ un groupe isopropyle et $R_6$ un groupe n-butylamino, et les atomes de carbone portant les substituants $R_3$, $R_4$ et $R_5$ sont en configuration S, et ses sels acceptables pour l'usage pharmaceutique.

**10.** Le composé selon revendication 1, 2 ou 3, de formule I dans laquelle $R_1$ représente un groupe 2(S)-pivaloyloxy-3-phényl-propionyle, A le radical bivalent de l'aminoacide L-histidine, $R_2$ l'hydrogène, $R_3$ un groupe isobutyle, $R_4$ un groupe hydroxy, $R_5$ un groupe cyclohexylméthyle et $R_6$ un groupe n-butylamino, et les atomes de carbone portant les substituants $R_3$ et $R_4$ sont en configuration S, et ses sels acceptables pour l'usage pharmaceutique.

**11.** Les composés de formule I de la revendication 1 énumérés ci-après :

N-(N-benzyl-indolyl-2-carbonyl)-His-Leu$\overset{C}{-}$Val-méthylamide,
N-(3(R,S)-benzyloxycarbonyl-4-alpha-naphtylbutyryl)-His-Leu$\overset{C}{-}$Val-méthylamide,
N-benzyloxycarbonyl-(p-benzyloxycarbonylamino-Phe)-His-Leu$\overset{C}{-}$Val-n-butylamide,
N-pivaloyl-(p-benzyloxycarbonylamino-Phe)-His-Leu$\overset{C}{-}$Val-n-butylamide,
N-(indolyl-2-carbonyl)-His-Leu$\overset{C}{-}$Val-4-[tris-(tert-butyl-diméthylsilyloxyméthyl)-méthylcarbamoyl]-butylamide,

N-(2-éthylaminocarbonyloxy-4-phényl-butyryl)-His-Cha$\overset{C}{-}$Val-n-butylamide,

et leurs sels acceptables pour l'usage pharmaceutique.

**12.** Compositions pharmaceutiques contenant des composés selon revendication 1, 2, 3 ou 11, de formule I, ou des sels acceptables pour l'usage pharmaceutique de ceux de ces composés qui portent des groupes salifiables, avec un véhicule pharmaceutique.

**13.** Utilisation des composés selon revendication 1, 2, 3 ou 11, de formule I, ou de sels acceptables pour l'usage pharmaceutique de composés de formule I, pour la préparation d'un médicament pour le traitement de l'hypertension sanguine et de l'insuffisance cardiaque.

**14.** Procédé de préparation des composés répondant à la formule I dans laquelle les symboles ont les significations indiquées dans la revendication 1, 2, 3 ou 11, caractérisé en ce que :

a) on condense un fragment d'un composé de formule I portant un groupe carboxy terminal ou un dérivé d'acide réactif d'un tel fragment avec un fragment complémentaire pour la formation du composé de formule I, portant un groupe amino libre, ou avec un dérivé réactif d'un tel fragment portant un groupe amino activé, les groupes fonctionnels libres présents dans les composants de la réaction étant éventuellement à l'état protégé, à l'exception des groupes qui participent à la réaction, avec formation d'une liaison amide, ou bien

b) on réduit en groupe hydroxy le groupe céto d'un composé de formule

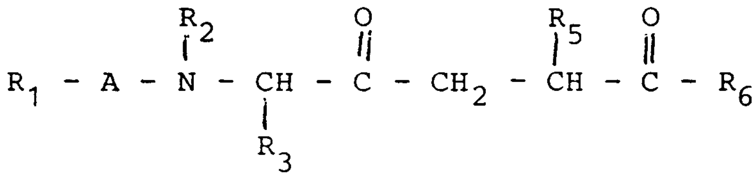 (II),

dans laquelle les symboles ont les significations indiquées ci-dessus et les groupes fonctionnels libres, à l'exception du groupe céto participant à la réaction, étant éventuellement à l'état protégé, par réaction avec un agent réducteur approprié, ou bien

c) on fait réagir un composé aldéhydique de formule

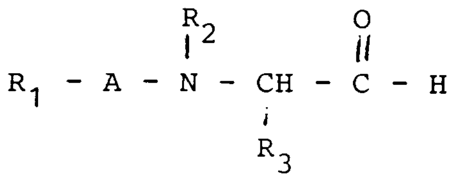 (III),

dans laquelle les symboles ont les significations indiquées ci-dessus et les groupes fonctionnels libres, à l'exception du groupe aldéhyde, sont éventuellement à l'état protégé, avec un dérivé organométallique de formule

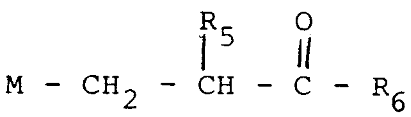 (IV),

dans laquelle les symboles ont les significations indiquées ci-dessus, et M représente un radical métallique, et on hydrolyse le produit formé par addition, ou bien

d) dans un composé de formule

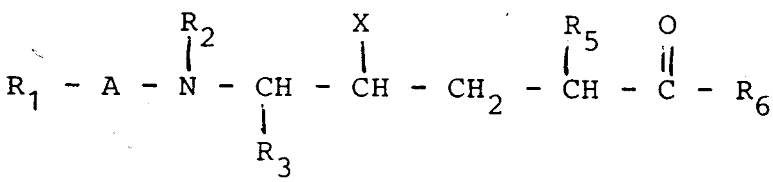 (V),

dans laquelle X représente un groupe éliminable nucléofuge, les autres symboles ayant les significations indiquées ci-dessus et les groupes fonctionnels libres étant éventuellement à l'état protégé, on échange le substituant X contre le substituant $R_4$ à l'aide d'un réactif introduisant ce substituant sous forme nucléophile, ou bien

e) dans un composé de formule

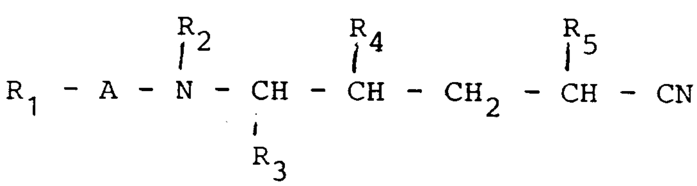 (VI),

dans laquelle les symboles ont les significations indiquées ci-dessus et les groupes fonctionnels

présents sont éventuellement à l'état protégé, on convertit le groupe cyano en un groupe carboxamido substitué à l'azote -(C = O)$R_6$, ou bien

f) on réduit un époxyde de formule

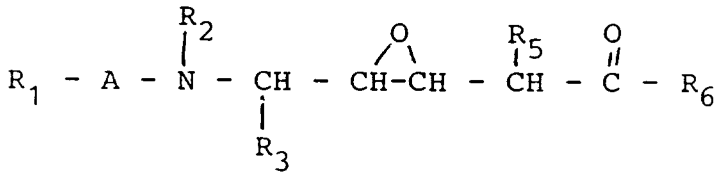

(VII),

dans laquelle les symboles ont les significations indiquées ci-dessus et les groupes fonctionnels libres sont éventuellement à l'état protégé, en l'alcool correspondant au moyen d'un agent réducteur régio-sélectif, et si on le désire

g) on élimine les groupes protecteurs existant dans un composé ainsi obtenu et/ou si on le désire, après la mise en oeuvre de l'un des procédés a) à f) ci-dessus ou d'un autre procédé quelconque de préparation d'un composé de formule I, on convertit un composé obtenu répondant à la formule I et portant un groupe salifiable en un sel ou un sel obtenu en le composé libre ou en un autre sel et/ou on sépare les mélanges d'isomères éventuellement obtenus et/ou dans un composé obtenu, répondant à la formule I, on inverse la configuration d'un atome de carbone chiral et/ou on convertit un composé de formule I selon l'invention en un autre composé de formule I selon l'invention.

**15.** Composés de formule

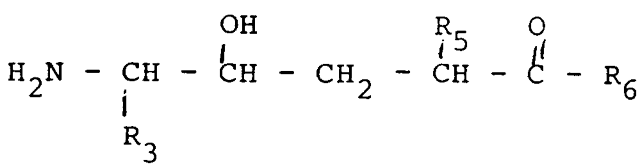

(VIII),

dans laquelle $R_3$ représente un groupe cyclohexylméthyle et $R_5$ et $R_6$ ont les significations indiquées dans la revendication 1, 2 ou 3, et leurs sels.

**16.** Procédé de préparation des composés de formule VIII de la revendication 15, dans laquelle les symboles ont les significations indiquées dans la revendication 15, caractérisé en ce que l'on convertit un composé de formule

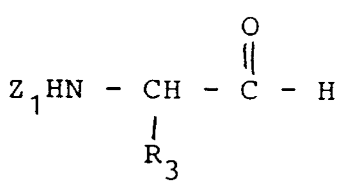

(IX),

dans laquelle $R_3$ a la signification indiquée ci-dessus et $Z_1$ représente un groupe protecteur du groupe amino, à l'aide d'un ylide de soufre, en un époxyde de formule

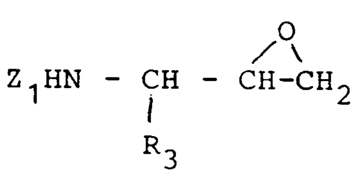

(X)

dans laquelle $R_3$ et $Z_1$ ont les significations indiquées ci-dessus, on fait réagir le composé X ainsi

obtenu, éventuellement après séparation des isomères, avec un réactif introduisant un groupe éliminable nucléofuge X, ce qui donne un composé de formule

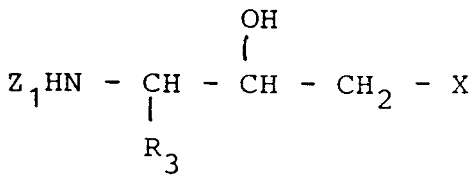

(XI)

dans laquelle $R_3$ et $Z_1$ ont les significations indiquées ci-dessus et X représente un groupe éliminable nucléofuge, qu'on fait réagir avec un composé carbonylé de formule

$R_a$ - (C = O) - $R_b$ (XII),

dans laquelle les symboles $R_a$ et $R_b$ représentent chacun, indépendamment l'un de l'autre, l'hydrogène, un groupe alkyle inférieur, aryle ou aryl-alkyle inférieur ou bien forment ensemble, par liaison, un pont alkylidène en C 4-C 12, ou avec un dérivé réactif d'un tel composé carbonylé, en présence d'un réactif acide, ce qui donne un composé de formule

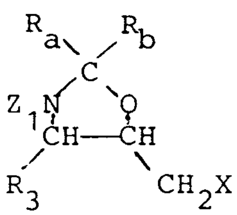

(XIII),

dans laquelle les symboles ont les significations indiquées ci-dessus, qu'on fait réagir avec un sel -ester d'acide carboxylique de formule

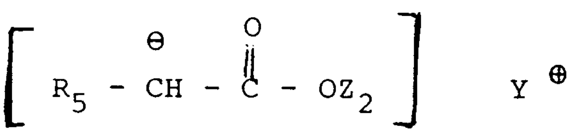

(XIV),

dans laquelle $R_5$ a les significations indiquées en référence à la formule I, $Z_2$ représente un groupe protecteur du groupe carboxy et $Y^{\oplus}$ représente un cation, par exemple un ion de métal alcalin tel que l'ion sodium ou de préférence l'ion lithium, ce qui donne un composé de formule

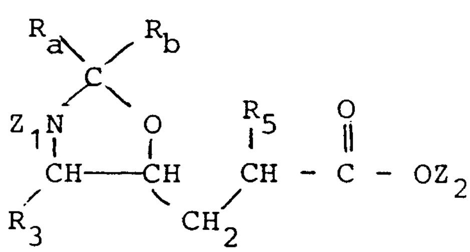

(XV),

dans laquelle les symboles ont les significations indiquées ci-dessus, d'où l'on élimine le groupe protecteur $Z_2$ du groupe carboxy, et/ou, lorsqu'il s'agit d'un mélange d'isomères, qu'on sépare en les isomères individuels, puis on amide ce composé portant un groupe carboxyle libre ($Z_2$ = H) à l'aide d'une amine $R_6$-H, ce qui donne un composé de formule

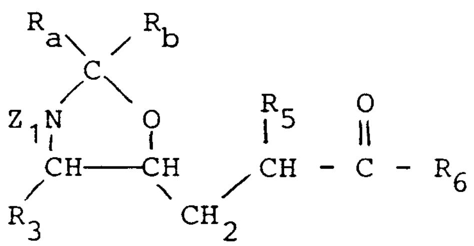

(XVI),

dans laquelle les symboles ont les significations indiquées ci-dessus, dont on ouvre le cycle à l'aide d'un réactif approprié de solvolyse, et d'où le cas échéant on élimine le groupe protecteur $Z_1$.

**Revendications pour l'Etat contractant suivant : AT**

**1.** Procédé de préparation d'un composé de formule

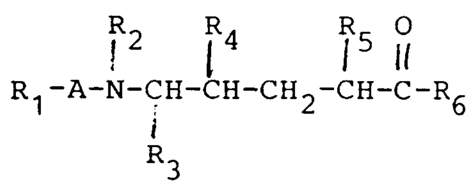

(I),

dans laquelle $R_1$ représente un groupe alcanoyle en C 1-C 7, phénoxy-alcanoyle en C 1-C 7, naphtoxy-alcanoyle en C 1-C 7, alpha-naphtoxy-carboxy-alcanoyle en C 1-C 7, alpha-naphtoxy-(alcoxy en C 1-C 7)-carbonyl-alcanoyle en C 1-C 7, alpha-naphtoxy-benzyloxycarbonyl-alcanoyle en C 1-C 7, alpha-naphtoxy-carbamoyl-alcanoyle en C 1-C 7, alpha-naphtoxy-cyano-alcanoyle en C 1-C 7, alpha-naphtoxy-di-(alkyle en C 1-C 7)-amino-alcanoyle en C 1-C 7, alpha-naphtoxy-oxo-alcanoyle en C 1-C 7, phényl-, alpha-naphtyl- ou bêta-naphtyl-alcanoyle en C 1-C 7 dans lesquels la partie alcanoyle en C 1-C 7 est non substituée ou peut être substituée par des groupes hydroxy, alcoxy en C 1-C 7, alcanoyloxy en C 1-C 7, carboxy, (alcoxy en C 1-C 7)-carbonyle, carbamoyle, (alkyle en C 1-C 7)-carbamoyle, di-(alkyle en C 1-C 7)-carbamoyle, cyano-, phosphono, di-(alcoxy en C 1-C 7)-phosphoryle, benzofurannyle et/ou oxo et éventuellement ramifiée, un groupe 2-phényléthyle, 3-phényl-2-propyle, 4-phényl-3-butyle, 2-alpha-naphtyléthyle, 3-alpha-naphtyl-2-propyle ou 4-alpha-naphtyl-3-butyle relié par l'intermédiaire d'un groupe carbonyle -CO- et qui est substitué en position 1 par un groupe acyloxy sous la forme d'un groupe éthylaminocarbonyloxy, 2-benzyloxycarbonylamino-2-méthyl-propionyloxy, 2-amino-2-méthyl-propionyloxy ou acétoacétoxy, par un groupe carboxy estérifié sous la forme d'un groupe benzyloxycarbonyle, par un groupe carbamoyle substitué sous la forme d'un groupe carboxyméthylcarbamoyle, tert-butoxycarbonylméthylcarbamoyle, 2-diméthylaminoéthylcarbamoyle, 3-hydroxy-2-propylcarbamoyle, 2,2-diméthoxyéthylcarbamoyle ou 5-amino-5-carboxypentylcarbamoyle, ou par un groupe phosphono estérifié sous la forme d'un groupe hydroxyméthoxyphosphoryle, un groupe 3-phényl-2-éthoxy-hydroxy-phosphoryl-propionyle, 3-phényl- ou 3-alpha-naphtyl-2-diméthylamino-méthyl-propionyle, 4-phényl-ou 4-alpha-naphtyl-3-benzyloxycarbonyl-butyryle, 2-benzyl-ou 2-alpha-naphtylméthyl-4,4-diméthyl-3-oxo-pentanoyle, 2-benzyl- ou 2-alpha-naphtylméthyl-5-diméthylamino-pentanoyle, 2-benzyl- ou 2-alpha-naphtylméthyl-5-diméthylamino-4-oxo-pentanoyle, 2-benzyl- ou 2-alpha-naphtylméthyl-5,5-diméthyl-4-oxo-hexanoyle,alpha,p-diaminophénylacétyle, alpha,p-dibenzyloxycarbonylaminophénylacétyle, alpha-pivaloylamino-p-benzyloxycarbonylaminophénylacétyle, 2-(o,o'-dichloranilino)-phénylacétyle, 2-(o,o-dichloro-N-benzylanilino)-phénylacétyle, alpha-ou bêta-naphtylcarbonyle, 1,8-naphtalène-dicarbonyle, pyrrolylcarbonyle, cyclohepta[b]pyrrolyl-5-carbonyle, indolylcarbonyle, 1-benzylindolyl-3-carbonyle, 4,5,6,7-tétrahydroindolyl-2-carbonyle, quinoléylcarbonyle ou oxamoyle,
A représente le radical bivalent des aminoacides leucine, norleucine, phénylalanine, N-méthyl-phénylalanine, bêta-phénylsérine, cyclohexylalanine, glutamine, histidine, ou N-méthylhistidine, $R_2$ représente l'hydrogène, $R_3$ représente un groupe isobutyle ou cyclohexylméthyle, $R_4$ représente un groupe hydroxy, $R_5$ représente un groupe isopropyle, cyclohexylméthyle, alpha-décahydronaphtyle ou diméthylamino, et
$R_6$ représente un groupe alkylamino en C 1-C 7, di-(alkyle en C 1-C 7)-amino, hydroxy-alkylamino en C

1-C 7, 2-phénoxyéthylamino, 2-(3-carbamoyl-4-hydroxyphénoxy)-éthylamino, carboxy-alkylamino ou amino-carboxy-alkylamino dans lesquels le groupe carboxy ne peut se trouver en position 1 de la partie alkyle, laquelle contient jusqu'à 10 atomes de carbone, (alcoxy en C 1-C 7)-carbonyl-alkylamino ou (-(alcanoyle en C 1-C 7)-amino-, (alcoxy en C 1-C 7)-carbonylamino- ou benzyloxycarbonylamino-)-(alcoxy en C 1-C 7)-carbonyl-alkylamino dans lesquels le groupe carbonyle ne peut se trouver en position 1 de la partie alkyle, laquelle contient jusqu'à 10 atomes de carbone, 4-tris-(hydroxyméthyl)-méthylcarbamoyl-n-butyl-amino, amino-(alkyle en C 1-C 7)-amino, di-(alkyle en C 1-C 7)-amino-(alkyle en C 1-C 7)-amino, (alcoxy en C 1-C 7)-carbonylamino-(alkyle en C 1-C 7)-amino, morpholino-(alkyle en C 1-C 7)-amino, cycloalkyl-(alkyle en C 1-C 7)-amino en C 4-C 10, benzylamino, pyridyl-(alkyle en C 1-C 7)-amino, imidazolyl-(alkyle en C 1-C 7)-amino ou 2-(2-[4-imidazolyl)-éthylamino]-éthylamino,

et des sels de ces composés qui portent des groupes salifiables, à l'exception du composé de formule I dans laquelle $R_1$ représente un groupe alpha-naphtoxyacétyle, A le radical bivalent de l'aminoacide histidine, $R_2$ l'hydrogène, $R_3$ un groupe isobutyle, $R_4$ un groupe hydroxy, $R_5$ un groupe isopropyle et $R_6$ un groupe cyclohexylméthylamino, caractérisé en ce que :

a) on condense un fragment d'un composé de formule I portant un groupe carboxy terminal ou un dérivé d'acide réactif d'un tel fragment avec un fragment complémentaire pour la formation du composé de formule I, portant un groupe amino libre, ou avec un dérivé réactif d'un tel fragment portant un groupe amino activé, les groupes fonctionnels libres présents dans les composants de la réaction étant éventuellement à l'état protégé, à l'exception des groupes qui participent à la réaction, avec formation d'une liaison amide, ou bien

b) on réduit en groupe hydroxy le groupe céto d'un composé de formule

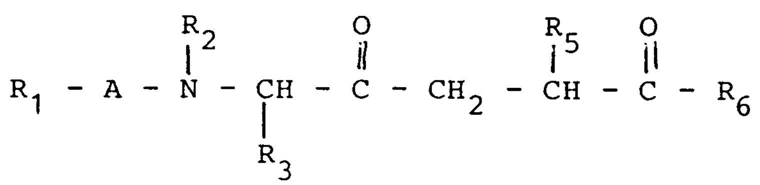

(II),

dans laquelle les symboles ont les significations indiquées ci-dessus et les groupes fonctionnels libres, à l'exception du groupe céto participant à la réaction, étant éventuellement à l'état protégé, par réaction avec un agent réducteur approprié, ou bien

c) on fait réagir un composé aldéhydique de formule

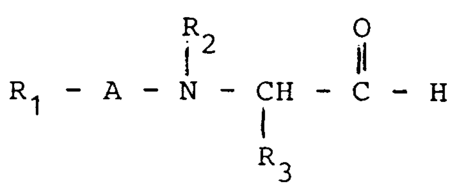

(III),

dans laquelle les symboles ont les significations indiquées ci-dessus et les groupes fonctionnels libres, à l'exception du groupe aldéhyde, sont éventuellement à l'état protégé, avec un dérivé organométallique de formule

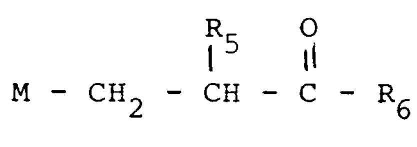

(IV),

dans laquelle les symboles ont les significations indiquées ci-dessus, et M représente un radical métallique, et on hydrolyse le produit formé par addition, ou bien

d) dans un composé de formule

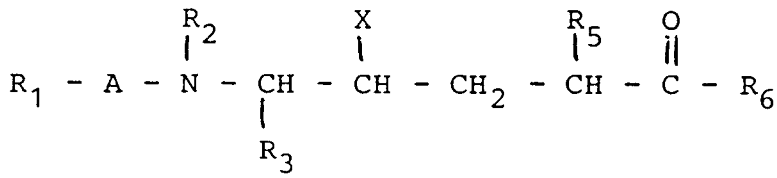 (V),

dans laquelle X représente un groupe éliminable nucléofuge, les autres symboles ayant les significations indiquées ci-dessus et les groupes fonctionnels libres étant éventuellement à l'état protégé, on échange le substituant X contre le substituant $R_4$ à l'aide d'un réactif introduisant ce substituant sous forme nucléophile, ou bien

e) dans un composé de formule

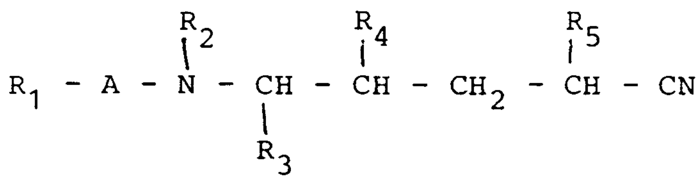 (VI),

dans laquelle les symboles ont les significations indiquées ci-dessus et les groupes fonctionnels présents sont éventuellement à l'état protégé, on convertit le groupe cyano en un groupe carboxamido substitué à l'azote $-(C=O)R_6$, ou bien

f) on réduit un époxyde de formule

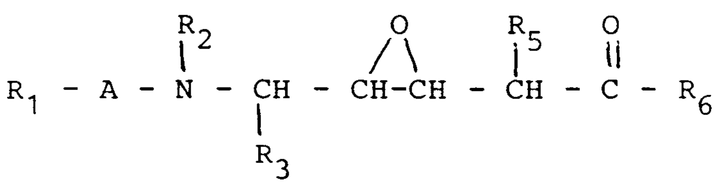 (VII),

dans laquelle les symboles ont les significations indiquées ci-dessus et les groupes fonctionnels libres sont éventuellement à l'état protégé, en l'alcool correspondant au moyen d'un agent réducteur régio-sélectif, et si on le désire

g) on élimine les groupes protecteurs existant dans un composé ainsi obtenu et/ou si on le désire, après la mise en oeuvre de l'un des procédés a) à f) ci-dessus ou d'un autre procédé quelconque de préparation d'un composé de formule I, on convertit un composé obtenu répondant à la formule I et portant un groupe salifiable en un sel ou un sel obtenu en le composé libre ou en un autre sel et/ou on sépare les mélanges d'isomères éventuellement obtenus et/ou dans un composé obtenu, répondant à la formule I, on inverse la configuration d'un atome de carbone chiral et/ou on convertit un composé de formule I selon l'invention en un autre composé de formule I selon l'invention.

**2.** Procédé de préparation d'un composé de formule I de la revendication 1, dans laquelle $R_1$ représente un groupe phénoxy-alcanoyle en C 1-C 7, naphtoxy-alcanoyle en C 1-C 7, phényl-alcanoyle en C 1-C 7 dans lesquels la partie alcanoyle en C 1-C 7 est non substituée ou peut être substituée par des groupes alcanoyloxy en C 1-C 7, carboxy, (alcoxy en C 1-C 7)-carbonyle, carbamoyle, (alkyle en C 1-C 7)-carbamoyle, cyano, di-(alcoxy en C 1-C 7)-phosphoryle ou alkyle en C 1-C 7 et oxo ou benzofurannyle et oxo et éventuellement ramifiée, un groupe naphtyl-alcanoyle en C 1-C 7 dans lequel la partie alcanoyle en C 1-C 7 est non substituée ou peut être substituée par des groupes alcanoyloxy en C 1-C 7, carboxy, (alcoxy en C 1-C 7)-carbonyle, carbamoyle, cyano ou alkyle en C 1-C 7 et oxo et éventuellement ramifiée, un groupe 2-tert-butylcarbamoyl-3-alpha-naphtyl-propionyle, 2-carboxy-méthylcarbamoyl-3-alpha-naphtyl-propionyle, indolyl-2-carbonyle ou cyclohepta[b]pyrrolyl-5-carbonyle. A représente le radical bivalent de l'aminoacide L-histidine, $R_2$ l'hydrogène, $R_3$ un groupe isobutyle ou cyclohexylméthyle, $R_4$ un groupe hydroxy, $R_5$ un groupe isopropyle ou cyclohexylméthyle et $R_6$ un

groupe alkylamino en C 1-C 7 ou amino-carboxy-alkylamino dans lesquels les substituants ne peuvent se trouver dans la position 1 du groupe alkyle, lequel contient jusqu'à 7 atomes de carbone, et les atomes de carbone portant les substituants $R_3$ et $R_4$ sont en configuration S, et des sels de ces composés qui contiennent des groupes salifiables, caractérisé en ce que :

a) on condense un fragment d'un composé de formule I portant un groupe carboxy terminal ou un dérivé d'acide réactif d'un tel fragment avec un fragment complémentaire pour la formation du composé de formule I, portant un groupe amino libre, ou avec un dérivé réactif d'un tel fragment portant un groupe amino activé, les groupes fonctionnels libres présents dans les composants de la réaction étant éventuellement à l'état protégé, à l'exception des groupes qui participent à la réaction, avec formation d'une liaison amide, ou bien

b) on réduit en groupe hydroxy le groupe céto d'un composé de formule

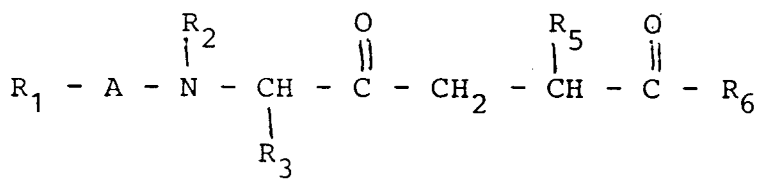 (II),

dans laquelle les symboles ont les significations indiquées ci-dessus et les groupes fonctionnels libres, à l'exception du groupe céto participant à la réaction, étant éventuellement à l'état protégé, par réaction avec un agent réducteur approprié, ou bien

c) on fait réagir un composé aldéhydique de formule

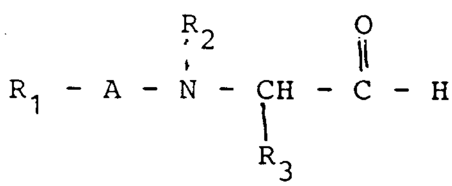 (III),

dans laquelle les symboles ont les significations indiquées ci-dessus et les groupes fonctionnels libres, à l'exception du groupe aldéhyde, sont éventuellement à l'état protégé, avec un dérivé organométallique de formule

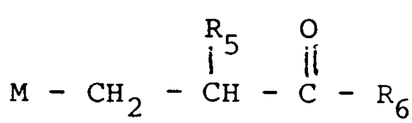 (IV),

dans laquelle les symboles ont les significations indiquées ci-dessus, et M représente un radical métallique, et on hydrolyse le produit formé par addition, ou bien

d) dans un composé de formule

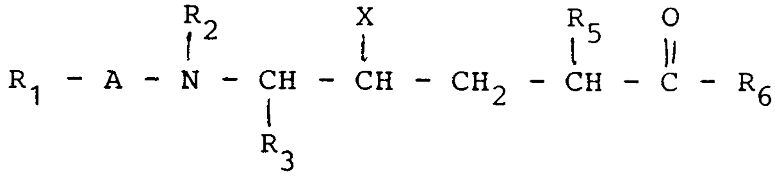 (V),

dans laquelle X représente un groupe éliminable nucléofuge, les autres symboles ayant les significations indiquées ci-dessus et les groupes fonctionnels libres étant éventuellement à l'état

protégé, on échange le substituant X contre le substituant $R_4$ à l'aide d'un réactif introduisant ce substituant sous forme nucléophile, ou bien

e) dans un composé de formule

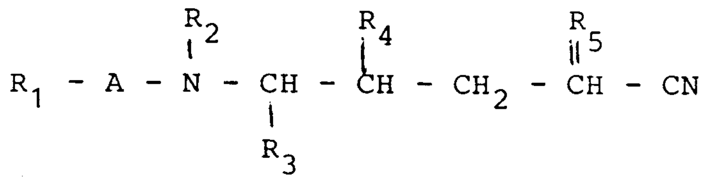

(VI),

dans laquelle les symboles ont les significations indiquées ci-dessus et les groupes fonctionnels présents sont éventuellement à l'état protégé, on convertit le groupe cyano en un groupe carboxamido substitué à l'azote $-(C=O)R_6$, ou bien

f) on réduit un époxyde de formule

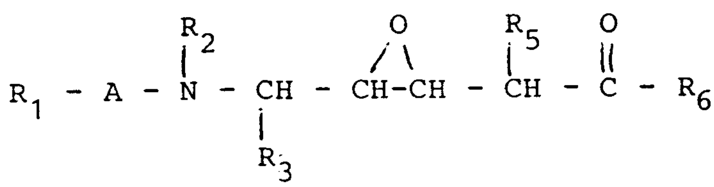

(VII),

dans laquelle les symboles ont les significations indiquées ci-dessus et les groupes fonctionnels libres sont éventuellement à l'état protégé, en l'alcool correspondant au moyen d'un agent réducteur régio-sélectif, et si on le désire

g) on élimine les groupes protecteurs existant dans un composé ainsi obtenu et/ou si on le désire, après la mise en oeuvre de l'un des procédés a) à f) ci-dessus ou d'un autre procédé quelconque de préparation d'un composé de formule I, on convertit un composé obtenu répondant à la formule I et portant un groupe salifiable en un sel ou un sel obtenu en le composé libre ou en un autre sel et/ou on sépare les mélanges d'isomères éventuellement obtenus et/ou dans un composé obtenu, répondant à la formule I, on inverse la configuration d'un atome de carbone chiral et/ou on convertit un composé de formule I selon l'invention en un autre composé de formule I selon l'invention.

**3.** Procédé de préparation d'un composé de formule I de la revendication 1, dans laquelle

a) $R_1$ représente un groupe N-benzyl-indolyl-2-carbonyle, A le radical bivalent de l'aminoacide histidine, $R_2$ l'hydrogène, $R_3$ un groupe isobutyle, $R_4$ un groupe hydroxy, $R_5$ un groupe isopropyle et $R_6$ un groupe méthylamino, ou bien

b) $R_1$ représente un groupe 3-(R,S)-benzyloxycarbonyl-4-alpha-naphtylbutyryle, A le radical bivalent de l'aminoacide histidine, $R_2$ l'hydrogène, $R_3$ un groupe isobutyle, $R_4$ un groupe hydroxy, $R_5$ un groupe isopropyle et $R_6$ un groupe méthylamino, ou bien

c) $R_1$ représente un groupe N-benzyloxycarbonyl-(p-benzyloxycarbonylamino-Phe), A le radical bivalent de l'aminoacide histidine, $R_2$ l'hydrogène, $R_3$ un groupe isobutyle, $R_4$ un groupe hydroxy, $R_5$ un groupe isopropyle et $R_6$ un groupe n-butylamino, ou bien

d) $R_1$ représente un groupe N-pivaloyl-(p-benzyloxycarbonylamino-Phe), A le radical bivalent de l'aminoacide histidine, $R_2$ l'hydrogène, $R_3$ un groupe isobutyle, $R_4$ un groupe hydroxy, $R_5$ un groupe isopropyle et $R_6$ un groupe n-butylamino, ou bien

e) $R_1$ représente un groupe indolyl-2-carbonyle, A le radical bivalent de l'aminoacide histidine, $R_2$ l'hydrogène, $R_3$ un groupe isobutyle, $R_4$ un groupe hydroxy, $R_5$ un groupe isopropyle et $R_6$ un groupe 4-[tris-tert-butyl-diméthylsilyloxyméthyl)-méthylcarbamoyl]-butylamino, ou bien

f) $R_1$ représente un groupe 2-éthylaminocarboxyloxy-4-phényl-butyryle, A le radical bivalent de l'aminoacide histidine, $R_2$ l'hydrogène, $R_3$ un groupe cyclohexylméthyle, $R_4$ un groupe hydroxy, $R_5$ un groupe isopropyle et $R_6$ un groupe n-butylamino, et des sels de ces composés qui portent des groupes salifiables, caractérisé en ce que :

a) on condense un fragment d'un composé de formule I portant un groupe carboxy terminal ou un dérivé d'acide réactif d'un tel fragment avec un fragment complémentaire pour la formation du

composé de formule I, portant un groupe amino libre, ou avec un dérivé réactif d'un tel fragment portant un groupe amino activé, les groupes fonctionnels libres présents dans les composants de la réaction étant éventuellement à l'état protégé, à l'exception des groupes qui participent à la réaction, avec formation d'une liaison amide, ou bien

b) on réduit en groupe hydroxy le groupe céto d'un composé de formule

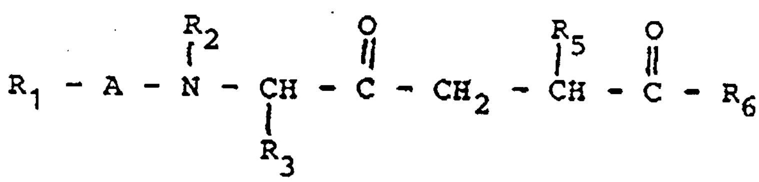

(II),

dans laquelle les symboles ont les significations indiquées ci-dessus et les groupes fonctionnels libres, à l'exception du groupe céto participant à la réaction, étant éventuellement A l'état protégé, par réaction avec un agent réducteur approprié, ou bien

c) on fait réagir un composé aldéhydique de formule

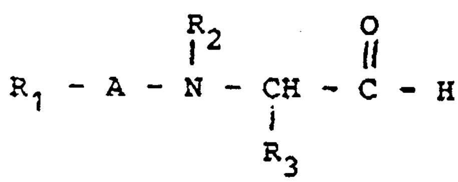

(III),

dans laquelle les symboles ont les significations indiquées ci-dessus et les groupes fonctionnels libres, à l'exception du groupe aldéhyde, sont éventuellement à l'état protégé, avec un dérivé organométallique de formule

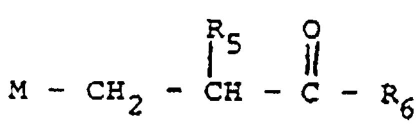

(IV),

dans laquelle les symboles ont les significations indiquées ci-dessus, et M représente un radical métallique, et on hydrolyse le produit formé par addition, ou bien

d) dans un composé de formule

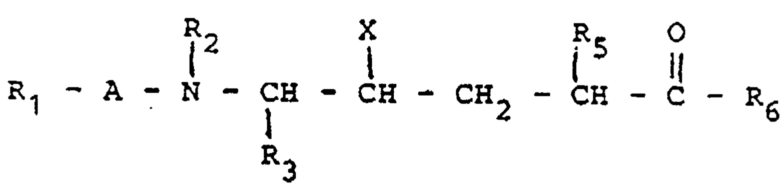

(V),

dans laquelle X représente un groupe éliminable nucléofuge, les autres symboles ayant les significations indiquées ci-dessus et les groupes fonctionnels libres étant éventuellement à l'état protégé, on échange le substituant X contre le substituant $R_4$ à l'aide d'un réactif introduisant ce substituant sous forme nucléophile, ou bien

e) dans un composé de formule

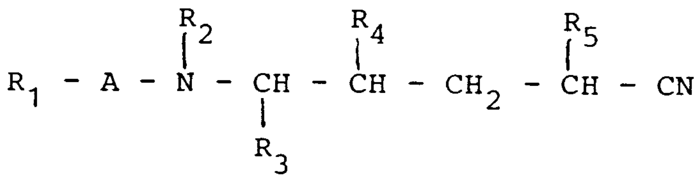 (VI),

dans laquelle les symboles ont les significations indiquées ci-dessus et les groupes fonctionnels présents sont éventuellement à l'état protégé, on convertit le groupe cyano en un groupe carboxamido substitué à l'azote $-(C=O)R_6$, ou bien

f) on réduit un époxyde de formule

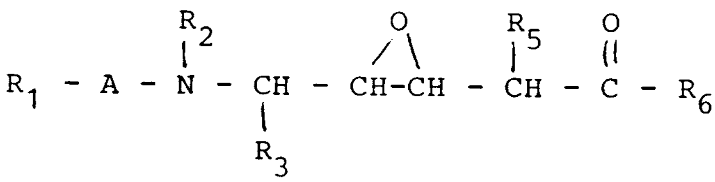 (VII),

dans laquelle les symboles ont les significations indiquées ci-dessus et les groupes fonctionnels libres sont éventuellement à l'état protégé, en l'alcool correspondant au moyen d'un agent réducteur régio-sélectif, et si on le désire

g) on élimine les groupes protecteurs existant dans un composé ainsi obtenu et/ou si on le désire, après la mise en oeuvre de l'un des procédés a) à f) ci-dessus ou d'un autre procédé quelconque de préparation d'un composé de formule I, on convertit un composé obtenu répondant à la formule I et portant un groupe salifiable en un sel ou un sel obtenu en le composé libre ou en un autre sel et/ou on sépare les mélanges d'isomères éventuellement obtenus et/ou dans un composé obtenu, répondant à la formule I, on inverse la configuration d'un atome de carbone chiral et/ou on convertit un composé de formule I selon l'invention en un autre composé de formule I selon l'invention.

**4.** Procédé de préparation d'un composé de formule I selon l'une des revendications 1 ou 2, caractérisé en ce que l'on condense un fragment d'un composé de formule I portant un groupe carboxy terminal ou un dérivé d'acide réactif d'un tel fragment avec un fragment portant un groupe amino libre, complémentaire pour la formation du composé de formule I, ou un dérivé réactif d'un tel fragment portant un groupe amino activé, les groupes fonctionnels libres présents dans les composants de la réaction, à l'exception des groupes participant à la réaction, étant éventuellement à l'état protégé, avec formation d'une liaison amide, et si on le désire, dans un composé ainsi obtenu, on élimine les groupes protecteurs présents en traitant par un acide ou par une base ou par hydrogénolyse et/ou on convertit un composé obtenu, répondant à la formule I et portant un groupe salifiable, en un sel, ou un sel obtenu en le composé libre ou en un autre sel, et/ou on sépare un mélange d'isomères éventuellement obtenu et/ou on convertit un composé de formule I selon l'invention en un autre composé de formule I selon l'invention.

**5.** Procédé de préparation d'un composé de formule I de la revendication 3, caractérisé en ce que l'on condense un fragment d'un composé de formule I portant un groupe carboxy terminal ou un dérivé d'acide réactif d'un tel fragment avec un fragment complémentaire pour la formation du composé de formule I, portant un groupe amino libre, ou un dérivé réactif d'un tel fragment portant un groupe amino activé, les groupes fonctionnels libres présents dans les composants de la réaction, à l'exception des groupes participant à la réaction, étant éventuellement à l'état protégé, avec formation d'une liaison amide, et si on le désire, on élimine les groupes protecteurs présents dans un composé ainsi obtenu à l'aide d'un acide ou d'une base ou par hydrogénolyse et/ou on convertit un composé obtenu répondant à la formule I et portant un groupe salifiable en un sel ou un sel obtenu en le composé libre ou en un autre sel, et/ou le cas échéant on sépare un mélange d'isomères et/ou on convertit un composé de formule I selon l'invention en un autre composé de formule I selon l'invention.

6. Procédé de préparation d'un composé de formule I selon l'une des revendications 1 ou 2, caractérisé en ce que l'on condense un fragment d'un composé de formule I portant un groupe carboxy terminal ou un ester activé dérivant d'un tel fragment, un anhydride réactif dérivant d'un tel fragment ou un amide cyclique réactif dérivant d'un tel fragment, avec un fragment complémentaire pour la formation du composé de formule I, portant un groupe amino libre, les groupes fonctionnels libres présents dans les composants de la réaction, à l'exception des groupes participant à la réaction, étant éventuellement à l'état protégé, en présence d'un agent de condensation choisi parmi un carbodiimide, un carbonyldiimidazole, un dérivé de 1,2-oxazolium, d'une 1,2-dihydroquinoléine ou un phosphate activé, et si on le désire en présence d'une base organique ou d'un carbonate de métal alcalin dans un solvant polaire aprotonique inerte, dans un intervalle de température de -40 à +100°, les esters activés du type aminoester ou amidoester étant si on le désire formés in situ par addition d'un dérivé N-hydroxyaminé ou N-hydroxyamidé, et si on le désire, dans un composé ainsi obtenu, on élimine les groupes protecteurs présents par traitement à l'aide d'un acide ou d'une base ou par hydrogénolyse et/ou on convertit un composé obtenu répondant à la formule I et portant un groupe salifiable en un sel ou un sel obtenu en le composé libre ou en un autre sel et/ou on sépare des mélanges d'isomères éventuellement obtenus et/ou on convertit un composé de formule I selon l'invention en un autre composé de formule I selon l'invention.

7. Procédé de préparation d'un composé de formule I selon la revendication 3, caractérisé en ce que l'on condense un fragment d'un composé de formule I portant un groupe carboxy terminal ou un ester activé dérivant d'un tel fragment, un anhydride réactif dérivant d'un tel fragment ou un amide cyclique réactif dérivant d'un tel fragment, avec un fragment complémentaire pour la formation du composé de formule I, portant un groupe amino libre, les groupes fonctionnels libres présents dans les composants de réaction, à l'exception des groupes participant à la réaction, étant éventuellement à l'état protégé, en présence d'un agent de condensation choisi parmi un carbodiimide, un carbonyldiimidazole, un dérivé de 1,2-oxazolium, une 1,2-dihydroquinoléine ou un phosphate activé, et si on le désire, en présence d'une base organique ou d'un carbonate de métal alcalin, dans un solvant polaire aprotonique inerte, dans un intervalle de température de -40 à +100°C, les esters activés du type aminoester ou amidoester étant si on le désire formés in situ par addition d'un dérivé N-hydroxyaminé ou N-hydroxyamidé, et si on le désire, on élimine les groupes protecteurs présents dans un composé obtenu en traitant par un acide ou par une base ou par hydrogénolyse, et/ou on convertit un composé obtenu répondant à la formule I et portant un groupe salifiable en un sel ou un sel obtenu en le composé libre ou en un autre sel, et/ou on sépare les mélanges d'isomères éventuellement obtenus et/ou on convertit un composé de formule I selon l'invention en un autre composé de formule I selon l'invention.

8. Procédé selon l'une des revendications 1, 2, 4 ou 6, caractérisé en ce que l'on choisit les composants de départ de manière à obtenir un composé de formule I dans laquelle $R_1$ représente un groupe 2(S)-pivaloyloxy-3-phénylpropionyle, 2(R)- et 2(S)-diméthoxyphosphoryl-3-phénylpropionyle, 2(R)- et 2(S)-benzyl-5,5-diméthyl-4-oxo-hexanoyle, 2(R)- et 2(S)-benzyl-4,4-diméthyl-3-oxo-pentanoyle, 2(R)- et 2(S)-tert-butylcarbamoyl-3-alpha-naphtyl-propionyle ou 2(R)- et 2(S)-éthoxycarbonyl-3-alpha-naphtyl-propionyle, indolyl-2-carbonyle ou cyclohepta[b]pyrrolyl-5-carbonyle, A représente le radical bivalent de l'aminoacide L-histidine, $R_2$ l'hydrogène, $R_3$ un groupe cyclohexylméthyle, $R_4$ un groupe hydroxy, $R_5$ un groupe isopropyle et $R_6$ un groupe alkylamino en C 1-C 7, et les atomes de carbone portant les substituants $R_3$, $R_4$ et $R_5$ sont en configuration S, et leurs sels acceptables pour l'usage pharmaceutique.

9. Procédé selon l'une des revendications 1, 2, 4, 6 ou 8, caractérisé en ce que l'on choisit les composants de départ de manière à former un composé de formule I dans laquelle $R_1$ représente le groupe 2(S)-pivaloyloxy-3-phénylpropionyle, A le radical bivalent de l'aminoacide L-histidine, $R_2$ l'hydrogène, $R_3$ un groupe cyclohexylméthyle, $R_4$ un groupe hydroxy, $R_5$ un groupe isopropyle et $R_6$ un groupe n-butylamino et les atomes de carbone portant les substituants $R_3$ et $R_4$ sont en configuration S, et leurs sels acceptables pour l'usage pharmaceutique.

10. Procédé selon l'une des revendications 1, 2, 4, 6 ou 8, caractérisé en ce que l'on choisit les composants de départ de manière à former un composé de formule I dans laquelle $R_1$ représente un groupe 2(R,S)-, 2(R)- ou 2(S)-diméthoxyphosphoryl-3-phénylpropionyle, A le radical bivalent de l'aminoacide L-histidine, $R_2$ l'hydrogène, $R_3$ un groupe cyclohexylméthyle, $R_4$ un groupe hydroxy, $R_5$ un groupe isopropyle et $R_6$ un groupe n-butylamino, et les atomes de carbone portant les substituants $R_3$

et $R_4$ et $R_5$ sont en configuration S, et leurs sels acceptables pour l'usage pharmaceutique.

**11.** Procéeé selon une des revendications 1, 2, 4, 6 ou 8, caractérisé en ce que l'on choisit les produits de départ de manière à former l'un des composés de formule I dans laquelle $R_1$ représente un groupe 2-(R,S)-, 2(R)- ou 2(S)-benzyl-5,5-diméthyl-4-oxohexanoyle, A le radical bivalent de l'aminoacide L-histidine, $R_2$ l'hydrogène, $R_3$ un groupe cyclohexylméthyle, $R_4$ un groupe hydroxy, $R_5$ un groupe isopropyle et $R_6$ un groupe n-butylamino, et les atomes de carbone portant les substituants $R_3$ $R_4$ et $R_5$ sont en configuration S, et leurs sels acceptables pour l'usage pharmaceutique.

**12.** Procédé selon l'une des revendications 1, 2, 4, 6 ou 8 caractérisé en ce que l'on choisit les produits de départ de manière à former le composé répondant à la formule I dans laquelle $R_1$ représente un groupe 2(R,S)-benzyl-4,4-diméthyl-3-oxo-pentanoyle, A le radical bivalent de l'aminoacide L-histidine, $R_2$ l'hydrogène, $R_3$ un groupe cyclohexylméthyle, $R_4$ un groupe hydroxy, $R_5$ un groupe isopropyle et $R_6$ un groupe n-butylamino et les atomes de carbone portant les substituants $R_3$, $R_4$ et $R_5$ sont en configuration S, et leurs sels acceptables pour l'usage pharmaceutique.

**13.** Procédé selon l'une des revendications 1, 2, 4, 6 ou 8 caractérisé en ce que l'on choisit les produits de départ de manière à former le composé répondant à la formule I dans laquelle $R_1$ représente un groupe 2(R,S)-éthoxycarbonyl-3-alpha-naphtyl-propionye, A le radical bivalent de l'aminoacide L-histidine, $R_2$ l'hydrogène, $R_3$ un groupe cyclohexylméthyle, $R_4$ un groupe hydroxy, $R_5$ un groupe isopropyle et $R_6$ un groupe n-butylamino et les atomes de carbone portant les substituants $R_3$, $R_4$ et $R_5$ sont en configuration S, et leurs sels acceptables pour l'usage pharmaceutique.

**14.** Procédé selon l'une des revendications 1, 2, 4, 6 ou 8 caractérisé en ce que l'on choisit les produits de départ de manière à former le composé répondant à la formule I dans laquelle $R_1$ représente un groupe cyclohepta[b]pyrrolyl-5-carbonyle, A le radical bivalent de l'aminoacide L-histidine, $R_2$ l'hydrogène, $R_3$ un groupe cyclohexylméthyle, $R_4$ un groupe hydroxy, $R_5$ un groupe isopropyle et $R_6$ un groupe n-butylamino et les atomes de carbone portant les substituants $R_3$, $R_4$ et $R_5$ sont en configuration S, et leurs sels acceptables pour l'usage pharmaceutique.

**15.** Procédé selon l'une des revendications 1, 2, 4, 6 ou 8 caractérisé en ce que l'on choisit les composants de départ de manière à former le composé répondant à la formule I dans laquelle $R_1$ représente un groupe 2(S)-pivaloyloxy-3-phénylpropionyle, A le radical bivalent de l'aminoacide L-histidine, $R_2$ l'hydrogène, $R_3$ un groupe isobutyle, $R_4$ un groupe hydroxy, $R_5$ un groupe cyclohexylméthyle et $R_6$ un groupe n-butylamino et les atomes de carbone portant les substituants $R_3$ et $R_4$ sont en configuration S, et leurs sels acceptables pour l'usage pharmaceutique.

**16.** Procédé de préparation de compositions pharmaceutiques, caractérisé en ce que l'on mélange un composé de formule I préparé par l'un des procédés des revendications 1 à 15 ou un sel acceptable pour l'usage pharmaceutique d'un tel composé de formule I portant des groupes salifiables, avec un véhicule pharmaceutique.

**17.** Procédé de préparation d'un composé de formule

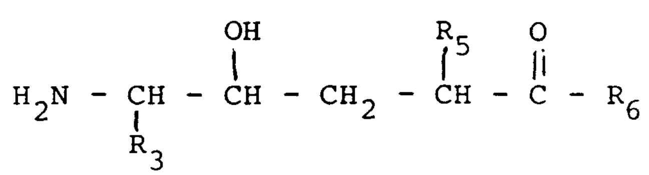

(VIII),

dans laquelle $R_3$ représente un groupe cyclohexylméthyle et $R_5$ et $R_6$ ont les significations indiquées dans la revendication 1 ou 2, et de leurs sels, caractérisé en ce que l'on convertit un composé de formule

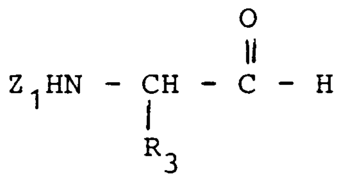 (IX),

dans laquelle $R_3$ a les significations indiquées ci-dessus et $Z_1$ représente un groupe protecteur du groupe amino, à l'aide d'un ylide du soufre, en un époxyde de formule

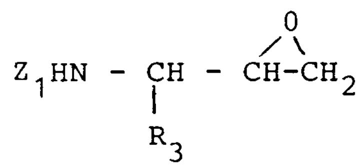 (X),

dans laquelle $R_3$ et $Z_1$ ont les significations indiquées ci-dessus, qu'on fait réagir, éventuellement après séparation des isomères, avec un réactif introduisant un groupe éliminable nucléofuge X, ce qui donne un composé de formule

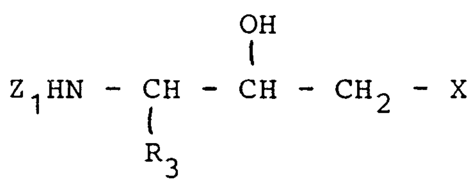 (XI),

dans laquelle $R_3$ et $Z_1$ ont les significations indiquées ci-dessus et X représente un groupe éliminable nucléofuge, qu'on fait réagir avec un dérivé carbonylé de formule

$R_a$ - (C = O) - $R_b$ (XII),

dans laquelle chacun des symboles $R_a$ et $R_b$ représente, indépendamment l'un de l'autre, l'hydrogène, un groupe alkyle inférieur, aryle ou aryl-alkyle inférieur ou forment ensemble un groupe alkylidène en C4-C12 formant éventuellement un pont, ou avec un dérivé réactif d'un tel composé carbonylé, en présence d'un réactif acide, ce qui donne un composé de formule

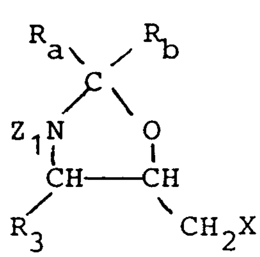 (XIII),

dans laquelle les symboles ont les significations indiquées ci-dessus, qu'on fait réagir avec un sel-ester d'acide carboxylique de formule

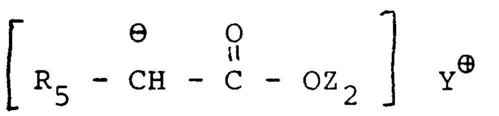 (XIV),

dans laquelle $R_5$ a les significations indiquées ci-dessus, $Z_2$ représente un groupe protecteur du groupe carboxy et $Y^{\oplus}$ représente un cation, par exemple un ion de métal alcalin tel que l'ion sodium ou, de préférence, l'ion lithium, ce qui donne un composé de formule

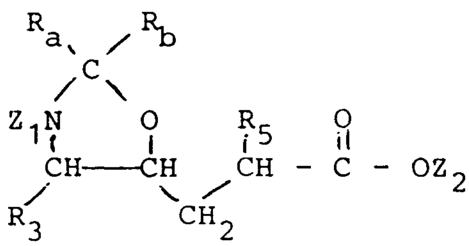 (XV),

dans laquelle les symboles ont les significations indiquées ci-dessus, d'où l'on élimine le groupe protecteur $Z_2$ du groupe carboxy, et/ou on sépare un mélange d'isomères en les isomères individuels, après quoi on amide le composé portant un groupe carboxy libre ($Z_2$ = H) à l'aide d'une amine $R_6$-H, ce qui donne un composé de formule

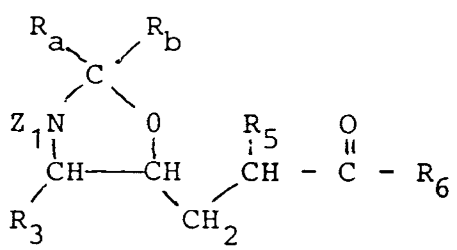 (XVI),

dans laquelle les symboles ont les significations indiquées ci-dessus, dont on ouvre le cycle au moyen d'un réactif de solvolyse approprié, et le cas échéant on élimine le groupe protecteur $Z_1$.